# EUROPÄISCHE PATENTSCHRIFT

(11) **EP 0 594 540 B1**
(45) Veröffentlichungstag und Bekanntmachung des Hinweises auf die Patenterteilung: **01.04.1998**
(21) Anmeldenummer: 93810724.0
(22) Anmeldetag: 14.10.1993
(51) Int. Cl.: C07K 5/02, A61K 38/55

(54) **Antiretrovirale Acyl-Verbindungen**
Antiretroviral acyl compounds
Composés acylés actifs contre les rétrovirus

(30) Priorität: 23.10.1992 CH 3312/92
(43) Veröffentlichungstag der Anmeldung: 27.04.1994
(73) Patentinhaber: Novartis AG, 4058 Basel (CH)
(72) Erfinder: Bold, Guido, Dr., CH-5264 Gipf-Oberfrick (CH); Fässler, Alexander, Dr., CH-3063 Ittigen (CH); Lang, Marc, Dr., F-68200 Mulhouse (FR)

(56) Entgegenhaltungen:
- EP-A- 0 432 695

## Beschreibung

Die Erfindung betrifft neue antiretroviral wirksame Verbindungen, Verfahren zur Herstellung dieser Verbindungen, pharmazeutische Präparate mit diesen Verbindungen, diese Verbindungen zur Anwendung in einem therapeutischen Verfahren zur Behandlung des menschlichen oder tierischen Körpers und die Verwendung dieser Verbindungen zur therapeutischen Behandlung des menschlichen oder tierischen Körpers oder zur Herstellung pharmazeutischer Präparate.

Es gibt eine ganze Reihe von Erkrankungen, die nach dem heutigen Wissensstand durch Retroviren hervorgerufen werden.

AIDS ist nach bisherigem Wissen eine durch das Retrovirus HIV (Human Immunodeficiency Virus") hervorgerufene Erkrankung des Immunsystems. Diese Erkrankung betrifft nach Schätzungen der WHO rund 10 Millionen Menschen, breitet sich immer weiter aus und führt praktisch stets zum Tod des Patienten.

Bisher konnten die Retroviren HIV-1 und HIV-2 (HIV steht für "Human Immunodeficiency Virus") als Ursache für die Erkrankung identifiziert und molekularbiologisch charakterisiert werden. Für die Therapie ist über die bisherigen begrenzten Möglichkeiten zur Linderung der AIDS-Symptome und gewisse präventive Möglichkeiten hinaus insbesondere die Suche nach Präparaten interessant, welche die Vermehrung des Virus selbst beeinträchtigen, ohne die intakten Zellen und Gewebe der Patienten zu schädigen.

Die retrovirale Protease ist ein proteolytisches Enzym, das wegen eines im aktiven Zentrum vorliegenden Aspartatrestes zu den Aspartat-Proteasen gezählt wird und im Vermehrungscyclus einer Reihe von Retroviren an der Reifung neuer infektiöser Virionen innerhalb von infizierten Zellen mitwirkt.

Beispielsweise weisen HIV-1 und HIV-2 jeweils in ihrem Genom einen Bereich auf, der für eine "gag-Protease" kodiert. Diese "gag-Protease" ist für die korrekte proteolytische Spaltung der Vorläuferproteine verantwortlich, die aus den für die "Group Specific Antigens" (gag) kodierenden Genomabschnitten hervorgehen. Hierbei werden die Strukturproteine des Viruskerns, englisch "Core", freigesetzt. Die "gag-Protease" selbst ist Bestandteil eines durch den pol-Genomabschnitt von HIV-1 und HIV-2 kodierten Vorläuferproteines, das auch die Abschnitte für die "Reverse Transcriptase" und die "Integrase" enthält und vermutlich autoproteolytisch gespalten wird.

Die "gag-Protease" spaltet das Hauptkernprotein ("Major Core Protein") p24 von HIV-1 und HIV-2 bevorzugt N-terminal von Prolinresten, z. B. in den bivalenten Radikalen Phe-Pro, Leu-Pro oder Tyr-Pro. Es handelt sich um eine Protease mit einem katalytisch aktiven Aspartatrest im aktiven Zentrum, eine sogenannte Aspartatprotease.

Aufgrund der zentralen Rolle der "gag-Protease" bei der Prozessierung der genannten "Core-Proteine" wird davon ausgegangen, dass eine wirksame Inhibierung dieses Enzyms in vivo den Zusammenbau reifer Virionen unterbindet, so dass entsprechende Inhibitoren therapeutisch eingesetzt werden können.

Generell gibt es zur Bekämpfung retroviraler Erkrankungen, wie AIDS, seit einiger Zeit Bemühungen, Verbindungen bereitzustellen, die in vivo als Hemmstoffe gegen die genannten retroviralen gag-Proteasen, insbesondere die gag-Protease von HIV-1 (HIV-1-Protease), aber auch gegen die von HIV-2 oder weitere AIDS-Viren, wirksam sind.

Hauptziel ist derzeit, solche Verbindungen zur Verfügung zu stellen, die bestmögliche pharmakokinetische Eigenschaften aufweisen.

Voraussetzung für die therapeutische Wirksamkeit in vivo ist nämlich das Erreichen guter Bioverfügbarkeit, z. B. einer guten Resorbierbarkeit und/oder eines hohen Blutspiegels, auch bei enteraler, wie oraler Verabreichung, um so an den infizierten Zellen ausreichend hohe Konzentrationen zu erreichen, und/oder einer günstigen Verteilung im Organismus.

Die Aufgabe der vorliegenden Erfindung besteht in der Bereitstellung strukturell neuer antiretroviral wirksamer Verbindungen mit verbesserten pharmakodynamischen Eigenschaften, die vorzugsweise eine bessere Resorption als die entsprechenden unmodifizierten antiretroviralen Verbindungen selbst bei enteraler, insbesondere oraler, Verabreichung zeigen und/oder zu Blutspiegeln insbesondere der unmodifizierten antiretroviral wirksamen Substanz führen, die höher sind als die bei entsprechender Verabreichung der unmodifizierten antiretroviral wirksamen Substanzen selbst erzielbaren.

Bei den erfindungsgemässen Verbindungen handelt es sich um solche der Formel I, 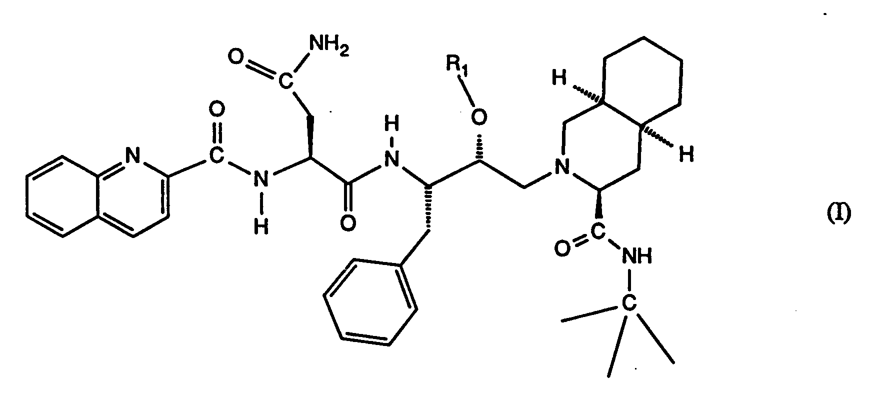 worin R₁ Acyl bedeutet, und Salze davon.

In der Europäischen Patentanmeldung EP 0 432 695 (publiziert am 19. Juni 1991) ist die Verbindung der Formel II, 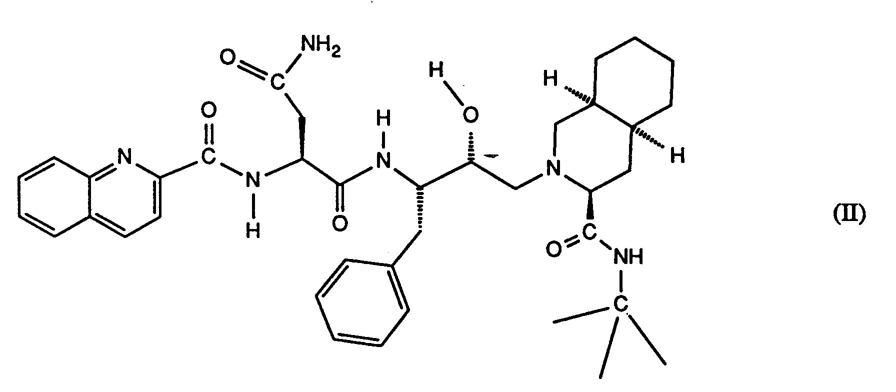 welche auch in EP 0 346 847 (publiziert am 19. Juni 1991) generisch umfasst ist und für die in EP 0 432 694 (publiziert am 11. Dez. 1989) ein Syntheseweg beschrieben ist, genannt. Diese Verbindung wird auch in den nachfolgend aufgeführten Publikationen genannt und beispielsweise auf ihre Wirkung in vitro, auf Zellkulturen und in vivo sowie auf Struktur/Wirkungsbeziehungen untersucht: Tucker, T. J., et al., J. Med. Chem. 35, 2525-2533 (1992); Roberts, N. A., et al., Biochem. Soc. Transact. 20, 513-516 (1992); Kräusslich, H.-G., J. Virology 66, 567-572 (1992); Martin, J. A., et al., Biochem. Biophys. Res. Commun. 176, 180 - 188 (1991); Krohn et al., J. Med. Chem. 34, 3340-3342 (1991); Holmes, H. C., et al., Antiviral Chem. & Chemother. 2, 287-293 (1991); Craig, J. C., et al., Antiviral Chem. & Chemother. 2, 181-186 (1991); Craig, J. C., et al., Antivaral Res. 16, 295-305 (1991); Roberts, N. A., et al., Science 248, 358-361 (1990); Overton, H. A., et al., Virology 179, 508-511 (1990); Muirhead, G. J., et al., Brit. J. Clin. Pharmacol. 34(2), 170P (1992); Williams, P. E. O., et al., Brit. J. Clin. Pharmacol. 34(2), 155P (1992); Shaw et al., Brit. J. Clin. Pharmacol. 34(2), 166P (1992); Johnson, V. A., et al., J. Infectious Dis. 166(5), 1143 (1992); Phylip, L. H., FEBS Lett. 314(3), 449 (1992); EP 0 432 695 (publiziert am 19.06.1991); EP 0 513 917 (publiziert am 19.11.1992); Thompson, W. J., J. Am. Chem. Soc. 115, 801 (1993); und EP 0 346 847 (publiziert am 20.12.1989).

In keiner dieser genannten Publikationen werden Verbindungen genannt oder nahegelegt, die an der freien Hydroxygruppe in Verbindungen der Formel II statt des Wasserstoffs einen anderen Rest enthalten.

Überraschenderweise wurde nun gefunden, dass Verbindungen der Formel II bei der Applikation an Säugetieren, vor allem bei oraler Applikation, in deutlich höherer Konzentration im Blut vorliegen, wenn eine Verbindung der Formel I mit durch R₁ modifizierter Hydroxygruppe verabreicht wird, als bei Verabreichung der entsprechenden freien Verbindung der Formel II.

Die in der Beschreibung der vorliegenden Erfindung verwendeten allgemeinen Ausdrücke und Bezeichnungen haben vorzugsweise die folgenden Bedeutungen:

Die erfindungsgemässen Verbindungen der Formel I können in verschiedenen isomeren Formen vorliegen, falls der Rest R₁ in verschiedenen isomeren Formen vorliegen kann. So können vorhandene asymmetrische Kohlenstoffatome in den Substituenten R₁ unabhängig voneinander in der (R)-, (S)- oder (R,S)-Konfiguration vorliegen, und/oder an Mehrfachbindungen, wie Doppelbindungen, kann cis/trans-Isomerie auftreten. Somit können die Verbindungen der Formel I als Isomerengemische, insbesondere als Diastereomerengemische oder als Racemate, oder als reine Isomeren, insbesondere als reine Enatiomere, vorliegen.

Der bei der Definition von Gruppen oder Resten, z. B. Niederalkyl, Niederalkoxycarbonyl etc., verwendete Ausdruck "Nieder" bedeutet, dass die so definierten Gruppen oder Reste, falls nicht ausdrücklich anders definiert, bis einschliesslich 7 und bevorzugt bis einschliesslich 4 C-Atome enthalten. Im Falle von Niederalkenyl und Niederalkinyl bedeutet "Nieder" das Vorliegen von wenigstens 2 bis maximal 7, vorzugsweise von 2 bis 4 Kohlenstoffatomen, im Falle von Niederalkenoyl oder Niederalkinoyl 3 bis 7, vorzugsweise 3 oder 4 Kohlenstoffatomen.

Acyl R₁ hat z.B. bis zu 25, bevorzugt bis zu 19 C-Atome, und ist in erster Linie die über ihr Carbonyl gebundene Acylgruppe einer Carbonsäure oder einer unsubstituierten oder substituierten Aminosäure, ferner Aminocarbonyl oder der über seine Aminocarbonylgruppe gebundene Rest einer N-substituierten Carbaminsäure oder der über Carbonyl gebundene Rest eines Halbesters der Kohlensäure.

Bevorzugte Acylgruppen R₁ einer Carbonsäure sind beispielsweise unsubstituiertes Alkanoyl, Alkenoyl oder Alkinoyl, oder substituiertes Alkanoyl, Alkenoyl oder Alkinoyl, insbesondere Octanoyl, Decanoyl, Dodecanoyl, oder Palmitoyl, unsubstituiertes oder substituiertes Niederalkanoyl, Niederalkenyol oder Niederalkinoyl, worin die Substituenten beispielsweise aus einem oder mehreren Resten, vorzugsweise aus bis zu drei Resten, insbesondere aus einem Rest oder zwei Resten ausgewählt aus der Gruppe Hydroxy, Niederalkoxy, Niederalkoxyniederalkoxy, Niederalkoxyniederalkoxyniederalkoxy, Phenoxy, Naphthoxy, Phenylniederalkoxy, 2-Halogenniederalkanoyl, wie 2-Chloracetyl, Amino-, Niederalkylamino- oder Diniederalkylamino-niederalkoxy-2-niederalkanoyl, wie Dimethylamino-niederalkoxyacetyl, Amino-, Niederalkylamino- oder Diniederalkylaminoniederalkoxyniederalkoxy-2-niederalkanoyl, wie Dimethylamino-(2-niederalkoxyethyl)-acetyl, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Carbamoyl, dessen Stickstoff Bestandteil eines 5- bis 7-gliedrigen heterocyclischen Ringes ist, der noch ein weiteres Heteroatom ausgewählt aus O, S, N und durch Niederalkyl, wie Methyl oder Ethyl, substituiertes N enthalten kann, z. B. Pyrrolidinocarbonyl, Morpholinocarbonyl, Thiomorpholinocarbonyl, Piperidin-1-ylcarbonyl, Piperazin-1-ylcarbonyl oder 4-Niederalkyl-piperazin-1-ylcarbonyl, wie 4-Methylpiperazin-1-ylcarbonyl, Cyano, Oxo, Cycloalkyl, z.B. C₃-C₈-Cycloalkyl, wie Cyclopropyl, Cyclobutyl, Cyclopentyl oder Cyclohexyl, Bicyloalkyl, z.B. C₆-C₁₂-Bicycloalkyl, wie Decahydronaphth-2-yl, endo- oder exo-2-Norbornyl, Bicyclo[2.2.]oct-2-yl oder Bicyclo[3.3.1.]non-9-yl, Tricycloalkyl, z.B. C₉-C₁₄-Tricycloalkyl, wie 1- oder 2-Adamantyl, Cycloalkenyl, z.B. C₄-C₈-Cycloalkenyl, wie 1-Cyclohexenyl oder 1,4-Cyclohexadienyl, Bicycloalkenyl z.B. 5-Norbornen-2-yl oder Bicyclo[2.2.2]octen-2-yl, Heterocyclyl, welches einen als solchen oder in ein- oder zweifach benz-, cyclopenta-, cyclohexa- oder cyclohepta-annellierter Form vorliegenden gesättigten, teilweise gesättigten oder ungesättigten Ring, der 3 bis 7, vorzugsweise 5 - 7 Ringatome enthält und bis zu vier Heteroatome ausgewählt aus Stickstoff, Schwefel und/oder Sauerstoff enthält, vorzugsweise 1 oder 2 der genannten Heteroatome, und unsubstituiert oder, insbesondere durch Niederalkyl, Niederalkanoyl, Hydroxy, Niederalkoxy, Phenylniederalkoxy, wie Benzyloxy, Hydroxyniederalkyl, wie Hydroxymethyl, Halogen, Cyano und/oder Trifluormethyl, substituiert sein kann, bedeutet, z. B. Pyrrolyl, 2,5-Dihydropyrrolyl, Furanyl, Thienyl, Tetrahydrofuranyl, Cyclohepta[b]pyrrolyl, Pyrrolidinyl, Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Chinolyl, Isochinolyl, Benzimidazolyl, Benzofuranyl, Pyridyl, Pyrimidinyl, Piperidinyl, Piperazin-1-yl, Morpholino, Thiomorpholino, S,S-Dioxothiomorpholino, 1,2-Dihydro- oder 1,2,3,4-Tetrahydrochinolyl, oder 1,2-Dihydro- oder 1,2,3,4-Tetrahydroisochinolyl, wobei die genannten Reste unsubstituiert oder wie oben substituiert sind, insbesondere durch Niederalkyl, z. B. in 4-Niederalkyl-piperazin-1-yl, wie 4-Methyl- oder 4-Ethyl-piperazin-1-yl, durch Niederalkanoyl, z. B. in 4-Niederalkanoyl-piperazin-1-yl, wie 4-Acetyl-piperazin-1-yl, oder durch Hydroxyniederalkyl, z.B. in 5-Hydroxymethylfuran-2-ylcarbonyl, und Aryl, vorzugsweise C₆-C₁₂-Aryl, z.B. Phenyl, Naphthyl, wie 1- oder 2-Naphthyl, Indanyl, wie 1-oder 2-Indanyl, Indenyl, wie Inden-1-yl, oder Fluorenyl, wie Fluoren-9-yl, wobei Aryl unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Heterocyclylniederalkyl, worin Heterocyclyl wie oben definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinomethyl oder Thiomorpholinomethyl, Cyano und/oder Nitro ein oder mehrfach, vorzugsweise einfach, substituiert ist, insbesondere Phenyl, welches durch einen der genannten Reste in p-Stellung substituiert ist, ausgewählt sind, z.B. Niederalkanoyl, wie Formyl, Acetyl, Propionyl, Butyryl, Methylpropionyl, Pivaloyl, n-Pentanoyl, Hexanoyl oder Heptanoyl, wie n-Heptanoyl, Hydroxyniederalkanoyl, z.B. β-Hydroxypropionyl, Niederalkoxyniederalkanoyl, z.B. Niederalkoxyacetyl oder Niederalkoxypropionyl, wie Methoxyacetyl, 3-Methoxypropionyl oder n-Butyloxyacetyl, Niederalkoxyniederalkoxyniederalkanyl, wie 2-(2-Methoxyethoxy)acetyl, Niederalkoxyniederalkoxyniederalkoxyniederalkanoyl, wie 2-(2-(2-Methoxyethoxy)ethoxy)acetyl, Phenoxyniederalkanoyl, z.B. Phenoxyacetyl, Naphthoxyniederalkanoyl, z.B. α- oder β-Naphthoxyacetyl, Phenylniederalkoxyniederalkanoyl, wie Benzyloxyacetyl, 2-Halogenniederalkanoyl, wie 2-Chloracetyl, Amino-, Niederalkylamino- oder Diniederalkylamino-niederalkoxy-2-niederalkanoyl, wie Dimethylamino-niederalkoxyacetyl, Amino-, Niederalkylamino-oder Diniederalkylamino-niederalkoxyniederalkoxy-2-niederalkanoyl, wie Dimethylamino-(2-niederalkoxyethoxy)acetyl, Niederalkanoyloxyniederalkanoyl, z.B. Niederalkanoyloxyacetyl oder Niederalkanoyloxypropionyl, wie Acetoxyacetyl oder β-Acetoxypropionyl, Halogenniederalkanoyl, z.B. α-Halogenacetyl, wie α-Chlor-, α-Brom-, α-Iod-α,α,α-Trifluor oder α,α,α-Trichloracetyl, oder Halogenpropionyl, wie β-Chlor- oder β-Brompropionyl, Carboxyniederalkanoyl, z.B. Carboxyacetyl oder 3-Carboxypropionyl, Niederalkoxycarbonylniederalkanoyl, z.B. Niederalkoxycarbonylacetyl oder Niederalkoxycarbonylpropionyl, wie Methoxycarbonylacetyl, β-Methoxycarbonylpropionyl, Ethoxycarbonylacetyl, β-Ethoxycarbonylpropionyl, tert-Butoxycarbonylacetyl oder β-tert-Butoxycarbonylpropionyl, Carbamoylniederalkanoyl, z.B. Carbamoylacetyl oder β-Carbamoylpropionyl, Niederalkylcarbamoylniederalkanoyl, z.B. Methylcarbamoylacetyl oder β-(N-Niederalkyl)carbamoylpropionyl, wie β-(N-Methyl)-, β-(N-Ethyl)-, β-(N-(n-Propyl))-carbamoyl- oder β-(N-(n-Hexyl))-carbamoylpropionyl, Diniederalkylcarbamoylniederalkanoyl, z.B. Dimethylcarbamoylacetyl, β-(N,N-(Di-niederalkyl)-carbamoyl)propionyl, wie β-(N,N-Dimethyl)-, β-(N,N-Diethyl)-, β-(N,N-Di-(n-propyl)-carbamoyl)-oder β-(N,N-Di-(n-hexyl))-carbamoylpropionyl, β-Pyrrolidinocarbonylpropionyl, β-Morpholinocarbonylpropionyl, β-Thiomorpholinocarbonylpropionyl, β-Piperidin-1-ylcarbonypropionyl, β-Piperazin-1-ylcarbonylpropionyl oder β-(4-Niederalkyl-piperazin-1-ylcarbonyl)-propionyl, wie β-(4-Methylpiperazin-1-ylcarbonyl)propionyl, Oxoniederalkanoyl, z.B. Acetoacetyl oder Propionylacetyl, Hydroxy-carboxy-niederalkanoyl, z.B. α-Hydroxy-α-carboxy-acetyl oder α-Hydroxy-β-carboxypropionyl, Hydroxy-niederalkoxycarbonyl-niederalkanoyl, z.B. α-Hydroxy-α-ethoxy- oder -methoxycarbonylacetyl oder α-Hydroxy-β-ethoxy- oder -methoxycarbonyl-propionyl, α-Acetoxy-α-methoxycarbonyl-acetyl, Dihydroxy-carboxy-niederalkanoyl, z.B. α,β-Dihydroxy-β-carboxypropionyl, Dihydroxy-niederalkoxycarbonyl-niederalkanoyl, z.B. α,β-Dihydroxy-β-ethoxy-oder -methoxycarbonyl-propionyl, α,β-Diacetoxy-β-methoxycarbonyl-propionyl, α-Naphthyloxy-carboxy-niederalkanoyl, z.B. 2-α-Naphthoxy-4-carboxy-butyryl, α-Naphthoxy-niederalkoxycarbonylniederalkanoyl, z.B. α-Naphthoxy-ethoxycarbonylacetyl, 2-α-Naphthoxy-3-ethoxycarbonyl-propionyl oder 2-α-Naphthoxy-4-tert-butoxycarbonylbutyryl, α-Naphthoxy-benzyloxycarbonyl-niederalkanoyl, z.B. 2-α-Naphthoxy-3-benzyloxycarbonyl-propionyl, α-Naphthoxy-carbamoyl-niederalkanoyl, z.B. 2-α-Naphthoxy-4-carbamoyl-butyryl, α-Naphthoxy-cyanoniederalkanoyl, z.B. α-Naphthoxy-cyano-acetyl oder 2-α-Naphthoxy-4-cyanobutyryl, α-Naphthoxy-oxo-niederalkanoyl, z.B. 2-α-Naphthoxy-4-oxo-pentanoyl, Heterocyclylniederalkanoyl, z.B. gegebenenfalls substituiertes Pyrrolylcarbonyl, z.B. 2- oder 3-Pyrrolylcarbonyl, Furylcarbonyl, z.B. 2-Furylcarbonyl, 5-Hydroxymethyl-furan-2-ylcarbonyl, Thienylcarbonyl, z.B. 2-Thienylcarbonyl, Pyridylniederalkanoyl, wie Pyridylcarbonyl, z.B. 2-, 3-oder 4-Pyridylcarbonyl, Pyridylacetyl, z.B. 2-Pyridylacetyl, oder Pyridylpropionyl, z.B. 3-(2-Pyridyl)propionyl, Chinolylcarbonyl, wie Chinolin-2-ylcarbonyl, Isochinolincarbonyl, wie Isochinolin-3-ylcarbonyl, gegebenenfalls substituiertes Indolylcarbonyl, z.B. 2-, 3- oder 5-Indolylcarbonyl, 1-Methyl-, 5-Methyl-, 5-Methoxy-, 5-Benzyloxy-, 5-Chlor- oder 4,5-Dimethylindolyl-2-carbonyl, Cyclohepta[b]pyrrolyl-5-carbonyl, Pyrrolidin-(2- oder 3-)yl-carbonyl (wobei Pyrrolidinyl-2-carbonyl (= Prolyl) vorzugsweise in der D- oder L-Form vorliegt), Hydroxypyrrolidinylcarbonyl, z.B. 3- oder 4-Hydroxypyrrolidinyl-2-carbonyl, Oxopyrrolidinylcarbonyl, z.B. 5-Oxopyrrolidinyl-2-carbonyl, Piperidinylcarbonyl, z.B. 2-, 3- oder 4-Piperidinylcarbonyl, 1,2,3,4-Tetrahydrochinolylcarbonyl, z.B. 1,2,3,4-Tetrahydrochinolyl-2-, -3- oder -4-carbonyl, oder 1,2,3,4-Tetrahydroisochinolylcarbonyl, z.B. 1,2,3,4-Tetrahydroisochinolyl-1-, -3- oder 4-carbonyl, Imidazolylniederalkanoyl, wie Imidazolylcarbonyl, z.B. Imidazol-1-ylcarbonyl oder Imidazol-4-ylcarbonyl, Imidazolylacetyl, z.B. 4-Imidazolylacetyl, oder Imidazolylpropionyl, z.B. 3-(4-Imidazolyl)propionyl, Morpholinocarbonyl, Thiomorpholinocarbonyl, Morpholinoacetyl, Thiomorpholinoacetyl, 4-Niederalkyl-1-piperazinoacetyl, wie 4-Methyl-piperazinoacetyl, Indolylacetyl oder Benzofuranylacetyl, Niederalkenoyl, z.B. Acryloyl, Vinylacetyl, Crotonoyl oder 3- oder 4-Pentenoyl, Niederalkinoyl, z.B. Propioloyl oder 2- oder 3-Butinoyl, Cycloalkylcarbonyl, z.B. Cyclopropyl-, Cyclobutyl-, Cyclopentyl- oder Cyclohexylcarbonyl, Bicycloalkylcarbonyl, z.B. Decahydronaphthyl-2-carbonyl, endo- oder exo-Norbornyl-2-carbonyl, Bicyclo[2.2.2]oct-2-ylcarbonyl oder Bicyclo[3.3.1]-non-9-ylcarbonyl, Tricycloalkylcarbonyl, z.B. 1- oder 2-Adamantylcarbonyl, Cycloalkenylcarbonyl, z.B. 1-Cyclohexenylcarbonyl oder 1,4-Cyclohexadienylcarbonyl, Bicycloalkenylcarbonyl, z.B. 5-Norbornen-2-ylcarbonyl oder Bicyclo[2.2.2]octen-2-ylcarbonyl, Cyclopropylacetyl, Cyclopentylacetyl, Cyclohexylacetyl oder 3-Cyclohexylpropionyl, Cycloalkylniederalkenoyl, z.B. Cyclohexylacryloyl, Cycloalkenylniederalkanoyl, z.B. 1-Cyclohexenylacetyl oder 1,4-Cyclohexadienylacetyl, Phenylniederalkanoyl, z.B. Benzoyl, Phenylacetyl oder 3-Phenylpropionyl, im Phenylrest unsubstituiert, mono- oder mehrfachsubstituiert durch Niederalkyl, z.B. Methyl, Haloniederalkyl, wie Chlor- oder Brommethyl, Halogen, z.B. Fluor oder Chlor, Hydroxy, Niederalkoxy, z.B. Methoxy, Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinomethyl, Thiomorpholinomethyl, Cyano und/oder Nitro, oder α-Naphthyl- oder β-Naphthyl-niederalkanoyl, worin Napthyl unsubstituiert, mono-oder mehrfachsubstituiert durch Niederalkyl, z.B. Methyl, Phenyl, Halogen, z.B. Chlor, Hydroxy, Niederalkoxy, z.B. Methoxy, und/oder Nitro ist, wobei Niederalkanoyl in Phenyl-, α-Naphthyl- oder β-Naphthyl-niederalkanoyl unsubstituiert oder substituiert z.B. durch Hydroxy, Niederalkoxy, Niederalkanoyloxy, Carboxy, Niederalkoxycarbonyl, Phenylniederalkoxycarbonyl, wie Benzyloxycarbonyl, Carbamoyl, Niederalkylcarbamoyl, Diniederalkylcarbamoyl, Cyano und/oder Oxo sein kann und gegebenenfalls verzweigt ist, z.B. 4-Chlormethyl-, 4-Brommethyl-, 4-Fluor-, 4-Chlor-, 4-Methoxy-, 4-Morpholinomethyl-, 4-Thiomorpholinomethyl-, 4-Cyano- oder 4-Nitrobenzoyl, α-Naphthylacetyl, β-Naphthylacetyl, Niederalkylphenylacetyl, wie 4-Methylphenylacetyl, Niederalkoxyphenylacetyl, wie 4-Methoxyphenylacetyl, 2-Niederalkoxy-2-phenylacetyl, wie (R)- oder (S)-2-Methoxy-2-phenylacetyl, 3-(p-Hydroxyphenyl)-propionyl, Diphenylacetyl, Di-(4-methoxyphenyl)-acetyl, Triphenylacetyl, 3-α- oder 3-β-Naphthylpropionyl, 3-Phenyl- oder 3-α-Naphthyl-2-hydroxy-propionyl, 3-Phenyl- oder 3-α-Naphthyl-2-niederalkoxypropionyl, wie 3-Phenyl- oder 3-α-Naphthyl-2-neopentyloxy-propionyl, 3-Phenyl-2-pivaloyloxy-oder 2-acetoxy-propionyl, 3-α-Naphthyl-2-pivaloyloxy- oder -2-acetoxy-propionyl, 3-Phenyl- oder 3-α-Naphthyl-2-carboxymethylpropionyl, 3-Phenyl- oder 3-α-Naphthyl-2-niederalkoxycarbonyl-propionyl, wie 3-α-Naphthyl-2-ethoxycarbonyl-propionyl, 3-Phenyl- oder 3-α-Naphthyl-2-benzyloxycarbonylmethyl-propionyl, 3-Phenyl-oder 3-α-Naphthyl-2-carbamoyl-propionyl, 3-Phenyl- oder 3-α-Naphthyl-2-tert-butylcarbamoyl-propionyl, 3-Phenyl- oder 3-α-Naphthyl-2-cyano-propionyl, 3-Phenyl- oder 3-α-Naphthyl-2-cyanomethyl-propionyl, 3-Phenyl- oder 3-α-Naphthyl-2-acetonyl-propionyl, 2-Benzyl- oder 2-α-Naphthylmethyl-4-cyano-butyryl, 4-Phenyl- oder 4-α-Naphthyl-3-carboxy-butyryl, 4-Phenyl- oder 4-α-Naphthyl-3-benzyloxycarbonyl-butyryl, 2-Benzyl-oder 2-α-Naphthylmethyl-4-oxo-pentanoyl, Phenylniederalkenoyl, z.B. β-Phenylacryloyl oder β-Phenylvinylacetyl, Naphthylcarbonyl, z.B. α- oder β-Naphthylcarbonyl, Indenylcarbonyl, z.B. 1-, 2- oder 3-Indenylcarbonyl, oder Indanylcarbonyl, z.B. 1-oder 2-Indanylcarbonyl.

Bevorzugte Acylgruppen R₁ eines Halbesters der Kohlensäure sind z.B. unsubstituiertes oder substituiertes Alkyloxycarbonyl, insbesondere Niederalkoxycarbonyl, z.B. Methoxy-, Ethoxy- oder tert-Niederalkoxycarbonyl, wie tert-Butoxycarbonyl, 2-Halogenniederalkoxycarbonyl, z.B. 2-Chlor-, 2-Brom-, 2-Iod- oder 2,2,2-Trichlorethoxycarbonyl; Arylniederalkoxycarbonyl, z.B. Arylmethoxy-carbonyl, worin Aryl vorzugsweise 6 bis 14 Kohlenstoffatome hat, unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Heterocyclylniederalkyl, worin Heterocyclyl wie oben als Substituent für Niederalkanoyl definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoylpiperazin-1-ylmethyl, wie 4-Acetyl-piperazin-1-ylmethyl, Morpholinomethyl oder Thiomorpholinomethyl, Cyano und/oder Nitro ein oder mehrfach, vorzugsweise einfach, substituiert ist, und insbesondere Phenyl, 1- oder 2-Naphthyl, Fluorenyl oder durch Niederalkyl, z.B. Methyl oder tert-Butyl, Niederalkoxy, z.B. Methoxy, Ethoxy oder tert-Butoxy, Hydroxy, Halogen, z.B. Fluor, Chlor oder Brom, und/oder Nitro mono- oder mehrfach substituiertes Phenyl ist, z.B. Phenylniederalkoxycarbonyl, wie Benzyloxycarbonyl, 4-Methoxybenzyloxycarbonyl, 4-Nitrobenzyloxycarbonyl, Diphenylniederalkoxycarbonyl, wie Diphenylmethoxycarbonyl, Di-(4-methoxyphenyl)-methoxycarbonyl, Trityloxycarbonyl oder Fluorenylniederalkoxycarbonyl, wie 9-Fluorenylmethoxycarbonyl; oder ferner Heterocyclylniederalkoxycarbonyl, worin Heterocyclyl wie oben als Substituent von Alkanoyl definiert ist, z.B. Furan-2-ylmethoxycarbonyl oder Pyridin-2-, - 3- oder -4-ylmethoxycarbonyl. Die jeweils unter die Definition von Acylgruppen R₁ eines Halbesters der Kohlensäure fallenden Definitionen können vorzugsweise bei allen vor-und nachstehend genannten Definitionen von Verbindungen der Formel I weggelassen werden.

Bevorzugte Acylgruppe R₁ einer N-substituierten Carbaminsäure ist ein Aminocarbonylrest, worin die Aminogruppe 1 bis 2 Substituenten trägt, die unabhängig voneinander aus unsubstituiertem oder substituiertem Niederalkyl, dessen Substituenten aus den oben für substituiertes Niederalkanoyl genannten ausgewählt sind und in der dort definierten Anzahl vorhanden sein können, vorzugsweise Substituenten ausgewählt aus Hydroxy, Niederalkoxy, Phenoxy, Naphthoxy, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo und C₆-C₁₂-Aryl, z.B. Phenyl, Naphthyl, wie 1- oder 2-Naphthyl, Indanyl, wie 1- oder 2-Indanyl, Indenyl, wie Inden-1-yl, oder Fluorenyl, wie Fluoren-9-yl, wobei Aryl unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono-oder Di-hydroxyniederalkylcarbamoyl, Haloniederalkyl, wie Trifluormethyl, Cyano und/oder Nitro ein oder mehrfach, vorzugsweise einfach, substituiert ist, insbesondere Phenyl, welches durch einen der genannten Reste in p-Stellung substituiert ist; insbesondere aus unsubstituiertem Niederalkyl, wie Methyl oder Ethyl; und Aryl, welches vorzugsweise 6 bis 14 Kohlenstoffatome hat und unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono-oder Diniederalkylcarbamoyl, Mono- oder Di-hydroxyniederalkylcarbamoyl, Haloniederalkyl, wie Trifluormethyl, Heterocyclylniederalkyl, worin Heterocyclyl wie oben als Substituent für Niederalkanoyl definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinomethyl oder Thiomorpholinomethyl, Cyano und/oder Nitro ein oder mehrfach, vorzugsweise einfach, substituiert ist, vorzugsweise entsprechend substituiertes Phenyl oder 1- oder 2-Naphthyl ausgewählt sind, wobei der Rest einer N-substituierten Carbaminsäure am Stickstoff nicht mehr als einen der genannten Arylreste tragen kann; insbesondere handelt es sich bei einer Acylgruppe R₁ einer N-substituierten Carbaminsäure um Mono-oder Diniederalkylaminocarbonyl, wie N-Methyl-, N-Ethyl-, N,N-Dimethyl- oder N,N-Diethylaminocarbonyl, oder Phenylniederalkylaminocarbonyl, worin Phenyl unsubstituiert oder durch die bei der Definition von Aryl genannten Reste substituiert ist, z.B. durch Niederalkyl, z.B. Methyl, Halogenniederalkyl, wie Chlor- oder Brommethyl oder Trifluormethyl, Halogen, z.B. Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl)- oder N-(4-Cyanobenzyl)-aminocarbonyl; besonders bevorzugt ist durch nur einen Rest am Stickstoff substituiertes Aminocarbonyl, z.B. N-Niederalkylaminocarbonyl, wie N-Methyl- oder N-Ethyl-aminocarbonyl, oder Phenylniederalkylaminocarbonyl, worin Phenyl unsubstituiert oder durch die bei der Definition von Aryl genannten Reste substituiert ist, z.B. durch Niederalkyl, wie Methyl, Halogenniederalkyl, wie Chlor-oder Brommethyl oder Trifluormethyl, Halogen, wie Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl)-oder N-(4-Cyanobenzyl)-aminocarbonyl. Die jeweils unter die Definition von Acylgruppen R₁ einer N-substituierten Carbaminsäure fallenden Definitionen und der Rest Aminocarbonyl R₁ können vorzugsweise bei allen vor- und nachstehend genannten Definitionen von Verbindungen der Formel I weggelassen werden.

Bevorzugte Acylgruppen R₁ einer unsubstituierten oder substituierten Aminosäure werden gebildet durch die über ihre Carbonylgruppe gebundenen Aminosäurereste einer α-, β-, γ-oder δ-Aminosäure, insbesondere
einer natürlichen α-Aminosäure mit der L-Konfiguration, wie sie normalerweise in Proteinen vorkommen, oder eines Epimeren einer solchen Aminosäure, d. h. mit der unnatürlichen D-Konfiguration, oder deren D,L-Isomerengemisch, eines Homologen einer solchen Aminosäure, z. B. worin die Aminosäurenseitenkette um eine oder zwei Methylengruppen verlängert oder verkürzt ist, worin die Aminogruppe in β-, γ- oder δ-Stellung vorliegt und/oder worin eine Methylgruppe durch Wasserstoff ersetzt ist, einer substituierten aromatischen Aminosäure, worin der aromatische Rest 6 - 14 Kohlenstoffatome hat, z. B. eines substituierten Phenylalanins oder Phenylglycins, worin der Phenyl-Substituent Niederalkyl, z. B. Methyl, Hydroxy, Niederalkoxy, z. B. Methoxy, Niederalkanoyloxy, z. B. Acetoxy, Amino, Niederalkylamino, z. B. Methylamino, Diniederalkylamino, z. B. Dimethylamino, Niederalkanoylamino, z. B. Acetylamino oder Pivaloylamino, Niederalkoxycarbonylamino, z. B. tert-Butoxycarbonylamino, Arylmethoxycarbonylamino, worin Aryl vorzugsweise 6 - 14 Kohlenstoffatome hat, z. B. Benzyloxycarbonylamino oder 9-Fluorenylmethoxycarbonylamino, Halogen, z. B. Fluor, Chlor, Brom oder Jod, Carboxy und/oder Nitro sein kann und ein- oder mehrfach vorkommt, eines benzannelierten Phenylalanins oder Phenylglycins, wie α-Naphthylalanins, oder eines hydrierten Phenylalanins oder Phenylglycins, wie Cyclohexylalanins oder Cyclohexylglycins. Im Rahmen der genannten Definitionen sind bereits zuvor erwähnte Acylgruppen von Aminosäuren, wie Prolyl, Indolin-2-carbonyl, 1,2,3,4-Tetrahydroisochinolin-3-carbonyl und trans-3- und trans-4-Hydroxyprolyl, welche bereits bei der Definition von substituiertem Niederalkanoyl R₁ vorkommen, ausgenommen, aber nur mit dem einzigen Zweck, die Überlappung der Definitionen zu vermeiden.

Diese Aminosäurereste können an freien Amino- oder Hydroxyfunktionen, vorzugsweise an einer freien Aminofunktion, durch einen der oben unter Acyl R₁ als Acylgruppe einer Carbonsäure oder eines Halbesters der Kohlensäure genannten Reste, durch unsubstituiertes oder substituiertes Alkyl, insbesondere Niederalkyl, wie Methyl, Ethyl, Isopropyl, n-Propyl oder n-Butyl, worin die Substituenten beispielsweise aus einem oder mehreren Resten, vorzugsweise aus bis zu drei Resten, insbesondere aus einem Rest ausgewählt aus der Gruppe Hydroxy, Niederalkoxy, Phenoxy, Naphthoxy, Niederalkanoyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo, Cycloalkyl, z.B. C₃-C₈-Cycloalkyl, wie Cyclopropyl, Cyclobutyl, Cyclopentyl oder Cyclohexyl, Bicyloalkyl, z.B. C₆-C₁₂-Bicycloalkyl, wie Decahydronaphth-2-yl, endo- oder exo-2-Norbornyl, Bicyclo[2.2.2]oct-2-yl oder Bicyclo[3.3.1.]non-9-yl, Tricycloalkyl, z.B. C₉-C₁₄-Tricycloalkyl, wie 1- oder 2-Adamantyl, Cycloalkenyl, z.B. C₄-C₈-Cycloalkenyl, wie 1-Cyclohexenyl oder 1,4-Cyclohexadienyl, Bicycloalkenyl z.B. 5-Norbornen-2-yl oder Bicyclo[2.2.2]octen-2-yl, Heterocyclyl, welches einen als solchen oder in ein-oder zweifach, insbesondere einfach, benz-, cyclopenta-, cyclohexa- oder cyclohepta-annellierter Form vorliegenden gesättigten, teilweise gesättigten oder ungesättigten Ring, der 3 bis 7, vorzugsweise 5 - 7 Ringatome enthält und bis zu vier Heteroatome ausgewählt aus Stickstoff, Schwefel und/oder Sauerstoff enthält, vorzugsweise 1 oder 2 der genannten Heteroatome, und unsubstituiert oder, insbesondere durch Niederalkyl, Niederalkanoyl, Hydroxy, Niederalkoxy, Phenylniederalkoxy, wie Benzyloxy, Hydroxyniederalkyl, wie Hydroxymethyl, Halogen, Cyano und/oder Trifluormethyl, substituiert sein kann, bedeutet, z. B. Pyrrolyl, 2,5-Dihydropyrrolyl, Furanyl, Thienyl, Tetrahydrofuranyl, Cyclohepta[b]pyrrolyl, Pyrrolidinyl, Imidazolyl, Imidazolidinyl, Pyrazolinyl, Pyrazolidinyl, Triazolyl, wie 1,2,3-, 1,2,4-oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Chinolyl, Isochinolyl, Benzimidazolyl, Benzofuranyl, Pyridyl, Pyrimidinyl, Piperidinyl, Piperazin-1-yl, Morpholino, Thiomorpholino, S,S-Dioxothiomorpholino, 1,2-Dihydro-oder 1,2,3 4-Tetrahydrochinolyl, oder 1,2-Dihydro- oder 1,2,3,4-Tetrahydroisochinolyl, wobei die genannten Reste unsubstituiert oder wie oben substituiert sind, insbesondere durch Niederalkyl, z. B. in 4-Niederalkyl-piperazin-1-yl, wie 4-Methyl- oder 4-Ethyl-piperazin-1-yl, oder durch Niederalkanoyl, z. B. in 4-Niederalkanoylpiperazin-1-yl, wie 4-Acetyl-piperazin-1-yl; und Aryl, vorzugsweise C₆-C₁₂-Aryl, z.B. Phenyl, Naphthyl, wie 1- oder 2-Naphthyl, Indanyl, wie 1- oder 2-Indanyl, Indenyl, wie Inden-1-yl, oder Fluorenyl, wie Fluoren-9-yl, wobei die genannten Arylreste unsubstituiert oder beispielsweise durch Niederalkyl, z.B. Methyl, Halogenniederalkyl, wie Chlor-oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Heterocyclylniederalkyl, worin Heterocyclyl wie oben definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinomethyl, oder Thiomorpholinomethyl, Cyano und/oder Nitro ein oder mehrfach, vorzugsweise einfach, substituiert sind, insbesondere Phenyl, welches durch einen der genannten Reste in p-Stellung substituiert ist, ausgewählt sind, insbesondere durch einen entsprechend substituierten Niederalkylrest, vor allem entsprechend substituiertes Methyl, vorzugsweise Benzyl, Diphenylmethyl, Trityl, 2- 3- oder 4-Pyridylmethyl, oder ferner durch einen der im Abschnitt über Verfahren als Schutzgruppen genannten Reste substituiert sein, oder an Carboxygruppen derivatisiert sein.

Besonders bevorzugt ist das über die Carbonylgruppe gebundene Radikal einer Aminosäure ausgewählt aus Glycin (H-Gly-OH), Alanin (H-Ala-OH), 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure oder 5-Aminohexansäure, Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxybuttersäure), Threonin (H-Thr-OH), Methionin (H-Met-OH), Cystein (H-Cys-OH), Phenylalanin (H-Phe-OH), Tyrosin (H-Tyr-OH), 4-Aminophenylalanin, 4-Chlorphenylalanin, 4-Carboxyphenylalanin, β-Phenylserin (β-Hydroxyphenylalanin), Phenylglycin, α-Naphthylalanin (H-Nal-OH), Cyclohexylalanin (H-Cha-OH), Cyclohexylglycin, Tryptophan (H-Trp-OH), Asparaginsäure (H-Asp-OH), Asparagin (H-Asn-OH), Aminomalonsäure, Aminomalonsäure-monoamid, Glutaminsäure (H-Glu-OH), Glutamin (H-Gln-OH), Histidin (H-His-OH), Arginin (H-Arg-OH), Lysin (HLys-OH), δ-Hydroxylysin, Ornithin (α,δ-Diaminovaleriansäure), 3-Aminopropansäure, α,γ-Diaminobuttersäure und α,β-Diaminopropionsäure, besonders bevorzugt der Rest einer aliphatischen Aminosäure ausgewählt aus Alanin, Valin, Norvalin, Leucin, 3-Aminopropionsäure, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure oder 5-Aminohexansäure und Isoleucin oder einer Aminosäure ausgewählt aus Glycin, Asparagin, Glutamin, Methionin, Lysin und Phenylalanin, wobei (ausser in Fällen, wo kein asymmetrisches Kohlenstoffatom vorliegt, z.B. bei Glycin) jede der genannten Aminosäuren in der D-, L- oder (D,L)-, vorzugsweise in der L-Form vorliegen kann,
eine α-Aminogruppe, falls vorhanden, unsubstituiert oder ein- oder zweifach N-alkyliert, z. B. durch Niederalkyl, wie Methyl, n-Propyl oder n-Butyl, durch Aminoniederalkyl, wie 3-Aminopropyl, durch Phenyl- oder Naphthylaminoniederalkyl, wie 3-Phenylaminopropyl, durch Phenylniederalkyl, wie Benzyl, durch Diphenylmethyl, durch Trityl, und/oder durch Heterocyclylniederalkyl, worin Heterocylcyl wie oben für eine Acylgruppe R₁ einer Carbonsäure definiert ist, insbesondere durch Heterocyclylmethyl, z.B. Furanylniederalkyl, wie 2-Furylmethyl, Thienylniederalkyl, wie 2-Thienylmethyl, Imidazolylniederalkyl, wie Imidazol-4-almethyl, oder 2-, 3- oder 4-Pyridylniederalkyl, wie 2-, 3- oder 4-Pyridylmethyl, und/oder N-acyliert, z. B. durch die oben bei der Definition von R₁ genannten Acylgruppen einer Carbonsäure, insbesondere durch unsubstituiertes oder substituiertes Niederalkanoyl, wie oben definiert, vor allem durch Acetyl, Propionyl, Pivaloyl, Heterocyclylniederalkanoyl, wie oben für Acyl R₁ definiert, z.B. Furan-2-ylcarbonyl, 5-Hydroxymethyl-furan-2-ylcarbonyl, 2-, 3- oder 4-Pyridylcarbonyl, Morpholinocarbonyl, Thiomorpholinocarbonyl, Indolylacetyl oder Benzofuranylacetyl, Arylniederalkanoyl, wie Benzoyl oder Phenylacetyl, oder die oben bei der Definition von R₁ genannten Acylgruppen eines Halbesters der Kohlensäure, insbesondere Niederalkoxycarbonyl, wie tert-Butoxycarbonyl, oder Arylniederalkoxycarbonyl, wie Benzyloxycarbonyl; vorliegt,
eine Carboxygruppe der Seitenkette frei, in veresterter oder amidierter Form, z. B. als Niederalkylestergruppe, wie Methoxycarbonyl oder tert-Butoxycarbonyl, als Arylestergruppe oder als Arylniederalkylestergruppe, worin Aryl Phenyl, 4-Nitrophenyl, Naphthyl, Fluorenyl oder Biphenylyl bedeutet, z. B. als 4-Nitrophenyloxycarbonyl, Benzyloxycarbonyl oder 9-Fluorenylmethoxycarbonyl, oder als Carbamoyl-, als Niederalkylcarbamoyl-, wie Methylcarbamoyl-, als Diniederalkylcarbamoyl-, wie Dimethylcarbamoyl-, als Mono- oder Di-(hydroxyniederalkyl)carbamoyl-, wie Hydroxymethylcarbamoyl-oder Di-(hydroxymethyl)carbamoyl-, oder als Mono- oder Di-(carboxyniederalkyl)carbamoyl-, wie Carboxymethylcarbamoyl- oder Di-(carboxymethyl)carbamoylgruppe vorliegt,
eine Aminogruppe der Seitenkette, die sich nicht in α-Stellung befindet, frei oder in alkylierter Form, z. B. als Mono- oder Diniederalkylamino, wie n-Butylamino oder Dimethylamino, oder in acylierter Form, z. B. als Niederalkanoylamino, wie Acetylamino oder Pivaloylamino, als Aminoniederalkanoylamino, wie 3-Amino-3,3-dimethylpropionylamino, als Arylniederalkanoylamino, worin Aryl 6 - 14 Kohlenstoffatome hat, z. B. Phenyl, Naphthyl oder Fluorenyl bedeutet, und unsubstituiert oder durch Niederalkyl, Hydroxy, Niederalkoxy, Carboxy, Carbamoyl oder Sulfamoyl substituiert ist, wie 4-Hydroxyphenylbutyryl, als Niederalkoxycarbonylamino, wie tert-Butoxycarbonylamino, als Arylmethoxycarbonylamino, worin Aryl 6 - 14 Kohlenstoffatome hat, wie als Benzyloxycarbonylamino oder 9-Fluorenylmethoxycarbonylamino, als Piperidyl-1-carbonyl, Morpholinocarbonyl, Thiomorpholinocarbonyl oder als S,S-Dioxothiomorpholinocarbonyl vorliegt, und/oder
eine Hydroxygruppe der Seitenkette frei, in veretherter oder veresterter Form, z. B. als Niederalkoxy-, wie Methoxy- oder tert-Butoxy-, als Arylniederalkoxy-, insbesondere Phenylniederalkoxy-, wie Benzyloxy-, als Niederalkanoyloxy-, wie Acetoxy-, oder als Niederalkyloxycarbonyloxygruppe, z. B. tert-Butyloxycarbonyloxygruppe, vorliegt.

Insbesondere sind Acylgruppen R₁ einer unsubstituierten oder substituierten Aminosäure ausgewählt aus Alanyl, N-Niederalkylalanyl, wie N-Methylalanyl, Phenylalanyl, N-(Benzyloxycarbonyl)-phenylalanyl, N-(9-Fluorenylmethoxycarbonyl)phenylalanyl, Aminoacetyl (Glycyl), N-Niederalkylaminoacetyl, N,N-Diniederalkylaminoacetyl, N-Niederalkyl-N-phenylniederalkylaminoacetyl, N-Niederalkyl-N-imidazolylniederalkylaminoacetyl, N-Niederalkyl-N-pyridylniederalkylaminoacetyl, N-Niederalkyl-N-niederalkoxycarbonylaminoacetyl, N-Phenylniederalkoxycarbonyl-N-niederalkylaminoacetyl, N-Morpholino- oder N-Thiomorpholino-niederalkylaminoacetyl, z.B. N-Methylaminoacetyl, N,N-Dimethylaminoacetyl, N-Methyl-N-(n-butyl)aminoacetyl, N-Methyl-N-benzylaminoacetyl, N-Methyl-N-[(2-, 3- oder 4-)pyridylmethyl]-aminoacetyl, wie N-Methyl-N-(2- oder 3-)pyridylmethylaminoacetyl, N-(Imidazol-4-ylmethyl)-N-methylaminoacetyl, N-Methyl-N-tert-butoxycarbonylaminoacetyl, N-Benzyloxycarbonyl-N-niederalkylaminoacetyl, N-Morpholinocarbonyl-aminoacetyl, 3-Aminopropionyl, 2-Aminobutyryl, 3-Aminobutyryl, 4-Aminobutyryl, 4-(N,N-Dimethylamino)butyryl, 3-Aminopentanoyl, 4-Aminopentanoyl, 5-Aminopentanoyl, 3-Aminohexanoyl, 4-Aminohexanoyl oder 5-Aminohexanoyl, Valyl, N-Phenylacetyl-valyl, N-Acetyl-valyl, N-(3-Phenylpropionyl)-valyl, N-(2-, 3- oder 4-Pyridylcarbonyl)-valyl, N-Methoxycarbonyl-valyl, N-Isobutoxycarbonyl-valyl, N-tert-Butoxycarbonyl-valyl, N-Benzyloxycarbonyl-valyl, N-(Morpholinocarbonyl)-valyl, Norvalyl, Leucyl, N-Acetyl-leucyl, N-(2-, 3- oder 4-Pyridylcarbonyl)-leucyl, N-(Benzyloxycarbonyl)-leucyl, Isoleucyl, N-Acetyl-isoleucyl, N-Propionyl-isoleucyl, N-(Benzyloxycarbonyl)-isoleucyl, N-(tert-Butoxycarbonyl)-isoleucyl, Methionyl, Lysyl, Glutamyl, γ-(N-Benzyloxycarbonyl)-glutamyl, Asparagyl und β-(N-Benzyloxycarbonyl)-asparagyl bevorzugt, wobei die Aminosäurereste (ausser in Fällen, wo kein asymmetrisches Kohlenstoffatom vorliegt, z.B. bei Gly) vorzugsweise in der (L)- oder ferner der (D)- oder (D,L)-Form vorliegen.

Befinden sich Hydroxy- oder Aminogruppen, die ein freies Wasserstoffatom aufweisen, an einem Kohlenstoffatom, von welchem eine Doppelbindung ausgeht, z.B. bei substituiertem Niederalkenyl oder Niederalkinyl, in Verbindungen der Formel I, so sind tautomere Formen (durch Keto/Enoltautomerie bzw. Imin/Enamintautomerie) möglich. Diese und ähnliche Tautomere, deren Auftreten dem Fachmann geläufig ist, sind im Rahmen der vorliegenden Anmeldung ebenfalls umfasst. Bevorzugt sind Verbindungen der Formel I, worin im Rest R₁ keine Tautomerie möglich ist (z.B. keine Bindung von -OH oder - NH-an Kohlenstoffatome, von denen eine Doppelbindung ausgeht).

In allen Fällen sind ausreichend stabile Verbindungen gemeint, nicht jedoch instabile Verbindungen, wie sie der Fachmann ohne weiteres erkennen kann. Ausreichend stabil sind dabei insbesondere solche Verbindungen, die isoliert, gelagert und/oder beispielsweise zu pharmazeutischen Präparaten verarbeitet werden können.

Salze von Verbindungen der Formel I sind insbesondere Säureadditionssalze, Salze mit Basen oder bei Vorliegen mehrerer salzbildender Gruppen gegebenenfalls auch Mischsalze oder innere Salze.

Salze sind in erster Linie die pharmazeutisch verwendbaren, nichttoxischen Salze von Verbindungen der Formel I.

Solche Salze werden beispielsweise von Verbindungen der Formel I mit einer sauren Gruppe, z. B. einer Carboxygruppe, gebildet und sind beispielsweise deren Salze mit geeigneten Basen, wie nicht-toxische, von Metallen der Gruppe Ia, Ib, IIa und IIb des Periodensystems der Elemente abgeleitete Metallsalze, in erster Linie geeignete Alkalimetall-, z. B. Lithium-, Natrium- oder Kalium-, oder Erdalkalimetallsalze, z. B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, insbesondere Mono-, Di- oder Triniederalkylaminen, oder mit quaternären Ammoniumverbindungen gebildet werden, z. B. mit N-Methyl-N-ethylamin, Diethylamin, Triethylamin, Mono-, Bis- oder Tris-(2-hydroxyniederalkyl)-aminen, wie Mono-, Bis- oder Tris-(2-hydroxyethyl)-amin, 2-Hydroxy-tert-butylamin oder Tris(hydroxymethyl)-methylamin, N,N-Diniederalkyl-N-(hydroxyniederalkyl)-aminen, wie N,N-Dimethyl-N-(2-hydroxyethyl)-amin oder Tri-(2-hydroxyethyl)-amin, N-Methyl-D-glucamin oder quaternären Ammoniumsalzen, wie Tetrabutylammoniumsalzen. Die Verbindungen der Formel I mit einer basischen Gruppe, z. B. einer tertiären Aminogruppe, können Säureadditionssalze bilden, beispielsweise mit anorganischen Säuren, z. B. Halogenwasserstoffsäure, wie Salzsäure, Schwefelsäure oder Phosphorsäure, oder mit organischen Carbon-, Sulfon-, Sulfo- oder Phosphosäuren oder N-substituierter Sulfaminsäuren, wie z. B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner mit Aminosäuren, wie z. B. den weiter vorn genannten α-Aminosäuren, sowie mit Methansulfonsäure, Ethansulfonsaure, 2-Hydroxyethansulfonsäure, Ethan-1,2-disulfonsäure, Benzolsulfonsäure, 4-Methylbenzolsulfonsäure, Naphthalin-2-sulfonsäure, 2- oder 3-Phosphoglycerat, Glucose-6-phosphat, N-Cyclohexylsulfaminsäure (unter Bildung der Cyclamate) oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Ausdrücke "Verbindungen" und "Salze" schliessen ausdrücklich auch einzelne Verbindungen oder einzelne Salze ein.

Die Verbindungen der Formel I haben wertvolle pharmakologische Eigenschaften. So werden aus ihnen nach Applikation an einen Warmblüter, wie Menschen, auf metabolischem Wege Verbindungen der Formel II freigesetzt, welche als antiretroviral wirksame Hemmstoffe von Aspartatproteasen, wie HIV-Protease, beschrieben sind und insbesondere zur Behandlung von AIDS als Hemmstoffe der Aspartatproteasen von HIV-1 und/oder HIV-2 (und gegebenenfalls weiteren Retroviren, die AIDS-analoge Symptome hervorrufen) geeignet sind (siehe EP 0 346 847 (publiziert am 19. Juni 1991) und die weiteren oben genannten Publikationen). Die Bindung der Verbindung der Formel II an HIV-1-Protease wird beispielsweise in J. Med. Chem. 34, 3340-3342 (1991) beschrieben, die Hemmwirkung auf HIV-1 und HIV-2-Proteasen und die antivirale Hemmwirkung in Zellkulturen in Science 248, 358-361 (1990). Auch eine Wirkung auf SIV-infizierte Zellen ist beschrieben (Biochem. Biophys. Res. Commun. 176, 180-188 (1991)).

Die Verbindungen der Formel II werden dabei im Körper des zu behandelnden Tieres, vorzugsweise eines Warmblüters einschliesslich eines Menschen, aus den Verbindungen der Formel I freigesetzt.

Mit Hilfe der Verbindungen der Formel I ist es möglich, insbesondere auch bei enteraler, vorzugsweise oraler, Verabreichung der Verbindungen der Formel I eine deutlich verbesserte Resorption gegenüber der Verbindung der Formel II und/oder eine höhere Blutkonzentration der Verbindung der Formel II zu erreichen, als dies bei Verabreichung der Verbindung der Formel II selbst unter sonst gleichen Bedingungen möglich ist. Auch wird es möglich sein, durch den eingeführten Rest R₁ beispielsweise die Verteilung des Wirkstoffes im Körper in nützlicher Weise zu beeinflussen. Somit wird die eingangs erwähnte Aufgabe, Vorstufen von Verbindungen mit antiretroviraler Wirkung mit verbessertem pharmakodynamischem Profil zur Behandlung retroviraler Erkrankungen, wie AIDS, zur Verfügung zu stellen, durch neue Verbindungen der Formel I gelöst.

Die vorteilhaften pharmakodynamischen Eigenschaften können beispielsweise wie folgt belegt werden:

Die zu untersuchenden Verbindungen der Formel I oder als Kontrolle die Vergleichsverbindung der Formel II werden in Dimethylsulfoxid (DMSO) in einer Konzentration von 240 mg/ml gelöst. Die resultierenden Lösungen werden mit 20% (w/v) Hydroxypropyl-β-cyclodextrin (HPβCD) verdünnt, um eine Konzentration der Testsubstanz von 12 mg/ml zu erhalten. Diese Lösung wird Mäusen durch künstliche Sonderernährung in einer Dosis von 120 mg/kg verabreicht. 30, 60, 90 und 120 min nach der Verabreichung werden die Tiere getötet und Blut entnommen. Pro Zeitpunkt werden drei bis vier Tiere untersucht. Das Blut wird heparinisiert und für die Analyse durch eine der folgenden beiden Methoden vorbereitet: Nach der ersten Methode wird Vollblut deproteinisiert durch Mischen eines Volumenteils Blut mit einem Volumenteil Acetonitril; nach Zentrifugation wird der Überstand durch Reversed-Phase-HPLC untersucht. Nach der zweiten Methode wird ein interner Standard zum heparinisierten Blut in einer Endkonzentration von 4 µM zugegeben. Das Blut wird zentrifugiert. 0,25 ml Plasma werden abgenommen und mit einem gleichen Volumen Acetonitril deproteinisiert. Nach Zentrifugation wird der Überstand unter Vakuum eingetrocknet und der Rückstand in 20 µl 3M NaCl-Lösung und 100 µl 0,05M Phthalat-Puffer mit einem pH von 3,0 suspendiert. Die Suspension wird erst mit 1 ml, dann mit 0,2 ml Diisopropylether extrahiert. Die Diisopropylether-Lösung wird zur Trockene eingedampft und der Rückstand in 50 % (v/v) wässrigem Acetonitril gelöst. Diese Lösung wird durch Reversed-Phase-HPLC untersucht.

Die Analyse mittels Reversed-Phase-HPLC wird mit einer 125 x 4,6 mm Nucleosil® C₁₈-Säule (Reversed-Phase-Material der Firma Macherey-Nagel, Düren, Bundesrep. Deutschland auf der Basis von mit Kohlenwasserstoffresten von 18 Kohlenstoffatomen derivatisiertem Kieselgel) durchgeführt, welche äquilibriert ist mit einer mobilen Phase von 40% Acetonitril in Wasser/0,1% Trifluoressigsäure. Die Flussrate ist 1 ml/min. Detektion erfolgt bei 215 nm. Standards für die Verbindungen in Blut werden analog den Blutproben aufgearbeitet und zur Erstellung von Standardkurven verwendet, aufgrund derer die in-vivo-Konzentrationen ermittelt werden.

Beim Vergleich der Verbindungen der Formel I mit denen der Formel II (aktive Komponente) sind folgende Resultate erhältlich: Die Konzentration der aktiven Komponente der Formel II im Blut von Mäusen ist nach oraler Verabreichung einer Verbindung der Formel I, beispielsweise der Verbindung der Formel I, worin R₁ Acetyl bedeutet, zu den meisten Zeitpunkten, vorzugsweise zu allen oben genannten Zeitpunkten, deutlich höher als bei Verabreichung der Verbindung der Formel II in unveresterter Form, beispielsweise mehr als dreimal so hoch. Alternativ oder ergänzend hierzu ist die Absorption der Verbindung der Formel I, beispielsweise derjenigen, worin R₁ Acetyl bedeutet, deutlich höher als die Absorption der Verbindung der Formel II, beispielsweise mehr als viermal so hoch. Auch ist er möglich, längere Zeit mit einer Verbindung der Formel I einen höheren Blutspiegel zu erhalten als mit der Verbindung der Formel II.

Die Verbindungen der vorliegenden Erfindung können auch Verwendung finden als gewerblich verkäufliche Vergleichssubstanzen beim Test anderer Aspartatproteaseinhibitoren, indem sie praktisch als Standardsubstanzen verwendet werden, um z.B. bei anderen verwendeten Tierarten einen Massstab zu haben, inwieweit erhaltene Blutspiegel von der Tierspezies abhängen. Dabei kann zum einen eine hier nicht genannte Verbindung mit dem oben genannten Mausmodell untersucht werden, zum anderen eine hier genannte Verbindung der Formel I als Vergleichssubstanz im Vergleich mit der zu untersuchenden Verbindung bei anderen Tiermodellen eingesetzt werden.

Bei den im nachfolgenden genannten Definitionen von Verbindungen der Formel I können in sinnvoller Weise, beispielsweise zur Ersetzung allgemeinerer durch speziellere Definitionen, Definitionen von Resten aus jeweils weiter oben genannten allgemeinen Definitionen eingesetzt werden oder auch einzelne Substituenten weggelassen werden.

Bevorzugt sind Verbindungen der Formel I, worin R₁ Octanoyl, Decanoyl, Dodecanoyl, Palmitoyl, unsubstituiertes oder substituiertes Niederalkanoyl, Niederalkenyol oder Niederalkinoyl, worin die Substituenten aus einem oder mehreren Resten, vorzugsweise aus bis zu drei Resten, insbesondere aus einem Rest oder ferner zwei Resten ausgewählt aus der Gruppe Hydroxy, Niederalkoxy, Niederalkoxyniederalkoxy, Niederalkoxyniederalkoxyniederalkoxy, Phenoxy, Naphthoxy, Phenylniederalkoxy, 2-Halogenniederalkanoyl, wie 2-Chloracetyl, Amino-, Niederalkylamino- oder Diniederalkylamino-niederalkoxy-2-niederalkanoyl, wie Dimethylamino-niederalkoxyacetyl, Amino-, Niederalkylamino-oder Diniederalkylamino-niederalkoxyniederalkoxy-2-niederalkanoyl, wie Dimethylamino-(2-niederalkoxyethoxy)acetyl, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Carbamoyl, dessen Stickstoff Bestandteil eines 5- bis 7-gliedrigen heterocyclischen Ringes ist, der noch ein weiteres Heteroatom ausgewählt aus O, S, N und durch Niederalkyl, wie Methyl oder Ethyl, substituiertes N enthalten kann, z. B. Pyrrolidinocarbonyl, Morpholinocarbonyl, Thiomorpholinocarbonyl, Piperidin-1-ylcarbonyl, Piperazin-1-ylcarbonyl oder 4-Niederalkyl-piperazin-1-ylcarbonyl, wie 4-Methylpiperazin-1-ylcarbonyl, Cyano, Oxo, Cycloalkyl, z.B. C₃-C₈-Cycloalkyl, wie Cyclopropyl, Cyclobutyl, Cyclopentyl oder Cyclohexyl, Bicyloalkyl, z.B. C₆-C₁₂-Bicycloalkyl, wie Decahydronaphth-2-yl, endo-oder exo-2-Norbornyl, Bicyclo[2.2.2]oct-2-yl oder Bicyclo[3.3.1.]non-9-yl, Tricycloalkyl, z.B. C₉-C₁₄-Tricycloalkyl, wie 1- oder 2-Adamantyl, Cycloalkenyl, z.B. C₄-C₈-Cycloalkenyl, wie 1-Cyclohexenyl oder 1,4-Cyclohexadienyl, Bicycloalkenyl z.B. 5-Norbornen-2-yl oder Bicyclo[2.2.2]octen-2-yl, Heterocyclyl, welches einen als solchen oder in einfach benz-, cyclopenta-, cyclohexa- oder cyclohepta-annellierter Form vorliegenden gesättigten, teilweise gesättigten oder ungesättigten Ring, der 5 - 7 Ringatome enthält und bis zu vier Heteroatome ausgewählt aus Stickstoff, Schwefel und/oder Sauerstoff enthält, vorzugsweise 1 oder 2 der genannten Heteroatome, und unsubstituiert oder, insbesondere durch Niederalkyl, Niederalkanoyl, Hydroxy, Niederalkoxy, Phenylniederalkoxy, wie Benzyloxy, Hydroxyniederalkyl, wie Hydroxymethyl, Halogen, Cyano und/oder Trifluormethyl, substituiert sein kann, bedeutet, z. B. Pyrrolyl, 2,5-Dihydropyrrolyl, Furanyl, Thienyl, Tetrahydrofuranyl, Cyclohepta[b]pyrrolyl, Pyrrolidinyl, Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Chinolyl, Isochinolyl, Benzimidazolyl, Benzofuranyl, Pyridyl, Pyrimidinyl, Piperidinyl, Piperazin-1-yl, Morpholino, Thiomorpholino, S,S-Dioxothiomorpholino, 1,2-Dihydro- oder 1,2,3,4-Tetrahydrochinolyl, oder 1,2-Dihydro- oder 1,2,3,4-Tetrahydroisochinolyl, wobei die genannten Reste unsubstituiert oder wie oben substituiert sind, insbesondere durch Niederalkyl, z. B. in 4-Niederalkyl-piperazin-1-yl, wie 4-Methyl- oder 4-Ethyl-piperazin-1-yl, durch Niederalkanoyl, z. B. in 4-Niederalkanoyl-piperazin-1-yl, wie 4-Acetyl-piperazin-1-yl, oder durch Hydroxyniederalkyl, z.B. in 5-Hydroxymethyl-furan-2-yl-carbonyl, und C₆-C₁₂-Aryl, z.B. Phenyl, Naphthyl, wie 1- oder 2-Naphthyl, Indanyl, wie 1-oder 2-Indanyl, Indenyl, wie Inden-1-yl, oder Fluorenyl, wie Fluoren-9-yl, worin Aryl unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Heterocyclylniederalkyl, worin Heterocyclyl wie oben definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinomethyl oder Thiomorpholinomethyl, Cyano und/oder Nitro ein- oder mehrfach, vorzugsweise einfach, substituiert ist, insbesondere Phenyl, welches durch einen der genannten Reste in p-Stellung substituiert ist, ausgewählt sind, Niederalkoxycarbonyl, z.B. Methoxy-, Ethoxy- oder tert-Niederalkoxycarbonyl, wie tert-Butoxycarbonyl, 2-Halogenniederalkoxycarbonyl, z.B. 2-Chlor-, 2-Brom-, 2-Iod- oder 2,2,2-Trichlorethoxycarbonyl; Arylniederalkoxycarbonyl, z.B. Arylmethoxy-carbonyl, worin Aryl 6 bis 14 Kohlenstoffatome hat und unsubstituiert unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Heterocyclylniederalkyl, worin Heterocyclyl wie oben als Substituent für Niederalkanoyl definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinomethyl oder Thiomorpholinomethyl, Cyano und/oder Nitro ein- oder mehrfach, vorzugsweise einfach, substituiert ist, insbesondere Phenyl, 1- oder 2-Naphthyl, Fluorenyl oder durch Niederalkyl, z.B. Methyl oder tert-Butyl, Niederalkoxy, z.B. Methoxy, Ethoxy oder tert-Butoxy, Hydroxy, Halogen, z.B. Fluor, Chlor oder Brom, und/oder Nitro mono- oder mehrfach substituiertes Phenyl ist, z.B. Phenylniederalkoxycarbonyl, wie Benzyloxycarbonyl, 4-Methoxybenzyloxycarbonyl, 4-Nitrobenzyloxycarbonyl, Diphenylniederalkoxycarbonyl, wie Diphenylmethoxycarbonyl, Di-(4-methoxyphenyl)-methoxycarbonyl, Trityloxycarbonyl, oder Fluorenylniederalkoxycarbonyl, wie 9-Fluorenylmethoxycarbonyl; oder ferner Heterocyclylniederalkoxycarbonyl, worin Heterocyclyl wie oben als Substituent von Niederalkanoyl definiert ist, z.B. Furan-2-ylmethoxycarbonyl oder Pyridin-2-, -3- oder -4-ylmethoxycarbonyl, oder das über die α-Carbonylgruppe gebundene Radikal einer Aminosäure ausgewählt aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure, 5-Aminohexansäure, Valin, Norvalin, Leucin, Isoleucin, Norleucin, Serin, Homoserin, Threonin, Methionin, Cystein, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure, besonders bevorzugt der Rest einer aliphatischen Aminosäure ausgewählt aus Alanin, Valin, Norvalin, Leucin, 3-Aminopropionsäure, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure, 5-Aminohexansäure und Isoleucin oder einer Aminosäure ausgewählt aus Glycin, Asparagin, Glutamin, Methionin, Lysin und Phenylalanin, wobei (ausser in Fällen, wo kein asymmetrisches Kohlenstoffatom vorliegt, z.B. bei Glycin) jede der genannten Aminosäuren in der D-, L- oder (D,L)-, vorzugsweise in der L-Form vorliegen kann,
eine α-Aminogruppe, falls vorhanden, unsubstituiert oder ein- oder zweifach N-alkyliert durch Niederalkyl, wie Methyl, n-Propyl oder n-Butyl, durch Aminoniederalkyl, wie 3-Aminopropyl, durch Phenyl- oder Naphthylaminoniederalkyl, wie 3-Phenylaminopropyl, durch Phenylniederalkyl, wie Benzyl, durch Diphenylmethyl, durch Trityl, und/oder durch Heterocyclylniederalkyl, worin Heterocylcyl wie oben für substituiertes Niederalkanoyl R₁ definiert ist, insbesondere durch Heterocyclylmethyl, z.B. Furanylniederalkyl, wie 2-Furylmethyl, Thienylniederalkyl, wie 2-Thienylmethyl, Imidazolylniederalkyl, wie Imidazol4-ylmethyl, oder 2-, 3- oder 4-Pyridylniederalkyl, wie 2-, 3-oder 4-Pyridylmethyl, und/oder N-acyliert, z. B. durch die oben bei der Definition von R₁ genannten unsubstituierten oder substituierten Niederalkanoylreste, vor allem durch Acetyl, Propionyl, Pivaloyl, Heterocyclylniederalkanoyl, wie oben für substituiertes Niederalkanoyl R₁ definiert, z.B. Furan-2-ylcarbonyl, 5-Hydroxymethyl-furan-2-ylcarbonyl, 2-, 3- oder 4-Pyridylcarbonyl, Morpholinocarbonyl, Thiomorpholinocarbonyl, Indolylacetyl oder Benzofuranylacetyl, Arylniederalkanoyl, wie Benzoyl oder Phenylacetyl, Niederalkoxycarbonyl, wie tert-Butoxycarbonyl, oder Arylniederalkoxycarbonyl, wie oben definiert, z.B. Phenylniederalkoxycarbonyl, wie Benzyloxycarbonyl;
eine Carboxygruppe der Seitenkette frei, als Niederalkylestergruppe, wie Methoxycarbonyl oder tert-Butoxycarbonyl, als Arylestergruppe oder als Arylniederalkylestergruppe, worin Aryl Phenyl, 4-Nitrophenyl, Naphthyl, Fluorenyl oder Biphenylyl bedeutet, z. B. als 4-Nitrophenyloxycarbonyl, Benzyloxycarbonyl oder 9-Fluorenylmethoxycarbonyl, als Carbamoyl-, als Niederalkylcarbamoyl-, wie Methylcarbamoyl-, als Diniederalkylcarbamoyl-, wie Dimethylcarbamoyl-, als Mono- oder Di-(hydroxyniederalkyl)carbamoyl-, wie Hydroxymethylcarbamoyl- oder Di-(hydroxymethyl)-carbamoyl-, oder als Mono- oder Di-(carboxyniederalkyl)carbamoyl-, wie Carboxymethylcarbamoyl-oder Di-(carboxymethyl)carbamoylgruppe vorliegt,
eine Aminogruppe der Seitenkette, die sich nicht in α-Stellung befindet, frei, als Mono-oder Diniederalkylamino, wie n-Butylamino oder Dimethylamino, als Niederalkanoylamino, wie Acetylamino oder Pivaloylamino, als Aminoniederalkanoylamino, wie 3-Amino-3,3-dimethylpropionylamino, als Arylniederalkanoylamino, worin Aryl 6 - 14 Kohlenstoffatome hat, z.B. Phenyl, Naphthyl oder Fluorenyl bedeutet, und unsubstituiert oder durch Niederalkyl, Hydroxy, Niederalkoxy, Carboxy, Carbamoyl oder Sulfamoyl substituiert ist, wie 4-Hydroxyphenylbutyryl, als Niederalkoxycarbonylamino, wie tert-Butoxycarbonylamino, als Arylmethoxycarbonylamino, worin Aryl 6 - 14 Kohlenstoffatome hat, wie als Benzyloxycarbonylamino oder 9-Fluorenylmethoxycarbonylamino, als Piperidyl-1-carbonyl, Morpholinocarbonyl, Thiomorpholinocarbonyl oder als S,S-Dioxothiomorpholinocarbonyl vorliegt, und/oder
eine Hydroxygruppe der Seitenkette frei, als Niederalkoxy-, wie Methoxy-oder tert-Butoxy-, als Phenylniederalkoxy-, wie Benzyloxy-, als Niederalkanoyloxy-, wie Acetoxy-, oder als Niederalkyloxycarbonyloxygruppe, z. B. tert-Butoxycarbonyloxygruppe, vorliegt, bedeutet, und Salze dieser Verbindungen, insbesondere pharmazeutisch verwendbare Salze davon. Ferner können auch die Verbindungen der Formel I, und Salze davon, bevorzugt sein, worin R₁ Aminocarbonyl oder einen Aminocarbonylrest bedeutet, worin die Aminogruppe 1 bis 2 Substituenten trägt, die unabhängig voneinander aus unsubstituiertem oder substituiertem Niederalkyl, dessen Substituenten aus Hydroxy, Niederalkoxy, Phenoxy, Naphthoxy, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo und C₆-C₁₂-Aryl, z.B. Phenyl, Naphthyl, wie 1- oder 2-Naphthyl, Indanyl, wie 1-oder 2-Indanyl, Indenyl, wie Inden-1-yl, oder Fluorenyl, wie Fluoren-9-yl, wobei Aryl unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Cyano und/oder Nitro ein oder mehrfach, vorzugsweise einfach, substituiert ist, insbesondere Phenyl, welches durch einen der genannten Reste in p-Stellung substituiert ist; insbesondere unsubstituiertem Niederalkyl, wie Methyl oder Ethyl; ausgewählt sind; und Aryl, welches 6 bis 14 Kohlenstoffatome hat und unsubstituiert oder durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Heterocyclylniederalkyl, worin Heterocyclyl wie oben als Substituent für Niederalkanoyl definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoylpiperazin-1-ylmethyl, wie 4-Acetyl-piperazin-1-ylmethyl, Morpholinomethyl oder Thiomorpholinomethyl, Cyano und/oder Nitro ein oder mehrfach, vorzugsweise einfach, substituiert ist, vorzugsweise entsprechend substituiertes Phenyl oder 1- oder 2-Naphthyl ausgewählt sind, wobei nicht mehr als einer der Substituenten des Aminocarbonylrestes Aryl bedeutet; insbesondere Aminocarbonyl, Mono- oder Diniederalkylaminocarbonyl, wie N-Methyl-, N-Ethyl-, N,N-Dimethyl- oder N,N-Diethylaminocarbonyl, oder Phenylniederalkylaminocarbonyl, worin Phenyl unsubstituiert oder durch die bei der Definition von Aryl genannten Reste substituiert ist, z.B. durch Niederalkyl, z.B. Methyl, Halogenniederalkyl, wie Chlor- oder Brommethyl oder Trifluormethyl, Halogen, z.B. Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl)- oder N-(4-Cyanobenzyl)-aminocarbonyl, besonders bevorzugt durch nur einen Rest am Stickstoff substituiertes Aminocarbonyl, z.B. N-Niederalkylaminocarbonyl, wie N-Methyl- oder N-Ethyl-aminocarbonyl, oder Phenylniederalkylaminocarbonyl, worin Phenyl unsubstituiert oder durch die bei der Definition von Aryl genannten Reste substituiert ist, z.B. durch Niederalkyl, wie Methyl, Halogenniederalkyl, wie Chlor- oder Brommethyl oder Trifluormethyl, Halogen, wie Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl)- oder N-(4-Cyanobenzyl)-aminocarbonyl.

Bevorzugt sind insbesondere Verbindungen der Formel I, worin R₁ unsubstituiertes oder substituiertes Niederalkanoyl, Niederalkenyol oder Niederakinoyl, worin die Substituenten aus einem oder mehreren Resten, vorzugsweise aus bis zu drei Resten, insbesondere aus einem Rest ausgewählt aus der Gruppe Hydroxy, Niederalkoxy, Phenoxy, Naphthoxy, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Carbamoyl, dessen Stickstoff Bestandteil eines 5- bis 7-gliedrigen heterocyclischen Ringes ist, der noch ein weiteres Heteroatom ausgewählt aus O, S, N und durch Niederalkyl, wie Methyl oder Ethyl, substituiertes N enthalten kann, z. B. Pyrrolidinocarbonyl, Morpholinocarbonyl, Thiomorpholinocarbonyl, Piperidin-1-ylcarbonyl, Piperazin-1-ylcarbonyl oder 4-Niederalkyl-piperazin-1-ylcarbonyl, wie 4-Methylpiperazin-1-ylcarbonyl, Cyano, Oxo, Cycloalkyl, z.B. C₃-C₈-Cycloalkyl, wie Cyclopropyl, Cyclobutyl, Cyclopentyl oder Cyclohexyl, Bicyloalkyl, z.B. C₆-C₁₂-Bicycloalkyl, wie Decahydronaphth-2-yl, endo- oder exo-2-Norbornyl, Bicyclo[2.2.2]oct-2-yl oder Bicyclo[3.3.1.]non-9-yl, Tricycloalkyl, z.B. C₉-C₁₄-Tricycloalkyl, wie 1-oder 2-Adamantyl, Cycloalkenyl, z.B. C₄-C₈-Cycloalkenyl, wie 1-Cyclohexenyl oder 1,4-Cyclohexadienyl, Bicycloalkenyl z.B. 5-Norbornen-2-yl oder Bicyclo[2.2.2]octen-2-yl, Heterocyclyl, welches vorzugsweise einen als solchen oder in einfach benz-, cyclopenta-, cyclohexa- oder cyclohepta-annellierter Form vorliegenden gesättigten, teilweise gesättigten oder ungesättigten Ring, der 5 - 7 Ringatome enthält und bis zu vier Heteroatome ausgewählt aus Stickstoff, Schwefel und/oder Sauerstoff enthält, vorzugsweise 1 oder 2 der genannten Heteroatome, und unsubstituiert oder, insbesondere durch Niederalkyl, Niederalkanoyl, Hydroxy, Niederalkoxy, Phenylniederalkoxy, wie Benzyloxy, Hydroxyniederalkyl, wie Hydroxymethyl, Halogen, Cyano und/oder Trifluormethyl, substituiert sein kann, bedeutet, z. B. Pyrrolyl, 2,5-Dihydropyrrolyl, Furanyl, Thienyl, Tetrahydrofuranyl, Cyclohepta[b]pyrrolyl, Pyrrolidinyl, Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Chinolyl, Isochinolyl, Benzimidazolyl, Benzofuranyl, Pyridyl, Pyrimidinyl, Piperidinyl, Piperazin-1-yl, Morpholino, Thiomorpholino, S,S-Dioxothiomorpholino, 1,2-Dihydro- oder 1,2,3,4-Tetrahydrochinolyl, oder 1,2-Dihydro- oder 1,2,3,4-Tetrahydroisochinolyl, wobei die genannten Reste unsubstituiert oder wie oben substituiert sind, insbesondere durch Niederalkyl, z. B. in 4-Niederalkyl-piperazin-1-yl, wie 4-Methyl- oder 4-Ethyl-piperazin-1-yl, durch Niederalkanoyl, z. B. in 4-Niederalkanoyl-piperazin-1-yl, wie 4-Acetyl-piperazin-1-yl, oder durch Hydroxyniederalkyl, z.B. in 5-Hydroxymethyl-furan-2-ylcarbonyl, und C₆-C₁₂-Aryl, z.B. Phenyl, Naphthyl, wie 1- oder 2-Naphthyl, Indanyl, wie 1-oder 2-Indanyl, Indenyl, wie Inden-1-yl, oder Fluorenyl, wie Fluoren-9-yl, worin Aryl unsubstituiert oder beispielsweise durch Niederalkyl, z.B. Methyl, Halogenniederalkyl, wie Chlor-oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Heterocyclylniederalkyl, worin Heterocyclyl wie oben definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinomethyl oder Thiomorpholinomethyl, Cyano und/oder Nitro ein- oder mehrfach, vorzugsweise einfach, substituiert ist, insbesondere Phenyl, welches durch einen der genannten Reste in p-Stellung substituiert ist, ausgewählt sind, Niederalkoxycarbonyl, z.B. Methoxy-, Ethoxy- oder tert-Niederalkoxycarbonyl, wie tert-Butoxycarbonyl, 2-Halogenniederalkoxycarbonyl, z.B. 2-Chlor-, 2-Brom-, 2-Iod- oder 2,2,2-Trichlorethoxycarbonyl; Arylniederalkoxycarbonyl, z.B. Arylmethoxy-carbonyl, worin Aryl 6 bis 14 Kohlenstoffatome hat und unsubstituiert unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Heterocyclylniederalkyl, worin Heterocyclyl wie oben als Substituent für Niederalkanoyl definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoylpiperazin-1-ylmethyl, wie 4-Acetyl-piperazin-1-ylmethyl, Morpholinomethyl oder Thiomorpholinomethyl, Cyano und/oder Nitro ein- oder mehrfach, vorzugsweise einfach, substituiert ist, insbesondere Phenyl, 1- oder 2-Naphthyl, Fluorenyl oder durch Niederalkyl, z.B. Methyl oder tert-Butyl, Niederalkoxy, z.B. Methoxy, Ethoxy oder tert-Butoxy, Hydroxy, Halogen, z.B. Fluor, Chlor oder Brom, und/oder Nitro mono- oder mehrfach substituiertes Phenyl ist, z.B. Phenylniederalkoxycarbonyl, wie Benzyloxycarbonyl, 4-Methoxybenzyloxycarbonyl, 4-Nitrobenzyloxycarbonyl, Diphenylniederalkoxycarbonyl, wie Diphenylmethoxycarbonyl, Di-(4-methoxyphenyl)-methoxycarbonyl, Trityloxycarbonyl, oder Fluorenylniederalkoxycarbonyl, wie 9-Fluorenylmethoxycarbonyl; oder ferner Heterocyclylniederalkoxycarbonyl, worin Heterocyclyl wie oben als Substituent von Niederalkanoyl definiert ist, z.B. Furan-2-ylmethoxycarbonyl oder Pyridin-2-, -3-oder 4-ylmethoxycarbonyl, oder das über die α-Carbonylgruppe gebundene Radikal einer Aminosäure ausgewählt aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure, 5-Aminohexansäure, Valin, Norvalin, Leucin, Isoleucin, Norleucin, Serin, Homoserin, Threonin, Methionin, Cystein, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure, besonders bevorzugt der Rest einer aliphatischen Aminosäure ausgewählt aus Alanin, Valin, Norvalin, Leucin, 3-Aminopropionsäure, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure, 5-Aminohexansäure und Isoleucin oder einer Aminosäure ausgewählt aus Glycin, Asparagin, Glutamin, Methionin, Lysin und Phenylalanin, wobei (ausser in Fällen, wo kein asymmetrisches Kohlenstoffatom vorliegt, z.B. bei Glycin) jede der genannten Aminosäuren in der D-, L- oder (D,L)-, vorzugsweise in der L-Form vorliegen kann,
eine α-Aminogruppe, falls vorhanden, unsubstituiert oder ein- oder zweifach N-alkyliert durch Niederalkyl, wie Methyl, n-Propyl oder n-Butyl, durch Aminoniederalkyl, wie 3-Aminopropyl, durch Phenyl- oder Naphthylaminoniederalkyl, wie 3-Phenylaminopropyl, durch Phenylniederalkyl, wie Benzyl, durch Diphenylmethyl, durch Trityl, und/oder durch Heterocyclylniederalkyl, worin Heterocylcyl wie oben für substituiertes Niederalkanoyl R₁ definiert ist, insbesondere durch Heterocyclylmethyl, z.B. Furanylniederalkyl, wie 2-Furylmethyl, Thienylniederalkyl, wie 2-Thienylmethyl, oder 2-, 3- oder 4-Pyridylniederalkyl, wie 2-, 3- oder 4-Pyridylmethyl, und/oder N-acyliert, z. B. durch die oben bei der Definition von R₁ genannten unsubstituierten oder substituierten Niederalkanoylreste, vor allem durch Acetyl, Propionyl, Pivaloyl, Heterocyclylniederalkanoyl, wie oben für substituiertes Niederalkanoyl R₁ definiert, z.B. Furan-2-ylcarbonyl, 5-Hydroxymethyl-furan-2-ylcarbonyl, 2-, 3- oder 4-Pyridylcarbonyl, Morpholinocarbonyl, Thiomorpholinocarbonyl, Indolylacetyl oder Benzofuranylacetyl, Arylniederalkanoyl, wie Benzoyl oder Phenylacetyl, Niederalkoxycarbonyl, wie tert-Butoxycarbonyl, oder Arylniederalkoxycarbonyl, wie oben definiert, z.B. Phenylniederalkoxycarbonyl, wie Benzyloxycarbonyl;
eine Carboxygruppe der Seitenkette frei, als Niederalkylestergruppe, wie Methoxycarbonyl oder tert-Butoxycarbonyl, als Arylestergruppe oder als Arylniederalkylestergruppe, worin Aryl Phenyl, 4-Nitrophenyl, Naphthyl, Fluorenyl oder Biphenylyl bedeutet, z. B. als 4-Nitrophenyloxycarbonyl, Benzyloxycarbonyl oder 9-Fluorenylmethoxycarbonyl, als Carbamoyl-, als Niederalkylcarbamoyl-, wie Methylcarbamoyl-, als Diniederalkylcarbamoyl-, wie Dimethylcarbamoyl-, als Mono- oder Di-(hydroxyniederalkyl)carbamoyl-, wie Hydroxymethylcarbamoyl- oder Di-(hydroxymethyl)-carbamoyl-, oder als Mono- oder Di-(carboxyniederalkyl)carbamoyl-, wie Carboxymethylcarbamoyl-oder Di-(carboxymethyl)carbamoylgruppe vorliegt,
eine Aminogruppe der Seitenkette, die sich nicht in α-Stellung befindet, frei, als Mono-oder Diniederalkylamino, wie n-Butylamino oder Dimethylamino, als Niederalkanoylamino, wie Acetylamino oder Pivaloylamino, als Aminoniederalkanoylamino, wie 3-Amino-3,3-dimethylpropionylamino, als Arylniederalkanoylamino, worin Aryl 6 - 14 Kohlenstoffatome hat, z.B. Phenyl, Naphthyl oder Fluorenyl bedeutet, und unsubstituiert oder durch Niederalkyl, Hydroxy, Niederalkoxy, Carboxy, Carbamoyl oder Sulfamoyl substituiert ist, wie 4-Hydroxyphenylbutyryl, als Niederalkoxycarbonylamino, wie tert-Butoxycarbonylamino, als Arylmethoxycarbonylamino, worin Aryl 6 - 14 Kohlenstoffatome hat, wie als Benzyloxycarbonylamino oder 9-Fluorenylmethoxycarbonylamino, als Piperidyl-1-carbonyl, Morpholinocarbonyl, Thiomorpholinocarbonyl oder als S,S-Dioxothiomorpholinocarbonyl vorliegt, und/oder
eine Hydroxygruppe der Seitenkette frei, als Niederalkoxy-, wie Methoxy- oder tert-Butoxy-, als Phenylniederalkoxy-, wie Benzyloxy-, als Niederalkanoyloxy-, wie Acetoxy-, oder als Niederalkyloxycarbonyloxygruppe, z. B. tert-Butoxycarbonyloxygruppe, vorliegt, bedeutet, und Salze dieser Verbindungen, insbesondere pharmazeutisch verwendbare Salze davon. Ferner können auch die Verbindungen der Formel I, und Salze davon, bevorzugt sein, worin R₁ Aminocarbonyl oder einen Aminocarbonylrest bedeutet, worin die Aminogruppe 1 bis 2 Substituenten trägt, die unabhängig voneinander aus unsubstituiertem oder substituiertem Niederalkyl, dessen Substituenten aus Hydroxy, Niederalkoxy, Phenoxy, Naphthoxy, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo und C₆-C₁₂-Aryl, z.B. Phenyl, Naphthyl, wie 1- oder 2-Naphthyl, Indanyl, wie 1-oder 2-Indanyl, Indenyl, wie Inden-1-yl, oder Fluorenyl, wie Fluoren-9-yl, wobei Aryl unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Cyano und/oder Nitro ein oder mehrfach, vorzugsweise einfach, substituiert ist, insbesondere Phenyl, welches durch einen der genannten Reste in p-Stellung substituiert ist; insbesondere unsubstituiertem Niederalkyl, wie Methyl oder Ethyl; ausgewählt sind; und aus Aryl, welches 6 bis 14 Kohlenstoffatome hat und unsubstituiert oder durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono-oder Di-hydroxyniederalkylcarbamoyl, Haloniederalkyl, wie Trifluormethyl, Heterocyclylniederalkyl, worin Heterocyclyl wie oben als Substituent für Niederalkanoyl definiert ist, insbesondere Heterocyclylmethyl, worin Heterocyclyl über ein Ringstickstoffatom gebunden ist, z.B. Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoylpiperazin-1-ylmethyl, wie 4-Acetyl-piperazin-1-ylmethyl, Morpholinomethyl oder Thiomorpholinomethyl, Cyano und/oder Nitro ein oder mehrfach, vorzugsweise einfach, substituiert ist, vorzugsweise entsprechend substituiertes Phenyl oder 1- oder 2-Naphthyl ausgewählt sind, wobei nicht mehr als einer der Substituenten des Aminocarbonylrestes Aryl bedeutet; insbesondere Aminocarbonyl, Mono- oder Diniederalkylaminocarbonyl, wie N-Methyl-, N-Ethyl-, N,N-Dimethyl- oder N,N-Diethylaminocarbonyl, oder Phenylniederalkylaminocarbonyl, worin Phenyl unsubstituiert oder durch die bei der Definition von Aryl genannten Reste substituiert ist, z.B. durch Niederalkyl, z.B. Methyl, Halogenniederalkyl, wie Chlor- oder Brommethyl oder Trifluormethyl, Halogen, z.B. Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl)- oder N-(4-Cyanobenzyl)-aminocarbonyl, besonders bevorzugt durch nur einen Rest am Stickstoff substituiertes Aminocarbonyl, z.B. N-Niederalkylaminocarbonyl, wie N-Methyl- oder N-Ethyl-aminocarbonyl, oder Phenylniederalkylaminocarbonyl, worin Phenyl unsubstituiert oder durch die bei der Definition von Aryl genannten Reste substituiert ist, z.B. durch Niederalkyl, wie Methyl, Halogenniederalkyl, wie Chlor- oder Brommethyl oder Trifluormethyl, Halogen, wie Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl)- oder N-(4-Cyanobenzyl)-aminocarbonyl.

Stärker bevorzugt sind Verbindungen der Formel I, worin R₁ Octanoyl, Decanoyl, Dodecanoyl, Palmitoyl, unsubstituiertes oder substituiertes Niederalkanoyl, worin die Substituenten aus einem oder bis zu drei Resten, insbesondere aus einem Rest oder ferner zwei Resten ausgewählt aus der Gruppe Hydroxy, Niederalkoxy, Niederalkoxyniederalkoxy, Niederalkoxyniederalkoxy-niederalkoxy, Phenoxy, Naphthoxy, Phenylniederalkoxy, 2-Halogenniederalkanoyl, wie 2-Chloracetyl, Amino-, Niederalkylamino- oder Diniederalkylamino-niederalkoxy-2-niederalkanoyl, wie Dimethylamino-niederalkoxyacetyl, Amino-, Niederalkylamino- oder Diniederalkylamino-niederalkoxyniederalkoxy-2-niederalkanoyl, wie Dimethylamino-(2-niederalkoxyethoxy)acetyl, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo, C₃-C₈-Cycloalkyl, wie Cyclopropyl, Cyclobutyl, Cyclopentyl oder Cyclohexyl, C₄-C₈-Cycloalkenyl, wie 1-Cyclohexenyl oder 1,4-Cyclohexadienyl; Pyrrolyl, 2,5-Dihydropyrrolyl, Furanyl, Thienyl, Tetrahydrofuranyl, Pyrrolidinyl, Imidazolyl, Imidazolidinyl, Pyrazolyl, Pyrazolidinyl, Tetrahydro-oxazolyl, Tetrahydro-isoxazolyl, Tetrahydro-thiazolyl, Tetrahydro-isothiazolyl, Indolyl, Isoindolyl, Chinolyl, Isochinolyl, Benzimidazolyl, Benzofuranyl, Pyridyl, Pyrimidinyl, Piperidinyl, Piperazin-1-yl, Morpholino, Thiomorpholino, S,S-Dioxothiomorpholino, 1,2-Dihydro-oder 1,2,3,4-Tetrahydrochinolyl oder 1,2-Dihydro- oder 1,2,3,4-Tetrahydroisochinolyl, wobei die genannten heterocyclischen Reste unsubstituiert oder durch Niederalkyl, Niederalkanoyl, Hydroxy, Niederalkoxy, Phenylniederalkoxy, wie Benzyloxy, Hydroxyniederalkyl, wie Hydroxymethyl, Halogen, Cyano und/oder Trifluormethyl substituiert sind, insbesondere durch Niederalkyl, z. B. in 4-Niederalkyl-piperazin-1-yl, wie 4-Methyl-oder 4-Ethyl-piperazin-1-yl, durch Niederalkanoyl, z.B. in 4-Niederalkanoylpiperazin-1-yl, wie 4-Acetyl-piperazin-1-yl, oder durch Hydroxyniederalkyl, z.B. in 5-Hydroxymethyl-furan-2-ylcarbonyl, und Aryl ausgewählt aus Phenyl, Naphthyl, wie 1-oder 2-Naphthyl, Indanyl, wie 1- oder 2-Indanyl, Indenyl, wie Inden-1-yl, und Fluorenyl, wie Fluoren-9-yl, wobei diese Reste unsubstituiert oder beispielsweise durch Niederalkyl, z.B. Methyl, Halogenniederalkyl, wie Chlor- oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1-ylmethyl, Morpholinomethyl, Thiomorpholinomethyl, Cyano und/oder Nitro ein oder mehrfach, vorzugsweise einfach, substituiert sind, insbesondere Phenyl, welches durch einen der genannten Reste in p-Stellung substituiert ist, ausgewählt sind, oder das über die α-Carbonylgruppe gebundene Radikal einer Aminosäure ausgewählt aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure, 5-Aminohexansäure, Valin, Norvalin, Leucin, Isoleucin, Norleucin, Serin, Homoserin, Threonin, Methionin, Cystein, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure, besonders bevorzugt der Rest einer aliphatischen Aminosäure ausgewählt aus Alanin, Valin, Norvalin, Leucin, 3-Aminopropionsäure, 2-Aminobuttersäure und Isoleucin oder einer Aminosäure ausgewählt aus Glycin, Asparagin, Glutamin, Methionin, Lysin und Phenylalanin, ferner auch einer Aminosäure ausgewählt aus 3-Aminobuttersäure, 4-Aminobuttersäure, 3-Aminopentansäure, 4-Aminopentansäure, 5-Aminopentansäure, 3-Aminohexansäure, 4-Aminohexansäure und 5-Aminohexansäure, wobei in allen Fällen (ausser bei Fehlen eines asymmetrischen Kohlenstoffatomes, z.B. bei Glycin) jede der genannten Aminosäuren in der D-, L- oder (D,L)-, vorzugsweise in der L-Form vorliegen kann,
eine α-Aminogruppe, falls vorhanden, unsubstituiert oder ein- oder zweifach N-alkyliert durch Niederalkyl, wie Methyl, n-Propyl oder n-Butyl, durch Aminoniederalkyl, wie 3-Aminopropyl, durch Phenyl- oder Naphthylaminoniederalkyl, wie 3-Phenylaminopropyl, durch Phenylniederalkyl, wie Benzyl, durch Diphenylmethyl, durch Trityl, durch Furanylniederalkyl, wie 2-Furylmethyl, durch Thienylniederalkyl, wie 2-Thienylmethyl, Imidazolylniederalkyl, wie Imidazol-4-ylmethyl und/oder durch 2-, 3- oder 4-Pyridylniederalkyl, wie 2-, 3- oder 4-Pyridylmethyl, und/oder N-acyliert durch die oben bei der Definition von R₁ genannten unsubstituierten oder substituierten Niederalkanoylreste, vor allem durch Acetyl, Propionyl, Pivaloyl, Furan-2-ylcarbonyl, 5-Hydroxymethyl-furan 2-ylcarbonyl, 2-, 3- oder 4-Pyridylcarbonyl, Morpholinocarbonyl, Thiomorpholinocarbonyl, Indolylacetyl oder Benzofuranylacetyl, Phenylniederalkanoyl, wie Benzoyl oder Phenylacetyl, Niederalkoxycarbonyl, wie tert-Butoxycarbonyl, oder Phenylniederalkoxycarbonyl, wie Benzyloxycarbonyl, vorliegt, bedeutet, und pharmazeutisch verwendbare Salze davon. Ferner können auch die Verbindungen der Formel I und deren Salze stärker bevorzugt sein, worin R₁ Aminocarbonyl, Mono- oder Diniederalkylaminocarbonyl, wie N-Methyl-, N-Ethyl-, N,N-Dimethyl- oder N,N-Diethylaminocarbonyl, vorzugsweise Mononiederalkylaminocarbonyl, oder Phenylniederalkylaminocarbonyl, worin Phenyl unsubstituiert oder durch die bei der Definition von Aryl genannten Reste substituiert ist, z.B. durch Niederalkyl, z.B. Methyl, Halogenniederalkyl, wie Chlor- oder Brommethyl oder Trifluormethyl, Halogen, z.B. Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl)-oder N-(4-Cyanobenzyl)-aminocarbonyl, bedeutet.

Stärker bevorzugt sind insbesondere Verbindungen der Formel I, worin R₁ unsubstituiertes oder substituiertes Niederalkanoyl, worin die Substituenten aus einem oder bis zu drei Resten, insbesondere aus einem Rest ausgewählt aus der Gruppe Hydroxy, Niederalkoxy, Phenoxy, Naphthoxy, Niederalkanoyloxy, Phenylniederalkanoyloxy, wie Benzoyloxy oder Phenylacetyloxy, Halogen, wie Fluor, Chlor, Brom oder Iod, insbesondere Fluor oder Chlor, Carboxy, Niederalkoxycarbonyl, Phenylniederalkoxycarbonyl, wie Benzyloxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo, C₃-C₈-Cycloalkyl, wie Cyclopropyl, Cyclobutyl, Cyclopentyl oder Cyclohexyl, C₄-C₈-Cycloalkenyl, wie 1-Cyclohexenyl oder 1,4-Cyclohexadienyl; Pyrrolyl, 2,5-Dihydropyrrolyl, Furanyl, Thienyl, Tetrahydrofuranyl, Pyrrolidinyl, Imidazolyl, Imidazolidinyl, Pyrazolinyl, Pyrazolidinyl, Tetrahydro-oxazolyl, Tetrahydro-isoxazolyl, Tetrahydro-thiazolyl, Tetrahydro-isothiazolyl, Indolyl, Isoindolyl, Chinolyl, Isochinolyl, Benzimidazolyl, Benzofuranyl, Pyridyl, Pyrimidinyl, Piperidinyl, Piperazin-1-yl, Morpholino, Thiomorpholino, S,S-Dioxothiomorpholino, 1,2-Dihydro- oder 1,2,3,4-Tetrahydrochinolyl oder 1,2-Dihydro-oder 1,2,3,4-Tetrahydroisochinolyl, wobei die genannten heterocyclischen Reste unsubstituiert oder durch Niederalkyl, Niederalkanoyl, Hydroxy, Niederalkoxy, Phenylniederalkoxy, wie Benzyloxy, Hydroxyniederalkyl, wie Hydroxymethyl, Halogen, Cyano und/oder Trifluormethyl substituiert sind, insbesondere durch Niederalkyl, z. B. in 4-Niederalkyl-piperazin-1-yl, wie 4-Methyl- oder 4-Ethyl-piperazin-1-yl, durch Niederalkanoyl, z.B. in 4-Niederalkanoyl-piperazin-1-yl, wie 4-Acetyl-piperazin-1-yl, oder durch Hydroxyniederalkyl, z.B. in 5-Hydroxymethyl-furan-2-ylcarbonyl, und Aryl ausgewählt aus Phenyl, Naphthyl, wie 1- oder 2-Naphthyl, Indanyl, wie 1- oder 2-Indanyl, Indenyl, wie Inden-1-yl, und Fluorenyl, wie Fluoren-9-yl, wobei diese Reste unsubstituiert oder beispielsweise durch Niederalkyl, z.B. Methyl, Halogenniederalkyl, wie Chlor-oder Brommethyl, Halogen, z.B. Fluor oder Chlor, Hydroxy, Niederalkoxy, wie Methoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Piperidinomethyl, Piperazin-1-ylmethyl, 4-Niederalkyl-piperazin-1-ylmethyl, wie 4-Methyl- oder 4-Ethyl-piperazin-1-ylmethyl, 4-Niederalkanoyl-piperazin-1-ylmethyl, wie 4-Acetyl-piperazin-1 -ylmethyl, Morpholinomethyl, Thiomorpholinomethyl, Cyano und/oder Nitro ein oder mehrfach, vorzugsweise einfach, substituiert sind, insbesondere Phenyl, welches durch einen der genannten Reste in p-Stellung substituiert ist, ausgewählt sind, oder das über die α-Carbonylgruppe gebundene Radikal einer Aminosäure ausgewählt aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure, 5-Aminohexansäure, Valin, Norvalin, Leucin, Isoleucin, Norleucin, Serin, Homoserin, Threonin, Methionin, Cystein, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure, besonders bevorzugt der Rest einer aliphatischen Aminosäure ausgewählt aus Alanin, Valin, Norvalin, Leucin, 3-Aminopropionsäure, 2-Aminobuttersäure und Isoleucin oder einer Aminosäure ausgewählt aus Glycin, Asparagin, Glutamin, Methionin, Lysin und Phenylalanin, ferner auch einer Aminosäure ausgewählt aus 3-Aminobuttersäure, 4-Aminobuttersäure, 3-Aminopentansäure, 4-Aminopentansäure, 5-Aminopentansäure, 3-Aminohexansäure, 4-Aminohexansäure und 5-Aminohexansäure, wobei in allen Fallen (ausser bei Fehlen eines asymmetrischen Kohlenstoffatomes, z.B. bei Glycin) jede der genannten Aminosäuren in der D-, L- oder (D-,L)-, vorzugsweise in der L-Form vorliegen kann,
eine α-Aminogruppe, falls vorhanden, unsubstituiert oder ein- oder zweifach N-alkyliert durch Niederalkyl, wie Methyl, n-Propyl oder n-Butyl, durch Aminoniederalkyl, wie 3-Aminopropyl, durch Phenyl- oder Naphthylaminoniederalkyl, wie 3-Phenylaminopropyl, durch Phenylniederalkyl, wie Benzyl, durch Diphenylmethyl, durch Trityl, durch Furanylniederalkyl, wie 2-Furylmethyl, durch Thienylniederalkyl, wie 2-Thienylmethyl, und/oder durch 2-, 3- oder 4-Pyridylniederalkyl, wie 2-, 3- oder 4-Pyridylmethyl, und/oder N-acyliert durch die oben bei der Definition von R₁ genannten unsubstituierten oder substituierten Niederalkanoylreste, vor allem durch Acetyl, Propionyl, Pivaloyl, Furan-2-ylcarbonyl, 5-Hydroxymethyl-furan-2-ylcarbonyl, 2-, 3- oder 4-Pyridylcarbonyl, Morpholinocarbonyl, Thiomorpholinocarbonyl, Indolylacetyl oder Benzofuranylacetyl, Phenylniederalkanoyl, wie Benzoyl oder Phenylacetyl, Niederalkoxycarbonyl, wie tert-Butoxycarbonyl, oder Phenylniederalkoxycarbonyl, wie Benzyloxycarbonyl, vorliegt, bedeutet, und pharmazeutisch verwendbare Salze davon. Ferner können auch die Verbindungen der Formel I und deren Salze stärker bevorzugt sein, worin R₁ Aminocarbonyl, Mono- oder Diniederalkylaminocarbonyl, wie N-Methyl-, N-Ethyl-, N,N-Dimethyl- oder N,N-Diethylaminocarbonyl, vorzugsweise Mononiederalkylaminocarbonyl, oder Phenylniederalkylaminocarbonyl, worin Phenyl unsubstituiert oder durch die bei der Definition von Aryl genannten Reste substituiert ist, z.B. durch Niederalkyl, z.B. Methyl, Halogenniederalkyl, wie Chlor- oder Brommethyl oder Trifluormethyl, Halogen, z.B. Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl)- oder N-(4-Cyanobenzyl)-aminocarbonyl, bedeutet.

Noch stärker bevorzugt sind Verbindungen der Formel I, worin R₁ Octanoyl, Decanoyl, Dodecanoyl, Palmitoyl, Niederalkanoyl, wie Formyl, Acetyl, Propionyl, Butyryl, Methylpropionyl, n-Pentanoyl, Pivaloyl, Hexanoyl oder Heptanoyl, Hydroxyniederalkanoyl, z.B. β-Hydroxypropionyl, Niederalkoxyniederalkanoyl, z.B. Niederalkoxyacetyl oder Niederalkoxypropionyl, wie Methoxyacetyl, 3-Methoxypropionyl oder n-Butyloxyacetyl, Niederalkoxyniederylkoxyniederalkanoyl, wie 2-(2-Methoxyethoxy)-acetyl, Niederalkoxyniederalkoxyniederalkoxyniederalkanoyl, wie 2-(2-(2-Methoxyethoxy)ethoxy)acetyl, Phenoxyniederalkanoyl, z.B. Phenoxyacetyl, Phenylniederalkoxyniederalkanoyl, wie Benzyloxyacetyl, 2-Halogenniederalkanoyl, wie 2-Chloracetyl, Amino-, Niederalkylamino- oder Diniederalkylamino-niederalkoxy-2-niederalkanoyl, wie Dimethylamino-niederalkoxyacetyl, Amino-, Niederalkylamino- oder Diniederalkylamino-niederalkoxyniederalkoxy-2-niederalkanoyl, wie Dimethylamino-(2-niederalkoxyethoxy)acetyl, Niederalkanoyloxyniederalkanoyl, z.B. Niederalkanoyloxyacetyl oder Niederalkanoyloxypropionyl, wie Acetoxyacetyl oder β-Acetoxypropionyl, Carboxyniederalkanoyl, wie Carboxyacetyl oder 3-Carboxypropionyl, Oxoniederalkanoyl, z.B. Acetoacetyl oder Propionylacetyl, 5-Hydroxymethyl-furan-2-ylcarbonyl, 2- oder 3-Pyrrolylcarbonyl, Furylcarbonyl, z.B. 2-Furylcarbonyl, Thienylcarbonyl, z.B. 2-Thienylcarbonyl, Pyridylniederalkanoyl, wie Pyridylcarbonyl, z.B. 2-, 3- oder 4-Pyridylcarbonyl, Pyridylacetyl, z.B. 2-Pyridylacetyl, oder Pyridylpropionyl, z.B. 3-(2-Pyridyl)-propionyl, Chinolylcarbonyl, wie Chinolin-2-ylcarbonyl, Isochinolylcarbonyl, wie Isochinolin-3-ylcarbonyl, 2-, 3- oder 5-Indolylcarbonyl, Pyrrolidinyl-(2- oder 3-)carbonyl, 2-, 3- oder 4-Piperidinylcarbonyl, 1,2,3,4-Tetrahydrochinolyl-2-, -3- oder-4-carbonyl, 1,2,3,4-Tetrahydroisochinolyl-1-, -3-oder 4-carbonyl, Imidazolylniederalkanoyl, wie Imidazolylcarbonyl, z.B. Imidazol-1-ylcarbonyl oder Imidazol-4-ylcarbonyl, Imidazolylacetyl, z.B. 4-Imidazolylacetyl, oder Imidazolylpropionyl, z.B. 3-(4-Imidazolyl)-propionyl, Pyrazolylniederalkanoyl, wie 1-Pyrazolylcarbonyl, Morpholinocarbonyl, Thiomorpholinocarbonyl, Morpholinoacetyl, Thiomorpholinoacetyl, 4-Niederalkyl-1-piperazinoacetyl, wie 4-Methyl-piperazinoacetyl, Indolylacetyl, Benzofuranylacetyl, Phenylniederalkanoyl, z.B. Benzoyl, Phenylacetyl oder 3-Phenylpropionyl, im Phenylrest unsubstituiert, mono- oder mehrfachsubstituiert durch Niederalkyl, z.B. Methyl, Haloniederalkyl, wie Chlor- oder Brommethyl, Halogen, z.B. Fluor oder Chlor, Hydroxy, Niederalkoxy, z.B. Methoxy, Piperidinomethyl, Piperazin-1-ylmethyl, 4-Niederalkyl-piperazin-1-ylmethyl, wie 4-Methyl- oder 4-Ethyl-piperazin-1-ylmethyl, Morpholinomethyl, Thiomorpholinomethyl, Cyano und/oder Nitro, z.B. 4-Chlormethyl-, 4-Brommethyl-, 4-Fluor-, 4-Chlor-, 4-Methoxy-, 4-Morpholinomethyl-, 4-Thiomorpholinomethyl-, 4-Cyano- oder 4-Nitrobenzoyl, 4-Methylphenylacetyl, 4-Methoxyphenylacetyl, 3-(p-Hydroxyphenyl)-propionyl oder 2-Niederalkoxy-2-phenylacetyl, wie (R)- oder (S)-2-Methoxy-2-phenylacetyl, oder das über die α-Carbonylgruppe gebundene Radikal einer aliphatischen Aminosäure ausgewählt aus Alanin, Valin, Norvalin, Leucin, 3-Aminopropionsäure, 2-Aminobuttersäure und Isoleucin oder einer Aminosäure ausgewählt aus Glycin, Asparagin, Glutamin, Methionin, Lysin und Phenylalanin, ferner auch einer Aminosäure ausgewählt aus 3-Aminobuttersäure, 4-Aminobuttersäure, 3-Aminopentansäure, 4-Aminopentansäure, 5-Aminopentansäure, 3-Aminohexansäure, 4-Aminohexansäure und 5-Aminohexansäure, wobei (ausser bei Fehlen asymmetrischer C-Atome, z.B. bei Glycin) jede der genannten Aminosäuren in der D-, L- oder (D,L)-, vorzugsweise in der L-Form vorliegen kann, eine α-Aminogruppe, falls vorhanden, unsubstituiert oder ein- oder zweifach N-alkyliert durch Niederalkyl, wie Methyl, n-Propyl oder n-Butyl, durch Phenylniederalkyl, wie Benzyl, durch Imidazolylniederalkyl, wie Imidazol-4-yl-methyl, und/oder durch 2-, 3- oder 4-Pyridylniederalkyl, wie 2-, 3- oder 4-Pyridylmethyl, und/oder N-acyliert durch Niederalkoxycarbonyl, wie tert-Butoxycarbonyl, oder durch Benzyloxycarbonyl, vorliegt, z.B. in Alanyl, N-Niederalkylalanyl, wie N-Methylalanyl, Phenylalanyl, Aminoacetyl (Glycyl), N-Niederalkylaminoacetyl oder N,N-Diniederalkylaminoacetyl, wie N-Methylaminoacetyl, N,N-Dimethylaminoacetyl oder N-Methyl-N-(n-butyl)aminoacetyl, N-Niederalkyl-N-phenylniederalkylaminoacetyl, wie N-Methyl-N-benzylaminoacetyl, N-Niederalkyl-N-pyridylniederalkylaminoacetyl, z.B. N-Methyl-N-[(2-, 3- oder 4-)pyridylmethyl]-aminoacetyl, wie N-Methyl-N-(2 oder 3-pyridylmethyl)aminoacetyl, N-(Imidazolylniederalkyl)-N-niederalkyl-aminoacetyl, wie N-(Imidazol-4-ylmethyl)-N-methylaminoacetyl, N-Phenylniederalkoxycarbonyl-N-niederalkylaminoacetyl, wie N-Benzyloxycarbonyl-N-methylaminoacetyl, 2-Aminobutyryl, 4-(N,N-Dimethylamino)butyryl, Valyl, Norvalyl, Leucyl, Isoleucyl, Methionyl, Lysyl, Glutamyl oder Asparagyl, worin die Aminosäurereste (ausser Glycin) vorzugsweise in der (L)-Form vorliegen, bedeutet, und pharmazeutisch verwendbare Salze davon. Ferner können auch die Verbindungen der Formel I und deren Salze stärker bevorzugt sein, worin R₁ Aminocarbonyl, Mono- oder Diniederalkylaminocarbonyl, wie N-Methyl-, N-Ethyl-, N,N-Dimethyl- oder N,N-Diethylaminocarbonyl, vorzugsweise Mononiederalkylaminocarbonyl, oder Phenylniederalkylaminocarbonyl, worin Phenyl unsubstituiert oder durch die bei der Definition von Aryl genannten Reste substituiert ist, z.B. durch Niederalkyl, z.B. Methyl, Halogenniederalkyl, wie Chlor- oder Brommethyl oder Trifluormethyl, Halogen, z.B. Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl) oder N-(4-Cyanobenzyl)-aminocarbonyl, bedeutet.

Noch stärker bevorzugt sind insbesondere Verbindungen der Formel I, worin R₁ Niederalkanoyl, wie Acetyl, Propionyl, Butyryl, Pivaloyl, Hexanoyl oder Heptanoyl, Hydroxyniederalkanoyl, z.B. β-Hydroxypropionyl, Niederalkoxyniederalkanoyl, z.B. Niederalkoxyacetyl oder Niederalkoxypropionyl, wie Methoxyacetyl oder β-Methoxypropionyl, Phenoxyniederalkanoyl, z.B. Phenoxyacetyl, Niederalkanoyloxyniederalkanoyl, z.B. Niederalkanoyloxyacetyl oder Niederalkanoyloxypropionyl, wie Acetoxyacetyl oder β-Acetoxypropionyl, Oxoniederalkanoyl, z.B. Acetoacetyl oder Propionylacetyl, 5-Hydroxymethyl-furan-2-ylcarbonyl, 2- oder 3-Pyrrolylcarbonyl, Furylcarbonyl, z.B. 2-Furylcarbonyl, Thienylcarbonyl, z.B. 2-Thienylcarbonyl, Pyridylcarbonyl, z.B. 2-, 3-oder 4-Pyridylcarbonyl, 2-, 3- oder 5-Indolylcarbonyl, Pyrrolidinyl-3-carbonyl, 2-, 3- oder 4-Piperidinylcarbonyl, 1,2,3,4-Tetrahydrochinolyl-2-, -3- oder -4-carbonyl, 1,2,3,4-Tetrahydroisochinolyl-1-, -3- oder 4-carbonyl, Imidazolylcarbonyl, wie Imidazol-1-ylcarbonyl, Morpholinocarbonyl, Thiomorpholinocarbonyl, Morpholinoacetyl, Thiomorpholinoacetyl, 4-Niederalkyl-1-piperazinoacetyl, wie 4-Methyl-piperazinoacetyl, Indolylacetyl, Benzofuranylacetyl, Phenylniederalkanoyl, z.B. Benzoyl, Phenylacetyl oder 3-Phenylpropionyl, im Phenylrest unsubstituiert, mono- oder mehrfachsubstituiert durch Niederalkyl, z.B. Methyl, Haloniederalkyl, wie Chlor- oder Brommethyl, Halogen, z.B. Fluor oder Chlor, Hydroxy, Niederalkoxy, z.B. Methoxy, Piperidinomethyl, Piperazin-1-yl-methyl, 4-Niederalkyl-piperazin-1-ylmethyl, wie 4-Methyl- oder 4-Ethyl-piperazin-1-yl-methyl, Morpholinomethyl, Thiomorpholinomethyl, Cyano und/oder Nitro, z.B. 4-Chlormethyl-, 4-Brommethyl-, 4-Fluor-, 4-Chlor-, 4-Methoxy-, 4-Morpholinomethyl-, 4-Thiomorpholinomethyl-, 4-Cyano- oder 4-Nitrobenzoyl, 4-Methylphenylacetyl, 4-Methoxyphenylacetyl oder 3-(p-Hydroxyphenyl)-propionyl, oder das über die α-Carbonylgruppe gebundene Radikal einer aliphatischen Aminosäure ausgewahlt aus Alanin, Valin, Norvalin, Leucin, 3-Aminopropionsäure, 2-Aminobuttersäure und Isoleucin oder einer Aminosäure ausgewählt aus Glycin, Asparagin, Glutamin, Methionin, Lysin und Phenylalanin, ferner auch einer Aminosäure ausgewählt aus 3-Aminobuttersäure, 4-Aminobuttersäure, 3-Aminopentansäure, 4-Aminopentansäure, 5-Aminopentansäure, 3-Aminohexansäure, 4-Aminohexansäure und 5-Aminohexansäure, wobei (ausser bei Fehlen asymmetrischer C-Atome, z.B. bei Glycin) jede der genannten Aminosäuren in der D-, L- oder (D,L)-, vorzugsweise in der L-Form vorliegen kann, eine α-Aminogruppe, falls vorhanden, unsubstituiert oder ein- oder zweifach N-alkyliert durch Niederalkyl, wie Methyl, n-Propyl oder n-Butyl, durch Phenylniederalkyl, wie Benzyl, und/oder durch 2-, 3- oder 4-Pyridylniederalkyl, wie 2-, 3- oder Pyridylmethyl, und/oder N-acyliert durch Niederalkoxycarbonyl, wie tert-Butoxycarbonyl, oder durch Benzyloxycarbonyl, vorliegt, z.B. in Alanyl, N-Niederalkylalanyl, wie N-Methylalanyl, Phenylalanyl, Aminoacetyl (Glycyl), N-Niederalkylaminoacetyl oder N,N-Diniederalkylaminoacetyl, wie N-Methylaminoacetyl, N,N-Dimethylaminoacetyl oder N-Methyl-N-(n-butyl)aminoacetyl, N-Niederalkyl-N-phenylniederalkylaminoacetyl, wie N-Methyl-N-benzylaminoacetyl, N-Niederalkyl-N-pyridylniederalkylaminoacetyl, z.B. N-Methyl-N-[(2-, 3- oder 4-)pyridylmethyl]-aminoacetyl, wie N-Methyl-N-(3-pyridylmethyl)aminoacetyl, N-Phenylniederalkoxycarbonyl-N-niederalkylaminoacetyl, wie N-Benzyloxycarbonyl-N-methylaminoacetyl, 2-Aminobutyryl, Valyl, Norvalyl, Leucyl, Isoleucyl, Methionyl, Lysyl, Glutamyl oder Asparagyl, worin die Aminosäurereste (ausser Glycin) vorzugsweise in der (L)-Form vorliegen, bedeutet, und pharmazeutisch verwendbare Salze davon. Ferner können auch die Verbindungen der Formel I und deren Salze stärker bevorzugt sein, worin R₁ Aminocarbonyl, Mono- oder Diniederalkylaminocarbonyl, wie N-Methyl-, N-Ethyl-, N,N-Dimethyl-oder N,N-Diethylaminocarbonyl, vorzugsweise Mononiederalkylaminocarbonyl, oder Phenylniederalkylaminocarbonyl, worin Phenyl unsubstituiert oder durch die bei der Definition von Aryl genannten Reste substituiert ist, z.B. durch Niederalkyl, z.B. Methyl, Halogenniederalkyl, wie Chlor- oder Brommethyl oder Trifluormethyl, Halogen, z.B. Fluor oder Chlor, Hydroxy, Niederalkoxy, wie Methoxy, Carboxy, und/oder Cyano, vorzugsweise durch bis zu drei unabhängig voneinander ausgewählte dieser Substituenten, insbesondere einen davon, z.B. in p-Stellung, wie in N-Benzyl-, N-(4-Fluorbenzyl)-, N-(4-Chlorbenzyl)-, N-(4-Trifluormethylbenzyl) oder N-(4-Cyanobenzyl)-aminocarbonyl, bedeutet.

Besonders bevorzugt sind Verbindungen der Formel I, worin R₁ Octanoyl, Decanoyl, Dodecanoyl, Palmitoyl, Niederalkanoyl, wie Acetyl, Propionyl, Butyryl, Methylpropionyl, n-Pentanoyl, Pivaloyl, Hexanoyl oder Heptanoyl, Niederalkoxyniederalkanoyl, wie Methoxyacetyl, 3-Methoxypropionyl oder 4-Butoxyacetyl, Niederalkoxyniederalkoxyniederalkanoyl, wie 2-(2-Methoxyethoxy)ethoxyacetyl, Niederalkoxyniederalkoxyniederalkanoyl, wie 2-(2-(2-Methoxyethoxy)ethoxy)acetyl, Phenoxyniederalkanoyl, wie Phenoxyacetyl, Phenylniederalkoxyniederalkanoyl, wie Benzyloxyacetyl, 2-Halogenniederalkanoyl, wie 2-Chloracetyl, Amino-, Niederalkylamino- oder Diniederalkylaminoniederalkoxy-2-niederalkanoyl, wie Dimethylamino-niederalkoxyacetyl, Amino-, Niederalkylamino-oder Diniederalkylamino-niederalkoxyniederalkoxy-2-niederalkanoyl, wie Dimethylamino-(2-niederalkoxyethoxy)acetyl, 2-Niederalkoxy-2-phenylacetyl, wie (R)-oder (S)-2-Methoxy-2-phenylacetyl, Furylcarbonyl, z.B. Furan-2-ylcarbonyl, Pyridylniederalkanoyl, z.B. Pyridylcarbonyl, wie 2-, 3- oder 4-Pyridylcarbonyl, Pyridylacetyl, wie 2-Pyridylacetyl, oder Pyridylpropionyl, wie 3-(Pyridin-2-yl)propionyl, Chinolylcarbonyl, wie Chinolin-2-ylcarbonyl, Isochinolylcarbonyl, wie Isochinolin-3-ylcarbonyl, Pyrrolidinyl-2-carbonyl, insbesondere L- oder D-Prolyl, Imidazolylniederalkanoyl, wie 4-Imidazolylcarbonyl, Imidazolylacetyl, z.B. 4-Imidazolylacetyl, oder Imidazolylpropionyl, wie 3-(4-Imidazolyl)propionyl, Pyrazolylniederalkanoyl, wie 1-Pyrazolylcarbonyl, Phenylniederalkanoyl, z.B. Benzoyl, Phenylacetyl oder 3-Phenylpropionyl, 4-Chlormethylbenzoyl, 4-Morpholinomethylbenzoyl, 4-Thiomorpholinomethylbenzoyl, Aminoacetyl, N-Niederalkylaminoacetyl oder N,N-Diniederalkylaminoacetyl, wie N-Methylaminoacetyl, N,N-Dimethylaminoacetyl oder N-Methyl-N-(n-butyl)aminoacetyl, N-Niederalkyl-N-phenylniederalkylaminoacetyl, wie N-Methyl-N-benzylaminoacetyl, N-Niederalkyl-N-benzyloxycarbonylaminoacetyl, wie N-Methyl-N-benzyloxyaminoacetyl, N-Imidazolylniederalkyl-N-niederalkylaminoacetyl, wie N-(Imidazol-4-ylmethyl)-N-methylaminoacetyl, N-Niederalkyl-N-pyridylniederalkylaminoacetyl, z.B. N-Methyl-N-[(2-, 3- oder 4-)pyridylmethyl]-aminoacetyl, wie N-Methyl-N-(2- oder 3-pyridylmethyl)aminoacetyl, oder 4-(N,N-Dimethylamino)butyryl bedeutet, und pharmazeutisch verwendbare Salze davon.

Besonders bevorzugt sind insbesondere Verbindungen der Formel I, worin R₁ Niederalkanoyl, wie Acetyl, Propionyl, Butyryl, Pivaloyl, Hexanoyl oder Heptanoyl, Furylcarbonyl, z.B. Furan-2-ylcarbonyl, Pyridylcarbonyl, z.B. 2-, 3- oder 4-Pyridylcarbonyl, Phenylniederalkanoyl, z.B. Benzoyl, Phenylacetyl oder 3-Phenylpropionyl, 4-Morpholinomethylbenzoyl, 4-Thiomorpholinomethylbenzoyl, Aminoacetyl, N-Niederalkylaminoacetyl, N,N-Diniederalkylaminoacetyl, wie N-Methylaminoacetyl, N,N-Dimethylaminoacetyl, N-Methyl-N-(n-butyl)aminoacetyl, N-Niederalkyl-N-phenylniederalkylaminoacetyl, wie N-Methyl-N-benzylaminoacetyl, oder N-Niederalkyl-N-pyridylniederalkylaminoacetyl, z.B. N-Methyl-N-[(2-, 3- oder 4-)pyridylmethyl]-aminoacetyl, wie N-Methyl-N-(3-pyridylmethyl)aminoacetyl, bedeutet, und pharmazeutisch verwendbare Salze davon.

Ganz besonders bevorzugt sind Verbindungen der Formel I, worin R₁ Niederalkanoyl, wie Acetyl, Niederalkoxyniederalkanoyl, wie Methoxyacetyl, Pyridylcarbonyl, wie Pyridin-2-ylcarbonyl, oder Furylcarbonyl, wie Furan-2-ylcarbonyl, bedeutet, und pharmazeutisch verwendbare Salze davon.

Ganz besonders bevorzugt sind insbesondere Verbindungen der Formel I, worin R₁ Niederalkanoyl, wie Acetyl, oder Furylcarbonyl, wie Furan-2-ylcarbonyl, bedeutet, und pharmazeutisch verwendbare Salze davon.

Ganz besonders bevorzugt sind auch Verbindungen der Formel I, worin R₁ Niederalkoxyniederalkanoyl, wie Methoxyacetyl, oder Pyridylcarbonyl, wie Pyridin-2-ylcarbonyl, bedeutet, und pharmazeutisch verwendbare Salze davon.

Sehr bevorzugt sind Verbindungen der Formel I, worin R₁ Niederalkanoyl bedeutet, insbesondere Acetyl, und pharmazeutisch verwendbare Salze davon.

Sehr bevorzugt sind die in den Beispielen genannten Verbindungen der Formel I und deren pharmazeutisch verwendbare Salze.

Die Verbindungen der Formel I und Salze von solchen Verbindungen mit mindestens einer salzbildenden Gruppe werden nach an sich bekannten Verfahren erhalten, z. B. indem man
a) eine Verbindung der Formel II, wie oben definiert, mit einer Carbonsäure der Formel III,

   R₁-OH (III)

   worin R₁ die genannten Bedeutungen hat, oder einem reaktionsfähigen Derivat davon umsetzt, wobei freie funktionelle Gruppen in den Ausgangsmaterialien der Formeln II und III, die nicht an der Reaktion teilnehmen sollen, erforderlichenfalls in geschützter Form vorliegen, und vorhandene Schutzgruppen abspaltet, oder
b) eine Aminoverbindung der Formel IV, 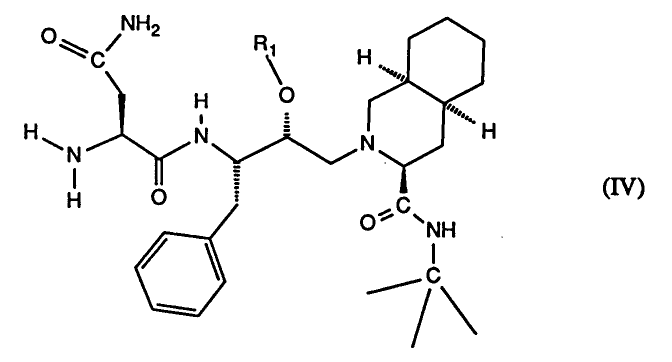 worin R₁ die genannten Bedeutungen hat, oder ein reaktionsfahiges Derivat davon, mit einer Carbonsäure der Formel V, 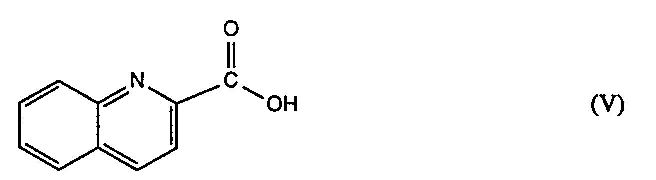 oder einem reaktionsfähigen Säurederivat davon, amidiert, wobei freie funktionelle Gruppen in den Ausgangsmaterialien der Formeln IV und V, die nicht an der Reaktion teilnehmen sollen, erforderlichenfalls in geschützter Form vorliegen, und vorhandene Schutzgruppen abspaltet, oder
c) eine Aminoverbindung der Formel VI, 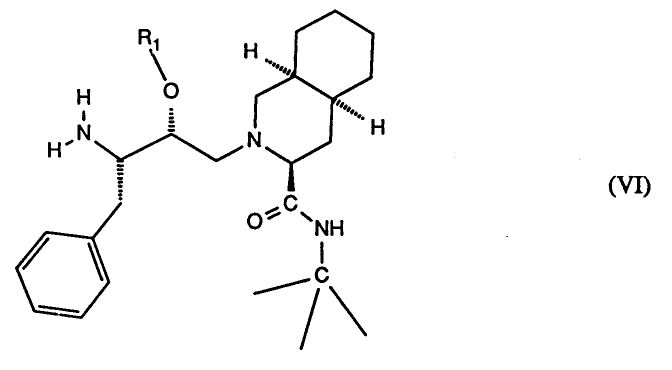 worin R₁ die genannten Bedeutungen hat, oder ein reaktionsfähiges Derivat davon, mit einer Carbonsäure der Formel VII, 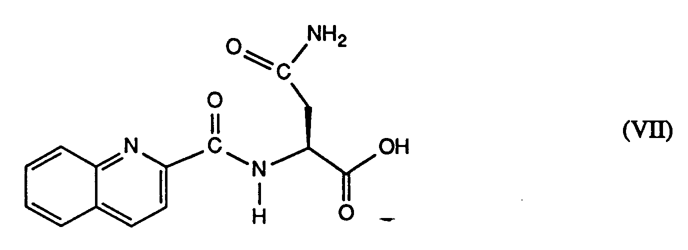 oder einem reaktionsfahigen Säurederivat davon, amidiert, wobei freie funktionelle Gruppen in den Ausgangsmaterialien der Formeln VI und VII, die nicht an der Reaktion teilnehmen sollen, erforderlichenfalls in geschützter Form vorliegen, und vorhandene Schutzgruppen abspaltet,
und gewünschtenfalls eine nach dem vorstehenden Verfahren erhältliche Verbindung der Formel I mit mindestens einer salzbildenden Gruppe in ihr Salz und/oder ein erhältliches Salz in die freie Verbindung oder in ein anderes Salz überführt und/oder gegebenenfalls erhältliche Isomerengemische von Verbindungen der Formel I auftrennt und/oder eine erfindungsgemässe Verbindung der Formel I in eine andere erfindungsgemässe Verbindung der Formel I umwandelt.

Die oben definierten Verfahren werden nachfolgend näher beschrieben:

### Verfahren a) Herstellung einer acylierten Verbindung

Die Herstellung eines Esters erfolgt beispielsweise in an sich bekannter Weise unter Verwendung einer Säure der Formel III, worin R₁ die genannten Bedeutungen ausser Aminocarbonyl oder dem Rest einer N-substituierten Carbaminsäure hat, oder eines reaktionsfähigen Derivates davon. Als reaktionsfähiges Derivat kommt beispielsweise eine Verbindung der Formel IIIa,

R₁-Z₁ (IIIa)

in Betracht, worin R₁ die zuletzt genannten Bedeutungen hat und Z₁ insbesondere reaktionsfahig aktiviertes Hydroxy bedeutet. Die freie Carbonsäure der Formel III kann beispielsweise durch starke Säuren, wie Halogenwasserstoff-, Schwefel-, Sulfon-, oder Carbonsäure oder saure Ionenaustauscher, z.B. durch Chlor-, Bromwasserstoff- oder Iodwasserstoffsäure, Schwefelsäure, eine gegebenenfalls, z.B. durch Halogen, substituierte Alkancarbonsäure oder durch eine Säure der Formel III, vorzugsweise mit einem Überschuss der Säure der Formel III, erforderlichenfalls unter Bindung von entstehendem Reaktionswasser durch wasserbindende Mittel, unter azeotroper Abdestillation des Reaktionswassers oder unter extraktiver Veresterung, durch Säureanhydride, insbesondere anorganische Säureanhydride, wie Carbonsäureanhydride, wie Niederalkancarbonsäureanhydride (ausser Ameisensäureanhydrid), z.B. Acetanhydrid, oder durch geeignete Aktivierungs-oder Kupplungsreagentien der nachstehend aufgeführten Art, insbesondere auch in situ, aktiviert werden. R₁-Z₁ kann auch für ein Carbonsäureazid (erhältlich beispielsweise durch Umsetzung eines entsprechenden Säureesters über das entsprechende Hydrazid und dessen Behandlung mit salpetriger Säure); für ein Carbonsäurehalogenid, insbesondere ein Säurechlorid oder -bromid, welches beispielsweise durch Umsetzung mit organischen Säurehalogeniden, insbesondere mit Oxalyldihalogeniden, wie Oxalyldichlorid, oder vor allem mit anorganischen Säurehalogeniden, z.B. mit Säurehalogeniden des Phosphors oder Schwefels, wie Phosphortrichlorid, Phosphortribromid, Phosphorpentachlorid, Phosphorpentabromid, Phosphoroxidchlorid, Phosphoroxidbromid, Thionylchlorid oder Thionylbromid, oder vor allem unter schonenden Bedingungen mit Tetraniederalkyl-α-halogenenaminen, wie Tetramethyl-α-halogen-enaminen, insbesondere 1-Chlor-N,N,2-trimethyl-1-propenamin (vorzugsweise durch Umsetzen in inerten Lösungsmitteln, insbesondere chlorierten Kohlenwasserstoffen, wie Methylenchlorid oder Chloroform, oder Ethern, wie Diethylether, Dioxan oder Tetrahydrofuran, bei bevorzugten Temperaturen zwischen - 78 und 50 °C, insbesondere bei -60 bis 30 °C, z.B. zwischen -10 ⁰C und Raumtemperatur (vgl. Devos, A., et al., J. C. S. Chem. Commun. 1979, 1180-81, und Haveaux, B., et al., Org. Synth. 59, 26 (1980)), wobei das erhaltene Säurehalogenid, z.B. das Säurechlorid der Formel IIIa, worin Z₁ Chlor bedeutet, auch direkt in situ weiterverarbeitet werden kann, beispielsweise durch Umsetzung mit der Verbindung der Formel II in Gegenwart von tertiären Stickstoffbasen, wie Pyridin und/oder 4-Dimethylaminopyridin (DMAP, welches vorzugsweise in katalytischen Mengen zugefügt wird), bei bevorzugten Temperaturen zwischen -20 und 50 °C, insbesondere zwischen 0 °C und Raumtemperatur) erhalten werden kann; für einen aktivierten Ester, wobei Z₁ insbesondere Cyanmethoxy, Nitrophenoxy, wie 4-Nitrophenoxy oder 2,4-Dinitrophenyoxy, oder Polyhalogenphenoxy, wie Pentachlorphenoxy, bedeutet; oder für ein symmetrisches oder vorzugsweise asymmetrisches Säureanhydrid stehen, welches beispielsweise durch Reaktion eines Salzes, z.B. eines Alkalimetallsalzes, wie des Natrium- oder Kaliumsalzes, einer Säure der Formel III oder ihres Reaktionspartners, vorzugsweise einer Niederalkancarbonsäure, wie Essigsäure, mit einem jeweils komplementaren Säurehalogenid, insbesondere im Falle der Reaktion mit einem Salz einer Carbonsäure der Formel III ein Carbonsäurehalogenid, z.B. -Chlorid, wie Acetylchlorid, und im Falle der Reaktion eines Carbonsäurehalogenides der Formel IIIa, worin Z₁ Halogen, z.B. Chlor oder Brom, bedeutet, mit einem Salz einer Niederalkancarbonsäure, insbesondere Natrium- oder Kaliumacetat, erhalten werden kann. Als Aktivierungs- und Kupplungsreagentien zur Aktivierung von Carbonsäuren der Formel III in situ können insbesondere Carbodiimide, z.B. N,N'-Di-C₁-C₄-alkyl- oder N,N'-Di-C₅-C₇-cycloalkyl-carbodiimid, wie Diisopropylcarbodiimid oder N,N'-Dicyclohexyl-carbodiimid, vorteilhaft unter Zusatz eines Aktivierungskatalysators, wie N-Hydroxysuccinimid oder gegebenenfalls, z.B. durch Halogen, C₁-C₇-Alkyl oder C₁-C₇-Alkoxy, substituiertes N-Hydroxy-benzotriazol oder N-Hydroxy-5-norbornen-2,3-dicarboxamid, C₁-C₄-Alkylhalogenformiat, z.B. Isobutylchlorformiat, geeignete Carbonylverbindungen, z.B. N,N-Carbonyldiimidazol, geeignete 1,2-Oxazoliumverbindungen, z.B. 2-Ethyl-5-phenyl-1,2-oxazolium-3'-sulfonat oder 2-tert-Butyl-5-methyl-isoxazolium-perchlorat, geeignete Acylaminoverbindungen, z.B. 2-Ethoxy-1-ethoxycarbonyl-1,2-dihydrochinolin, oder geeignete Phosphorylcyanamide bzw. -azide, z.B. Diethylphosphorylcyanamid oder Diphenylphosphorylazid, ferner Triphenylphosphin-disulfid oder 1-C₁-C₄-Alkyl-2-halogeno-pyridinium-halogenide, z.B. 1-Methyl-2-chlorpyridinium-iodid, eingesetzt werden.

Z₁ bedeutet bevorzugt Halogen, wie Chlor oder Brom, sowie Acyloxy, z.B. Niederalkanoyloxy, wie Acetyloxy.

Für den Spezialfall der Einführung eines über seine Carbonylgruppe an den bindenden Sauerstoff geknüpften Acylrestes eines Halbesters der Kohlensäure eignen sich insbesondere die Verbindungen der Formel IIIa, worin Z₁ Halogen, wie Chlor, bedeutet, welche z.B. hergestellt werden können durch Umsetzung der komplementären Alkohole, beispielsweise unsubstituierter oder substituierte Alkylalkohole, Arylniederalkylalkohole oder Heterocyclylniederalkylalkohole, worin die Reste wie bei der Definition von unsubstituiertem oder substituiertem Alkoxycarbonyl, Arylniederalkoxycarbonyl oder Heterocyclylniederalkoxycarbonyl R₅ definiert, mit Phosgen oder ferner Analogen davon, welche statt Chlor andere Halogenatome, insbesondere Brom, enthalten, vorzugsweise in Gegenwart von tertiären Stickstoffbasen, wie Pyridin oder Triethylamin, und in inerten Lösungsmitteln, z.B. chlorierten Kohlenwasserstoffen, wie Methylenchlorid oder Chloroform, Ethern, wie Diethylether, Tetrahydrofuran oder Dioxan, oder Carbonsäureamiden, wie Dimethylformamid. Auch entsprechende N-Carbonylazolide der Formel IIIa (Z₁= N-haltiger Heterocyclus, wie 1-Imidazolido) sind geeignet, welche beispielsweise durch Umsetzung mit den entsprechenden N,N'-carbonyldiazoliden, wie N,N-Carbonyldiimidazol, erhalten werden unter Bedingungen, wie gerade für Phosgen und Analoge mit anderen Halogenatomen beschrieben. Die Reaktion von Verbindungen der Formel II mit entsprechenden Verbindungen der Formel IIIa findet dann ebenfalls unter diesen Bedingungen statt (vgl. Staab, H. A., Angew. Chemie 74, 407 (1962)).

Für den Spezialfall der Einführung von Aminocarbonyl R₁ oder eines N-substituierten Carbaminsäurerestes als Acylgruppe R₁ eignet sich als aktiviertes Säurederivat insbesondere das entsprechende Isocyanat der Formel IIIb,

Q-N=C=O (IIIb)

worin Q eine Schutzgruppe, z.B. Trihalogenacetyl, wie Trifluor- oder Trichloracetyl, oder einen der oben bei der Definition von einem Aminocarbonylrest R₁, worin die Aminogruppe 1 bis 2 Substituenten trägt, genannten unsubstituierten oder substituierten Niederalkylreste oder Arylreste bedeutet, wobei man, wenn Q eine Aminoschutzgruppe bedeutet, nach der Umsetzung mit der Verbindung der Formel II die entsprechende Verbindung der Formel I, worin R₁ freies Aminocarbonyl bedeutet, durch Abspaltung einer Schutzgruppe Q erhalten kann, wie unten bei der Freisetzung von durch Acyl geschütztem Amino beschrieben, insbesondere durch saure Hydrolyse, oder, wenn Q einen der genannten substituierten oder unsubstituierten Niederalkyl- oder Arylreste bedeutet, eine entsprechende Verbindung der Formel I mit am Stickstoff monosubstituiertem Aminocarbonyl. Sowohl Aminocarbonyl als auch N-monosubstituiertes Aminocarbonyl kann in N-disubstituiertes Aminocarbonyl überführt werden, indem man alkyliert, wie unten bei den zusätzlichen Verfahrensmassnahmen beschrieben.

Die Reaktionen können unter an sich bekannten Reaktionsbedingungen, bei üblichen Temperaturen, in An- oder, insbesondere im Falle der Verwendung von Niederalkanoylanhydriden zur Aktivierung der Carbonsäure der Formel III, Abwesenheit von inerten Lösungs- oder Verdünnungsmitteln durchgeführt werden, beispielsweise in Säureamiden, z.B. Carbonsäuramiden, wie Dimethylformamid, Dimethylacetamid oder 1,3-Dimethyl-3,4,5,6-tetrahydro-2(1H)-pyrimidinon (DMPU), oder Amiden anorganischer Säuren, wie Hexamethylphosphorsäuretriamid, Ethern, z.B. cyclischen Ethern, wie Tetrahydrofuran oder Dioxan, oder acyclischen Ethern, wie Diethylether oder Ethylenglykoldimethylether, halogenierten Kohlenwasserstoffen, wie Haloniederalkanen, z.B. Methylenchlorid oder Chloroform, Ketonen, wie Aceton, Nitrilen, wie Acetonitril, Säureanhydriden, wie Acetanhydrid, Estern, wie Essigsäureethylester, Bisalkansulfinen, wie Dimethylsulfoxid, Stickstoffheterocyclen, wie Pyridin, oder Gemischen dieser Lösungsmittel, insbesondere in wasserfreien Lösungsmitteln oder Lösungsmittelgemischen, wobei die jeweils geeigneten Lösungsmittel für die oben genannten Reaktionen ausgewählt werden können, soweit sinnvoll und zweckmässig, unter Verwendung von Salzen der eingesetzten Verbindungen, insbesondere von Metallsalzen eingesetzter Cabonsäuren, wie den Alkali- oder Erdalkalimetallsalzen, z.B. Natrium- oder Kaliumsalzen, in Ab- oder Anwesenheit von Katalysatoren, Kondensationsmitteln oder neutralisierenden Agentien, je nach Art der Reaktion und/oder der Reaktionsteilnehmer unter atmosphärischem Druck oder in einem geschlossenen Gefäss, unter Normaldruck oder ferner unter erhöhtem Druck, z.B. bei dem Druck, der im Reaktionsgemisch unter den Reaktionsbedingungen in einem geschlossenen Rohr entsteht, und/oder in einer inerten Atmosphäre, z.B. unter einer Argon- oder Stickstoffatmosphäre. Bevorzugt sind Reaktionsbedingungen, die den in den Beispielen genannten analog sind. Auch kann das Acylierungsmittel, z.B. ein Carbonsäurehalogenid oder ein Carbonsäureanhydrid, selbst als Lösungsmittel dienen. Der Reaktionsablauf wird zweckmässig mittels üblicher analytischer Methoden, insbesondere mit Dünnschichtchromatographie, verfolgt.

Die erfindungsgemässe Umsetzung wird, soweit nichts anderes angegeben ist, vorzugsweise unter milden Bedingungen, insbesondere bei Temperaturen zwischen 0 °C und 60 °C, z.B. bei Raumtemperatur oder leicht erhöhten Temperaturen bis etwa 45 °C, z.B. etwa bei Raumtemperatur durchgeführt. Falls die Umsetzung mit einer Verbindung der Formel R₁-Z₁ durchgeführt wird, worin Z₁ Halogen bedeutet, oder mit einem aktivierten Derivat einer Verbindung der Formel II, wie dem entsprechenden Chlorkohlensäureester, zur Herstellung einer Verbindung der Formel I, worin R₁ Aminocarbonyl oder der Rest einer N-substituierten Carbaminsäure ist, wird vorteilhaft in Gegenwart eines säurebindenden Mittels, wie einer nichtacylierbaren Base, insbesondere einer tertiären Stickstoffbase, wie N-Methylmorpholin, 4-Dimethylaminopyridin, Triethylamin oder Ethyldiisopropylamin, gearbeitet. Sowohl bei der Umsetzung mit einem Carbonsäurehalogenid der Formel IIIa, worin Z₁ Halogen, wie Chlor oder Brom bedeutet, als auch der Umsetzung mit einem Anhydrid, insbesondere einem symmetrischen Anhydrid (Z₁ = O-R₁), verwendet man insbesondere einen Ueberschuss der entsprechenden Verbindung der Formel IIIa (Halogenid bzw. R₁-O-R₁), beispielsweise den mehr als 1,05-fachen Überschuss. Bevorzugt sind jeweils spezifisch genannte Reaktionsbedingungen.

Zu den Schutzgruppen für funktionelle Gruppen in Ausgangsmaterialien, deren Umsetzung vermieden werden soll, insbesondere Carboxy-, Amino-, Hydroxy- und/oder Mercaptogruppen, zählen insbesondere diejenigen Schutzgruppen (conventional protecting groups), die üblicherweise bei der Synthese von Peptid-Verbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d. h. ohne unerwünschte Nebenreaktionen abspaltbar sind, beispielsweise solvolytisch, reduktiv, photolytisch oder auch enzymatisch, z. B. auch unter physiologischen Bedingungen. Als Schutzgruppen werden nur solche Reste bezeichnet, die in den Endstoffen nicht vorhanden sind.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben-Weyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Monosaccharide und Derivate", Georg Thieme Verlag, Stuttgart 1974, beschrieben.

Eine Carboxygruppe ist z. B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche bevorzugt in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, ist beispielsweise Methoxycarbonyl oder Ethoxycarbonyl.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z. B. tert-Butoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise Arylmethoxycarbonyl mit einem oder zwei Arylresten, worin Aryl unsubstituiertes oder z. B. durch Niederalkyl, z. B. tert-Niederalkyl, wie tert-Butyl, Niederalkoxy, z. B. Methoxy, Hydroxy, Halogen, z. B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z. B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z. B. Di-(4-methoxyphenyl)-methoxycarbonyl, ferner durch eine Niederalkylgruppe verestertes Carboxy, wobei die Niederalkylgruppe in 1- oder 2-Stellung durch geeignete Substituenten substituiert ist, wie 1-Niederalkoxyniederalkoxycarbonyl, z. B. Methoxymethoxycarbonyl, 1-Methoxyethoxycarbonyl oder 1-Ethoxyethoxycarbonyl, 1-Niederalkylthioniederalkoxycarbonyl, z. B. 1-Methylthiomethoxycarbonyl oder 1-Ethylthioethoxycarbonyl, Aroylmethoxycarbonyl, worin die Aroylgruppe gegebenenfalls beispielsweise durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-Iodethoxycarbonyl, sowie 2-(trisubstituiertes Silyl)-niederalkoxycarbonyl, worin die Substituenten unabhängig voneinander je einen gegebenenfalls, z. B. durch Niederalkyl, Niederalkoxy, Aryl, Halogen, und/oder Nitro substituierten aliphatischen, araliphatischen, cycloaliphatischen oder aromatischen Kohlenwasserstoffrest bedeuten, beispielsweise gegebenenfalls wie oben substituiertes Niederalkyl, Phenylniederalkyl, Cycloalkyl oder Phenyl, z. B. 2-Triniederalkylsilylniederalkoxycarbonyl, wie 2-Triniederalkylsilylethoxycarbonyl, z. B. 2-Trimethylsilylethoxycarbonyl oder 2-(Di-n-butyl-methyl-silyl)-ethoxycarbonyl, oder 2-Triarylsilylethoxycarbonyl, wie Triphenylsilylethoxycarbonyl.

Eine Carboxygruppe wird auch als organische Silyloxycarbonylgruppe geschützt. Eine organische Silyloxycarbonylgruppe ist beispielsweise eine Triniederalkylsilyloxycarbonylgruppe, z. B. Trimethylsilyloxycarbonyl. Das Siliciumatom der Silyloxycarbonylgruppe kann auch durch zwei Niederalkyl-, z. B. Methylgruppen, und eine Amino- oder Carboxygruppe eines zweiten Moleküls der Formel I substituiert sein. Verbindungen mit solchen Schutzgruppen lassen sich z. B. mit Dimethylchlorsilan als Silylierungsmittel herstellen.

Eine Carboxygruppe wird auch in Form eines inneren Esters mit einer in geeignetem Abstand, z. B. in γ-Stellung, zur Carboxygruppe im Molekül vorliegenden Hydroxygruppe, d. h. in Form eines Lactons, vorzugsweise eines γ-Lactons, geschützt.

Eine geschützte Carboxygruppe ist bevorzugt tert-Niederalkoxycarbonyl, z. B. tert-Butoxycarbonyl, Benzyloxycarbonyl, 4-Nitrobenzyloxycarbonyl, 9-Fluorenylmethoxycarbonyl oder Diphenylmethoxycarbonyl.

Eine geschützte Aminogruppe ist durch eine Aminoschutzgruppe geschützt, z. B. in Form einer Acylamino-, Arylmethylamino-, veretherten Mercaptoamino-, 2-Acyl-niederalk-1-enylamino oder Silylaminogruppe oder als Azidogruppe.

In einer Acylaminogruppe ist Acyl beispielsweise der Acylrest einer organischen Carbonsäure mit z. B. bis zu 18 Kohlenstoffatomen, insbesondere einer gegebenenfalls, z.B durch Halogen oder Aryl, substituierten Niederalkancarbonsäure oder gegebenenfalls, z. B. durch Halogen, Niederalkoxy oder Nitro, substituierten Benzoesäure, oder bevorzugt eines Kohlensäurehalbesters. Solche Acylgruppen sind vorzugsweise Niederalkanoyl, wie Formyl, Acetyl, Propionyl oder Pivaloyl, Halogenniederalkanoyl, z. B. 2-Halogenacetyl, wie 2-Chlor-, 2-Brom-, 2-Iod-, 2,2,2-Trifluor- oder 2,2,2-Trichloracetyl, gegebenenfalls z. B. durch Halogen, Niederalkoxy oder Nitro substituiertes Benzoyl, wie Benzoyl, 4-Chlorbenzoyl, 4-Methoxybenzoyl oder 4-Nitrobenzoyl, Niederalkoxycarbonyl, vorzugsweise in 1-Stellung des Niederalkylrestes verzweigtes oder in 1- oder 2-Stellung geeignet substituiertes Niederalkoxycarbonyl, z. B. tert-Niederalkoxycarbonyl, wie tert-Butoxycarbonyl, Arylmethoxycarbonyl mit einem, zwei oder drei Arylresten, die gegebenenfalls, z. B. durch Niederalkyl, insbesondere tert-Niederalkyl, wie tert-Butyl, Niederalkoxy, wie Methoxy, Hydroxy, Halogen, wie Chlor, und/oder Nitro mono- oder polysubstituiertes Phenyl darstellen, z. B. Benzyloxycarbonyl, 4-Nitrobenzyloxycarbonyl, Diphenylmethoxycarbonyl, 9-Fluorenylmethoxycarbonyl oder Di-(4-methoxyphenyl)methoxycarbonyl, Aroylmethoxycarbonyl, worin die Aroylgruppe vorzugsweise gegebenenfalls, z. B. durch Halogen, wie Brom, substituiertes Benzoyl darstellt, z. B. Phenacyloxycarbonyl, 2-Halogen-niederalkoxycarbonyl, z. B. 2,2,2-Trichlorethoxycarbonyl, 2-Bromethoxycarbonyl oder 2-Iodethoxycarbonyl, 2-(trisubstituiertes Silyl)-niederalkoxycarbonyl, z. B. 2-Triniederalkylsilylniederalkoxycarbonyl, wie 2-Trimethylsilylethoxycarbonyl oder 2-(Di-n-butyl-methyl-silyl)-ethoxycarbonyl, oder Triarylsilylniederalkoxycarbonyl, z. B. 2-Triphenylsilylethoxycarbonyl.

In einer Arylmethylaminogruppe, z. B. einer Mono-, Di- oder insbesondere Triarylmethylaminogruppe, sind die Arylreste insbesondere gegebenenfalls substituierte Phenylreste. Solche Gruppen sind z. B. Benzyl-, Diphenylmethyl- oder insbesondere Tritylamino.

In einer veretherten Mercaptoaminogruppe liegt die Mercaptogruppe in erster Linie als substituiertes Arylthio oder Arylniederalkylthio, worin Aryl beispielsweise gegebenenfalls z. B. durch Niederalkyl, wie Methyl oder tert.-Butyl, Niederalkoxy, wie Methoxy, Halogen, wie Chlor, und/oder Nitro substituiertes Phenyl ist, z. B. 4-Nitrophenylthio, vor.

In einem als Aminoschutzgruppe verwendbaren 2-Acyl-niederalk-1-enylrest ist Acyl z. B. der entsprechende Rest einer Niederalkancarbonsäure, einer gegebenenfalls z. B. durch Niederalkyl, wie Methyl oder tert-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-niederalk-1-en-2-yl, z. B. 1-Niederalkanoyl-prop-1-en-2-yl, wie 1-Acetyl-prop-1-en-2-yl, oder Niederalkoxycarbonyl-niederalk-1-en-2-yl, z. B. Niederalkoxycarbonyl-prop-1-en-2-yl, wie 1-Ethoxycarbonyl-prop-1-en-2-yl.

Eine Silylaminogruppe ist beispielsweise eine Triniederalkylsilylaminogruppe, z. B. Trimethylsilylamino oder tert-Butyl-dimethylsilylamino. Das Siliciumatom der Silylaminogruppe kann auch nur durch zwei Niederalkylgruppen, z. B. Methylgruppen, und die Aminogruppe oder Carboxylgruppe eines zweiten Moleküls der Formel I substituiert sein. Verbindungen mit solchen Schutzgruppen lassen sich z. B. mit den entsprechenden Chlorsilanen, wie Dimethylchlorsilan, als Silylierungsmittel herstellen.

Eine Aminogruppe kann auch durch Überführung in die protonierte Form geschützt werden; als entsprechende Anionen kommen in erster Linie diejenigen von starken anorganischen Säuren, wie von Schwefelsäure, Phosphorsäure oder Halogenwasserstoffsäuren, z. B. das Chlor- oder Bromanion, oder von organischen Sulfonsäuren, wie p-Toluolsulfonsäure, in Frage.

Bevorzugte Aminoschutzgruppen sind Niederalkoxycarbonyl, Phenylniederalkoxycarbonyl, Fluorenylniederalkoxycarbonyl, 2-Niederalkanoyl-niederalk-1-en-2-yl oder Niederalkoxycarbonyl-niederalk-1-en-2-yl, besonders bevorzugt tert-Butoxycarbonyl oder Benzyloxycarbonyl.

Eine Hydroxygruppe kann beispielsweise durch eine Acylgruppe, z. B. unsubstituiertes oder durch Halogen, wie Chlor, substituiertes Niederalkanoyl, wie Acetyl oder 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Triphenylmethoxycarbonyl. Eine Hydroxygruppe kann ferner durch Triniederalkylsilyl, z. B. Trimethylsilyl, Triisopropylsilyl oder tert-Butyl-dimethylsilyl, eine leicht abspaltbare verethernde Gruppe, z. B. eine Alkylgruppe, wie tert-Niederalkyl, z. B. tert-Butyl, einen oxa- oder einen thiaaliphatischen oder -cycloaliphatischen, insbesondere 2-oxa- oder 2-thiaaliphatischen oder - cycloaliphatischen, Kohlenwasserstoffrest, beispielsweise 1-Niederalkoxyniederalkyl oder 1-Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, wie 2-Tetrahydrofuryl oder 2-Tetrahydropyranyl, oder ein entsprechendes Thiaanaloges, sowie durch 1-Phenylniederalkyl, wie Benzyl, Diphenylmethyl oder Trityl, wobei die Phenylreste beispielsweise durch Halogen, z. B. Chlor, Niederalkoxy, z. B. Methoxy, und/oder Nitro substituiert sein können, geschützt sein.

Zwei in einem Molekül vorkommende, insbesondere benachbarte Hydroxylgruppen oder eine benachbarte Hydroxy- und Aminogruppe können beispielsweise durch bivalente Schutzgruppen, wie eine vorzugsweise, etwa durch ein oder zwei Niederalkylreste oder Oxo, substituierte Methylengruppe, geschützt sein, z. B. durch unsubstituiertes oder substituiertes Alkyliden, z. B. Niederalkyliden, wie Isopropyliden, Cycloalkyliden, wie Cyclohexyliden, eine Carbonylgruppe oder Benzyliden.

Eine in Nachbarstellung zu einer Carboxygruppe stehende Hydroxygruppe kann durch Bildung eines inneren Esters (Lacton), insbesondere eines γ-Lactones, geschützt sein.

Bevorzugt ist eine geschützte Hydroxygruppe durch Triniederalkylsilyl oder als Lacton geschützt, insbesondere durch tert-Butyl-dimethylsilyl.

Eine Mercaptogruppe, wie z. B. in Cystein, kann insbesondere durch S-Alkylierung mit gegebenenfalls substituierten Alkylresten, Silylierung, Thioacetalbildung, S-Acylierung oder durch die Bildung asymmetrischer Disulfidgruppierungen geschützt sein. Bevorzugte Mercaptoschutzgruppen sind beispielsweise gegebenenfalls im Phenylrest, z. B. durch Methoxy oder Nitro, substituiertes Benzyl, wie 4-Methoxybenzyl, gegebenenfalls im Phenylkest, z. B. durch Methoxy, substituiertes Diphenylmethyl, wie Di-(4-methoxyphenyl)-methyl, Triphenylmethyl, Pyridyldiphenylmethyl, Trimethylsilyl, Benzylthiomethyl, Tetrahydropyranyl, Acylaminomethyl, wie Acetamidomethyl, iso-Butyrylacetamidomethyl oder 2-Chloracetamidomethyl, Benzoyl, Benzyloxycarbonyl oder Alkyl-, insbesondere Niederalkylaminocarbonyl, wie Ethylaminocarbonyl, sowie Niederalkylthio, wie S-Ethylthio oder S-tert-Butylthio, oder S-Sulfo.

Die Abspaltung der Schutzgruppen, die nicht Bestandteil des gewünschten Endproduktes der Formel I sind, z. B. der Carboxy-, Amino-, Hydroxy- und/oder Mercaptoschutzgruppen, erfolgt in an sich bekannter Weise, z. B. mittels Solvolyse, insbesondere Hydrolyse, Alkoholyse oder Acidolyse, oder mittels Reduktion, insbesondere Hydrogenolyse oder mittels anderer Reduktionsmittel, sowie Photolyse, gegebenenfalls stufenweise oder gleichzeitig, wobei auch enzymatische Methoden verwendet werden können. Die Abspaltung der Schutzgruppen ist beispielsweise in den weiter vorn im Abschnitt über "Schutzgruppen" genannten Standardwerken beschrieben.

So kann beispielsweise geschütztes Carboxy, z. B. tert-Niederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure, Chlorwasserstoff oder Trifluoressigsäure, gegebenenfalls unter Zugabe einer nukleophilen Verbindung, wie Phenol oder Anisol, in freies Carboxy überführt werden. Gegebenenfalls substituiertes Benzyloxycarbonyl kann z. B. mittels Hydrogenolyse, d.h. durch Behandeln mit Wasserstoff in Gegenwart eines metallischen Hydrierkatalysators, wie eines Palladiumkatalysators, freigesetzt werden. Ferner kann geeignet substituiertes Benzyloxycarbonyl, wie 4-Nitrobenzyloxycarbonyl, auch durch Reduktion, z. B. durch Behandeln mit einem Alkalimetall-, wie Natrium-dithionit, oder mit einem reduzierenden Metall, z. B. Zink, oder einem reduzierenden Metallsalz, wie einem Chrom-II-salz, z. B. Chrom-II-chlorid, üblicherweise in Gegenwart eines Wasserstoff-abgebenden Mittels, das zusammen mit dem Metall nascierenden Wasserstoff erzeugen kann, wie einer Säure, in erster Linie einer geeigneten Carbonsäure, wie einer gegebenenfalls, z. B. durch Hydroxy, substituierten Niederalkancarbonsäure, z. B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-(trisubstituiertes Silyl)-niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalimetallfluorid, z. B. Natrium-oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylniederalkylammoniumfluorid, z. B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkylsilyloxycarbonyl, z. B. Trimethylsilyloxycarbonyl, geschütztes Carboxy kann in üblicher Weise solvolytisch, z. B. durch Behandeln mit Wasser, einem Alkohol oder Säure, oder ausserdem Fluorid, wie oben beschrieben, freigesetzt werden. Verestertes Carboxy kann auch enzymatisch freigesetzt werden, beispielsweise durch Esterasen oder geeignete Peptidasen, z. B. verestertes Arginin oder Lysin, wie Lysinmethylester, mittels Trypsin. Als innerer Ester, wie als γ-Lacton, geschütztes Carboxy kann durch Hydrolyse in Gegenwart eines Hydroxid-haltigen Base, wie Erdalkalihydroxides oder insbesondere eines Alkalimetallhydroxides, z. B. NaOH, KOH oder LiOH, besonders LiOH, freigesetzt werden, wobei gleichzeitig die entsprechend geschützte Hydroxygruppe freigesetzt wird.

Eine geschützte Aminogruppe setzt man in an sich bekannter und je nach Art der Schutzgruppen in verschiedenartiger Weise, vorzugsweise mittels Solvolyse oder Reduktion, frei. Niederalkoxycarbonylamino, wie tert-Butoxycarbonylamino, kann in Gegenwart von Säuren, beispielsweise Mineralsäuren, z. B. Halogenwasserstoff, wie Chlorwasserstoff oder Bromwasserstoff, insbesondere Bromwasserstoff, oder von Schwefel- oder oder Phosphorsäure, vorzugsweise von Chlorwasserstoff, in polaren Lösungsmitteln, wie Wasser oder einer Carbonsäure, wie Essigsäure, oder Ethern, bevorzugt cyclischen Ethern, wie Dioxan, 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z. B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z. B. Natrium-dithionit, gespalten werden. Gegebenenfalls substituiertes Diphenylmethoxycarbonylamino, tert-Niederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z. B. Ameisen- oder Trifluoressigsäure, gegebenenfalls substituiertes Benzyloxycarbonylamino z. B. mittels Hydrogenolyse, d.h. durch Behandeln mit Wasserstoff in Gegenwart eines geeigneten Hydrierkatalysators, wie eines Palladiumkatalysators, bevorzugt in polaren Lösungsmitteln, wie Diniederalkylniederalkanoylamiden, z. B. Dimethylformamid, Ethern, wie cyclischen Ethern, z. B. Dioxan, oder Alkoholen, wie Methanol, Ethanol oder Propanol, wobei Methanol besonders bevorzugt ist, gegebenenfalls substituiertes Triarylmethylamino oder Formylamino z. B. durch Behandeln mit einer Säure, wie Mineralsäure, z. B. Chlorwasserstoffsäure, oder einer organischen Säure, z. B. Ameisen-, Essig-oder Trifluoressigsäure, gegebenenfalls in Gegenwart von Wasser, und eine als Silylamino geschützten Aminogruppe z. B. mittels Hydrolyse oder Alkoholyse freigesetzt werden. Eine durch 2-Halogenacetyl, z. B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionssproduktes freigesetzt werden. Eine durch 2-(trisubstituiertes Silyl)-niederalkoxycarbonyl, wie 2-Triniederalkylsilylniederalkoxycarbonyl, geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z. B. durch Reduktion in freies Amino überführt, beispielsweise durch katalytische Hydrierung mit Wasserstoff in Gegenwart eines Hydrierkatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z. B. Methylenchlorid, oder auch in Wasser oder einem Gemisch von Wasser und einem organischen Lösungsmittel, wie einem Alkohol oder Dioxan, bei etwa 20 °C bis 25 °C, oder auch unter Kühlen oder Erwärmen, durchgeführt.

Eine durch eine geeignete Acylgruppe, eine Triniederalkylsilylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z. B. durch basische Hydrolyse, eine durch tert-Niederalkyl oder durch einen 2-oxa- oder 2-thia-aliphatischen oder -cycloaliphatischen Kohlenwasserstoffrest geschützte Hydroxy- bzw. Mercaptogruppe durch Acidolyse, z. B. durch Behandeln mit einer Mineralsäure oder einer starken Carbonsäure, z. B. Trifluoressigsäure, freigesetzt. Durch Pyridyldiphenylmethyl geschütztes Mercapto kann z. B. durch Quecksilber-II-salze bei pH 2-6 oder durch Zink/Essigsäure oder elektrolytische Reduktion, durch Acetamidomethyl oder iso-Butyrylamidomethyl geschütztes Mercapto z. B. durch Reaktion mit Quecksilber-II-salzen bei pH 2-6, durch 2-Chloracetamidomethyl geschütztes Mercapto z. B. durch 1-Piperidinothiocarboxamid, S-Ethylthio, S-tert-Butylthio und S-Sulfo z. B. durch Thiolyse mit Thiophenol, Thioglycolsäure, Natrium-thiophenolat oder 1,4-Dithiothreitol freigesetzt werden. Zwei Hydroxygruppen oder eine benachbarte Amino- und Hydroxygruppe, die zusammen mittels einer bivalenten Schutzgruppe, vorzugsweise z. B. einer durch Niederalkyl ein-oder zweifach substituierten Methylengruppe, wie durch Niederalkyliden, z. B. Isopropyliden, Cycloyalkyliden, z. B. Cyclohexyliden, oder Benzyliden, geschützt sind, können durch saure Solvolyse, besonders in Gegenwart einer Mineralsäure oder einer starken organischen Säure, freigesetzt werden. Eine Triniederalkylsilylgruppe wird ebenfalls durch Acidolyse, z. B. durch Mineralsäure, bevorzugt Fluorwasserstoffsäure, oder eine starke Carbonsäure abgespalten. 2-Halogenniederalkoxycarbonyl wird durch die oben genannten Reduktionsmittel, z. B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt. Veresterte Hydroxygruppen, z. B. Niederalkanoyloxy, wie Acetyloxy, können auch durch Esterasen freigesetzt werden, acyliertes Amino beispielsweise durch geeignete Peptidasen.

Die Temperaturen für die Freisetzung der geschützten funktionellen Gruppen liegen vorzugsweise zwischen -80 und 100 °C, besonders bevorzugt zwischen -20 und 50 °C, beispielsweise zwischen 10 und 35 ⁰C, wie im Bereich der Raumtemperatur.

Beim Vorhandensein von mehreren geschützten funktionellen Gruppen können, wenn erwünscht, die Schutzgruppen so gewählt werden, dass gleichzeitig mehr als eine solche Gruppe abgespalten werden kann, beispielsweise acidolytisch, wie durch Behandeln mit Trifluoressigsäure, oder mit Wasserstoff und einem Hydrierkatalysator, wie einem Palladium-Kohle-Katalysator. Umgekehrt können die Gruppen auch so gewählt werden, dass sie nicht alle gleichzeitig, sondern in gewünschter Reihenfolge abgespalten werden können, wobei die entsprechenden Zwischenprodukte erhalten werden.

Die Ausgangsverbindung der Formel II ist erhältlich aus 2-[3(S)-Amino-2(R)-hydroxy-4-phenylbutyl]-N-tert-butyl-decahydro-(4aS, 8aS)-isochinolin-3(S)-carboxamid (herstellbar analog EP 0 432 695, Beispiel 33) welches auch beispielsweise durch Umsetzung einer Verbindung der Formel VIII, 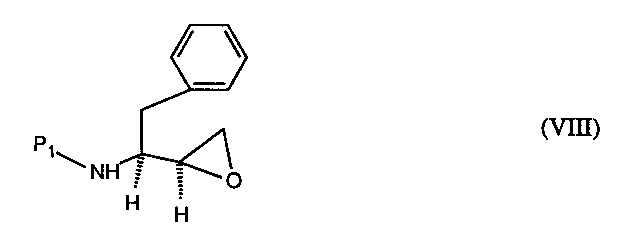 worin P₁ eine Aminoschutzgruppe, wie oben unter Verfahren a) definiert, insbesondere tert-Butoxycarbonyl oder Benzyloxycarbonyl, bedeutet, welche gemäss Evans et al., J. Org. Chem. 50, 4615-4625 (1985) hergestellt werden kann, mit N-tert-Butyldecahydro-(4aS,8aS)-isochinolin-3(S)carboxamid (hergestellt gemäss EP 0 432 694), beispielsweise in einem polaren Lösungsmittel, wie einem Alkohol, z. B. Methanol oder Ethanol, bei erhöhter Temperatur, z. B. 40 bis 90 °C, wie Rückflusstemperatur, unter Erhalt einer Verbindung der Formel IX, 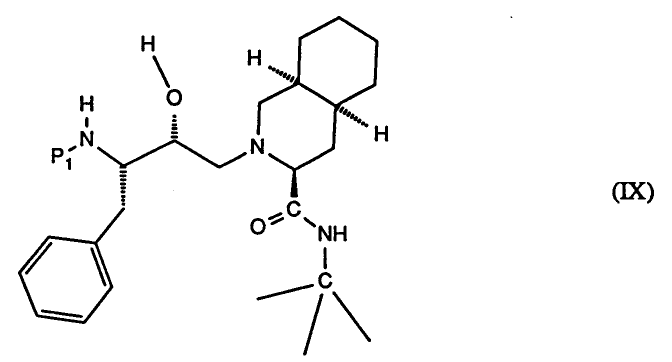 worin P₁ die zuletzt genannten Bedeutungen hat, und anschliessende Abspaltung der Aminoschutzgruppe P₁ erhalten werden kann, beispielsweise im Falle von tert-Butoxycarbonyl P₁ durch Abspaltung mit einer Säure, wie einer Mineralsäure, z.B. einer Halogenwasserstoffsäure, wie Chlorwasserstoffsäure, oder in Gegenwart einer starken organischen Säure, wie Ameisensäure, in organischen Lösungsmitteln, beispielsweise einem Ether, z.B. einem cyclischen Ether, wie Dioxan, oder im Falle einer flüssigen organischen Säure, wie Ameisensäure, ohne Lösungsmittel; oder im Falle von Benzyloxycarbonyl durch Hydrierung in Gegenwart eines Katalysators, beispielsweise eines Edelmetallkatalysators, z.B. an einen Träger, wie Kohle, Silicagel oder Aluminiumoxid gebunden, vorzugsweise Pd auf Aktivkohle, bei Normaldruck oder erhöhtem Druck, vorzugsweise etwa bei Normaldruck, in einem polaren Lösungsmittel, z.B. einem Alkohol, wie Methanol oder Ethanol, bei 0 bis 50 °C, vorzugsweise etwa bei Raumtemperatur, und die erhaltene Verbindung {N-tert-Butyl-decahydro-2-[[2(R)-hydroxy-4-phenyl-3(S)amino]butyl]-(4aS,8aS)-isochinolin-3(S)carboxamid}, oder ein Salz davon, z.B. das Salz einer der zuletzt genannten Säuren, entweder mit durch eine Aminoschutzgruppe P₂ N-geschütztem L-Asparagin, z.B. α-(N-Benzyloxycarbonyl-)-L-asparagin, oder einem reaktionsfähigen Säurederivat davon, insbesondere dem p-Nitrophenylester, amidiert, wie zur Amidierung unter Verfahren b) beschrieben, vorzugsweise in Anwesenheit einer tertiären Stickstoffbase, wie Triethylamin oder N-Ethyldiisopropylamin, insbesondere in einem Säureamid, z.B. Dimethylformamid, bei Temperaturen zwischen 0 und 50 °C, vorzugsweise etwa bei Raumtemperatur, wobei man die Verbindung der Formel X, 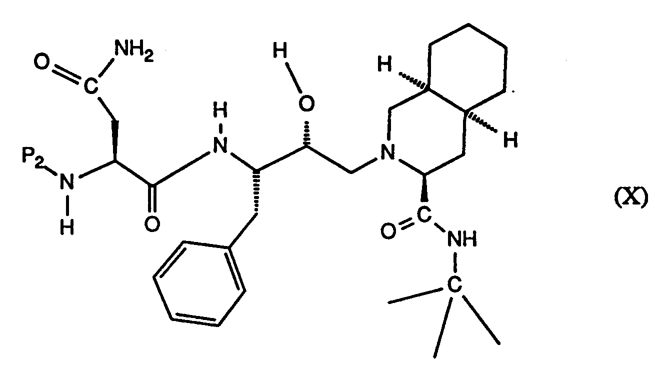 worin P₂ eine Aminoschutzgruppe, wie unter Verfahren a) definiert, insbesondere Benzyloxycarbonyl, bedeutet, erhält, die dann unter Schutzgruppenabspaltung in die entsprechende freie Aminoverbindung, bei Benzyloxycarbonyl P₂ beispielsweise durch Hydrierung in Gegenwart eines Katalysators, beispielsweise eines Edelmetallkatalysators, z.B. an einen Träger, wie Kohle, Silicagel oder Aluminiumoxid gebunden, vorzugsweise Pd auf Aktivkohle, bei Normaldruck oder erhöhtem Druck, vorzugsweise bei Normaldruck, in einem polaren Lösungsmittel, z. B. einem Alkohol, wie Methanol oder Ethanol, bei 0 bis 50 °C, vorzugsweise etwa bei Raumtemperatur, überführt wird, welche dann durch Umsetzung mit Chinolin-2-carbonsäure amidiert wird unter Reaktionsbedingungen analog den unter Verfahren b) genannten unter Erhalt der Verbindung der Formel II.

Alternativ kann die Verbindung der Formel II auch nach den in EP 0 432 695 (publiziert am 19.06.1991) genannten Herstellungsverfahren erhalten werden.

Die Ausgangsverbindungen der Formel III sind kommerziell erhältlich, bekannt oder nach an sich bekannten Verfahren herstellbar.

Als Beispiel erwähnt sei die Herstellung von durch Heterocyclylmethyl, worin Heterocyclyl über ein Ringstickstoffatom gebunden ist, substituiertem Arylniederalkanoyl, welche vorzugsweise durch Umsetzung eines durch Halogenmethyl, wie Chlor- oder Brommethyl, substituierten Arylniederalkanoylrestes, wie Chlormethylbenzoyl oder Brommethylbenzoyl, mit einer entsprechenden heterocyclischen Stickstoffbase, wie Piperidin, Piperazin, 1-Niederalkylpiperazin, 1-Niederalkanyolpiperazin oder insbesondere Morpholin oder Thiomorpholin, unter nukleophiler Substitution des Halogenatomes erfolgt.

Die Herstellung von Aminosäurederivaten der Formel III, worin die α-Aminogruppe durch einen Rest ausgewählt aus Phenylniederalkyl oder Heterocyclylniederalkyl alkyliert ist, ist beispielsweise möglich durch reduktive Aminierung der (erforderlichenfalls an weiteren Gruppen, die nicht an der Reaktion teilnehmen sollen, geschützten) Aminosäure, welche eine primäre oder sekundäre α-Aminogruppe hat, mit einem Phenylniederalkylketon oder -aldehyd, wie Benzaldehyd, oder Heterocyclylniederalkylketon- oder - aldehyd, beispielsweise Heterocyclylaldehyd, z.B. Furanaldehyd, wie Furan-2-aldehyd, oder Pyridinaldehyd, wie Pyridin-3-aldehyd, oder Imidazolylaldehyd, wie Imidazol-4-ylaldehyd (gegebenenfalls N-geschützt, z.B. durch Trityl, welches wie oben beschrieben in der Verbindung der Formel III oder dem geschützten Endprodukt der Formel I abgespalten werden kann, wie oben beschrieben) beispielsweise unter katalytischer Hydrierung, z.B. in Gegenwart eines Schwermetallkatalysators, wie Raney-Nickel, bei Normaldruck oder Drucken von 1 bis 100 bar, vorzugsweise bei etwa 100 bar, oder unter Reduktion mittels komplexer Borhydride, wie Natriumcyanoborhydrid.

Die Isocyanate der Formel IIIb lassen sich beispielsweise aus den entsprechenden Aminvorstufen durch Umwandlung der Aminogruppe in die Isocyanatogruppe herstellen, z.B. durch Umsetzung mit Phosgen in der Hitze, z.B. bei Rückflussbedingungen, oder durch Zutropfen des flüssigen oder in einem Losungsmittel gelösten primären, sekundären oder tertiären Amins zu einem Überschuss von Phosgen in einem geeigneten Lösungsmittel (Toluol, Xylol, Ligroin, Chlorbenzen, α-Chlornaphthalen usw.) unter Kühlung (beispielsweise auf - 50 bis 0 °C), wobei sich intermediär eine Mischung aus Carbamoylchlorid und Aminhydrochlorid bildet, die dann bei erhöhter Temperatur (besipielsweise bei 50 °C bis Rückflusstemperatur) weiter bis zur vollständigen Lösung phosgeniert wird, unter HCl-Eliminierung.

### Verfahren b) Amidierung (Kondensation zur Herstellung einer Amidbindung)

In Ausgangsmaterialien der Formel IV und/oder V sind funktionelle Gruppen, die nicht an der Reaktion teilnehmen sollen, erforderlichenfalls durch Schutzgruppen geschützt. Die Schutzgruppen und ihre Einführung sind wie oben unter Verfahren a) beschrieben.

Die Carbonsäure der Formel V liegt entweder mit freier Carboxygruppe vor oder als reaktionsfähiges Derivat hiervon, beispielsweise als von der freien Carboxyverbindung abgeleiteter aktivierter Ester, als reaktionsfähiges Anhydrid, oder ferner als reaktionsfähiges cyclisches Amid. Die reaktionsfähigen Derivate können auch in situ gebildet werden.

Aktivierte Ester von der Verbindung der Formel V mit einer Carboxygruppe sind insbesondere am Verknüpfungskohlenstoffatom des veresternden Restes ungesättigte Ester, z.B. vom Vinylester-Typ, wie Vinylester (erhältlich z. B. durch Umesterung eines entsprechenden Esters mit Vinylacetat; Methode des aktivierten Vinylesters), Carbamoylester (erhältlich z. B. durch Behandeln der entsprechenden Säure mit einem Isoxazoliumreagens; 1,2-Oxazolium- oder Woodward-Methode), oder 1-Niederalkoxyvinylester (erhältlich z. B. durch Behandeln der entsprechenden Säure mit einem Niederalkoxyacetylen; Ethoxyacetylen-Methode), oder Ester vom Amidinotyp, wie N,N'-disubstituierte Amidinoester (erhältlich z. B. durch Behandeln der entsprechenden Säure mit einem geeigneten N,N'-disubstituierten Carbodiimid, z. B. N,N'-Dicyclohexylcarbodiimid; Carbodiimid-Methode), oder N,N-disubstituierte Amidinoester (erhältlich z. B. durch Behandeln der entsprechenden Säure mit einem N,N-disubstituierten Cyananmid; Cyanamid-Methode), geeignete Arylester, insbesondere durch elektronenanziehende Substituenten substituierte Phenylester (erhältlich z. B. durch Behandeln der entsprechenden Säure mit einem geeignet substituierten Phenol, z. B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z. B. durch Behandeln der entsprechenden Säure mit Chloracetonitril in Gegenwart einer Base; Cyanmethylester-Methode), Thioester, insbesondere gegebenenfalls, z. B. durch Nitro, substituierte Phenylthioester (erhältlich z. B. durch Behandeln der entsprechenden Säure mit gegebenenfalls, z. B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z. B. durch Behandeln der entsprechenden Säure mit einer N-Hydroxyamino- bzw. N-Hydroxyamidoverbindung, z. B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benztriazin-4-on, z. B. nach der Anhydrid- oder Carbodiimid-Methode; Methode der aktivierten N-Hydroxyester). Auch innere Ester, z. B. γ-Lactone, sind einsetzbar.

Anhydride der Säure können symmetrisch oder vorzugsweise gemischte Anhydride dieser Säure sein, z. B. Anhydride mit anorganischen Säuren, wie Säurehalogenide, insbesondere Säurechloride (erhältlich z. B. durch Behandeln der entsprechenden Säure mit Thipnylchlorid, Phosphorpentachlorid oder Oxalylchlorid; Säurechloridmethode), Azide (erhältlich z. B. aus einem entsprechenden Säureester über das entsprechende Hydrazid und dessen Behandlung mit salpetriger Säure; Azidmethode), Anhydride mit Kohlensäurehalbestern, z. B. Kohlensäureniederalkylhalbestern (erhältlich z. B. durch Behandeln der entsprechenden Säure mit Chlorameisensäureniederalkylestern oder mit einem 1-Niederalkoxycarbonyl-2-niederalkoxy-1,2-dihydrochinolin; Methode der gemischten O-Alkylkohlensäureanhydride), oder Anhydride mit dihalogenierter, insbesondere dichlorierter Phosphorsäure (erhältlich z. B. durch Behandeln der entsprechenden Säure mit Phosphoroxychlorid; Phosphoroxychloridmethode), Anhydride mit anderen Phosphorsäurederivaten (z. B. solchen, die man mit Phenyl-N-phenylphosphoramidochloridat erhalten kann oder durch Umsetzung von Alkylphosphorsäureamiden in Gegenwart von Sulfonsäureanhydriden und/oder racemisierungssenkenden Additiven, wie NHydroxybenztriazol, oder in Gegenwart von Cyanphosphonsäurediethylester) oder mit Phosphorigsäurederivaten, oder Anhydride mit organischen Säuren, wie gemischte Anhydride mit organischen Carbonsäuren (erhältlich z. B. durch Behandeln der entsprechenden Säure mit einem gegebenenfalls substituierten Niederalkan- oder Phenylniederalkancarbonsäurehalogenid, z. B. Phenylessigsäure-, Pivalinsäure- oder Trifluoressigsäurechlorid; Methode der gemischten Carbonsäureanhydride) oder mit organischen Sulfonsäuren (erhältlich z. B. durch Behandeln eines Salzes, wie eines Alkalimetallsalzes, der entsprechenden Säure mit einem geeigneten organischen Sulfonsäurehalogenid, wie Niederalkan- oder Aryl-, z. B. Methan- oder p-Toluolsulfonsäurechlorid; Methode der gemischten Sulfonsäureanhydride) sowie symmetrische Anhydride (erhältlich z. B. durch Kondensation der entsprechenden Säure in Gegenwart eines Carbodiimides oder von 1-Diethylaminopropin; Methode der symmetrischen Anhydride).

Geeignete cyclische Amide sind insbesondere Amide mit fünfgliedrigen Diazacyclen aromatischen Charakters, wie Amide mit Imidazolen, z. B. Imidazol (erhältlich z. B. durch Behandeln der entsprechenden Säure mit N,N'-Carbonyldiimidazol; Imidazol-Methode), oder Pyrazol, z. B. 3,5-Dimethylpyrazol (erhältlich z. B. über das Säurehydrazid durch Behandeln mit Acetylaceton; Pyrazolid-Methode).

Wie erwähnt, können Derivate einer Carbonsäure, die als Acylierungsmittel verwendet werden, auch in situ gebildet werden. So kann man z. B. N,N'-disubstituierte Amidinoester in situ bilden, indem man das Gemisch des Ausgangsmaterials der Formel IV und der als Acylierungsmittel verwendeten Säure der Formel V in Gegenwart eines geeigneten N,N'-disubstituierten Carbodiimids, z. B. N,N'-Cyclohexylcarbodiimid, zur Reaktion bringt, beispielsweise in Gegenwart einer geeigneten Base, wie Triethylamin. Weiter kann man Amino- oder Amidoester der als Acylierungsmittel verwendeten Säuren in Gegenwart des zu acylierenden Ausgangsmaterials der Formel IV bilden, indem man das Gemisch der entsprechenden Säure- und Amino-Ausgangsstoffe in Gegenwart eines N,N'-disubstituierten Carbodiimids, z. B. N,N'-Dicyclohexylcarbodiimid, und eines N-Hydroxyamins oder N-Hydroxy-amids, z. B. N-Hydroxysuccinimid, gegebenenfalls in Anwesenheit einer geeigneten Base, z. B. 4-Dimethylamino-pyridin, umsetzt. Ferner kann man durch Umsetzung mit N,N,N',N'-Tetraalkyluroniumverbindungen, wie O-Benztriazol-1-yl-N,N,N',N'-tetra-methyl-uronium-hexafluorphosphat, Aktivierung in situ erreichen. Schliesslich können Phosphorsäureanhydride der Carbonsäuren der Formel V in situ hergestellt werden, indem man ein Alkylphosphorsäureamid, wie Hexamethylphosphorsäuretriamid, in Gegenwart eines Sulfonsäureanhydrides, wie 4-Toluolsulfonsäureanhydrid, mit einem Salz, wie einem Tetrafluoroborat, z. B. Natriumtetrafluoroborat, oder mit einem anderen Abkömmling des Hexamethylphosphorsäuretriamides, wie Benzotriazol-1-yl-oxy-tris-(dimethylamino)-phosphonium-hexafluorid, vorzugsweise in Gegenwart eines racemisierungssenkenden Additives, wie N-Hydroxybenztriazol, umsetzt.

Die Aminogruppe von Verbindungen der Formel IV, die an der Reaktion teilnimmt, trägt vorzugsweise mindestens ein reaktionsfähiges Wasserstoffatom, insbesondere, wenn die damit reagierende Carboxygruppe in reaktionsfahiger Form vorliegt; die Verbindung der Formel IV kann aber auch selbst als reaktionsfähiges Derivat vorliegen, d.h. mit der Aminogruppe in reaktionsfähiger Form, z. B. durch Reaktion mit einem Phosphit, wie Diethylchlorphosphit, 1,2-Phenylenchlorphosphit, Ethyldichlorphosphit, Ethylenchlorphosphit oder Tetraethylpyrophosphit. Ein Derivat einer solchen Verbindung mit einer Aminogruppe ist z. B. auch ein Carbaminsäurehalogenid, wobei die an der Reaktion teilnehmende Aminogruppe durch Halogencarbonyl, z. B. Chlorcarbonyl, substituiert ist.

Die Kondensation zur Herstellung einer Amidbindung kann in an sich bekannter Weise durchgeführt werden, beispielsweise wie in Standardwerken, wie "Houben-Weyl, Methoden der organischen Chemie", 4. Auflage, Band 15/II (1974), Band IX (1955) Band E 11 (1985), Georg Thieme Verlag, Stuttgart, "The Peptides" (E. Gross und J. Meienhofer, Hg.), Band 1 und 2, Academic Press, London und New York, 1979/1980, oder M. Bodansky, "Principles of Peptide Synthesis", Springer-Verlag, Berlin 1984, beschrieben.

Die Kondensation einer freien Carbonsäure mit dem entsprechenden Amin kann vorzugsweise in Gegenwart eines der üblichen Kondensationsmittel durchgeführt werden. Übliche Kondensationsmittel sind z. B. Carbodiimide, beispielsweise Diethyl-, Dipropyl-, N-Ethyl-N'-(3-dimethylaminopropyl)-carbodiimid oder insbesondere Dicyclohexylcarbodiimid, ferner geeignete Carbonylverbindungen, beispielsweise Carbonylimidazol, 1,2-Oxazoliumverbindungen, z. B. 2-Ethyl-5-phenyl-1,2-oxazolium-3'-sulfonat und 2-tert-Butyl-5-methylisoxazoliumperchlorat, oder eine geeignete Acylaminoverbindung, z. B. 2-Ethoxy-1-ethoxycarbonyl-1,2-dihydrochinolin, N,N,N',N'-Tetraalkyluroniumverbindungen, wie O-Benzotriazol-1-yl-N,N,N',N'-tetra-methyluronium-hexafluorphosphat, ferner aktivierte Phosphorsäurederivate, z. B. Diphenylphosphorylazid, Diethylphosphorylcyanid, Phenyl-N-phenylphosphoroamidochloridat, Bis-(2-oxo-3-oxazolidinyl)phosphinsäurechlorid oder 1-Benztriazolyloxy-tris-(dimethylamino)-phosphonium-hexafluorophosphat.

Gewünschtenfalls wird eine organische Base zugegeben, z. B. ein Triniederalkylamin mit voluminösen Resten, z. B. Ethyldiisopropylamin, und/oder eine heterocyclische Base, z. B. Pyridin, 4-Dimethylaminopyridin oder bevorzugt N-Methylmorpholin.

Die Kondensation aktivierter Ester, reaktionsfähiger Anhydride oder reaktionsfähiger cyclischer Amide mit den entsprechenden Aminen wird üblicherweise in Gegenwart einer organischen Base, z. B. einfachen Triniederalkylaminen, z. B. Triethylamin oder Tributylamin, oder einer der vorstehend genannten organischen Basen durchgeführt. Gewünschtenfalls wird zusätzlich noch ein Kondensationsmittel verwendet, wie für freie Carbonsäuren beschrieben ist.

Die Kondensation von Säureanhydriden mit Aminen kann z. B. in Gegenwart von anorganischen Carbonaten, z. B. Ammonium- oder Alkalimetallcarbonaten oder -hydrogen-carbonaten, wie Natrium- oder Kaliumcarbonat oder -hydrogencarbonat (üblicherweise zusammen mit einem Sulfat), erfolgen, die Reaktion von Sulfonsäurehalogeniden, wie Sulfonsäurechloriden, in Gegenwart von Hydroxiden, z. B. Alkalimetallhydroxiden, wie Natriumhydroxid oder Kaliumhydroxid.

Carbonsäurehalogenide, beispielsweise das von der Säure der Formel V abgeleitete Chlorkohlensäurederivat, werden mit den entsprechenden Aminen vorzugsweise in Gegenwart eines organischen Amins, z. B. der vorstehend genannten Triniederalkylamine oder heterocyclischen Basen, gegebenenfalls in Gegenwart eines Hydrogensulfates, kondensiert.

Die Kondensation wird vorzugsweise in einem inerten, aprotischen, vorzugsweise wasserfreien, Lösungsmittel oder Lösungsmittelgemisch durchgeführt, beispielsweise in einem Carbonsäureamid, z. B. Formamid oder Dimethylformamid, einem halogenierten Kohlenwasserstoff, z. B. Methylenchlorid, Tetrachlorkohlenstoff oder Chlorbenzol, einem Keton, z. B. Aceton, einem cyclischen Ether, z. B. Tetrahydrofuran, einem Ester, z. B. Essigsäureethylester, oder einem Nitril, z. B. Acetonitril, oder in einer Mischung davon, gegebenenfalls bei erniedrigter oder erhöhter Temperatur, z. B. in einem Temperaturbereich von etwa -40 °C bis etwa +100 °C, bevorzugt von etwa -10 °C bis etwa +50 °C, und ohne Inertgas oder unter Inertgas-, z. B. Stickstoff- oder Argonatmosphäre.

Auch wässrige, beispielsweise alkoholische, z. B. Ethanol, oder aromatische Lösungsmittel oder Lösungsmittelgemische, z. B. Benzol oder Toluol, sind möglich. Bei Gegenwart von Alkalihydroxiden als Basen kann gegebenenfalls auch Aceton zugesetzt werden.

Die Kondensation kann auch gemäss der als Festphasen-Synthese bekannten Technik erfolgen, die auf R. Merrifield zurückgeht und beispielsweise in Angew. Chem. 97, 801 - 812 (1985), Naturwissenschaften 71, 252 - 258 (1984) oder in R. A. Houghten, Proc. Natl. Acad. Sci. USA 82, 5131 - 5135 (1985) beschrieben ist.

Die Freisetzung durch Schutzgruppen geschützter funktioneller Gruppen in den erhaltenen Verbindungen der Formel I mit geschützten Funktionen erfolgt gegebenenfalls nach einer oder mehreren der unter Verfahren a) genannten Methoden.

Die Herstellung einer Ausgangsverbindung der Formel IV geschieht vorzugsweise aus einer Verbindung der Formel VI, die wie unter Verfahren c) beschrieben hergestellt werden kann, indem man entweder mit durch eine Aminoschutzgruppe P₂ N-geschütztem L-Asparagin, z.B. α-(N-Benzyloxycarbonyl-)-L-asparagin, oder einem reaktionsfähigen Säurederivat davon, insbesondere dem p-Nitrophenylester, vorzugsweise in Anwesenheit einer tertiären Stickstoffbase, wie Triethylamin oder N-Ethyldiisopropylamin amidiert, wie oben beschrieben, insbesondere in einem Säureamid, z.B. Dimethylformamid, bei Temperaturen zwischen 0 und 50 °C, vorzugsweise bei Raumtemperatur, wobei man die durch P₂ N-geschützte Verbindung der Formel IV erhält, die durch Abspaltung der Schutzgruppe P₂ unter den für die Schutzgruppenabspaltung aus einer Verbindung der Formel IX genannten Bedingungen in die Verbindung der Formel IV umgewandelt wird.

### Verfahren c) Amidierung (Kondensation zur Herstellung einer Amidbindung)

In Ausgangsmaterialien der Formel VI und/oder VII sind funktionelle Gruppen, die nicht an der Reaktion teilnehmen sollen, erforderlichenfalls durch Schutzgruppen geschützt. Die Schutzgruppen und ihre Einführung sind wie oben unter Verfahren a) beschrieben.

Die freien Carbonsäuren und ihre reaktionsfähigen Derivate und die verwendeten Verfahren zur Amidierung (Kondensation) sind vollständig analog zu den unter Verfahren b) beschriebenen, wenn man anstelle von Aminoverbindungen der Formel IV solche der Formel VI und anstelle von Carbonsäuren der Formel V solche der Formel VII einsetzt.

Die Freisetzung durch Schutzgruppen geschützter funktioneller Gruppen in den erhaltenen Verbindungen der Formel I mit geschützten Funktionen erfolgt gegebenenfalls nach einer oder mehreren der unter Verfahren a) genannten Methoden.

Es ist zu berücksichtigen, dass diese Reaktion unter Umständen durch Acylwanderung des Restes R₁ zu der freien Aminogruppe in der Verbindung der Formel VI behindert wird. Die Verfahrensvariante ist daher vorzugsweise auf solche Ausgangsmaterilien und Reaktionsbedingungen beschränkt, die eine Umsetzung ohne störende Acylwanderung erlauben.

Die Herstellung einer Ausgangsverbindung der Formel VI erfolgt vorzugsweise durch Umsetzung einer Verbindung der oben definierten Formel IX mit einer Carbonsäure der ebenfalls bereits definierten Formel III, oder einem reaktionsfähigen Derivat davon, unter Reaktionsbedingungen analog den für Verfahren a) beschriebenen, wobei man ein durch P₁ geschütztes Derivat einer Verbindung der Formel VI erhält, aus welchem die Schutzgruppe, beispielsweise tert-Butoxycarbonyl oder Benzyloxycarbonyl, vorzugsweise wie bei der Schutzgruppenabspaltung aus einer Verbindung der Formel IX beschrieben, abgespalten wird unter Erhalt der Verbindung der Formel VI.

Eine Ausgangsverbindung der Formel VII wird beispielsweise erhalten, indem man ein carboxygeschütztes Derivat von L-Asparagin, z.B. mit einer der unter Verfahren a) geschützten Carboxyschutzgruppen geschützt, mit Chinolin-2-carbonsäure amidiert unter den oben genannten Amidierungsbedingungen, vorzugsweise den Bedingungen, die zur Herstellung von Verbindungen der Formel II aus einer Verbindung der Formel X nach Abspaltung der Schutzgruppe P₂ genannt worden sind, und die Carboxyschutzgruppe abspaltet, wobei man die Verbindung der Formel VII erhält.

Vorzugsweise wird die Verfahrensvariante c) nicht verwendet, sondern stattdessen eines der Verfahren a) oder b) angewendet, um Verbindungen der Formel I zu erhalten.

Alle weiteren für eines der vorstehend genannten Verfahren genannten Ausgangsverbindungen sind kommerziell erhältlich, bekannt oder nach an sich bekannten Verfahren herstellbar.

Sämtliche genannte Reaktionen können unter an sich bekannten Reaktionsbedingungen, vorzugsweise den oben genannten Bedingungen, bei üblichen Temperaturen, in An-oderAbwesenheit von inerten Lösungs- oder Verdünnungsmitteln durchgeführt werden, beispielsweise in Säureamiden, z.B. Carbonsäuramiden, wie Dimethylformamid, Dimethylacetamid oder 1,3-Dimethyl-3,4,5,6-tetrahydro-2(1H)-pyrimidinon (DMPU), oder Amiden anorganischer Säuren, wie Hexamethylphosphorsäuretriamid, Ethern, z.B. cyclischen Ethern, wie Tetrahydrofuran oder Dioxan, oder acyclischen Ethern, wie Diethylether oder Ethylenglykoldimethylether, halogenierten Kohlenwasserstoffen, wie Haloniederalkanen, z.B. Methylenchlorid oder Chloroform, Ketonen, wie Aceton, Nitrilen, wie Acetonitril, Säureanhydriden, wie Acetanhydrid, Estern, wie Essigsäureethylester, Bisalkansulfinen, wie Dimethylsulfoxid, Stickstoffheterocyclen, wie Pyridin, oder Gemischen dieser Lösungsmittel, insbesondere in wasserfreien Lösungsmitteln oder Lösungsmittelgemischen, wobei die jeweils geeigneten Lösungsmittel für die oben genannten Reaktionen ausgewählt werden können, soweit sinnvoll und zweckmässig, unter Verwendung von Salzen der eingesetzten Verbindungen, insbesondere von Metallsalzen eingesetzter Cabonsäuren, wie den Alkali- oder Erdalkalimetallsalzen, z.B. Natrium- oder Kaliumsalzen, in Ab- oder Anwesenheit von Katalysatoren, Kondensationsmitteln oder neutralisierenden Agentien, je nach Art der Reaktion und/oder der Reaktionsteilnehmer unter atmosphärischem Druck oder in einem geschlossenen Gefäss, unter Normaldruck oder ferner unter erhöhtem Druck, z.B. bei dem Druck, der im Reaktionsgemisch unter den Reaktionsbedingungen in einem geschlossenen Rohr entsteht, und/oder in einer inerten Atmosphäre, z.B. unter einer Argon- oder Stickstoffatmosphäre. Bevorzugt sind Reaktionsbedingungen, die den in den Beispielen genannten analog sind. Auch kann das Acylierungsmittel, z.B. ein Carbonsäurehalogenid oder ein Carbonsäureanhydrid, selbst als Lösungsmittel dienen. Der Reaktionsablauf wird zweckmässig mittels üblicher analytischer Methoden, insbesondere mit Dünnschichtchromatographie, verfolgt.

### Zusätzliche Verfahrensmassnahmen

Bei den zusätzlichen Verfahrensmassnahmen, die gewünschtenfalls durchgeführt werden, können funktionelle Gruppen der Ausgangsverbindungen, die nicht an der Reaktion teilnehmen sollen, erforderlichenfalls in geschützter Form, beispielsweise durch eine oder mehrere der oben unter Verfahren a) genannten Schutzgruppen, vorliegen. Die Schutzgruppen können nach den unter Verfahren a) genannten Methoden abgespalten werden.

Salze von Verbindungen der Formel I können in an sich bekannter Weise hergestellt werden. So kann man Salze von Verbindungen der Formel I mit sauren Gruppen z. B. durch Behandeln mit Metallverbindungen, wie Alkalimetallsalzen von geeigneten organischen Carbonsäuren, z. B. dem Natriumsalz der 2-Ethyl-hexansäure, mit organischen Alkali- oder Erdalkalimetallverbindungen, wie den entsprechenden Hydroxiden, Carbonaten oder Hydrogencarbonaten, wie Natrium- und Kaliumhydroxid, -carbonat oder -hydrogencarbonat, mit entsprechenden Calciumverbindungen oder mit Ammoniak oder einem geeigneten organischen Amin bilden, wobei man vorzugsweise stöchiometrische Mengen oder nur einen kleinen Überschuss des salzbildenden Mittels verwendet. Die bevorzugten Säureadditionssalze von Verbindungen der Formel I erhält man in üblicher Weise, z. B. durch Behandeln mit einer Säure, z.B. einer organischen Säure oder einer anorganischen Säure, oder mit einem geeigneten Ionenaustauscher. Innere Salze von Verbindungen der Formel I, welche saure und basische salzbildende Gruppen enthalten, z.B. eine freie Carboxygruppe und eine freie Aminogruppe, können beispielsweise durch Neutralisieren von Salzen, wie Säureadditionssalzen, auf den isoelektrischen Punkt, z. B. mit schwachen Basen, oder durch Behandeln mit Ionenaustauschern gebildet werden.

Salze können in üblicher Weise in die freien Verbindungen überführt werden, Metall-und Ammoniumsalze beispielsweise durch Behandeln mit geeigneten Säuren oder sauren Ionenaustauschern, und Säureadditionssalze beispielsweise durch Behandeln mit einem geeigneten basischen Mittel oder basischen Ionenaustauschern.

Die Umwandlung eines Salzes einer Verbindung der Formel I in ein anderes Salz erfolgt beispielsweise durch Überführung eines Salzes in die freie Verbindung, wie zuletzt beschrieben, und anschliessende Umwandlung der freien Verbindung in ein anderes Salz, wie zuletzt beschrieben, oder direkt durch Umwandlung eines Salzes in ein anderes, beispielsweise durch Umsalzung mittels Chromatographie, z.B. mittels Gelpermeationschromatographie, oder aus einer Lösung mit einem Überschuss des zur Bildung des neuen Salzes nötigen Gegenions.

Stereoisomerengemische, also Gemische von Diastereomeren und/oder Enantiomeren, wie beispielsweise racemische Gemische, können in an sich bekannter Weise durch geeignete Trennverfahren in die entsprechenden Isomeren aufgetrennt werden. So können Diastereomerengemische durch fraktionierte Kristallisation, Chromatographie, Lösungsmittelverteilung etc. in die einzelnen Diastereomeren aufgetrennt werden. Racemate können nach Überführung der optischen Antipoden in Diastereomere, beispielsweise durch Umsetzung mit optisch aktiven Verbindungen, z. B. optisch aktiven Säuren oder Basen, durch Chromatographie an mit optisch aktiven Verbindungen belegten Säulenmaterialien oder durch enzymatische Methoden, z. B. durch selektive Umsetzung nur eines der beiden Enantiomeren, voneinander getrennt werden. Diese Trennung kann sowohl auf der Stufe eines der Ausgangsprodukte als auch bei den Verbindungen der Formel I selbst erfolgen.

In einer erhältlichen Verbindung der Formel I kann man eine Amino- oder Carboxamidgruppe substituieren, eine in freier oder in reaktionsfähiger Form vorliegende Carboxygruppe verestern oder amidieren bzw. eine veresterte oder amidierte Carboxygruppe in eine freie Carboxygruppe überführen.

Die Substitution einer Carboxamidgruppe oder einer primären oder sekundären Aminogruppe, beispielsweisezu Diniederalkylcarbamoyl oder Mono- oder Di-hydroxyniederalkylcarbamoyl, in Verbindungen der Formel I, in denen der Stickstoff der umzusetzenden Aminogruppen an Wasserstoff gebunden ist, erfolgt z. B. durch Alkylierung.

Geeignete Mittel zur Alkylierung einer Carboxamidgruppe in einer Verbindung der Formel I sind beispielsweise Diazoverbindungen, z. B. Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z. B. in Gegenwart eines Edelmetalls in fein verteilter Form, z. B. Kupfer, oder eines Edelmetallsalzes, z. B. Kupfer(I)-chlorid oder Kupfer(II)-sulfat.

Alkylierungsmittel sind auch in der Deutschen Offenlegungsschrift 2 331 133 genannt, z. B. Alkylhalogenide, Sulfonsäureester, Meerweinsalze oder 1-substituierte 3-Aryltriazene, welche man unter den dort genannten Reaktionsbedingungen mit einer Verbindung der Formel I mit einer Carboxamidgruppe umsetzen kann.

Weitere Alkylierungsmittel sind ausgewählt aus entsprechenden unsubstituierten oder substituierten Niederalkylverbindungen, die zur Einführung wie oben für den Rest R₁ einer N-substituierten Carbaminsäure genannter substituierter oder unsubstituierter Niederalkylreste dienen, die einen zusätzlichen Substituenten X tragen, worin X eine Abgangsgruppe ist. Eine Abgangsgruppe ist insbesondere eine nukleofuge Abgangsgruppe ausgewählt aus mit einer starken anorganischen oder organischen Säure verestertem Hydroxy, wie mit einer Mineralsäure, z. B. Halogenwasserstoffsäure, wie Chlorwasserstoff-, Bromwasserstoff- oder Iodwasserstoffsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trimethansulfon- oder p-Toluolsulfonsäure, verestertes Hydroxy oder mit Stickstoffwasserstoffsäure verestertes Hydroxy.

Beispielsweise kann man eine der Verbindungen mit einem Substituenten X, worin X für eine Abgangsgruppe mit hoher Polarisierbarkeit der Elektronenhülle, z. B. für Brom oder Iod, steht, in einem polaren aprotischen Lösungsmittel, z. B. Aceton, Acetonitril, Nitromethan, Dimethylsulfoxid oder Dimethylformamid, umsetzen. Die Substitutionsreaktion wird gegebenenfalls bei erniedrigter oder erhöhter Temperatur, z. B. in einem Temperaturbereich von etwa -40 ° bis etwa 100 °C, bevorzugt von etwa -10 ° bis etwa 50 °C, und gegebenenfalls unter Inertgas, z. B. Stickstoff- oder Argonatmosphäre, durchgeführt.

Zur Veresterung oder Amidierung einer Carboxygruppe in einer Verbindung der Formel I, beispielsweise zur Amidierung einer freien Carboxygruppe einer Aminosäure, wie Glu oder Asp, mit Ammoniak, Niederalkylamin oder Diniederalkylamin, kann man, wenn erwünscht, die freie Säure verwenden oder die freie Säure in eines der oben genannten reaktionsfahigen Derivate überführen und mit einem Alkohol, Ammoniak, einem primären oder einem sekundären Amin umsetzen, oder man kann zur Veresterung die freie Säure oder ein reaktionsfähiges Salz, z. B. das Cäsiumsalz, mit einem reaktionsfähigen Derivat eines Alkohols umsetzen. Beispielsweise kann man das Cäsiumsalz einer Carbonsäure mit einem dem Alkohol entsprechenden Halogenid oder Sulfonsäureester umsetzen. Die Veresterung der Carboxygruppe kann auch mit anderen üblichen Alkylierungsmitteln erfolgen, z. B. mit Diazomethan, Alkylhalogeniden, Sulfonsäureestern, Meerweinsalzen oder 1-substituierten 3-Aryltriazenen, etc.

Zur Überführung einer veresterten oder amidierten Carboxygruppe in die freie Carboxygruppe kann eine der oben bei der Abspaltung der Carboxyschutzgruppen beschriebenen Methoden oder gewünschtenfalls eine alkalische Verseifung unter üblichen, wie den im Organikum, 17. Auflage, VEB Deutscher Verlag der Wissenschaften, Berlin 1988, genannten Reaktionsbedingungen angewendet werden.

In einer Verbindung der Formel I kann man eine vorhandene freie Aminogruppe acylieren, beispielsweise zur Einführung eines der für R₁ ausser Wasserstoff genannten Reste. Die Acylierung erfolgt nach der oben unter Verfahren a) oder einer der für Schutzgruppen genannten Methoden oder beispielsweise nach einem der im Organikum, 17. Auflage, VEB Deutscher Verlag der Wissenschaften, Berlin (Ost) 1988, genannten Verfahren.

In einer Verbindung der Formel I, worin R₁ einen Aminoacylrest bedeutet, welcher eine primäre oder sekundäre α-Aminogruppe hat, kann die α-Aminogruppe durch einen Rest ausgewählt aus Niederalkyl, Phenylniederalkyl oder Heterocyclylniederalkyl alkyliert werden, indem man beispielsweise durch reduktive Aminierung die Aminogruppe des (erforderlichenfalls an weiteren Gruppen, die nicht an der Reaktion teilnehmen sollen, geschützten) Aminosäurerestes mit einem Niederalkylaldehyd oder -keton, einem Phenylniederalkylaldehyd oder -keton oder einem Heterocyclylniederalkylaldehyd oder -keton, beispielsweise Formaldehyd, Heterocyclylaldehyd, z.B. Furanaldehyd, wie Furan-2-aldehyd, Pyridinaldehyd, wie Pyridin-3-aldehyd, oder Imidazolylaldehyd, wie Imidazol-4-yl-aldehyd (gegebenenfalls N-geschützt, z.B. durch Trityl, welches wie oben beschrieben aus der geschützten Verbindung der Formel I abgespalten werden kann) beispielsweise unter katalytischer Hydrierung, z.B. in Gegenwart eines geeigneten Schwermetallkatalysators, wie Raney-Nickel, bei Normaldruck oder Drucken von 1 bis 100 bar, vorzugsweise bei etwa 100 bar, oder unter Reduktion mittels komplexer Borhydride, wie Natriumcyanoborhydrid, umsetzt.

In einer Verbindung der Formel I, worin R₁ 2-Halogenniederalkanoyl, wie Chloracetyl, bedeutet, kann das Halogenatom erforderlichenfalls, falls es sich insbesondere um Chlor oder Brom handelt, durch Finkelstein-Reaktion in einem polaren aprotischen Lösungsmittel, insbesondere einem Keton, wie Aceton, mit Natriumiodid in die entsprechende Verbindung der Formel I mit 2-Iodniederalkanoyl, insbesondere 2-Iodacetyl, überführt werden. 2-Halogenniederalkanoyl, insbesondere 2-Iodacetyl, kann dann durch Umsetzung mit einem Amino-, Niederalkylamino- oder Diniederalkylamino-niederalkanol oder einem Amino-, Niederalkylamino- oder Diniederalkylamino-niederalkoxyniederalkanol, vorzugsweise in Gegenwart von Basen, wie Carbonaten oder Hydrogencarbonaten, z.B. Alkalimetallcarbonate oder -hydrogencarbonate, wie Natrium- oder Kaliumcarbonat oder Natrium- oder Kaliumhydrogencarbonat, in den zuletzt genannten Lösungsmitteln, oder ferner (insbesondere falls es sich um 2-Chlor- oder 2-Bromniederalkanoyl handelt), auch der entsprechenden (erforderlichenfalls N-geschützten) Alkalimetallalkoholate der Amino-, Niederalkylamino- oder Diniederalkylamino-niederalkanole oder Amino-, Niederalkylamino- oder Diniederalkylamino-niederalkoxyniederalkanole, herstellbar z.B. aus dem entsprechenden Alkohol und einem Alkalimetall, wie Natrium oder Kalium, in einem geeigneten, halogenfreien aprotischen Lösungsmittel in die Verbindung der Formel I überführt werden, worin R₁ Amino-, Niederalkylamino- oder Diniederalkylamino-niederalkoxy-2-niederalkanoyl, wie Dimethylamino-niederalkoxyacetyl, oder Amino-, Niederalkylamino- oder Diniederalkylamino-niederalkoxyniederalkoxy-2-niederalkanoyl, wie Dimethylamino-(2-niederalkoxyethoxy)acetyl, bedeutet.

In einer erhältlichen Verbindung der Formel I, worin die Substituenten die genannten Bedeutungen haben und mindestens eine freie Hydroxygruppe vorhanden ist und die übrigen funktionellen Gruppen in geschützter Form vorliegen, kann man die freie Hydroxygruppe acylieren oder verethern.

Die Acylierung kann erfolgen mit acylierenden Reagentien nach einer der unter Verfahren a) bis e) oder nach einer der für Schutzgruppen genannten Methoden oder nach einem der im Organikum, 17. Auflage, VEB Deutscher Verlag der Wissenschaften, Berlin (Ost) 1988, genannten Verfahren.

Die Veretherung kann mit den oben genannten Alkylierungsmitteln und unter den gleichen Reaktionsbedingungen erfolgen, z. B. mit Diazomethan, Alkylhalogeniden, Sulfonsäureestern, Meerweinsalzen, 1-substituierten 3-Aryltriazenen, etc.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -80°C bis etwa 200°C, vorzugsweise von etwa -20°C bis etwa 150°C, z.B. bei Raumtemperatur bis zu der Rückflusstemperatur, bei Schmelzen bei bis zu 220 °C, unter atmosphärischem Druck oder in einem geschlossenen Gefäss, gegebenenfalls unter Druck, z.B. bei dem Druck, der im Reaktionsgemisch unter den Reaktionsbedingungen in einem geschlossenen Rohr entsteht, und/oder in einer inerten Atmosphäre, z.B. unter einer Argon- oder Stickstoffatmosphäre. Bevorzugt sind die jeweils spezifisch genannten Reaktionsbedingungen.

Lösungs- und Verdünnungsmittel sind beispielsweise Wasser, Alkohole, z.B. Niederalkylhydroxide, wie Methanol, Ethanol oder Propanol, Diole, wie Ethylenglykol, Triole, wie Glycerin, oder Arylalkohole, wie Phenol, Säureamide, z.B. Carbonsäuramide, wie Dimethylformamid, Dimethylacetamid oder 1,3-Dimethyl-3,4,5,6-tetrahydro-2(1H)-pyrimidinon (DMPU), oder Amide anorganischer Säuren, wie Hexamethylphosphorsäuretriamid, Ether, z.B. cyclische Ether, wie Tetrahydrofuran oder Dioxan, oder acyclische Ether, wie Diethylether oder Ethylenglykoldimethylether, halogenierte Kohlenwasserstoffe, wie Haloniederalkane, z.B. Methylenchlorid oder Chloroform, Ketone, wie Aceton, Nitrile, wie Acetonitril, Säureanhydride, wie Acetanhydrid, Ester, wie Essigsäureethylester, Bisalkansulfine, wie Dimethylsulfoxid, Stickstoffheterocyclen, wie Pyridin, Kohlenwasserstoffe, z.B. Niederalkane, wie Heptan, oder Aromaten, wie Benzol oder Toluol, oder Gemische dieser Lösungsmittel, wobei die jeweils geeigneten Lösungsmittel für die oben genannten Reaktionen ausgewählt werden können.

Infolge der engen Beziehung zwischen den Verbindungen der Formel I und ihren Vorstufen in freier Form und in Form von Salzen sind vor- und nachstehend unter den Verbindungen und Zwischenverbindungen sinn- und zweckmässig auch die entsprechenden Salze bzw. freien Verbindungen zu verstehen, sofern die Verbindungen eine oder mehrere salzbildende Gruppen enthalten. Ausgangs- und Zwischenverbindungen können auch in geschützter Form vorliegen, soweit dies erforderlich, ist, wobei die Schutzgruppen zu geeigneten Zeitpunkten abgespalten werden können. Schutzgruppen und ihre Abspaltung sind insbesondere wie oben definiert.

Die Verbindungen, einschliesslich ihrer Salze, können auch in Form von Hydraten erhalten werden, oder ihre Kristalle können z.B. das zur Kristallisation verwendete Lösungsmittel einschliessen.

Die Erfindung betrifft auch diejenigen Ausführungsformen des Verfahrens, bei denen man von einer auf irgend einer Stufe als Zwischenprodukt erhältlichen Verbindung ausgeht und die fehlenden Schritte durchführt oder das Verfahren auf irgendeiner Stufe abbricht oder einen Ausgangsstoff unter den Reaktionsbedingungen bildet oder in Form eines reaktionsfähigen Derivates oder Salzes verwendet oder eine nach dem erfindungsgemässen Verfahren erhältliche Verbindung unter den Verfahrensbedingungen erzeugt und in situ weiterverarbeitet. Dabei geht man vorzugsweise von solchen Ausgangsstoffen aus, die zu den Verbindungen führen, die oben als bevorzugt beschrieben sind.

### Pharmazeutische Präparate:

Die Erfindung betrifft auch pharmazeutische Präparate mit Verbindungen der Formel I (diese werden als Wirkstoff bezeichnet).

Die pharmakologisch verwendbaren Verbindungen der vorliegenden Erfindung können z. B. zur Herstellung von pharmazeutischen Präparaten verwendet werden, welche eine wirksame Menge des Wirkstoffes zusammen oder im Gemisch mit einer signifikanten Menge von anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie nasalen, bukkalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Krankheit sowie der Applikationsweise ab.

Die Erfindung betrifft auch pharmazeutische Präparate und eine Methode zur Behandlung von durch Retroviren verursachten Krankheiten, z. B. von AIDS, insbesondere, wenn HIV-1 oder HIV-2 die Erkrankung verursacht, dadurch gekennzeichnet, dass eine antiretroviral wirksame Menge einer erfindungsgemässen Verbindung der Formel I, insbesondere an einen Warmblüter, z. B. Menschen, der wegen einer der genannten Erkrankungen, insbesondere AIDS, einer derartigen Behandlung bedarf, verabreicht wird. Die an Warmblüter, z. B. Menschen von etwa 70 kg Körpergewicht, zu verabreichenden Dosismengen liegen zwischen etwa 3 mg und etwa 10 g, vorzugsweise zwischen etwa 10 mg und etwa 4 g, z. B. bei ungefahr 25 mg bis 2,0 g pro Person und Tag, verteilt auf vorzugsweise 1 bis 5, insbesondere 1 bis 3 Einzeldosen, die z. B. gleich gross sein können. Üblicherweise erhalten Kinder die halbe Dosis von Erwachsenen.

Die pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z. B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z. B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen oder Dispersionen, und zwar insbesondere isotonische wässrige Lösungen, Dispersionen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z. B. mittels konventioneller Lösungs-oder Lyophilisierungsverfahren, hergestellt. Die genannten Lösungen, Dispersionen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z. B. Laurinsäure, Tridecylsäure, Myristinsäure, Pentadecylsäure, Palmitinsäure, Margarinsäure, Stearinsäure, Arachidinsäure, Behensäure oder entsprechende ungesättigte Säuren, wie z. B. Ölsäure, Elaidinsäure, Erucasäure, Brasidinsäure oder Linolsäure, enthalten, gegebenenfalls unter Zusatz von Antioxidantien, wie z. B. Vitamin E, β-Carotin oder 3,5-Di-tert-butyl-4-hydroxytoluol. Die Alkoholkomponente dieser Fettsäureester hat maximal 6 Kohlenstoffatome und ist ein ein- oder mehrwertiger, z. B. ein-, zwei- oder dreiwertiger Alkohol, z. B. Methanol, Ethanol, Propanol, Butanol oder Pentanol oder deren Isomere, vor allem aber Glycol und Glycerin. Als Fettsäureester sind daher beispielsweise zu nennen: Ethyloleat, Isopropylmyristat, Isopropylpalmitat, "Labrafil M 2375" (Polyoxyethylenglycerintrioleat der Firma Gattefossé, Paris), "Miglyol 812" (Triglycerid gesättigter Fettsäuren der Kettenlänge C₈ bis C₁₂ der Firma Hüls AG, Deutschland), besonders aber vegetabile Öle wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und, wenn erwünscht oder notwendig, nach Zugabe von geeigneten Hilfsstoffen, zu Tabletten, Dragée-Kernen oder Kapseln verarbeitet, oder durch Herstellung von Dispersionen, vorzugsweise mit Phospholipiden, die in Gläschen abgefüllt werden. Dabei kann man die Wirkstoffe auch in Kunststoffträger einbauen, die sie dosiert abgeben oder diffundieren lassen.

Geeignete Trägerstoffe sind insbesondere Füllstoffe, wie Zucker, z. B. Lactose, Saccharose, Mannit oder Sorbit, Cellulosepräparate, und/oder Calciumphosphate, z. B. Tricalciumphosphat oder Calciumhydrogenphosphat, ferner Bindemittel, wie Stärkekleister unter Verwendung z. B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z. B. Kieselsäure, Talk, Stearinsäure oder Salze davon, wie Magnesium- oder Calciumstearat, und/oder Polyethylenglycol. Dragée-Kerne werden mit geeigneten, gegebenenfalls magensaftresistenten Überzügen versehen, wobei man u.a. konzentrierte Zuckerlösungen, welche gegebenenfalls arabischen Gummi, Talk, Polyvinylpyrrolidon, Polyethylenglycol und/oder Titandioxid enthalten, Lacklösungen in geeigneten organischen Lösungsmitteln oder, zur Herstellung von magensaftresistenten Überzügen, Lösungen von geeigneten Cellulosepräparaten, wie Ethylcellulosephthalat oder Hydroxypropylmethylcellulosephthalat, verwendet. Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granulats, z. B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Ölen, Paraffinöl oder flüssigen Polyethylenglykolen, gelöst oder suspendiert, wobei ebenfalls Stabilisatoren und/oder antibakterielle Mittel zugefügt sein können. Den Tabletten oder Dragée-Überzügen und den Kapselhüllen können Farbstoffe oder Pigmente, z. B. zur Identifizierung oder zur Kennzeichnung verschiedener Wirkstoffdosen, beigefügt werden.

Als pharmazeutische Präparate besonders bevorzugt sind durch Phospholipide stabilisierte Dispersionen des Wirkstoffes, vorzugsweise zur oralen Applikation, enthaltend
a) ein Phospholipid oder mehrere Phospholipide der Formel 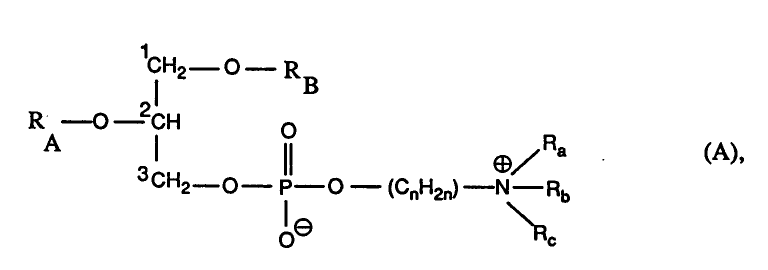 worin R_{A} C₁₀₋₂₀-Acyl, R_{B} Wasserstoff oder C₁₀₋₂₀-Acyl, Rₐ, R_{b} und R_{c} Wasserstoff oder C₁₋₄-Alkyl und n eine ganze Zahl von zwei bis vier darstellen, gewünschtenfalls b) ein weiteres Phospholipid oder mehrere weitere Phospholipide
c) den Wirkstoff und
d) eine pharmazeutisch annehmbare Trägerflüssigkeit und gewünschtenfalls weitere Hilfsstoffe und/oder Konservierungsmittel.

Das Herstellungsverfahren für diese Dispersionen ist dadurch gekennzeichnet, dass man eine Lösung oder Suspension der Komponenten a) und c) oder a), b) und c), vorzugsweise von a) und b) in einem Gewichtsverhältnis von 20 : 1 bis 1 : 5, insbesondere von 5 : 1 bis 1 : 1, durch Verdünnung mit Wasser in eine Dispersion umwandelt, anschliessend das organische Lösungsmittel entfernt, beispielsweise durch Zentrifugation, Gelfiltration, Ultrafiltration oder insbesondere durch Dialyse, z. B. tangentiale Dialyse, vorzugsweise gegen Wasser, die erhaltene Dispersion, vorzugsweise nach Zugabe von Hilfsstoffen oder Konservierungsmitteln, erforderlichenfalls unter Einstellung eines annehmbaren pH-Wertes durch Zugabe von pharmazeutisch annehmbaren Puffern, wie Phosphatsalzen oder organischen Säuren (rein oder gelöst in Wasser), wie Essigssäure oder Zitronensäure, vorzugsweise zwischen pH 3 und 6, z. B. pH 4 - 5, falls sie nicht bereits die richtige Wirkstoffkonzentration hat, konzentriert, vorzugsweise auf eine Wirkstoffkonzentration von 2 bis 30 mg/ml, insbesondere von 10 bis 20 mg/ml, wobei die Konzentrierung vorzugsweise nach den zuletzt genannten Methoden zur Entfernung eines organischen Lösungsmittels erfolgt, insbesondere durch Ultrafiltration, z. B. unter Verwendung einer Apparatur zur Durchführung tangentialer Dialyse und Ultrafiltration.

Die nach diesem Verfahren herstellbare, durch Phospholipide stabilisierte Dispersion ist bei Zimmertemperatur mindestens mehrere Stunden stabil, reproduzierbar bezüglich des Mengenanteils der Komponenten und toxikologisch unbedenklich und daher insbesondere für die orale Applikation am Menschen geeignet.

Die Grössenordnung der erhaltenen Partikel in der Dispersion ist variabel und liegt vorzugsweise zwischen ca. 1,0 x 10⁻⁸ bis ca. 1,0 x 10⁻⁵ m, insbesondere zwischen etwa 1 x 10⁻⁷ und etwa 2 x 10⁻⁶ m.

Die Nomenklatur der Phospholipide der Formel I und die Bezifferung der C-Atome erfolgt anhand der in Eur. J. of Biochem. 79, 11-21 (1977) "Nomenclature of Lipids" von der IUPAC-IUB Commission on Biochemical Nomenclature (CBN) gegebenen Empfehlungen (sn-Nomenklatur, stereospecific numbering).

In einem Phospholipid der Formel A sind R_{A} und R_{B} mit den Bedeutungen C₁₀₋₂₀-Acyl vorzugsweise geradkettiges C₁₀₋₂₀-Alkanoyl mit einer geraden Anzahl an C-Atomen und geradkettiges C₁₀₋₂₀-Alkenoyl mit einer Doppelbindung und einer geraden Anzahl an C-Atomen.

Geradkettiges C₁₀₋₂₀-Alkanoyl R_{A} und R_{B} mit einer geraden Anzahl an C-Atomen sind beispielsweise n-Dodecanoyl, n-Tetradecanoyl, n-Hexadecanoyl oder n-Octadecanoyl.

Geradkettiges C₁₀₋₂₀-Alkenoyl R_{A} und R_{B} mit einer Doppelbindung und einer geraden Anzahl an C-Atomen sind beispielsweise 6-cis-, 6-trans-, 9-cis- oder 9-trans-dodecenoyl, -tetradecenoyl, -hexadecenoyl, -octadecenoyl oder -icosenoyl, insbesondere 9-cis-octadecenoyl (Oleoyl).

In einem Phospholipid der Formel A ist n eine ganze Zahl von zwei bis vier, vorzugsweise zwei. Die Gruppe der Formel -(CₙH₂ₙ)- stellt unverzweigtes oder verzweigtes Alkylen dar, z.B. 1,1-Ethylen, 1,1-, 1,2- oder 1,3-Propylen oder 1,2-, 1,3- oder 1,4-Butylen. Bevorzugt ist 1,2-Ethylen (n=2).

Phospholipide der Formel A sind beispielsweise natürlich vorkommende Kephaline, worin Rₐ, R_{b} und R_{c} Wasserstoff bedeuten oder natürlich vorkommende Lecithine, worin Rₐ, R_{b} und R_{c} Methyl bedeuten, z.B. Kephalin oder Lecithin aus Sojabohnen, Rinderhirn, Rinderleber oder Hühnerei mit verschiedenen oder identischen Acylgruppen R_{A} und R_{B} oder Mischungen davon.

Bevorzugt sind synthetische, im wesentlichen reine Phospholipide der Formel A mit verschiedenen oder identischen Acylgruppen R_{A} und R_{B}.

Der Begriff "synthetisches" Phospholipid der Formel A definiert Phospholipide, welche bezüglich R_{A} und R_{B} eine einheitliche Zusammensetzung haben. Solche synthetischen Phospholipide sind vorzugsweise die unten definierten Lecithine und Kephaline, deren Acylgruppen R_{A} und R_{B} eine definierte Struktur haben und von einer definierten Fettsäure mit einem Reinheitsgrad höher als ca. 95 % abgeleitet sind. R_{A} und R_{B} können gleich oder verschieden und ungesättigt oder gesättigt sein. Bevorzugt ist R_{A} gesättigt, z.B. n-Hexadecanoyl, und R_{B} ungesättigt, z.B. 9-cis-Octadecenoyl (= Oleoyl).

Der Begriff "natürlich vorkommende" Phospholipide der Formel A definiert Phospholipide, welche bezüglich R_{A} und R_{B} keine einheitliche Zusammensetzung haben. Solche natürlichen Phospholipide sind ebenfalls Lecithine und Kephaline, deren Acylgruppen R_{A} und R_{B} strukturell undefinierbar und von natürlich vorkommenden Fettsäuregemischen abgeleitet sind.

Der Begriff "im wesentlichen reines" Phospholipid definiert einen Reinheitsgrad von mehr als 70 % (Gew.) des Phospholipids der Formel A, welcher anhand geeigneter Bestimmungsmethoden, z.B. papierchromatographisch, nachweisbar ist.

Besonders bevorzugt sind synthetische, im wesentlichen reine Phospholipide der Formel A, worin R_{A} die Bedeutung geradkettiges C₁₀₋₂₀-Alkanoyl mit einer geraden Anzahl an C-Atomen und R_{B} die Bedeutung geradkettiges C₁₀₋₂₀-Alkenoyl mit einer Doppelbindung und einer geraden Anzahl an C-Atomen haben. Rₐ, R_{b} und R_{c} sind Methyl und n ist zwei.

In einem besonders bevorzugten Phospholipid der Formel A bedeuten R_{A} n-Dodecanoyl, n-Tetradecanoyl, n-Hexadecanoyl oder n-Octadecanoyl und R_{B} 9-cis-Dodecenoyl, 9-cis-Tetradecenoyl, 9-cis-Hexadecenoyl, 9-cis-Octadecenoyl oder 9-cis-Icosenoyl. Rₐ, R_{b} und R_{c} sind Methyl und n ist zwei.

Ein ganz besonders bevorzugtes Phospholipid der Formel A ist synthetisches 1-n-Hexadecanoyl-2-(9-cis-octadecenoyl)-3-sn-phosphatidylcholin mit einer Reinheit von mehr als 95 %.

Bevorzugte natürliche, im wesentlichen reine Phospholipide der Formel A sind insbesondere Lecithin (L-α-Phosphatidylcholin) aus Sojabohnen oder Hühnerei.

Für die Acylreste in den Phospholipiden der Formeln A sind auch die in Klammern angegebenen Bezeichnungen gebräuchlich: 9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 11-cis-Octadecenoyl (Vaccenoyl), 9-cis-Icosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl).

Weitere Phospholipide sind vorzugsweise Ester von Phosphatidsäure (3-sn-Phosphatidsäure) mit den genannten Aclyresten, wie Phosphatidylserin und Phosphatidylethanolamin.

Schwerlösliche Wirkstoffe können auch als wasserlösliche, pharmazeutisch annehmbare Salze vorliegen, wie oben definiert.

In der Trägerflüssigkeit d) sind die Komponenten a), b) und c) oder a) und c) als Liposomen so enthalten, dass sich mehrere Tage bis Wochen keine Feststoffe oder feste Aggregate wie Mizellen zurückbilden und die Flüssigkeit mit den genannten Komponenten, gegebenenfalls nach Filtration, vorzugsweise oral, applizierbar ist.

In der Trägerflüssigkeit d) können pharmazeutisch annehmbare, nicht toxische Hilfsstoffe enthalten sein, z.B. wasserlösliche Hilfsstoffe welche zur Herstellung von isotonischen Bedingungen geeignet sind, z.B. ionische Zusätze wie Kochsalz oder nichtionische Zusätze (Gerüstbildner) wie Sorbit, Mannit oder Glucose oder wasserlösliche Stabilisatoren für die Liposomendispersion wie Lactose, Fructose oder Sucrose.

Zusätzlich zu den wasserlöslichen Hilfsstoffen können in der Trägerflüssigkeit für flüssige pharmazeutische Formulierungen verwendbare Emulgatoren, Netzmittel oder Tenside vorhanden sein, insbesondere Emulgatoren wie Ölsäure, nichtionische Tenside vom Typ Fettsäure-Polyhydroxyalkoholester wie Sorbitanmonolaurat, -oleat, -stearat oder -palmitat, Sorbitantristearat oder -trioleat, Polyoxyethylen-Addukte von Fettsäure-Polyhydroxyalkoholestern wie Polyoxyethylen-sorbitanmonolaurat, -oleat, -stearat, -palmitat, -tristearat oder -trioleat, Polyethylenglycol-Fettsäureester wie Polyoxyethylstearat, Polyethylenglycol-400-stearat, Polyethylenglycol-2000-stearat, insbesondere Ethylenoxid-Propylenoxid Blockpolymere vom Typ Pluronic® (Wyandotte Chem. Corp.) oder Synperonic® (ICI).

Bevorzugte Konservierungsmittel sind z. B. Antioxidantien, wie Ascorbinsäure, oder Microbizide, wie Sorbinsäure oder Benzoesäure.

Die folgenden Beispiele dienen zur Illustration der Erfindung, schränken deren Umfang jedoch in keiner Weise ein.

Temperaturen werden in Celsiusgraden (°C) angegeben. Sofern keine Temperatur angegeben ist, erfolgt die Reaktion bei Raumtemperatur. Die R_{f}-Werte, die das Verhältnis von Laufstrecke der jeweiligen Substanz zur Laufstrecke der Laufmittelfront angeben, werden auf Kieselgeldünnschichtplatten durch Dünnschichtchromatographie (DC) in folgenden Lösungsmittelsystemen ermittelt:
- A: Essigsäureethylester
- B: Methylenchlorid/Ethanol/Triethylamin 90:10:2
- C: Methylenchlorid/THF 1:1

Die Abkürzung "R_{f}(A)" bedeutet beispielsweise, dass der R_{f}-Wert im Lösungsmittelsystem A ermittelt wurde.

Das Mengenverhältnis von Lösungs- und Elutionsmitteln zueinander ist stets in Volumenanteilen angegeben.

### HPLC-Gradienten:

- I: 20 % → 100 % a) in b) während 35 min.
- II: 20 % → 100 % a) in b) während 20 min.

Laufmittel a): Acetonitril + 0,05 % TFA; Laufmittel b): Wasser + 0,05 % TFA. Säule (250 x 4,6mm) gefüllt mit "Reversed-Phase"-Material C₁₈-Nucleosil® (5 µm mittlere Korngrösse, mit Octadecylsilanen kovalent derivatisiertes Silicagel, Macherey & Nagel, Düren, BRD). Detektion durch UV-Absorption bei 215 nm. Die Retentionszeiten (t_{Ret}) werden in Minuten angegeben. Fliessgeschwindigkeit 1 ml/min.

Die weiteren verwendeten Kurzbezeichnungen und Abkürzungen haben die folgenden Bedeutungen:
- abs.: absolut (gibt an, dass Lösungsmittel wasserfrei ist)
- atm: physikalische Atmosphären
(Druckeinheit) - 1 atm entspricht 1,013 bar
- Boc: tert-Butoxycarbonyl
- BOP: Benzotriazol-1-yl-oxy-tris-(di-methylamino)-phosponium-hexafluor-phosphat
- DC: Dünnschichtchromatographie
- DIPE: Diisopropylether
- DMAP: 4-Dimethylaminopyridin
- DMF: Dimethylformamid
- ges.: gesättigt
- h: Stunde(n)
- HOBt: 1-Hydroxy-benztriazol
- HPLC: Hochleistungs-Flüssigchromatographie
- HV: Hochvakuum
- min: Minute(n)
- MS: Massenspektroskopie
- NMM: N-Methyl-morpholin
- RT: Raumtemperatur
- Sole: gesättigte Natriumchloridlösung
- TFA: Trifluoressigsäure
- THF: Tetrahydrofuran
- Z: Benzyloxycarbonyl

Massenspektroskopische Messwerte werden entweder durch konventionelle MS oder nach der "Fast-Atom-Bombardment" (FAB-MS)-Methode erhalten. Die Massenangaben beziehen sich im ersten Fall auf das unprotonierte Molekülion (M)⁺ oder das protonierte Molekülion (M+H)⁺.

Die Werte für Protonen-Kernresonanzspektroskopie (¹H-NMR) werden in ppm (parts per million) bezogen auf Tetramethylsilan als internen Standard angegeben. s = Singulett, d = Dublett, t = Triplett, q = Quartett, m = Multiplett, dd = Doppeldublett.

### Beispiel 1: N-tert.-Butyl-decahydro-2-[2(R)-acetyloxy-4-phenyl-3(S)-[[N-(2-chinolylcarbonyl)-L-asparaginyl]amino]butyl]-(4aS,8aS)-isochinolin-3(S)-carboxamid

Unter Stickstoffatmosphäre wird eine Lösung von 81 mg (0.12 mMol) N-tert.-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid in 2 ml THF abs. und 50 µl (0.36 mMol) Triethylamin bei RT mit 2 Kristallen DMAP und 17 µl (0.18 mMol) Acetanhydrid versetzt. Nach 18 h giesst man das Reaktionsgemisch auf Eis, zieht mit 3 Portionen Essigsäureethylester aus, wäscht die organischen Phasen mit ges. NaHCO₃-Lösung, Wasser und Sole, trocknet sie mit Na₂SO₄ und dampft sie ein. Digerieren des Rohproduktes in DIPE liefert die Titelverbindung: DC R_{f}(A)=0,44; t_{Ret}=18,9 min; FAB-MS (M+H)⁺=713;
¹H-NMR (200 MHz, CD₃OD): 1.34 (s, (H₃C)₃C), 1.2-2.0 (m, ca. 13 H), 2.08 (s, H₃CCO), 2.2-2.44 (m, 2 H), 2.6-2.8 (m, 5 H), 2.95-3.13 (m, 2 H), 4.43 (m, HC), 5.30 (m, HC), 6.89 (m, 1H), 7.04 (t, J=7 Hz, 2 H), 7.24 (d, J=7 Hz, 2 H), 7.70 (m, 1 H), 7.85 (m, 1 H), 8.02 (d, J=7 Hz, 1 H), 8.17 (m, 2 H), 8.47 (d, J=7 Hz, 1 H).

Die Ausgangsmaterialien werden wie folgt hergestellt:

### 1a) N-tert.-Butyl-decahydro-2-[2(R)-hydroxy-4-phenyl-3(S)-[tert.-butoxycarbonylamino]butyl]-(4aS,8aS)-isochinolin-3(S)-carboxamid

Unter Stickstoffatmosphäre werden in einer Ampulle 1,105 g (4.195 mMol) 2(S)-[1(S)-(Boc-amino)-2-phenylethyl]-oxiran (Herstellung analog B.K. Handa, P.J. Machin, S. Redshaw, G.J. Thomas - European Patent Appln. 0346847 (1989), wobei anstelle der Benzyloxycarbonyl-Schutzgruppe die tert-Butoxycarbonylgruppe verwendet wird, oder nach Evans et al., J. Org. Chem. 50, 4615-4625 (1985)) und 1.18 g (4.95mMol) N-tert.-Butyl-decahydro-(4aS,8aS)-isochinolin-3(S)-carboxamid (Herstellung siehe K.E.B. Parkes, S. Redshaw, G.J. Thomas, European Patent Appln. 0432694A2 (1990)) in 21 ml Ethanol während 16 h auf 90°C erhitzt. Säulenchromatographie (SiO₂, Hexan/Essigsäureethylester 5:1 + 2 % Triethylamin) des Eindampfrückstandes liefert die Titelverbindung: t_{Ret}=18,1 min; FAB-MS (M+H)⁺=502; ¹H-NMR (200 MHz, CD₃OD): 1.1-2.23 (m, 14 H), 1.26 und 1.33 (2 s, 2 (H₃C)₃C), 2.57-2.70 (m, 2 H), 2.73-2.84 (m, 1 H), 2.93-3.14 (m, 2 H), 3.73-3.90 (m, 2 H), 7.1-7.3 (m, HC_{arom.}).

### 1b) N-tert.-Butyl-decahydro-2-[2(R)-hydroxy-4-phenyl-3(S)-aminobutyl]-(4aS,8aS)-isochinolin-3(S)-carboxamid · HCl

Unter Stickstoffatmosphäre löst man 0.6 g (1.2 mMol) N-tert.-Butyl-decahydro-2-[2(R)-hydroxy-4-phenyl-3(S)-[tert.-butoxycarbonylamino]butyl]-(4aS,8aS)-isochinolin-3(S)-carboxamid in 12 ml Dioxan, versetzt mit 12 ml 4N HCl-Lösung in Dioxan und rührt während 2 h bei RT. Lyophilisation liefert die Titelverbindung: ¹H-NMR (300 MHz, CD₃OD): 1.37 (s, (H₃C)₃C), 1.2-1.9 (m, 10 H), 1.9-2.2 (m, 4 H), 2.81-3.03 (m, 2 H), 3.14-3.27 (m, 2 H), 3.44 (m, 1 H), 4.07 (m, 1 H), 4.53 (m, 1 H), 7.27-7.43 (m, HC_{arom.}).

Alternativer Syntheseweg: Die Titelverbindung in freier Form (kein HCl-Salz) wird erhalten, indem man 109 mg (0.217 mMol) der Titelverbindung aus Beispiel 1 a), gelöst in 0,75 ml konzentrierter Ameisensäure, rührt. Nach 16 h bei RT dampft man die Ameisensäure am Hochvakuum ab und verteilt den Rückstand zwischen 3 Protionen Essigsäureethylester, ges. NaHCO₃-Lösung und Sole. Trocknen der organischen Phasen mit Na₂SO₄ und Eindampfen liefert die Titelverbindung: ¹H-NMR (200 MHz, CD₃OD): u. a. 1,32 (s, 9H); 2,27 (d, 12 Hz, 1H); 2,37 (dd, 12 Hz, 4 Hz, 1H); 2,57-2,78 (m, 3H), 2,99 (d, 12 Hz, 1H), 3.1-3.4 (m, 2H), 3.68 (m, 1H).

### 1c) N-tert.-Butyl-decahydro-2-[2(R)-hydroxy-4-phenyl-3(S)-[[N-benzyloxycarbonyl-L-asparaginyl]amino]butyl]-(4aS,8aS)-isochinolin-3(S)-carboxamid

Gelöst in 13 ml NMM 0.3 N in DMF und 2.24 ml (13 mMol) N-Ethyldiisopropylamin werden 0.624 g (1.35 mMol) N-tert.-Butyl-decahydro-2-[2(R)-hydroxy-4-phenyl-3(S)-amino]butyl]-(4aS,8aS)-isochinolin-3(S)-carboxamid · HCl mit 0.762 g (1.97 mMol) Z-Asparagin-p-nitrophenylester (Bachem, Bubendorf/Schweiz) während 4 h bei RT umgesetzt. Das Reaktionsgemisch wird am HV eingedampft und der Rückstand verteilt zwischen 3 Portionen Methylenchlorid, 2 Portionen 5%-iger K₂CO₃-Lösung und Sole. Mehrmaliges Lösen in wenig DMF und Fällen mit eiskaltem DIPE liefert die Titelverbindung: t_{Ret}=15,7 min; FAB-MS (M+H)⁺=650.

### 1d) N-tert.-Butyl-decahydro-2-[2(R)-hydroxy-4-phenyl-3(S)-[[L-asparapinyl]amino]- butyl]-(4aS,8aS)-isochinolin-3(S)-carboxamid

Gelöst in 20 ml Methanol werden 0.69 g (1.06 mMol) N-tert.-Butyl-decahydro-2-[2(R)-hydroxy-4-phenyl-3(S)-[[N-benzyloxycarbonyl-L-asparaginyl]amino]butyl]-(4aS,8aS)-isochinolin-3(S)-carboxamid in Gegenwart von 140 mg 10 % Palladium auf Kohle bei 1 atm Wasserstoffdruck und RT während 5 h hydriert. Abfiltrieren des Katalysators und Eindampfen liefert die Titelverbindung: ¹H-NMR (300 MHz, CD₃OD): 1.33 (s, (H₃C)₃C), 1.2-2.24 (m, 15 H), 2.45 (dd, J₁=15 Hz, J₂=5 Hz, 1 H), 2.55-2.80 (m, 3 H), 3.02-3.14 (m, 2 H), 3.54 (dd, J₁=8 Hz, J₂=5 Hz, 1 H), 3.87 (m, 1 H), 4.22-4.32 (m, 1 H), 7.1-7.34 (m, HC_{arom.}).

### 1e) N-tert.-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid

Eine Lösung von 246 mg (1.42 mMol) Chinaldinsäure, 487 mg (0.945 mMol) N-tert.-Butyl-decahydro-2-[2(R)-hydroxy-4-phenyl-3(S)-[[L-asparaginyl]amino]butyl]-(4aS,8aS)-isochinolin-3(S)-carboxamid, 627 mg (1.42 mMol) BOP und 192 mg (1.42 mMol) HOBT wird bei RT in 7.5 ml 0.3 M NMM in DMF gelöst und 18 h bei RT gerührt. Das Reaktionsgemisch wird am HV eingedampft und der Rückstand verteilt zwischen 3 Portionen Methylenchlorid, 2 Portionen ges. Natriumbicarbonatlösung und Sole. Trocknen der vereinigten organischen Phasen über Natriumsulfat, Eindampfen und Säulenchromatographie (SiO₂, Methylenchlorid/Ethanol/Essigsäure 90:10:1 → Methylenchlorid/Ethanol/NH_{3aq} 90:10:1) des Rückstandes liefert die Titelverbindung: DC R_{f}(B)=0,50; t_{Ret}=16,0min; FAB-MS (M+H)⁺=671, ¹H-NMR (300 MHz, CD₃OD): 1.31 (s, (H₃C)₃C), 1.1-2.4 (m, 14 H), 2.6-2.9 (m, 5 H), 3.04 (m, 2 H), 3.92 (m, 1 H), 4.23 (m, 1 H), 4.92 (t, J=7 Hz, 1 H), 6.74 (t, J=7 Hz, 1 H), 6.91 (t, J=8 Hz, 2 H), 7.19 (d, J=8 Hz, 2 H), 7.69 (m, 1 H), 7.84 (m, 1 H), 7.99 (t, J=8 Hz, 1 H), 8.14 (d, J=9 Hz, 1 H), 8.17 (d, J=9 Hz, 1 H), 8.48 (d, J=9 Hz, 1 H).

Alternativ kann die Titelverbindung auch hergestellt werden, indem man (analog EP 0 432 695, publiziert am 19.06.1991, Beispiel 3) eine Lösung von N-(2-Chinolylcarbonyl-*M8M* L-asparagin und N-tert.-Butyl-decahydro-2-[2(R)-hydroxy-4-phenyl-3(S)-aminobutyl]-(4aS,8aS)-isochinolin-3(S)-carboxamid (herstellbar wie in dem Alternativverfahren unter 1 b) beschrieben) in Tetrahydrofuran auf - 10 °C kühlt und 3-Hydroxy-1,2,3-benzotriazin-4(3H)on (Fluka, Schweiz) und Dicyclohexylcarbodiimid zugibt, die Mischung bei der genannten Temperatur für 2 h und bei 20 ⁰C für weitere 16 h rührt, dann mit Essigsäureethylester verdünnt und filtriert. Das Filtrat wird dann mit gesättigter Natriumbicarbonatlösung und Sole gewaschen und eingedampft. Der Rückstand wird über eine Kieselgelsäule mit 4 Volumenprozent Methanol in Dichlormethan gereinigt und die Titelverbindung erhalten.

### Beispiel 2: N-tert.-Butyl-decahydro-2-[2(R)-(furan-2-carbonyloxy)-4-phenyl-3(S)-[[N-(2-chinolylcarbonyl)-L-asparaginyl]amino]butyl]-(4aS,8aS)-isochinolin-3(S)-carboxamid

40 mg (0.06 mMol) N-tert.-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid (Beispiel 1e) werden mit einer Lösung von 8.8 µl (0.06 mMol) Furan-2-carbonsäurechlorid, 0.5 mg DMAP, 34 µl (0.2 mMol) N-Ethyldiisopropylamin in 5 ml Dioxan versetzt. Nach 2 Tagen bei RT verteilt man zwischen 3 Portionen Essigsäureethylester, NaHCO₃ ges. und Sole. Trocknen der org. Phasen mit Natriumsulfat, Eindampfen und Säulenchromatographie (SiO₂, Methylenchlorid/THF 5:1) liefert die Titelverbindung: t_{Ret}=19.0 min; FAB-MS (M+H)⁺=765.

### Beispiel 3:

Analog zu einem der oben angeführten Beispiele oder nach den genannten Verfahren können auch die folgenden Acyl-Derivate von N-tert.-Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid hergestellt werden:
3a) N-tert.-Butyl-decahydro-2-[2(R)-pivaloyloxy-4-phenyl-3(S)-[[N-(2-chinolylcarbonyl)-L-asparaginyl]amino]butyl]-(4aS,8aS)-isochinolin-3(S)-carboxamid,
3b) N-tert.-Butyl-decahydro-2-[2(R)-(N-methylaminoacetyloxy)-4-phenyl-3(S)-[[N-(2-chinolylcarbonyl)-L-asparaginyl]amino]butyl]-(4aS,8aS)-isochinolin-3(S)-carboxamid,
3c) N-tert.-Butyl-decahydro-2-[2(R)-(N-benzyloxycarbonyl-N-methylaminoacetyloxy)-4-phenyl-3(S)-[[N-(2-chinolylcarbonyl)-L-asparaginyl]amino]butyl]-(4aS,8aS)-isochinolin-3(S)-carboxamid,
3d) N-tert.-Butyl-decahydro-2-[2(R)-(N-dimethylaminoacetyloxy)-4-phenyl-3(S)-[[N-(2-chinolylcarbonyl)-L-asparaginyl]amino]butyl]-(4aS,8aS)-isochinolin-3(S)-carboxamid,
3e) N-tert.-Butyl-decahydro-2-[2(R)-(N-n-butyl-N-methylaminoacetyloxy)-4-phenyl-3(S)-[[N-(2-chinolylcarbonyl)-L-asparaginyl]amino]butyl]-(4aS,8aS)-isochinolin-3(S)-carboxamid,
3f) N-tert.-Butyl-decahydro-2-[2(R)-(N-methyl-N-benzylaminoacetyloxy)-4-phenyl-3(S)-[[N-(2-chinolylcarbonyl)-L-asparaginyl]amino]butyl]-(4aS,8aS)-isochinolin-3(S)-carboxamid,
3g) N-tert.-Butyl-decahydro-2-[2(R)-(N-methyl-N-3-pyridylmethyl-aminoacetyloxy)-4-phenyl-3(S)-[[N-(2-chinolylcarbonyl)-L-asparaginyl]amino]butyl]-(4aS,8aS)-isochinolin-3(S)-carboxamid,
3h) N-tert.-Butyl-decahydro-2-[2(R)-(3-pyridylcarbonyloxy)-4-phenyl-3(S)-[[N-(2-chinolylcarbonyl)-L-asparaginyl]amino]butyl]-(4aS,8aS)-isochinolin-3(S)-carboxamid, und/oder
3i) N-tert.-Butyl-decahydro-2-[2(R)-(4-(N-morpholinyl-methyl)-benzoyloxy)-4-phenyl-3(S)-[[N-(2-chinolylcarbonyl)-L-asparaginyl]amino]butyl]-(4aS,8aS)-iso-chinolin-3(S)-carboxamid.

### Beispiel 4:

Analog zu einem der oben angeführten Beispiele oder nach den genannten Verfahren können auch die folgenden Acyl-Derivate von N-tert.-Butyl-decahydro-2-[2(R)-hydroxy4-phenyl-3(S)-[[N-(2-chinolylcarbonyl)-L-asparaginyl]amino]butyl]-(4aS,8aS)-isochinolin-3(S)-carboxamid hergestellt werden:
4a) N-tert.-Butyl-decahydro-2-[2(R)-propionyloxy-4-phenyl-3(S)-[[N-(2-chinolylcarbonyl)-L-asparaginyl]amino]butyl]-(4aS,8aS)-isochinolin-3(S)-carboxamid;
4b) N-tert.-Butyl-decahydro-2-[2(R)-butyryloxy-4-phenyl-3(S)-[[N-(2-chinolylcarbonyl)-L-asparaginyl]amino]butyl]-(4aS,8aS)-isochinolin-3(S)-carboxamid,
4c) N-tert.-Butyl-decahydro-2-[2(R)-methylpropionyloxy-4-phenyl-3(S)-[[N-(2-chinolylcarbonyl)-L-asparaginyl]amino]butyl]-(4aS,8aS)-isochinolin-3(S)-carboxamid,
4d) N-tert.-Butyl-decahydro-2-[2(R)-pentanoyloxy-4-phenyl-3(S)-[[N-(2-chinolylcarbonyl)-L-asparaginyl]amino]butyl]-(4aS,8aS)-isochinolin-3(S)-carboxamid,
4e) N-tert.-Butyl-decahydro-2-[2(R)-octanoyloxy-4-phenyl-3(S)-[[N-(2-chinolylcarbonyl)-L-asparaginyl]amino]butyl]-(4aS,8aS)-isochinolin-3(S)-carboxamid,
4f) N-tert.-Butyl-decahydro-2-[2(R)-decanoyloxy-4-phenyl-3(S)-[[N-(2-chinolylcarbonyl)-L-asparaginyl]amino]butyl]-(4aS,8aS)-isochinolin-3(S)-carboxamid,
4g) N-tert.-Butyl-decahydro-2-[2(R)-dodecanoyloxy-4-phenyl-3(S)-[[N-(2-chinolylcarbonyl)-L-asparaginyl]amino]butyl]-(4aS,8aS)-isochinolin-3(S)-carboxamid,
4h) N-tert.-Butyl-decahydro-2-[2(R)-palmitoyloxy-4-phenyl-3(S)-[[N-(2-chinolylcarbonyl)-L-asparaginyl]amino]butyl]-(4aS,8aS)-isochinolin-3(S)-carboxamid,
4i) N-tert.-Butyl-decahydro-2-[2(R)-(3-carboxypropionyloxy)-4-phenyl-3(S)-[[N-(2-chinolylcarbonyl)-L-asparaginyl]amino]butyl]-(4aS,8aS)-isochinolin-3(S)-carboxamid,
4j N-tert.-Butyl-decahydro-2-[2(R)-(3-methoxypropionyloxy)-4-phenyl-3(S)-[[N-(2-chinolylcarbonyl)-L-asparaginyl]amino]butyl]-(4aS,8aS)-isochinolin-3(S)-carboxamid,
4k) N-tert.-Butyl-decahydro-2-[2(R)-benzyloxyacetyloxy-4-phenyl-3(S)-[[N-(2-chinolylcarbonyl)-L-asparaginyl]amino]butyl]-(4aS,8aS)-isochinolin-3(S)-carboxamid,
4l) N-tert.-Butyl-decahydro-2-[2(R)-{(S)-α-methoxy-α-phenylacetyloxy}-4-phenyl-3(S)-[[N-(2-chinolylcarbonyl)-L-asparaginyl]amino]butyl]-(4aS,8aS)-isochinolin-3(S)-carboxamid,
4m) N-tert.-Butyl-decahydro-2-[2(R)-{(R)-α-methoxy-α-phenylacetyloxy}-4-phenyl-3(S)-[[N-(2-chinolylcarbonyl)-L-asparaginyl]amino]butyl]-(4aS,8aS)-isochinolin-3(S)-carboxamid,
4n) N-tert.-Butyl-decahydro-2-[2(R)-{2-(2-methoxyethoxy)acetyloxy}-4-phenyl-3(S)-[[N-(2-chinolylcarbonyl)-L-asparaginyl]amino]butyl]-(4aS,8aS)-isochinolin-3(S)-carboxamid,
4o) N-tert.-Butyl-decahydro-2-[2(R)-{n-butyloxyacetyloxy}-4-phenyl-3(S)-[[N-(2-chinolylcarbonyl)-L-asparaginyl]amino]butyl]-(4aS,8aS)-isochinolin-3(S)-carboxamid,
4p) N-tert.-Butyl-decahydro-2-[2(R)-{2-(2-(2-methoxyethoxy)ethoxy)acetyloxy}-4-phenyl-3(S)-[[N-(2-chinolylcarbonyl)-L-asparaginyl]amino]butyl]-(4aS,8aS)-isochinolin-3(S)-carboxamid,
4q) N-tert.-Butyl-decahydro-2-[2(R)-{2-pyridylacetyloxy}-4-phenyl-3(S)-[[N-(2-chinolylcarbonyl)-L-asparaginyl]amino]butyl]-(4aS,8aS)-isochinolin-3(S)-carboxamid,
4r) N-tert.-Butyl-decahydro-2-[2(R)-{3-(2-pyridyl)propionyloxy}-4-phenyl-3(S)-[[N-(2-chinolylcarbonyl)-L-asparaginyl]amino]butyl]-(4aS,8aS)-isochinolin-3(S)-carboxamid,
4s) N-tert.-Butyl-decahydro-2-[2(R)-{4-imidazolylcarbonyloxy}-4-phenyl-3(S)-[[N-(2-chinolylcarbonyl)-L-asparaginyl]amino]butyl]-(4aS,8aS)-isochinolin-3(S)-carboxamid,
4t) N-tert.-Butyl-decahydro-2-[2(R)-{4-imidazolylacetyloxy}-4-phenyl-3(S)-[[N-(2-chinolylcarbonyl)-L-asparaginyl]amino]butyl]-(4aS,8aS)-isochinolin-3(S)-carboxamid,
4u) N-tert.-Butyl-decahydro-2-[2(R)-{3-(4-imidazolyl)propionyloxy}-4-phenyl-3(S)-[[N-(2-chinolylcarbonyl)-L-asparaginyl]amino]butyl]-(4aS,8aS)-isochinolin-3(S)-carboxamid,
4v) N-tert.-Butyl-decahydro-2-[2(R)-chinolin-2-ylcarbonyloxy-4-phenyl-3(S)-[[N-(2-chinolylcarbonyl)-L-asparaginyl]amino]butyl]-(4aS,8aS)-isochinolin-3(S)-carboxamid,
4w) N-tert.-Butyl-decahydro-2-[2(R)-phenoxyacetyloxy-4-phenyl-3(S)-[[N-(2-chinolylcarbonyl)-L-asparaginyl]amino]butyl]-(4aS,8aS)-isochinolin-3(S)-carboxamid,
4x) N-tert.-Butyl-decahydro-2-[2(R)-pyridin-4-carbonyloxy-4-phenyl-3(S)-[[N-(2-chinolylcarbonyl)-L-asparaginyl]amino]butyl]-(4aS ,8aS)-isochinolin-3(S)-carboxamid,
4y) N-tert.-Butyl-decahydro-2-[2(R)-pyridin-2-carbonyloxy-4-phenyl-3(S)-[[N-(2-chinolylcarbonyl)-L-asparaginyl]amino]butyl]-(4aS ,8aS)-isochinolin-3(S)-carboxamid,

### Beispiel 5:

Analog zu einem der oben angeführten Beispiele oder nach den genannten Verfahren können auch die folgenden Acyl-Derivate von N-tert.-Butyl-decahydro-2-[2(R)-hydroxy4-phenyl-3(S)-[[N-(2-chinolylcarbonyl)-L-asparaginyl]amino]butyl]-(4aS,8aS)-isochinolin-3(S)-carboxamid hergestellt werden:
5a) N-tert.-Butyl-decahydro-2-[2(R)-{1-pyrazolylacetyloxy}-4-phenyl-3(S)-[[N-(2-chinolylcarbonyl)-L-asparaginyl]amino]butyl]-(4aS,8aS)-isochinolin-3(S)-carboxamid,
5b) N-tert.-Butyl-decahydro-2-[2(R)-pyrazin-2-ylcarbonyloxy-4-phenyl-3(S)-[[N-(2-chinolylcarbonyl)-L-asparaginyl]amino]butyl]-(4aS,8aS)-isochinolin-3(S)-carboxamid,
5c) N-tert.-Butyl-decahydro-2-[2(R)-isochinolin-3-carbonyloxy-4-phenyl-3(S)-[[N-(2-chinolylcarbonyl)-L-asparaginyl]amino]butyl]-(4aS,8aS)-isochinolin-3(S)-carboxamid,
5d) N-tert.-Butyl-decahydro-2-[2(R)-aminoacetyloxy-4-phenyl-3(S)-[[N-(2-chinolylcarbonyl)-L-asparaginyl]amino]butyl]-(4aS,8aS )-isochinolin-3(S)-carboxamid,
5e) N-tert.-Butyl-decahydro-2-[2(R)-{(L)-pyrrolidin-2-carbonyloxy}-4-phenyl-3(S)-[[N-(2-chinolylcarbonyl)-L-asparaginyl]amino]butyl]-(4aS,8aS)-isochinolin-3(S)-carboxamid,
5f) N-tert.-Butyl-decahydro-2-[2(R)-{N-(imidazolyl-4-methyl)-N-methylaminoacetyloxy}-4-phenyl-3(S)-[[N-(2-chinolylcarbonyl)-L-asparaginyl]amino]butyl]-(4aS,8aS)-isochinolin-3(S)-carboxamid,
5g) N-tert.-Butyl-decahydro-2-[2(R)-{N-(pyridin-2-ylmethyl)-N-methylaminoacetyloxy-4-phenyl-3(S)-[[N-(2-chinolylcarbonyl)-L-asparaginyl]amino]butyl]-(4aS,8aS)-isochinolin-3(S)-carboxamid,
5h) N-tert.-Butyl-decahydro-2-[2(R)-{N,N-(4-dimethylaminobutyryl)oxy}-4-phenyl-3(S)-[[N-(2-chinolylcarbonyl)-L-asparaginyl]amino]butyl]-(4aS,8aS)-isochinolin-3(S)-carboxamid,
5i) N-tert.-Butyl-decahydro-2-[2(R)-benzoyloxy-4-phenyl-3(S)-[[N-(2-chinolylcarbonyl)-L-asparaginyl]amino]butyl]-(4aS,8aS)-isochinolin-3(S)-carboxamid,
5j) N-tert.-Butyl-decahydro-2-[2(R)-{4-chlormethylbenzoyloxy}-4-phenyl-3(S)-[[N-(2-chinolylcarbonyl)-L-asparaginyl]amino]butyl]-(4aS,8aS)-isochinolin-3(S)-carboxamid,
5k) N-tert.-Butyl-decahydro-2-[2(R)-{3-(N,N-dimethylaminopropyl)oxyxyacetylo}-4 -phenyl-3(S)-[[N-(2-chinolylcarbonyl)-L-asparaginyl]amino]butyl]-(4aS,8aS)-isochinolin-3(S)-carboxamid, oder
5l) N-tert.-Butyl-decahydro-2-[2(R)-{2-[3-(N,N-dimethylaminopropy loxy)ethoxy]acetyloxy}-4-phenyl-3(S)-[[N-(2-chinolylcarbonyl)-L-asparaginyl]amino]butyl]-(4aS,8aS)-isochinolin-3(S)-carboxamid.

### Beispiel 6: N-tert.Butyl-decahydro-2-[2(R)-methoxyacetyloxy-4-phenyl-3(S)-[[N-(2-chinolylcarbonyl)-L-asparaginyl]amino] butyl]-(4aS,8aS)-isochinolin-3(S)-carboxamid

Eine Lösung von 10 mg (0,015 mMol) N-tert.Butyl-decahydro-2-[2(R)-hydroxy-4-phenyl-3(S)-[[N-(2-chinolylcarbonyl)-L-asparaginyl]amino]butyl]-(4aS,8aS)-isochinolin-3(S)-carboxamid (Beispiel 1e) in 0,24 ml Dioxan/Pyridin 5:1 wird unter Stickstoffatmosphäre bei 0°C mit 4 µl (0,046 mMol) Methoxyessigsäurechlorid und 0,2 mg (0,002 mMol) DMAP versetzt. Da bei HPLC nach 18 h bei RT noch Ausgangsmaterial gefunden wird, werden in 2 Portionen nochmals total 35 µl Pyridin und 35 µl Methoxyessigsäurechlorid zugegeben. Wenn gemäss HPLC alles Ausgangsmaterial umgesetzt ist, verteilt man das Reaktionsgemisch zwischen 3 Portionen Essigsäureethylester, ges.

NaHCO₃-Lsg, Wasser und Sole, trocknet die organische Phase in Na₂SO₄ und dampft sie ein. Digerieren aus Hexan/Diisopropylether im Ultraschallbad und Stehenlassen bei 0°C liefert die reine Titelverbindung: t_{Ret}(II)=13,1 min; FAB-MS (M+H)⁺=743.

### Beispiel 7: N-tert.Butyl-decahydro-2-[2(R)-(pyridin-2-carbonyloxy)-4-phenyl-3(S)-[[N-(2-chinolylcarbonyl)-L-asparaginyl]amino]butyl]-(4aS,8aS)-isochinolin-3(S)-carboxamid

Unter Stickstoffatmosphäre wird eine Lösung von 30 mg (0,24 mMol) 2-Picolinsäure (Pyridin-2-carbonsäure) in 1,6 ml Methylenchlorid bei 0°C mit 20 µl (0,144 mMol) 1-Chlor-N,N,2-trimethyl-1-propenamin *[B. Haveaux, A. Dekoker, M. Rens, A.R. Sidani, J.* *Toye, L. Ghosez, M. Murakami, M. Yoshioka, and W. Nagata,* Organic Syntheses **59**, 26 (1980)*]* versetzt und bei RT während 45 min ins Säurechlorid überführt. Danach fügt man 0,4 ml Pyridin, einer katalytischen Menge 4-Dimethylaminopyridin und 40 mg (0,06 mMol) N-tert.Butyl-decahydro2-[2(R)-hydroxy-4-phenyl-3(S)-[[N-(2-chinolylcarbonyl)-L-asparaginyl]amino]butyl]-(4aS,8aS)-isochinolin-3(S)-carboxamid (Beispiel 1e) zu und rührt während 18 h bei RT. Da laut HPLC noch Ausgangsmaterial vorliegt, werden nochmals 0,12 mMol 2-Picolinsäurechlorid in 0,8 ml Methylenchlorid wie oben hergestellt und zusammen mit 0,2 ml Pyridin zugesetzt. Nach weiteren 18 h bei RT verteilt man das Reaktionsgemisch zwischen 3 Portionen Methylenchlorid, 2 Portionen ges. NaHCO₃-Lsg, Wasser und Sole, trocknet die organischen Phasen mit Na₂SO₄ und dampft sie ein. Eine Säulenchromatographie (SiO₂, Methylenchlorid/THF 1:1) und Digerieren aus Diisopropylether liefert die reine Titelverbindung: DC R_{f}(C)=0,28; t_{Ret}(II)=13,2 min; FAB-MS (M+H)⁺=776.

### Beispiel 8: Gelatine-Lösung

Eine sterilfiltrierte wässrige Lösung einer der in den vorausgehenden Beispielen 1 bis 7 genannten Verbindungen der Formel I, die zusätzlich 20 % Cyclodextrin enthält, und eine sterile, mit Phenol konservierte Gelatinelösung werden unter Erwärmen unter aseptischen Bedingungen so vermischt, dass 1,0 ml Lösung folgender Zusammensetzung resultiert:

| | |
|---|---|
| Wirkstoff | 3 mg |
| Gelatine | 150,0 mg |
| Phenol | 4,7 mg |
| dest. Wasser mit 20 % Cyclodextrinen | 1,0 ml |

### Beispiel 9: Sterile Trockensubstanz zur Injektion

Man löst 5 mg einer der in den vorausgehenden Beispielen 1 bis 7 genannten Verbindungen der Formel I in 1 ml einer wässrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

### Beispiel 10: Nasenspray

In einer Mischung von 3,5 ml Myglyol 812® und 0,08 g Benzylalkohol werden 500 mg fein gemahlenes (<5,0 µm) Pulver einer der in den vorausgehenden Beispielen 1 bis 7 genannten Verbindungen der Formel I suspendiert. Diese Suspension wird in einen Behälter mit Dosierventil eingefüllt. Es werden 5,0 g Freon 12® unter Druck durch das Ventil in einen Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

### Beispiel 11: Lacktabletten

Für die Herstellung von 10 000 Tabletten enthaltend je 100 mg Verbindung der Formel I werden folgende Bestandteile verarbeitet:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | quantum satis |

Ein Gemisch von einer der in den vorausgehenden Beispielen 1 bis 7 genannten Verbindungen der Formel I, 50 g Maisstärke und kolloidaler Kieselsäure wird mit Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45 ° während 30 Min. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

### Beispiel 12: Oral verabreichbare Dispersion 1

625 mg einer der in den vorausgehenden Beispielen 1 bis 7 genannten Verbindungen der Formel lund 625 mg POPC (1-Palmitoyl-2-oleoyl-phosphatidylcholin = 1-Hexadecanoyl-2-(9-cis-octadecenoyl)-3-sn-phosphatidylcholin) werden in 25 ml Ethanol gelöst. Die Lösung wird mit der zehnfachen Menge Wasser verdünnt. Hierzu wird die ethanolische Lösung bei Raumtemperatur mit einer Rate von 10 ml/min zu der vorgelegten Menge Wasser zugetropft. Das Ethanol wird aus der Mischung durch tangentiale Dialyse ("Cross Flow Filtration") gegen 1750 ml Wasser (System: Minitan®, 700 cm²- Polyethersulphon-Membran mit einer Ausschlussgrenze von 100 kD, der Firma Millipore (USA)) entfernt. Die Mischung wird unter Verwendung desselben Systems auf 15 mg Wirkstoff konzentriert durch Ultrafiltration. Nach Zugabe von 1,24 mg/ml Zitronensäure und 1,24 mg/ml Dinatriumhydrogenphosphat·2 H₂O zur Einstellung auf pH 4,2 und von 1 mg/ml Sorbinsäure als antimikrobiellem Konservierungsmittel wird die Dispersion erneut auf 15 mg/ml aufkonzentriert und in Gläschen, beispielsweise mit 20 ml Inhalt, abgefüllt. Die Dispersionspartikel haben einen Durchmesser von 0,1 - 2 µm. Sie sind bei +2 bis 8 °C mindestens ein halbes Jahr lang stabil und geeignet zur oralen Verabreichung.

### Beispiel 13: Oral verabreichbare Dispersion 2

Die Herstellung erfolgt analog Beispiel 12, jedoch unter Verwendung von 25 mg der Verbindung der Formel I und 50 mg POPC zur Herstellung der ethanolischen Lösung.

### Beispiel 14: Oral verabreichbare Dispersion 3

Die Herstellung erfolgt analog Beispiel 12, jedoch unter Verwendung von 25 mg der Verbindung der Formel I und 125 mg POPC zur Herstellung der ethanolischen Lösung.

### Beispiel 15: Oral verabreichbare Dispersion 4

Die Herstellung erfolgt analog Beispiel 12, jedoch unter Verwendung von 50 mg der Verbindung der Formel I und 50 mg POPC zur Herstellung der ethanolischen Lösung.

### Beispiel 16: Oral verabreichbare Dispersion 5

Die Herstellung erfolgt analog einem der Beispiele 12 bis 15, jedoch unter Verwendung von der Verbindung der Formel I und Phosphatidylcholin aus Soja oder Phosphatidylcholin aus Eigelb (70 - 100 % rein) anstelle von POPC zur Herstellung der ethanolischen Lösung. Gewünschtenfalls wird ein Antioxidans, wie Ascorbinsäure, in einer Konzentration von 5 mg/ml zugegeben.

## Patentansprüche

1. Verbindungen der Formel I, 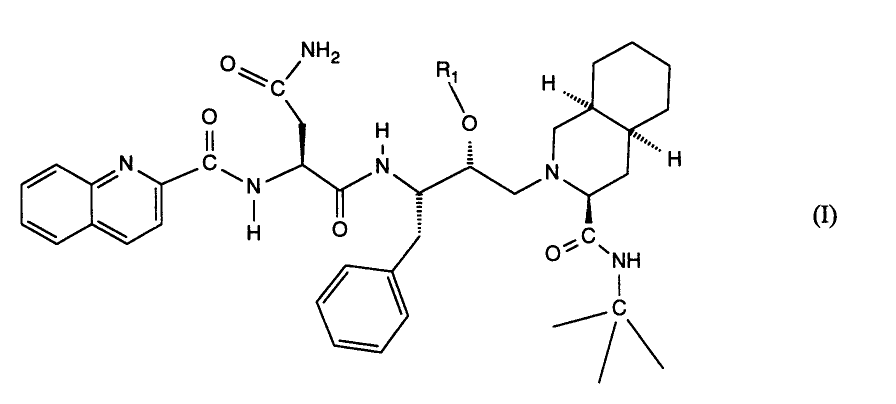 worin R₁ Octanoyl, Decanoyl, Dodecanoyl, Palmitoyl, unsubstituiertes oder substituiertes Niederalkanoyl, Niederalkenyol oder Niederalkinoyl, worin die Substituenten aus einem oder mehreren Resten ausgewählt aus der Gruppe Hydroxy, Niederalkoxy, Niederalkoxyniederalkoxy, Niederalkoxyniederalkoxyniederalkoxy, Phenoxy, Naphthoxy, Phenylniederalkoxy, 2-Halogenniederalkanoyl, Amino-, Niederalkylamino- oder Diniederalkylamino-niederalkoxy-2-niederalkanoyl, Amino-, Niederalkylamino- oder Diniederalkylamino-niederalkoxyniederalkoxy-2-niederalkanoyl, Niederalkanoyloxy, Phenylniederalkanoyloxy, Halogen, Carboxy, Niederalkoxycarbonyl, Phenylniederalkoxycarbonyl, Carbamoyl, Niederalkylcarbamoyl, Hydroxyniederalkylcarbamoyl, Diniederalkylcarbamoyl, Bis(hydroxyniederalkyl)carbamoyl, Carbamoyl, dessen Stickstoff Bestandteil eines 5- bis 7-gliedrigen heterocyclischen Ringes ist, der noch ein weiteres Heteroatom ausgewählt aus O, S, N und durch Niederalkyl, wie Methyl oder Ethyl, substituiertem N enthalten kann, Cyano, Oxo, Cycloalkyl, Bicyloalkyl, Tricycloalkyl, Cycloalkenyl, Bicycloalkenyl, Heterocyclyl, welches einen gesättigten, teilweise gesättigten oder ungesättigten einfachen Ring bedeutet, der 5 - 7 Ringatome und bis zu vier Heteroatome ausgewählt aus Stickstoff, Schwefel und/oder Sauerstoff enthält, wobei der Ring entweder als solcher vorliegt oder einfach benzanneliert, cyclopenta-, cyclohexa- oder cyclohepta-anneliert sein kann; und unsubstituiert oder insbesondere durch Niederalkyl, Niederalkanoyl, Hydroxy, Niederalkoxy, Phenylniederalkoxy, Hydroxyniederalkyl, Halogen, Cyano und/oder Trifluormethyl substituiert sein kann, und C₆-C₁₂-Aryl, welches unsubstituiert oder durch Niederalkyl, Halogenniederalkyl, Halogen, Hydroxy, Niederalkoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, Heterocyclylniederalkyl, worin Heterocyclyl wie oben definiert ist, Cyano und/oder Nitro ein oder mehrfach substituiert ist; Niederalkoxycarbonyl, 2-Halogenniederalkoxycarbonyl, Arylniederalkoxycarbonyl, worin Aryl 6 bis 14 Kohlenstoffatome hat und unsubstituiert oder durch Niederalkyl, Halogenniederalkyl, Halogen, Hydroxy, Niederalkoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, Heterocyclylniederalkyl, worin Heterocyclyl wie oben als Substituent für Niederalkanoyl definiert ist, Cyano und/oder Nitro ein oder mehrfach substituiert ist, Heterocyclylniederalkoxycarbonyl, worin Heterocyclyl wie oben als Substituent von Niederalkanoyl definiert ist; Aminocarbonyl, einen Aminocarbonylrest, worin die Aminogruppe 1 bis 2 Substituenten trägt, die unabhängig voneinander aus unsubstituiertem oder substituiertem Niederalkyl, worin die Substituenten aus Hydroxy, Niederalkoxy, Phenoxy, Naphthoxy, Niederalkanoyloxy, Phenylniederalkanoyloxy, Halogen, Carboxy, Niederalkoxycarbonyl, Phenylniederalkoxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo und C₆-C₁₂-Aryl, welches unsubstituiert oder beispielsweise durch Niederalkyl, Halogenniederalkyl, Halogen, Hydroxy, Niederalkoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Cyano und/oder Nitro ein oder mehrfachsubstituiert ist, und aus Aryl, welches 6 bis 14 Kohlenstoffatome hat und unsubstituiert oder durch Niederalkyl, Halogenniederalkyl, Halogen, Hydroxy, Niederalkoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, Heterocyclylniederalkyl, worin Heterocyclyl wie oben als Substituent für Niederalkanoyl definiert ist, Cyano und/oder Nitro ein oder mehrfach substituiert ist, ausgewählt sind; wobei nicht mehr als einer der Substituenten des Aminocarbonylrestes Aryl bedeuten kann; oder das über eine Carboxygruppe gebundene Radikal einer Aminosäure ausgewählt aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure oder 5-Aminohexansäure, Valin, Norvalin, Leucin, Isoleucin, Norleucin, Serin, Homoserin, Threonin, Methionin, Cystein, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure, wobei (ausser in Fällen, wo kein asymmetrisches Kohlenstoffatom vorliegt) jede der genannten Aminosäuren in der D-, L- oder (D,L)-Form vorliegen kann,
eine α-Aminogruppe, falls vorhanden, unsubstituiert oder ein- oder zweifach N-alkyliert durch Niederalkyl, durch Aminoniederalkyl, durch Phenyl- oder Naphthylaminoniederalkyl, durch Phenylniederalkyl, durch Diphenylmethyl, durch Trityl, und/oder durch Heterocyclylniederalkyl, worin Heterocylcyl wie oben für substituiertes Niederalkanoyl R₁ definiert ist, und/oder N-acyliert durch einen oben bei der Definition von R₁ genannten unsubstituierten oder substituierten Niederalkanoylrest, durch Niederalkoxycarbonyl oder durch Phenylniederalkoxycarbonyl; vorliegt,
eine Carboxygruppe der Seitenkette frei, als Niederalkylestergruppe, als Arylestergruppe oder als Arylniederalkylestergruppe, worin Aryl Phenyl, 4-Nitrophenyl, Naphthyl, Fluorenyl oder Biphenylyl bedeutet, als Carbamoyl-, als Niederalkylcarbamoyl-, als Diniederalkylaminocarbamoyl-, als Mono- oder Di-(hydroxyniederalkyl)carbamoyl- oder als Mono- oder Di-(carboxyniederalkyl)carbamoyl-gruppe vorliegt,
eine Aminogruppe der Seitenkette, die sich nicht in α-Stellung befindet, frei, als Mono- oder Diniederalkylamino, als Niederalkanoylamino, als Aminoniederalkanoylamino, als Arylniederalkanoylamino, worin Aryl 6 - 14 Kohlenstoffatome hat und unsubstituiert oder durch Niederalkyl, Hydroxy, Niederalkoxy, Carboxy, Carbamoyl oder Sulfamoyl substituiert ist, als Niederalkoxycarbonylamino, als Arylmethoxycarbonylamino, worin Aryl 6 - 14 Kohlenstoffatome hat, als Piperidyl-1-carbonyl, Morpholinocarbonyl, Thiomorpholinocarbonyl oder als S,S-Dioxothiomorpholinocarbonyl vorliegt, und/oder
eine Hydroxygruppe der Seitenkette frei, als Niederalkoxy-, als Phenylniederalkoxy-, als Niederalkanoyloxy-, oder als Niederalkyloxycarbonyloxygruppe vorliegt, bedeutet, und Salze davon.

2. Verbindungen gemäss Anspruch 1 der Formel I, worin R₁ unsubstituiertes oder substituiertes Niederalkanoyl, Niederalkenyol oder Niederalkinoyl, worin die Substituenten aus einem oder mehreren Resten ausgewählt aus der Gruppe Hydroxy, Niederalkoxy, Phenoxy, Naphthoxy, Niederalkanoyloxy, Phenylniederalkanoyloxy, Halogen, Carboxy, Niederalkoxycarbonyl, Phenylniederalkoxycarbonyl, Carbamoyl, Niederalkylcarbamoyl, Hydroxyniederalkylcarbamoyl, Diniederalkylcarbamoyl, Bis(hydroxyniederalkyl)carbamoyl, Carbamoyl, dessen Stickstoff Bestandteil eines 5- bis 7-gliedrigen heterocyclischen Ringes ist, der noch ein weiteres Heteroatom ausgewählt aus O, S, N und durch Niederalkyl, wie Methyl oder Ethyl, substituiertem N enthalten kann, Cyano, Oxo, Cycloalkyl, Bicyloalkyl, Tricycloalkyl, Cycloalkenyl, Bicycloalkenyl, Heterocyclyl, welches einen gesättigten, teilweise gesättigten oder ungesättigten einfachen Ring bedeutet, der 5 - 7 Ringatome und bis zu vier Heteroatome ausgewählt aus Stickstoff, Schwefel und/oder Sauerstoff enthält, wobei der Ring entweder als solcher vorliegt oder einfach benzanneliert, cyclopenta-, cyclohexa- oder cyclohepta-anneliert sein kann; und unsubstituiert oder insbesondere durch Niederalkyl, Niederalkanoyl, Hydroxy, Niederalkoxy, Phenylniederalkoxy, Hydroxyniederalkyl, Halogen, Cyano und/oder Trifluormethyl substituiert sein kann, und C₆-C₁₂-Aryl, welches unsubstituiert oder durch Niederalkyl, Halogenniederalkyl, Halogen, Hydroxy, Niederalkoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, Heterocyclylniederalkyl, worin Heterocyclyl wie oben definiert ist, Cyano und/oder Nitro ein oder mehrfach substituiert ist; Niederalkoxycarbonyl, 2-Halogenniederalkoxycarbonyl, Arylniederalkoxycarbonyl, worin Aryl 6 bis 14 Kohlenstoffatome hat und unsubstituiert oder durch Niederalkyl, Halogenniederalkyl, Halogen, Hydroxy, Niederalkoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, Heterocyclylniederalkyl, worin Heterocyclyl wie oben als Substituent für Niederalkanoyl definiert ist, Cyano und/oder Nitro ein oder mehrfach substituiert ist, Heterocyclylniederalkoxycarbonyl, worin Heterocyclyl wie oben als Substituent von Niederalkanoyl definiert ist; Aminocarbonyl, einen Aminocarbonylrest, worin die Aminogruppe 1 bis 2 Substituenten trägt, die unabhängig voneinander aus unsubstituiertem oder substituiertem Niederalkyl, worin die Substituenten aus Hydroxy, Niederalkoxy, Phenoxy, Naphthoxy, Niederalkanoyloxy, Phenylniederalkanoyloxy, Halogen, Carboxy, Niederalkoxycarbonyl, Phenylniederalkoxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo und C₆-C₁₂-Aryl, welches unsubstituiert oder beispielsweise durch Niederalkyl, Halogenniederalkyl, Halogen, Hydroxy, Niederalkoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, wie Trifluormethyl, Cyano und/oder Nitro ein oder mehrfachsubstituiert ist, und aus Aryl, welches 6 bis 14 Kohlenstoffatome hat und unsubstituiert oder durch Niederalkyl, Halogenniederalkyl, Halogen, Hydroxy, Niederalkoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, Heterocyclylniederalkyl, worin Heterocyclyl wie oben als Substituent für Niederalkanoyl definiert ist, Cyano und/oder Nitro ein oder mehrfach substituiert ist, ausgewählt sind; wobei nicht mehr als einer der Substituenten des Aminocarbonylrestes Aryl bedeuten kann; oder das über eine Carboxygruppe gebundene Radikal einer Aminosäure ausgewählt aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure oder 5-Aminohexansäure, Valin, Norvalin, Leucin, Isoleucin, Norleucin, Serin, Homoserin, Threonin, Methionin, Cystein, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure, wobei (ausser in Fällen, wo kein asymmetrisches Kohlenstoffatom vorliegt) jede der genannten Aminosäuren in der D-, L- oder (D,L)-Form vorliegen kann,
eine α-Aminogruppe, falls vorhanden, unsubstituiert oder ein- oder zweifach N-alkyliert durch Niederalkyl, durch Aminoniederalkyl, durch Phenyl- oder Naphthylaminoniederalkyl, durch Phenylniederalkyl, durch Diphenylmethyl, durch Trityl, und/oder durch Heterocyclylniederalkyl, worin Heterocylcyl wie oben für substituiertes Niederalkanoyl R₁ definiert ist, und/oder N-acyliert durch einen oben bei der Definition von R₁ genannten unsubstituierten oder substituierten Niederalkanoylrest, durch Niederalkoxycarbonyl oder durch Phenylniederalkoxycarbonyl; vorliegt,
eine Carboxygruppe der Seitenkette frei, als Niederalkylestergruppe, als Arylestergruppe oder als Arylniederalkylestergruppe, worin Aryl Phenyl, 4-Nitrophenyl, Naphthyl, Fluorenyl oder Biphenylyl bedeutet, als Carbamoyl-, als Niederalkylcarbamoyl-, als Diniederalkylaminocarbamoyl-, als Mono- oder Di-(hydroxyniederalkyl)carbamoyl- oder als Mono- oder Di-(carboxyniederalkyl)carbamoyl-gruppe vorliegt,
eine Aminogruppe der Seitenkette, die sich nicht in α-Stellung befindet, frei, als Mono- oder Diniederalkylamino, als Niederalkanoylamino, als Aminoniederalkanoylamino, als Arylniederalkanoylamino, worin Aryl 6 - 14 Kohlenstoffatome hat und unsubstituiert oder durch Niederalkyl, Hydroxy, Niederalkoxy, Carboxy, Carbamoyl oder Sulfamoyl substituiert ist, als Niederalkoxycarbonylamino, als Arylmethoxycarbonylamino, worin Aryl 6 - 14 Kohlenstoffatome hat, als Piperidyl-1-carbonyl, Morpholinocarbonyl, Thiomorpholinocarbonyl oder als S,S-Dioxothiomorpholinocarbonyl vorliegt, und/oder
eine Hydroxygruppe der Seitenkette frei, als Niederalkoxy-, als Phenylniederalkoxy-, als Niederalkanoyloxy-, oder als Niederalkyloxycarbonyloxygruppe vorliegt, bedeutet, und Salze davon.

3. Verbindungen gemäss einem der Ansprüche 1 und 2 der Formel I, worin R₁ Octanoyl, Decanoyl, Dodecanoyl, Palmitoyl, unsubstituiertes oder substituiertes Niederalkanoyl, worin die Substituenten aus einem oder bis zu drei Resten aus der Gruppe Hydroxy, Niederalkoxy, Niederalkoxyniederalkoxy, Niederalkoxyniederalkoxyniederalkoxy, Phenoxy, Naphthoxy, Phenylniederalkoxy, Niederalkanoyloxy, Phenylniederalkanoyloxy, Amino-, Niederalkylamino- oder Diniederalkylamino-niederalkoxy-2-niederalkanoyl, Amino-, Niederalkylamino- oder Diniederalkylamino-niederalkoxyniederalkoxy-2-niederalkanoyl, Halogen, Carboxy, Niederalkoxycarbonyl, Phenylniederalkoxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo, C₃-C₈-Cycloalkyl, C₄-C₈-Cycloalkenyl; Pyrrolyl, 2,5-Dihydropyrrolyl, Furanyl, Thienyl, Tetrahydrofuranyl, Pyrrolyl, Pyrrolidinyl, Imidazolyl, Imidazolidinyl, Pyrazolyl, Pyrazolidinyl, Tetrahydro-oxazolyl, Tetrahydro-isoxazolyl, Tetrahydro-thiazolyl, Tetrahydro-isothiazolyl, Indolyl, Isoindolyl, Chinolyl, Isochinolyl, Benzimidazolyl, Benzofuranyl, Pyridyl, Pyrimidinyl, Piperidinyl, Piperazin-1-yl, Morpholino, Thiomorpholino, S,S-Dioxothiomorpholino, 1,2-Dihydro- oder 1,2,3,4-Tetrahydrochinolyl oder 1,2-Dihydro- oder 1,2,3,4-Tetrahydroisochinolyl, wobei die genannten heterocyclischen Reste unsubstituiert oder durch Niederalkyl, Niederalkanoyl, Hydroxy, Niederalkoxy, Phenylniederalkoxy, Hydroxyniederalkyl, Halogen, Cyano und/oder Trifluormethyl substituiert sind; und Aryl ausgewählt aus Phenyl, Naphthyl, Indanyl, Indenyl und Fluorenyl, wobei diese Reste unsubstituiert oder durch Niederalkyl, Halogenniederalkyl, Halogen, Hydroxy, Niederalkoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, Piperidinomethyl, Piperazin-1-ylmethyl, 4-Niederalkyl-piperazin-1-ylmethyl, 4-Niederalkanoyl-piperazin-1-ylmethyl, Morpholinomethyl, Thiomorpholinomethyl, Cyano und/oder Nitro ein oder mehrfach substituiert sind, ausgewählt sind, oder das über eine Carboxygruppe gebundene Radikal einer Aminosäure ausgewählt aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure oder 5-Aminohexansäure, Valin, Norvalin, Leucin, Isoleucin, Norleucin, Serin, Homoserin, Threonin, Methionin, Cystein, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan,Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure, wobei (ausser in Fällen, wo kein asymmetrisches Kohlenstoffatom vorliegt) jede der genannten Aminosäuren in der D-, L- oder (D,L)-Konfiguration vorliegen kann, worin
eine α-Aminogruppe, falls vorhanden, unsubstituiert oder ein- oder zweifach N-alkyliert durch Niederalkyl, durch Aminoniederalkyl, durch Phenyl- oder Naphthylaminoniederalkyl, durch Phenylniederalkyl, durch Diphenylmethyl, durch Trityl, durch Furanylniederalkyl, durch Thienylniederalkyl, durch Imidazolylniederalkyl und/oder durch 2-, 3- oder 4-Pyridylniederalkyl, und/oder N-acyliert durch einen oben bei der Definition von R₁ genannten unsubstituierten oder substituierten Niederalkanoylrest, durch Niederalkoxycarbonyl oder durch Phenylniederalkoxycarbonyl vorliegt, bedeutet, und pharmazeutisch verwendbare Salze davon.

4. Verbindungen gemäss Anspruch 2 der Formel I, worin R₁ unsubstituiertes oder substituiertes Niederalkanoyl, worin die Substituenten aus einem oder bis zu drei Resten aus der Gruppe Hydroxy, Niederalkoxy, Phenoxy, Naphthoxy, Niederalkanoyloxy, Phenylniederalkanoyloxy, Halogen, Carboxy, Niederalkoxycarbonyl, Phenylniederalkoxycarbonyl, Carbamoyl, Niederalkylcarbamoyl, Hydroxyniederalkylcarbamoyl, Diniederalkylcarbamoyl, Bis(hydroxyniederalkyl)carbamoyl, Cyano, Oxo, C₃-C₈-Cycloalkyl, C₄-C₈-Cycloalkenyl; Pyrrolyl, 2,5-Dihydropyrrolyl, Furanyl, Thienyl, Tetrahydrofuranyl, Pyrrolyl, Pyrrolidinyl, Imidazolyl, Imidazolidinyl, Pyrazolyl, Pyrazolidinyl, Tetrahydro-oxazolyl, Tetrahydro-isoxazolyl, Tetrahydro-thiazolyl, Tetrahydro-isothiazolyl, Indolyl, Isoindolyl, Chinolyl, Isochinolyl, Benzimidazolyl, Benzofuranyl, Pyridyl, Pyrimidinyl, Piperidinyl, Piperazin-1-yl, Morpholino, Thiomorpholino, S,S-Dioxothiomorpholino, 1,2-Dihydro- oder 1,2,3,4-Tetrahydrochinolyl oder 1,2-Dihydro- oder 1,2,3,4-Tetrahydroisochinolyl, wobei die genannten heterocyclischen Reste unsubstituiert oder durch Niederalkyl, Niederalkanoyl, Hydroxy, Niederalkoxy, Phenylniederalkoxy, Hydroxyniederalkyl, Halogen, Cyano und/oder Trifluormethyl substituiert sind; und Aryl ausgewählt aus Phenyl, Naphthyl, Indanyl, Indenyl und Fluorenyl, wobei diese Reste unsubstituiert oder durch Niederalkyl, Halogenniederalkyl, Halogen, Hydroxy, Niederalkoxy, Niederalkanoyloxy, Carboxy, Niederalkyloxycarbonyl, Phenylniederalkoxycarbonyl, Carbamoyl, Mono- oder Diniederalkylcarbamoyl, Mono- oder Di-hydroxyniederalkylcarbamoyl, Haloniederalkyl, Piperidinomethyl, Piperazin-1-ylmethyl, 4-Niederalkylpiperazin-1-ylmethyl, 4-Niederalkanoyl-piperazin-1-ylmethyl, Morpholinomethyl, Thiomorpholinomethyl, Cyano und/oder Nitro ein oder mehrfach substituiert sind, ausgewählt sind, oder das über eine Carboxygruppe gebundene Radikal einer Aminosäure ausgewählt aus Glycin, Alanin, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure oder 5-Aminohexansäure, Valin, Norvalin, Leucin, Isoleucin, Norleucin, Serin, Homoserin, Threonin, Methionin, Cystein, Phenylalanin, Tyrosin, 4-Aminophenylalanin, 4-Chlorphenylalanin, 4-Carboxyphenylalanin, β-Phenylserin, Phenylglycin, α-Naphthylalanin, Cyclohexylalanin, Cyclohexylglycin, Tryptophan, Asparaginsäure, Asparagin, Aminomalonsäure, Aminomalonsäure-monoamid, Glutaminsäure, Glutamin, Histidin, Arginin, Lysin, δ-Hydroxylysin, Ornithin, 3-Aminopropansäure, α,γ-Diaminobuttersäure und α,β-Diaminopropionsäure, wobei (ausser in Fällen, wo kein asymmetrisches Kohlenstoffatom vorliegt) jede der genannten Aminosäuren in der D-, L- oder (D,L)-Konfiguration vorliegen kann, worin
eine α-Aminogruppe, falls vorhanden, unsubstituiert oder ein- oder zweifach N-alkyliert durch Niederalkyl, durch Aminoniederalkyl, durch Phenyl- oder Naphthylaminoniederalkyl, durch Phenylniederalkyl, durch Diphenylmethyl, durch Trityl, durch Furanylniederalkyl, durch Thienylniederalkyl und/oder durch 2-, 3- oder 4-Pyridylniederalkyl, und/oder N-acyliert durch einen oben bei der Definition von R₁ genannten unsubstituierten oder substituierten Niederalkanoylrest, durch Niederalkoxycarbonyl oder durch Phenylniederalkoxycarbonyl vorliegt, bedeutet, und pharmazeutisch verwendbare Salze davon.

5. Verbindungen der Formel I gemäss Anspruch 1, worin R₁ Octanoyl, Decanoyl, Dodecanoyl, Palmitoyl, Niederalkanoyl, Hydroxyniederalkanoyl, Niederalkoxyniederalkanoyl, Niederalkoxyniederylkoxyniederalkanoyl, Niederalkoxyniederalkoxyniederalkoxyniederalkanoyl, Phenoxyniederalkanoyl, Phenylniederalkoxyniederalkanoyl, Amino-, Niederalkylamino- oder Diniederalkylamino-niederalkoxy-2-niederalkanoyl, Amino-, Niederalkylamino- oder Diniederalkylamino-niederalkoxyniederalkoxy-2-niederalkanoyl,Niederalkanoyloxyniederalkanoyl, Carboxyniederalkanoyl, Oxoniederalkanoyl, 5-Hydroxymethyl-furan-2-ylcarbonyl, 2- oder 3-Pyrrolylcarbonyl, Furylcarbonyl, Thienylcarbonyl, Pyridylniederalkanoyl, Chinolylcarbonyl, Isochinolylcarbonyl, 2-, 3- oder 5-Indolylcarbonyl, Pyrrolidinyl-(2- oder 3-)carbonyl, 2-, 3- oder 4-Piperidinylcarbonyl, 1,2,3,4-Tetrahydrochinolyl-2-, -3- oder -4-carbonyl, 1,2,3,4-Tetrahydroisochinolyl-1-, -3- oder 4-carbonyl, Imidazolylniederalkanoyl, Pyrazolylniederalkanoyl, Morpholinocarbonyl, Thiomorpholinocarbonyl, Morpholinoacetyl, Thiomorpholinoacetyl, 4-Niederalkyl-1-piperazinoacetyl, Indolylacetyl, Benzofuranylacetyl, Phenylniederalkanoyl, Phenylrest unsubstituiert, mono- oder mehrfachsubstituiert durch Niederalkyl, Haloniederalkyl, Halogen, Hydroxy, Niederalkoxy, Piperidinomethyl, Piperazin-1-ylmethyl, 4-Niederalkyl-piperazin-1-ylmethyl, Morpholinomethyl, Thiomorpholinomethyl, Cyano und/oder Nitro, oder das über eine Carboxygruppe gebundene Radikal einer aliphatischen Aminosäure ausgewählt aus Alanin, Valin, Norvalin, Leucin, 3-Aminopropionsäure, 2-Aminobuttersäure und Isoleucin oder einer Aminosäure ausgewählt aus Glycin, Asparagin, Glutamin, Methionin, Lysin, Phenylalanin, 3-Aminobuttersäure, 4-Aminobuttersäure, 3-Aminopentansäure, 4-Aminopentansäure, 5-Aminopentansäure, 3-Aminohexansäure, 4-Aminohexansäure und 5-Aminohexansäure, wobei (ausser bei Fehlen asymmetrischer C-Atome) jede der genannten Aminosäuren in der D-, L- oder (D,L)-Form vorliegen kann, eine α-Aminogruppe, falls vorhanden, unsubstituiert oder ein- oder zweifach N-alkyliert durch Niederalkyl, durch Phenylniederalkyl, durch 2-, 3- oder 4-Pyridylniederalkyl, Imidazolylniederalkyl, und/oder durch 2-, 3- oder 4-Pyridylniederalkyl, wie 2-, 3- oder 4-Pyridylmethyl, und/oder N-acyliert durch Niederalkoxycarbonyl,oder durch Benzyloxycarbonyl vorliegt, und pharmazeutisch verwendbare Salze davon.

6. Verbindungen gemäss Anspruch 1 der Formel I, worin R₁ Niederalkanoyl, Hydroxyniederalkanoyl, Niederalkoxyniederalkanoyl, Phenoxyniederalkanoyl, Niederalkanoyloxyniederalkanoyl, Oxoniederalkanoyl, 5-Hydroxymethyl-furan-2-ylcarbonyl, 2- oder 3-Pyrrolylcarbonyl, Furylcarbonyl, z.B. 2-Furylcarbonyl, Thienylcarbonyl, Pyridylcarbonyl, 2-, 3- oder 5-Indolylcarbonyl, Pyrrolidinyl-3-carbonyl, 2-, 3- oder 4-Piperidinylcarbonyl, 1,2,3,4-Tetrahydrochinolyl-2-, -3- oder -4-carbonyl, 1,2,3,4-Tetrahydroisochinolyl-1-, -3- oder 4-carbonyl, Imidazolylcarbonyl, Morpholinocarbonyl, Thiomorpholinocarbonyl, Morpholinoacetyl, Thiomorpholinoacetyl, 4-Niederalkyl-1-piperazinoacetyl, Indolylacetyl, Benzofuranylacetyl, Phenylniederalkanoyl, im Phenylrest unsubstituiert, mono- oder mehrfachsubstituiert durch Niederalkyl, Haloniederalkyl, Halogen, Hydroxy, Niederalkoxy, Piperidinomethyl, Piperazin-1-ylmethyl, 4-Niederalkylpiperazin-1-ylmethyl, Morpholinomethyl, Thiomorpholinomethyl, Cyano und/oder Nitro, oder das über eine Carboxygruppe gebundene Radikal einer aliphatischen Aminosäure ausgewählt aus Alanin, Valin, Norvalin, Leucin, 3-Aminopropionsäure, 2-Aminobuttersäure, 3-Aminobuttersäure, 4-Aminobuttersäure, 3-Aminopentansäure, 4-Aminopentansäure, 5-Aminopentansäure, 3-Aminohexansäure, 4-Aminohexansäure, 5-Aminohexansäure, und Isoleucin oder einer Aminosäure ausgewählt aus Glycin, Asparagin, Glutamin, Methionin, Lysin, Prolin oder Phenylalanin, bedeutet, wobei (ausser in Fällen, wo kein asymmetrisches Kohlenstoffatom vorliegt) jede der genannten Aminosäuren in der D-, L- oder (D,L)-Form vorliegen kann; eine α-Aminogruppe, falls vorhanden, unsubstituiert oder ein- oder zweifach N-alkyliert durch Niederalkyl, durch Phenylniederalkyl, und/oder durch 2-, 3- oder 4-Pyridylniederalkyl, und/oder N-acyliert durch Niederalkoxycarbonyl oder durch Phenylniederalkoxycarbonyl vorliegt, und pharmazeutisch verwendbare Salze davon.

7. Verbindungen der Formel I gemäss Anspruch 1, worin R₁ Octanoyl, Decanoyl, Dodecanoyl, Palmitoyl, Niederalkanoyl, Niederalkoxyniederalkanoyl, Niederalkoxyniederalkoxyniederalkanoyl, Niederalkoxyniederalkoxyniederalkanoyl, Phenoxyniederalkanoyl, Phenylniederalkoxyniederalkanoyl, Amino-, Niederalkylamino- oder Diniederalkylamino-niederalkoxy-2-niederalkanoyl, Amino-, Niederalkylamino- oder Diniederalkylamino-niederalkoxyniederalkoxy-2-niederalkanoyl, 2-Niederalkoxy-2-phenylacetyl, Furylcarbonyl, Pyridylniederalkanoyl, Chinolylcarbonyl, Isochinolylcarbonyl, Pyrrolidinyl-2-carbonyl, Imidazolylniederalkanoyl, Pyrazolylniederalkanoyl, Phenylniederalkanoyl, 4-Chlormethylbenzoyl, 4-Morpholinomethylbenzoyl, 4-Thiomorpholinomethylbenzoyl, Aminoacetyl, N-Niederalkylaminoacetyl, N,N-Diniederalkylaminoacetyl, N-Niederalkyl-N-phenylniederalkylaminoacetyl, N-Niederalkyl-N-benzyloxycarbonylaminoacetyl, N-Imidazolylniederalkyl-N-niederalkylaminoacetyl, N-Niederalkyl-N-pyridylniederalkylaminoacetyl oder 4-(N,N-Dimethylamino)butyryl bedeutet, und pharmazeutisch verwendbare Salze davon.

8. Verbindungen gemäss Anspruch 1 der Formel I, worin R₁ Niederalkanoyl, Furylcarbonyl, Pyridylcarbonyl, Phenylniederalkanoyl, 4-Morpholinomethylbenzoyl, 4-Thiomorpholinomethylbenzoyl, Aminoacetyl, N-Niederalkylaminoacetyl, N,N-Diniederalkylaminoacetyl, N-Niederalkyl-N-phenylniederalkylaminoacetyl, N-Niederalkyl-N-pyridylniederalkylaminoacetyl oder N-Phenylniederalkoxycarbonyl-N-niederalkylaminoacetyl bedeutet, und pharmazeutisch verwendbare Salze davon.

9. Verbindungen der Formel I gemäss Anspruch 1, worin R₁ Niederalkanoyl, Niederalkoxyniederalkanoyl, Pyridylcarbonyl oder Furylcarbonyl bedeutet, und pharmazeutisch verwendbare Salze davon.

10. Verbindungen gemäss Anspruch 1 der Formel I, worin R₁ Niederalkanoyl oder Furylcarbonyl bedeutet, und pharmazeutisch verwendbare Salze davon.

11. Verbindungen gemäss Anspruch 1 der Formel I, worin R₁ Niederalkoxyniederalkanoyl oder Pyridylcarbonyl bedeutet, und pharmazeutisch verwendbare Salze davon.

12. Verbindungen gemäss Anspruch 1 der Formel I, worin R₁ Niederalkanoyl bedeutet, und pharmazeutisch verwendbare Salze davon.

13. Verbindungen der Formel I gemäss Anspruch 1, ausgewählt aus den Verbindungen mit der Bezeichnung N-tert.-Butyl-decahydro-2-[2(R)-(furan-2-carboxy)-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-pivaloyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-(N-methylaminoacetoxy)-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-(N-benzyloxycarbonyl-N-methylaminoacetoxy)-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2[2(R)-(N-dimethylaminoacetoxy)-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-(N-n-butyl-N-methylaminoacetoxy)-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-(N-methyl-N-benzylaminoacetoxy)-4-phenyl-3(S)-[[N-(2-chinolylcarbonyl)-L-asparaginyl]amino]butyl]-(4aS,8aS)-isochinolin-3(S)-carboxamid,
N-tert.Butyl-decahydro-2-[2(R)-(N-methyl-N-3-pyridylmethyl-aminoacetoxy)-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-(3-pyridylcarboxy)-4-phenyl-3(S)-[[N-(2-chinolylcarbonyl)-L-asparaginyl]amino]butyl]-(4aS,8aS)-isochinolin-3(S)-carboxamid und
N-tert.-Butyl-decahydro-2-[2(R)-(4-(N-morpholinyl-methyl)-benzoyloxy)-4-phenyl-3(S)-[[N-(2-chinolylcarbonyl)-L-asparaginyl]amino]butyl]-(4aS,8aS)isochinolin-3(S)-carboxamid,
oder pharmazeutisch verwendbare Salze davon.

14. Verbindungen der Formel I gemäss Anspruch 1, ausgewählt aus den Verbindungen mit der Bezeichnung N-tert.-Butyl-decahydro-2-[2(R)-propionyloxy-4-phenyl-3(S)-[[N-(2-chinolylcarbonyl)-L-asparaginyl]amino]bu tyl]-(4aS,8aS)-isochinolin-3(S)-carboxamid; N-tert.-Butyl-decahydro-2-[2(R)-butyryloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-methylpropionyloxy-4-phenyl-3(S)-[[N-(2-chinolylcarbonyl)-L-asparaginyl]amino]butyl]-(4aS,8aS)-isochinolin-3(S)-carboxamid,
N-tert.Butyl-decahydro-2-[2(R)-pentanoyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-octanoyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-decanoyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-dodecanoyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-palmitoyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-(3-carboxypropionyloxy)-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-(3-methoxypropionyloxy)-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-benyloxyacetyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-{(S)-α-methoxy-α-phenylacetyloxy}-4-phenyl-3(S)-[[N-(2-chinolylcarbonyl)-L-asparaginyl]amino]butyl]-(4aS,8aS)-isochinolin-3(S)-carboxamid, N-tert.-Butyl-decahydro-2-[2(R)-{(R)-α-methoxy-α-phenylacetyloxy}-4-phenyl-3(S)-[[N-(2-chinolylcarbonyl)-L-asparaginyl]amino]butyl]-(4aS,8aS)-isochinolin-3(S)-carboxamid, N-tert.-Butyl-decahydro-2-[2(R)-{2-(2-methoxyethoxy)acetyloxy}-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-{n-butyloxyacetyloxy}-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-{2-(2-(2-methoxyethoxy)ethoxy)acetoxy}-4-phenyl-3(S)-[(N-(2-chinolylcarbonyl)-L-asparaginyl]amino]butyl]-(4aS,8aS)-isochinolin-3(S)-carboxamid, N-tert.-Butyl-decahydro-2-[2(R)-{2-pyridylacetyloxy}-4-phenyl-3(S)-[[N-(2-chinolylcarbonyl)-L-asparaginyl]amino]butyl]-(4aS,8aS)-isochinolin-3(S)-carboxamid, N-tert.-Butyl-decahydro-2-[2(R)-{3-(2-pyridyl)propionyloxy}-4-phenyl-3-(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-{4-imidazolylcarbonyloxy}-4-phenyl-3(S)-[[N-(2-chinolylcarbonyl)-L-asparaginyl]amino]butyl]-(4aS,8aS)-isochinolin-3(S)-carboxamid
N-tert.-Butyl-decahydro-2-(2(R)-{4-imidazolylacetyloxy}-4-phe nyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-{3-(4-imidazolyl)propionyloxy}-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-chinolin-2-ylcarbonyloxy-4-phenyl-3(S)-[[N-(2-chinolylcarbonyl)-L-asparaginyl]amino]butyl]-(4aS,8aS)-isochinolin-3(S)carboxamid,
N-tert.-Butyl-decahydro-2-[2(R)-phenoxyacetyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-pyridin-4-carbonyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-pyridin-2-carbonyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-{1-pyrazolylacetyloxy}-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-pyrazin-2-ylcarbonyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-isochinolin-3-carbonyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-aminoacetyloxy-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-{(L)-pyrrolidin-2-carbonyloxy}-4-phenyl-3(S)-[[N-(2-chinolylcarbonyl)-L-asparaginyl]amino]butyl]-(4aS,8aS)-isochinolin-3(S)-carboxamid,
N-tert.-Butyl-decahydro-2-[2(R)-{N-(imidazolyl-4-methyl)-N-methylaminoacetyloxy}-4-phenyl-3(S)-[[N-(2-chinolylcarbonyl)-L-asparaginyl]amino]butyl]-(4aS,8aS)-isochinolin-3(S)-carboxamid, N-tert.-Butyl-decahydro-2-[2(R)-{N-(pyridin-2-ylmethyl)-N-methylaminoacetyloxy-4-phenyl-3(S)-[[N-(2-chinolylcarbonyl)-L-asparaginyl]amino]butyl]-(4aS,8aS)-isochinolin-3(S)-carboxamid, N-tert.-Butyl-decahydro-2-[2(R)-{N,N-(4-dimethylaminobutyryl)oxy}-4-phenyl-3(S)-[[N-(2-chinolylcarbonyl)-L-asparaginyl]amino]butyl]-(4aS,8aS)-isochinolin-3(S)-carboxamid, N-tert.-Butyl-decahydro-2-[2(R)-benzoyloxy-4-phenyl-3(S)-[[N-(2-chinolylcarbonyl)-L-asparaginyl]amino]butyl]-(4aS,8aS)-isochinolin-3(S)-carboxamid, N-tert.-Butyl-decahydro-2-[2(R)-{4-chlormethylbenzoyloxy}-4-phenyl-3(S)-[[N-(2-chinolylcarbonyl)-L-asparaginyl]amino]butyl]-(4aS,8aS)-isochinolin-3(S)-carboxamid, N-tert.-Butyl-decahydro-2-[2(R)-{3-(N,N-dimethylaminopropyl)oxyacetyl}-4 -phenyl-3(S)-[[N-(2-chinolylcarbonyl)-L-asparaginyl]amino]butyl]-(4aS,8aS)-isochinolin-3(S)-carboxamid und N-tert.-Butyl-decahydro-2-[2(R)-{2-[3-(N,N-dimethylaminopropyloxy)ethoxy]acetyl}-4 -phenyl-3(S)-[[N-(2-chinolylcarbonyl)-L-asparaginyl]amino]butyl]-(4aS,8aS)-isochinolin-3(S)-carboxamid, oder pharmazeutisch verwendbare Salze davon.

15. Eine Verbindung der Formel I gemäss Anspruch 1, worin R₁ Acetyl bedeutet, und pharmazeutisch verwendbare Salze davon.

16. Eine Verbindung der Formel I gemäss Anspruch 1, worin R₁ Furan-2-ylcarbonyl bedeutet, und pharmazeutisch verwendbare Salze davon.

17. Eine Verbindung der Formel I gemäss Anspruch 1, worin R₁ Methoxyacetyl bedeutet, und pharmazeutisch verwendbare Salze davon.

18. Eine Verbindung der Formel I gemäss Anspruch 1, worin R₁ Pyridin-2-ylcarbonyl bedeutet, und pharmazeutisch verwendbare Salze davon

19. Eine Verbindung der Formel I oder ein Salz davon gemäss einem der Anprüche 1 - 18 zur Anwendung in einem Verfahren zur therapeutischen Behandlung des menschlichen oder tierischen Körpers.

20. Verwendung einer Verbindung gemäss einem der Ansprüche 1 - 18 der Formel I, oder eines pharmazeutischen Salzes davon, zur Herstellung von pharmazeutischen Präparaten zur Behandlung von AIDS.

21. Pharmazeutische Präparate enthaltend Verbindungen der Formel I gemäss einem der Ansprüche 1 - 18 oder ein pharmazeutisch verwendbares Salz einer solchen Verbindung mit mindestens einer salzbildenden Gruppe zusammen mit pharmazeutisch verwendbarem Trägermaterial.

22. Verfahren zur Herstellung einer Verbindung der Formel I nach Anspruch 1, und von Salzen von solchen Verbindungen, dadurch gekennzeichnet, dass man
a) eine Verbindung der Formel II, 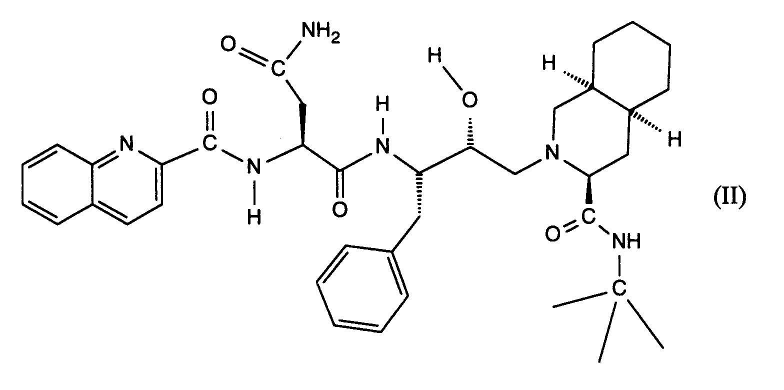 mit einer Carbonsäure der Formel III,
R₁-OH (III)
worin R₁ die in Anspruch 1 genannten Bedeutungen hat, oder einem reaktionsfähigen Derivat davon umsetzt, wobei freie funktionelle Gruppen in den Ausgangsmaterialien der Formeln II und III, die nicht an der Reaktion teilnehmen sollen, erforderlichenfalls in geschützter Form vorliegen, und vorhandene Schutzgruppen abspaltet, oder
b) eine Aminoverbindung der Formel IV, 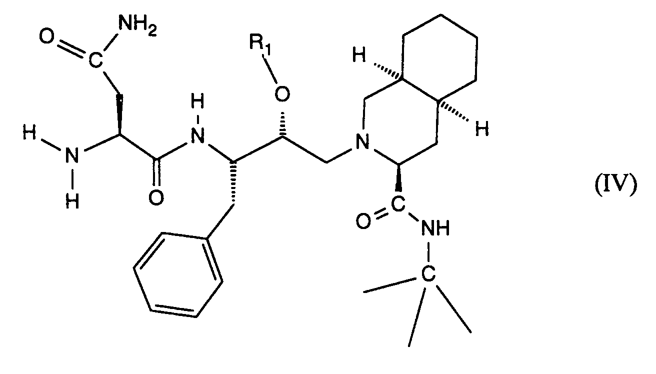 worin R₁ die genannten Bedeutungen hat, oder ein reaktionsfähiges Derivat davon, mit einer Carbonsäure der Formel V, 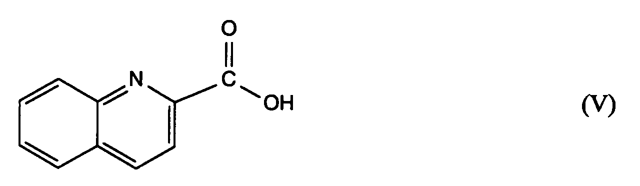 oder einem reaktionsfähigen Säurederivat davon, amidiert, wobei freie funktionelle Gruppen in den Ausgangsmaterialien der Formeln IV und V, die nicht an der Reaktion teilnehmen sollen, erforderlichenfalls in geschützter Form vorliegen, und vorhandene Schutzgruppen abspaltet, oder
c) eine Aminoverbindung der Formel VI, 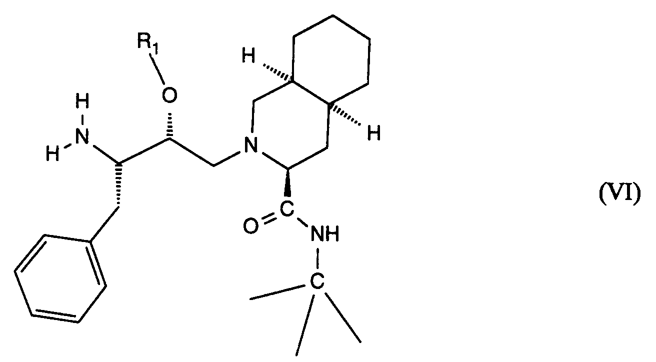 worin R₁ die genannten Bedeutungen hat, oder ein reaktionsfähiges Derivat davon, mit einer Carbonsäure der Formel VII, 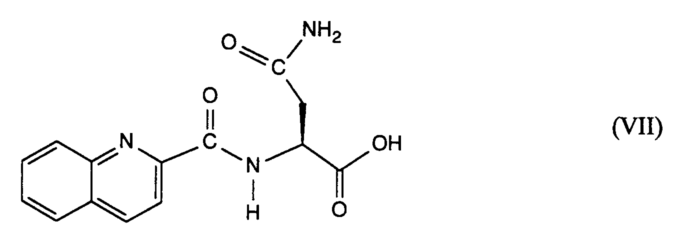 oder einem reaktionsfähigen Säurederivat davon, amidiert, wobei freie funktionelle Gruppen in den Ausgangsmaterialien der Formeln VI und VII, die nicht an der Reaktion teilnehmen sollen, erforderlichenfalls in geschützter Form vorliegen, und vorhandene Schutzgruppen abspaltet,
und gewünschtenfalls eine nach dem vorstehenden Verfahren erhältliche Verbindung der Formel I mit mindestens einer salzbildenden Gruppe in ihr Salz und/oder ein erhältliches Salz in die freie Verbindung oder in ein anderes Salz überführt und/oder gegebenenfalls erhältliche Isomerengemische von Verbindungen der Formel I auftrennt und/oder eine erfindungsgemässe Verbindung der Formel I in eine andere erfindungsgemässe Verbindung der Formel I umwandelt.

## Claims

1. A compound of formula I 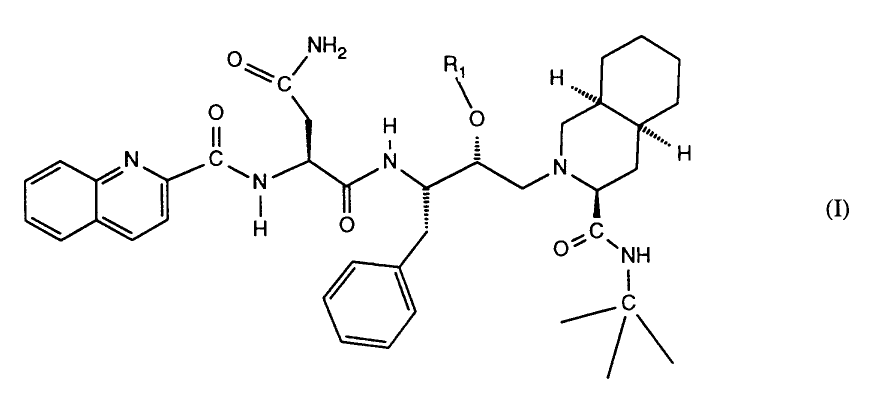 wherein R₁ is octanoyl, decanoyl, dodecanoyl, palmitoyl, unsubstituted or substituted lower alkanoyl, lower alkenoyl or lower alkynoyl,
wherein the substituents are selected from one or more radicals selected from the group consisting of hydroxy, lower alkoxy, lower alkoxy-lower alkoxy, lower alkoxy-lower alkoxy-lower alkoxy, phenoxy, naphthyloxy, phenyl-lower alkoxy, 2-halo-lower alkanoyl, amino-, lower alkylamino- or di-lower alkylamino-lower alkoxy-2-lower alkanoyl, amino-, lower alkylamino- or di-lower alkylamino-lower alkoxy-lower alkoxy-2-lower alkanoyl, lower alkanoyloxy, phenyl-lower alkanoyloxy, halogen, carboxy, lower alkoxycarbonyl, phenyl-lower alkoxycarbonyl, carbamoyl, lower alkylcarbamoyl, hydroxy-lower alkylcarbamoyl, di-lower alkylcarbamoyl, bis(hydroxy-lower alkyl)carbamoyl, carbamoyl the nitrogen atom of which is a constituent of a 5- to 7-membered heterocyclic ring that may contain a further hetero atom selected from oxygen, sulfur, nitrogen and lower alkyl-substituted, such as methyl- or ethyl-substituted, nitrogen, cyano, oxo, cycloalkyl, bicycloalkyl, tricycloalkyl, cycloalkenyl bicycloalkenyl, heterocyclyl, which is a saturated, partially saturated or unsaturated single ring containing from 5 to 7 ring atoms and up to four hetero atoms selected from nitrogen, sulfur and oxygen, the ring either being present as such or in once benzofused, cyclopenta-, cyclohexa- or cyclohepta-fused form, heterocyclyl being unsubstituted or substituted especially by lower alkyl, lower alkanoyl, hydroxy, lower alkoxy, phenyl-lower alkoxy, hydroxy-lower alkyl, halogen, cyano and/or by trifluoromethyl, and C₆-C₁₂aryl, which is unsubstituted or is mono- or poly-substituted by lower alkyl, halo-lower alkyl, halogen, hydroxy, lower alkoxy, lower alkanoyloxy, carboxy, lower alkoxycarbonyl, phenyl-lower alkoxycarbonyl, carbamoyl, mono- or di-lower alkylcarbamoyl, mono- or di-hydroxy-lower alkylcarbamoyl, halo-lower alkyl, heterocyclyl-lower alkyl wherein heterocyclyl is as defined above, cyano and/or by nitro;
lower alkoxycarbonyl, 2-halo-lower alkoxycarbonyl, aryl-lower alkoxycarbonyl wherein aryl has from 6 to 14 carbon atoms and is unsubstituted or is mono- or poly-substituted by lower alkyl, halo-lower alkyl, halogen, hydroxy, lower alkoxy, lower alkanoyloxy, carboxy, lower alkoxycarbonyl, phenyl-lower alkoxycarbonyl, carbamoyl, mono- or di-lower alkylcarbamoyl, mono- or di-hydroxy-lower alkylcarbamoyl, halo-lower alkyl, heterocyclyl-lower alkyl wherein heterocyclyl is as defined above as a substituent of lower alkanoyl, cyano and/or by nitro; heterocyclyl-lower alkoxycarbonyl wherein heterocyclyl is as defined above as a substituent of lower alkanoyl; aminocarbonyl, an aminocarbonyl radical wherein the amino group carries 1 or 2 substituents selected independently of one another from unsubstituted or substituted lower alkyl wherein the substituents are selected from hydroxy, lower alkoxy, phenoxy, naphthyloxy, lower alkanoyloxy, phenyl-lower alkanoyloxy, halogen, carboxy, lower alkoxycarbonyl, phenyl-lower alkoxycarbonyl, carbamoyl, lower alkylcarbamoyl, hydroxy-lower alkylcarbamoyl, di-lower alkylcarbamoyl, bis(hydroxy-lower alkyl)carbamoyl, cyano, oxo and C₆-C₁₂aryl which is unsubstituted or is mono- or poly-substituted, for example, by lower alkyl, halo-lower alkyl, halogen, hydroxy, lower alkoxy, lower alkanoyloxy, carboxy, lower alkoxycarbonyl, phenyl-lower alkoxycarbonyl, carbamoyl, mono- or di-lower alkylcarbamoyl, mono- or di-hydroxy-lower alkylcarbamoyl, halo-lower alkyl, such as trifluoromethyl, cyano and/or by nitro, and aryl which has from 6 to 14 carbon atoms and is unsubstituted or is mono- or poly-substituted by lower alkyl, halo-lower alkyl, halogen, hydroxy, lower alkoxy, lower alkanoyloxy, carboxy, lower alkoxycarbonyl, phenyl-lower alkoxycarbonyl, carbamoyl, mono- or di-lower alkylcarbamoyl, mono- or di-hydroxy-lower alkylcarbamoyl, halo-lower alkyl, heterocyclyl-lower alkyl wherein heterocyclyl is as defined above as a substituent of lower alkanoyl, cyano and/or by nitro, not more than one of the substituents of the aminocarbonyl radical being aryl;
or the residue, bonded *via* a carboxy group, of an amino acid selected from glycine, alanine, 2-aminobutyric acid, 3-aminobutyric acid, 4-aminobutyric acid, 3-aminopentanoic acid, 4-aminopentanoic acid, 5-aminopentanoic acid, 3-aminohexanoic acid, 4-aminohexanoic acid or 5-aminohexanoic acid, valine, norvaline, leucine, isoleucine, norleucine, serine, homoserine, threonine, methionine, cysteine, phenylalanine, tyrosine, 4-aminophenylalanine, 4-chlorophenylalanine, 4-carboxyphenylalanine, β-phenylserine, phenylglycine, α-naphthylalanine, cyclohexylalanine, cyclohexylglycine, tryptophan, aspartic acid, asparagine, aminomalonic acid, aminomalonic acid monoamide, glutamic acid, glutamine, histidine, arginine, lysine, δ-hydroxylysine, ornithine, 3-aminopropanoic acid, α,γ-diaminobutyric acid and α,β-diaminopropionic acid, it being possible for each of the mentioned amino acids to be in the D-, L- or (D,L)-form (except in cases where there is no asymmetric carbon atom),
an α-amino group, if present, is unsubstituted or is mono- or di-N-alkylated by lower alkyl, by amino-lower alkyl, by phenyl- or naphthyl-amino-lower alkyl, by phenyl-lower alkyl, by diphenylmethyl, by trityl and/or by heterocyclyl-lower alkyl wherein heterocyclyl is as defined above for substituted lower alkanoyl R₁, and/or is N-acylated by an unsubstituted or substituted lower alkanoyl radical mentioned above in the definition of R₁, by lower alkoxycarbonyl or by phenyl-lower alkoxycarbonyl;
a carboxy group of the side chain is present in free form, in the form of a lower alkyl ester group, an aryl ester group or an aryl-lower alkyl ester group, wherein aryl is phenyl, 4-nitrophenyl, naphthyl, fluorenyl or biphenylyl, or in the form of a carbamoyl, a lower alkylcarbamoyl, a di-lower alkylaminocarbamoyl, a mono- or di-(hydroxy-lower alkyl)carbamoyl or a mono- or di-(carboxy-lower alkyl)carbamoyl group,
an amino group of the side chain that is not in the α-position is present in free form or in the form of mono- or di-lower alkylamino, lower alkanoylamino, amino-lower alkanoylamino, aryl-lower alkanoylamino wherein aryl has from 6 to 14 carbon atoms and is unsubstituted or substituted by lower alkyl, hydroxy, lower alkoxy, carboxy, carbamoyl or by sulfamoyl, lower alkoxycarbonylamino, arylmethoxycarbonylamino wherein aryl has from 6 to 14 carbon atoms, piperidyl-1-carbonyl, morpholinocarbonyl, thiomorpholinocarbonyl or S,S-dioxothiomorpholinocarbonyl, and/or
a hydroxy group of the side chain is present in free form or in the form of a lower alkoxy, phenyl-lower alkoxy, lower alkanoyloxy or lower alkoxycarbonyloxy group, or a salt thereof.

2. A compound according to claim 1 of formula I wherein R₁ is unsubstituted or substituted lower alkanoyl, lower alkenoyl or lower alkynoyl,
wherein the substituents are selected from one or more radicals selected from the group consisting of hydroxy, lower alkoxy, phenoxy, naphthyloxy, lower alkanoyloxy, phenyl-lower alkanoyloxy, halogen, carboxy, lower alkoxycarbonyl, phenyl-lower alkoxycarbonyl, carbamoyl, lower alkylcarbamoyl, hydroxy-lower alkylcarbamoyl, di-lower alkylcarbamoyl, bis(hydroxy-lower alkyl)carbamoyl, carbamoyl the nitrogen atom of which is a constituent of a 5 to 7-membered heterocyclic ring that may contain a further hetero atom selected from oxygen, sulfur, nitrogen and lower alkyl-substituted, such as methyl- or ethyl-substituted, nitrogen, cyano, oxo, cycloalkyl, bicycloalkyl, tricycloalkyl, cycloalkenyl, bicycloalkenyl, heterocyclyl, which is a saturated, partially saturated or unsaturated single ring containing from 5 to 7 ring atoms and up to four hetero atoms selected from nitrogen, sulfur and oxygen, the ring either being present as such or in once benzofused, cyclopenta-, cyclohexa- or cyclohepta-fused form, heterocyclyl being unsubstituted or substituted especially by lower alkyl, lower alkanoyl, hydroxy, lower alkoxy, phenyl-lower alkoxy, hydroxy-lower alkyl, halogen, cyano and/or by trifluoromethyl, and C₆-C₁₂aryl which is unsubstituted or is mono- or poly-substituted by lower alkyl, halo-lower alkyl, halogen, hydroxy, lower alkoxy, lower alkanoyloxy, carboxy, lower alkoxycarbonyl, phenyl-lower alkoxycarbonyl, carbamoyl, mono- or di-lower alkylcarbamoyl, mono- or di-hydroxy-lower alkylcarbamoyl, halo-lower alkyl, heterocyclyl-lower alkyl wherein heterocyclyl is as defined above, cyano and/or by nitro;
lower alkoxycarbonyl, 2-halo-lower alkoxycarbonyl, aryl-lower alkoxycarbonyl wherein aryl has from 6 to 14 carbon atoms and is unsubstituted or is mono- or poly-substituted by lower alkyl, halo-lower alkyl, halogen, hydroxy, lower alkoxy, lower alkanoyloxy, carboxy, lower alkoxycarbonyl, phenyl-lower alkoxycarbonyl, carbamoyl, mono- or di-lower alkylcarbamoyl, mono- or di-hydroxy-lower alkylcarbamoyl, halo-lower alkyl, heterocyclyl-lower alkyl wherein heterocyclyl is as defined above as a substituent of lower alkanoyl, cyano and/or by nitro; heterocyclyl-lower alkoxycarbonyl wherein heterocyclyl is as defined above as a substituent of lower alkanoyl; aminocarbonyl, an aminocarbonyl radical wherein the amino group carries 1 or 2 substituents selected independently of one another from unsubstituted or substituted lower alkyl, wherein the substituents are selected from hydroxy, lower alkoxy, phenoxy, naphthyloxy, lower alkanoyloxy, phenyl-lower alkanoyloxy, halogen, carboxy, lower alkoxycarbonyl, phenyl-lower alkoxycarbonyl, carbamoyl, lower alkylcarbamoyl, hydroxy-lower alkylcarbamoyl, di-lower alkylcarbamoyl, bis(hydroxy-lower alkyl)carbamoyl, cyano, oxo and C₆-C₁₂aryl, which is unsubstituted or is mono- or poly-substituted, for example, by lower alkyl, halo-lower alkyl, halogen, hydroxy, lower alkoxy, lower alkanoyloxy, carboxy, lower alkoxycarbonyl, phenyl-lower alkoxycarbonyl, carbamoyl, mono- or di-lower alkylcarbamoyl, mono- or di-hydroxy-lower alkylcarbamoyl, halo-lower alkyl, such as trifluoromethyl, cyano and/or by nitro, and aryl which has from 6 to 14 carbon atoms and is unsubstituted or is mono- or poly-substituted by lower alkyl, halo-lower alkyl, halogen, hydroxy, lower alkoxy, lower alkanoyloxy, carboxy, lower alkoxycarbonyl, phenyl-lower alkoxycarbonyl, carbamoyl, mono- or di-lower alkylcarbamoyl, mono- or di-hydroxy-lower alkylcarbamoyl, halo-lower alkyl, heterocyclyl-lower alkyl wherein heterocyclyl is as defined above as a substituent of lower alkanoyl, cyano and/or by nitro, not more than one of the substituents of the aminocarbonyl radical being aryl;
or the residue, bonded *via* a carboxy group, of an amino acid selected from glycine, alanine, 2-aminobutyric acid, 3-aminobutyric acid, 4-aminobutyric acid, 3-aminopentanoic acid, 4-aminopentanoic acid, 5-aminopentanoic acid, 3-aminohexanoic acid, 4-aminohexanoic acid or 5-aminohexanoic acid, valine, norvaline, leucine, isoleucine, norleucine, serine, homoserine, threonine, methionine, cysteine, phenylalanine, tyrosine, 4-aminophenylalanine, 4-chlorophenylalanine, 4-carboxyphenylalanine, β-phenylserine, phenylglycine, α-naphthylalanine, cyclohexylalanine, cyclohexylglycine, tryptophan, aspartic acid, asparagine, aminomalonic acid, aminomalonic acid monoamide, glutamic acid, glutamine, histidine, arginine, lysine, δ-hydroxylysine, ornithine, 3-aminopropanoic acid, α,γ-diaminobutyric acid and α,β-diaminopropionic acid, it being possible for each of the mentioned amino acids to be in the D-, L- or (D,L)-form (except in cases where there is no asymmetric carbon atom),
an α-amino group, if present, is unsubstituted or is mono- or di-N-alkylated by lower alkyl, by amino-lower alkyl, by phenyl- or naphthyl-amino-lower alkyl, by phenyl-lower alkyl, by diphenylmethyl, by trityl and/or by heterocyclyl-lower alkyl wherein heterocyclyl is as defined above for substituted lower alkanoyl R₁, and/or is N-acylated by an unsubstituted or substituted lower alkanoyl radical mentioned above in the definition of R₁, by lower alkoxycarbonyl or by phenyl-lower alkoxycarbonyl;
a carboxy group of the side chain is present in free form, in the form of a lower alkyl ester group, an aryl ester group or an aryl-lower alkyl ester group, wherein aryl is phenyl, 4-nitrophenyl, naphthyl, fluorenyl or biphenylyl, or in the form of a carbamoyl, a lower alkylcarbamoyl, a di-lower alkylaminocarbamoyl, a mono- or di-(hydroxy-lower alkyl)carbamoyl or a mono- or di-(carboxy-lower alkyl)carbamoyl group,
an amino group of the side chain that is not in the α-position is present in free form or in the form of mono- or di-lower alkylamino, lower alkanoylamino, amino-lower alkanoylamino, aryl-lower alkanoylamino wherein aryl has from 6 to 14 carbon atoms and is unsubstituted or substituted by lower alkyl, hydroxy, lower alkoxy, carboxy, carbamoyl or by sulfamoyl, lower alkoxycarbonylamino, arylmethoxycarbonylamino wherein aryl has from 6 to 14 carbon atoms, piperidyl-1-carbonyl, morpholinocarbonyl, thiomorpholinocarbonyl or S,S-dioxothiomorpholinocarbonyl; and/or
a hydroxy group of the side chain is present in free form or in the form of a lower alkoxy, phenyl-lower alkoxy, lower alkanoyloxy or lower alkoxycarbonyloxy group; or a salt thereof.

3. A compound according to either claim 1 or claim 2 of formula I wherein R₁ is octanoyl, decanoyl, dodecanoyl, palmitoyl, unsubstituted or substituted lower alkanoyl,
wherein the substituents are selected from one to three radicals selected from the group consisting of hydroxy, lower alkoxy, lower alkoxy-lower alkoxy, lower alkoxy-lower alkoxy-lower alkoxy, phenoxy, naphthyloxy, phenyl-lower alkoxy, lower alkanoyloxy, phenyl-lower alkanoyloxy, amino-, lower alkylamino- or di-lower alkylamino-lower alkoxy-2-lower alkanoyl, amino-, lower alkylamino- or di-lower alkylamino-lower alkoxy-lower alkoxy-2-lower alkanoyl, halogen, carboxy, lower alkoxycarbonyl, phenyl-lower alkoxycarbonyl, carbamoyl, lower alkylcarbamoyl, hydroxy-lower alkylcarbamoyl, di-lower alkylcarbamoyl, bis(hydroxy-lower alkyl)carbamoyl, cyano, oxo, C₃-C₈cycloalkyl, C₄-C₈cycloalkenyl, pyrrolyl, 2,5-dihydropyrrolyl, furanyl, thienyl, tetrahydrofuranyl, pyrrolyl, pyrrolidinyl, imidazolyl, imidazolidinyl, pyrazolyl, pyrazolidinyl, tetrahydro-oxazolyl, tetrahydro-isoxazolyl tetrahydro-thiazolyl, tetrahydro-isothiazolyl, indolyl, isoindolyl, quinolyl, isoquinolyl, benzimidazolyl, benzofuranyl, pyridyl, pyrimidinyl, piperidinyl, piperazin-1-yl, morpholino, thiomorpholino, S,S-dioxothiomorpholino, 1,2-dihydro- or 1,2,3,4-tetrahydro-quinolyl or 1,2-dihydro- or 1,2,3,4-tetrahydro-isoquinolyl, the said heterocyclic radicals being unsubstituted or substituted by lower alkyl, lower alkanoyl, hydroxy, lower alkoxy, phenyl-lower alkoxy, hydroxy-lower alkyl, halogen, cyano and/or by trifluoromethyl, and aryl selected from phenyl, naphthyl, indanyl, indenyl and fluorenyl, those radicals being unsubstituted or mono- or poly-substituted by lower alkyl, halo-lower alkyl, halogen, hydroxy, lower alkoxy, lower alkanoyloxy, carboxy, lower alkoxycarbonyl, phenyl-lower alkoxycarbonyl, carbamoyl, mono- or di-lower alkylcarbamoyl, mono- or di-hydroxy-lower alkylcarbamoyl, halo-lower alkyl, piperidinomethyl, piperazin-1-ylmethyl, 4-lower alkylpiperazin-1-ylmethyl, 4-lower alkanoyl-piperazin-1-ylmethyl, morpholinomethyl, thiomorpholinomethyl, cyano and/or by nitro;
or the residue, bonded *via* a carboxy group, of an amino acid selected from glycine, alanine, 2-aminobutyric acid, 3-aminobutyric acid, 4-aminobutyric acid, 3-aminopentanoic acid, 4-aminopentanoic acid, 5-aminopentanoic acid, 3-aminohexanoic acid, 4-aminohexanoic acid or 5-aminohexanoic acid, valine, norvaline, leucine, isoleucine, norleucine, serine, homoserine, threonine, methionine, cysteine, phenylalanine, tyrosine, 4-aminophenylalanine, 4-chlorophenylalanine, 4-carboxyphenylalanine, β-phenylserine, phenylglycine, α-naphthylalanine, cyclohexylalanine, cyclohexylglycine, tryptophan, aspartic acid, asparagine, aminomalonic acid, aminomalonic acid monoamide, glutamic acid, glutamine, histidine, arginine, lysine, δ-hydroxylysine, ornithine, 3-aminopropanoic acid, α,γ-diaminobutyric acid and α,β-diaminopropionic acid, it being possible for each of the mentioned amino acids to be in the D-, L- or (D,L)-configuration (except in cases where there is no asymmetric carbon atom),
an α-amino group, if present, is unsubstituted or is mono- or di-N-alkylated by lower alkyl, by amino-lower alkyl, by phenyl- or naphthyl-amino-lower alkyl, by phenyl-lower alkyl, by diphenylmethyl, by trityl, by furanyl-lower alkyl, by thienyl-lower alkyl, by imidazolyl-lower alkyl and/or by 2-, 3- or 4-pyridyl-lower alkyl, and/or is N-acylated by an unsubstituted or substituted lower alkanoyl radical mentioned above in the definition of R₁, by lower alkoxycarbonyl or by phenyl-lower alkoxycarbonyl; or a pharmaceutically acceptable salt thereof.

4. A compound according claim 2 of formula I wherein R₁ is unsubstituted or substituted lower alkanoyl,
wherein the substituents are selected from one to three radicals selected from the group consisting of hydroxy, lower alkoxy, phenoxy, naphthyloxy, lower alkanoyloxy, phenyl-lower alkanoyloxy, halogen, carboxy, lower alkoxycarbonyl, phenyl-lower alkoxycarbonyl, carbamoyl, lower alkylcarbamoyl, hydroxy-lower alkylcarbamoyl, di-lower alkylcarbamoyl, bis(hydroxy-lower alkyl)carbamoyl, cyano, oxo, C₃-C₈cycloalkyl, C₄-C₈cycloalkenyl, pyrrolyl, 2,5-dihydropyrrolyl, furanyl, thienyl, tetrahydrofuranyl, pyrrolyl, pyrrolidinyl, imidazolyl, imidazolidinyl, pyrazolyl, pyrazolidinyl, tetrahydrooxazolyl, tetrahydro-isoxazolyl, tetrahydro-thiazolyl, tetrahydro-isothiazolyl, indolyl, isoindolyl, quinolyl, isoquinolyl, benzimidazolyl, benzofuranyl, pyridyl, pyrimidinyl, piperidinyl, piperazin-1-yl, morpholino, thiomorpholino, S,S-dioxothiomorpholino, 1,2-dihydro- or 1,2,3,4-tetrahydro-quinolyl or 1,2-dihydro- or 1,2,3,4-tetrahydroisoquinolyl, the said heterocyclic radicals being unsubstituted or substituted by lower alkyl, lower alkanoyl, hydroxy, lower alkoxy, phenyl-lower alkoxy, hydroxy-lower alkyl, halogen, cyano and/or by trifluoromethyl, and aryl selected from phenyl, naphthyl, indanyl, indenyl and fluorenyl, those radicals being unsubstituted or mono- or poly-substituted by lower alkyl, halo-lower alkyl, halogen, hydroxy, lower alkoxy, lower alkanoyloxy, carboxy, lower alkoxycarbonyl, phenyl-lower alkoxycarbonyl, carbamoyl, mono- or di-lower alkylcarbamoyl, mono- or di-hydroxy-lower alkylcarbamoyl, halo-lower alkyl, piperidinomethyl, piperazin-1-ylmethyl, 4-lower alkyl-piperazin-1-ylmethyl, 4-lower alkanoyl-piperazin-1-ylmethyl, morpholinomethyl, thiomorpholinomethyl, cyano and/or by nitro;
or the residue, bonded *via* a carboxy group, of an amino acid selected from glycine, alanine, 2-aminobutyric acid, 3-aminobutyric acid, 4-aminobutyric acid, 3-aminopentanoic acid, 4-aminopentanoic acid, 5-aminopentanoic acid, 3-aminohexanoic acid, 4-aminohexanoic acid or 5-aminohexanoic acid, valine, norvaline, leucine, isoleucine, norleucine, serine, homoserine, threonine, methionine, cysteine, phenylalanine, tyrosine, 4-aminophenylalanine, 4-chlorophenylalanine, 4-carboxyphenylalanine, β-phenylserine, phenylglycine, α-naphthylalanine, cyclohexylalanine, cyclohexylglycine, tryptophan, aspartic acid, asparagine, aminomalonic acid, aminomalonic acid monoamide, glutamic acid, glutamine histidine, arginine, lysine, δ-hydroxylysine, ornithine, 3-aminopropanoic acid, α,γ-diaminobutyric acid and α,β-diaminopropionic acid, it being possible for each of the mentioned amino acids to be in the D-, L- or (D,L)-configuration (except in cases where there is no asymmetric carbon atom), wherein
an α-amino group, if present, is unsubstituted or is mono- or di-N-alkylated by lower alkyl, by amino-lower alkyl, by phenyl- or naphthyl-amino-lower alkyl, by phenyl-lower alkyl, by diphenylmethyl, by trityl, by furanyl-lower alkyl, by thienyl-lower alkyl and/or by 2-, 3- or 4-pyridyl-lower alkyl, and/or is N-acylated by an unsubstituted or substituted lower alkanoyl radical mentioned above in the definition of R₁, by lower alkoxycarbonyl or by phenyl-lower alkoxycarbonyl;
or a pharmaceutically acceptable salt thereof.

5. A compound of formula I according to claim 1 wherein R₁ is octanoyl, decanoyl, dodecanoyl, palmitoyl, lower alkanoyl, hydroxy-lower alkanoyl, lower alkoxy-lower alkanoyl, lower alkoxy-lower alkoxy-lower alkanoyl, lower alkoxy-lower alkoxy-lower alkoxy-lower alkanoyl, phenoxy-lower alkanoyl, phenyl-lower alkoxy-lower alkanoyl, amino-, lower alkylamino- or di-lower alkylamino-lower alkoxy-2-lower alkanoyl, amino-, lower alkylamino- or di-lower alkylamino-lower alkoxy-lower alkoxy-2-lower alkanoyl, lower alkanoyloxy-lower alkanoyl, carboxy-lower alkanoyl, oxo-lower alkanoyl, 5-hydroxymethyl-furan-2-ylcarbonyl, 2- or 3-pyrrolylcarbonyl, furylcarbonyl, thienylcarbonyl, pyridyl-lower alkanoyl, quinolylcarbonyl, isoquinolylcarbonyl, 2-, 3- or 5-indolylcarbonyl, pyrrolidinyl-(2- or 3-)carbonyl, 2-, 3- or 4-piperidinylcarbonyl, 1,2,3,4-tetrahydroquinolyl-2-, -3- or -4-carbonyl, 1,2,3,4-tetrahydroisoquinolyl-1-, -3- or -4-carbonyl, imidazolyl-lower alkanoyl, pyrazolyl-lower alkanoyl, morpholinocarbonyl thiomorpholinocarbonyl, morpholinoacetyl, thiomorpholinoacetyl, 4-lower alkyl-1-piperazinoacetyl, indolylacetyl, benzofuranylacetyl, phenyl-lower alkanoyl that is unsubstituted or mono- or poly-substituted in the phenyl radical by lower alkyl, halo-lower alkyl, halogen, hydroxy, lower alkoxy, piperidinomethyl, piperazin-1-ylmethyl, 4-lower alkylpiperazin-1-ylmethyl, morpholinomethyl, thiomorpholinomethyl, cyano and/or by nitro; or the residue, bonded *via* a carboxy group, of an aliphatic amino acid selected from alanine, valine, norvaline, leucine, 3-aminopropionic acid, 2-aminobutyric acid and isoleucine, or of an amino acid selected from glycine, asparagine, glutamine, methionine, lysine, phenylalanine, 3-aminobutyric acid, 4-aminobutyric acid, 3-aminopentanoic acid, 4-aminopentanoic acid, 5-aminopentanoic acid, 3-aminohexanoic acid, 4-aminohexanoic acid and 5-aminohexanoic acid, it being possible for each of the mentioned amino acids to be in the D-, L- or (D,L)-form (except where there are no asymmetric carbon atoms), and an α-amino group, if present, is unsubstituted or is mono- or di-N-alkylated by lower alkyl, by phenyl-lower alkyl, by 2-, 3- or 4-pyridyl-lower alkyl, by imidazolyl-lower alkyl and/or by 2-, 3- or 4-pyridyl-lower alkyl, such as 2-, 3- or 4-pyridylmethyl, and/or is N-acylated by lower alkoxycarbonyl or by benzyloxycarbonyl,
or a pharmaceutically acceptable salt thereof.

6. A compound according to claim 1 of formula I wherein R₁ is lower alkanoyl, hydroxy-lower alkanoyl, lower alkoxy-lower alkanoyl, phenoxy-lower alkanoyl, lower alkanoyloxy-lower alkanoyl, oxo-lower alkanoyl, 5-hydroxymethyl-furan-2-ylcarbonyl, 2- or 3-pyrrolylcarbonyl, furylcarbonyl, for example 2-furylcarbonyl, thienylcarbonyl, pyridylcarbonyl, 2-, 3- or 5-indolylcarbonyl, pyrrolidinyl-3-carbonyl, 2-, 3- or 4-piperidinylcarbonyl, 1,2,3,4-tetrahydroquinolyl-2-, -3- or -4-carbonyl, 1,2,3,4-tetrahydroisoquinolyl-1-, -3- or -4-carbonyl, imidazolylcarbonyl, morpholinocarbonyl, thiomorpholinocarbonyl, morpholinoacetyl, thiomorpholinoacetyl, 4-lower alkyl-1-piperazinoacetyl, indolylacetyl, benzofuranylacetyl, phenyl-lower alkanoyl that is unsubstituted or mono- or poly-substituted in the phenyl radical by lower alkyl, halo-lower alkyl, halogen, hydroxy, lower alkoxy, piperidinomethyl, piperazin-1-ylmethyl, 4-lower alkyl-piperazin-1-yl-methyl, morpholinomethyl, thiomorpholinomethyl, cyano and/or by nitro; or the residue, bonded *via* a carboxy group, of an aliphatic amino acid selected from alanine, valine, norvaline, leucine, 3-aminopropionic acid, 2-aminobutyric acid, 3-aminobutyric acid, 4-aminobutyric acid, 3-aminopentanoic acid, 4-aminopentanoic acid, 5-aminopentanoic acid, 3-aminohexanoic acid, 4-aminohexanoic acid, 5-aminohexanoic acid and isoleucine, or of an amino acid selected from glycine, asparagine, glutamine, methionine, lysine, proline and phenylalanine, it being possible for each of the mentioned amino acids to be in the D-, L- or (D,L)-form (except in cases where there is no asymmetric carbon atom); an α-amino group, if present, is unsubstituted or is mono- or di-N-alkylated by lower alkyl, by phenyl-lower alkyl and/or by 2-, 3- or 4-pyridyl-lower alkyl, and/or is N-acylated by lower alkoxycarbonyl or by phenyl-lower alkoxycarbonyl,
or a pharmaceutically acceptable salt thereof.

7. A compound of formula I according to claim 1 wherein R₁ is octanoyl, decanoyl, dodecanoyl, palmitoyl, lower alkanoyl, lower alkoxy-lower alkanoyl, lower alkoxy-lower alkoxy-lower alkanoyl, lower alkoxy-lower alkoxy-lower alkanoyl, phenoxy-lower alkanoyl, phenyl-lower alkoxy-lower alkanoyl, amino-, lower alkylamino- or di-lower alkylamino-lower alkoxy-2-lower alkanoyl, amino-, lower alkylamino- or di-lower alkylamino-lower alkoxy-lower alkoxy-2-lower alkanoyl, 2-lower alkoxy-2-phenylacetyl furylcarbonyl, pyridyl-lower alkanoyl, quinolylcarbonyl, isoquinolylcarbonyl, pyrrolidinyl-2-carbonyl, imidazolyl-lower alkanoyl, pyrazolyl-lower alkanoyl, phenyl-lower alkanoyl, 4-chloromethylbenzoyl, 4-morpholinomethylbenzoyl, 4-thiomorpholinomethylbenzoyl, aminoacetyl, N-lower alkylaminoacetyl, N,N-di-lower alkylaminoacetyl N-lower alkyl-N-phenyl-lower alkylaminoacetyl, N-lower alkyl-N-benzyloxycarbonylaminoacetyl, N-imidazolyl-lower alkyl-N-lower alkylaminoacetyl, N-lower alkyl-N-pyridyl-lower alkylaminoacetyl or 4-(N,N-dimethylamino)butyryl, or a pharmaceutically acceptable salt thereof.

8. A compound according to claim 1 of formula I wherein R₁ is lower alkanoyl, furylcarbonyl, pyridylcarbonyl, phenyl-lower alkanoyl, 4-morpholinomethylbenzoyl, 4-thiomorpholinomethylbenzoyl, aminoacetyl, N-lower alkylaminoacetyl, N,N-di-lower alkylaminoacetyl, N-lower alkyl-N-phenyl-lower alkylaminoacetyl, N-lower alkyl-N-pyridyl-lower alkylaminoacetyl or N-phenyl-lower alkoxycarbonyl-N-lower alkylaminoacetyl or a pharmaceutically acceptable salt thereof.

9. A compound of formula I according to claim 1 wherein R₁ is lower alkanoyl, lower alkoxy-lower alkanoyl, pyridylcarbonyl or furylcarbonyl, or a pharmaceutically acceptable salt thereof.

10. A compound according to claim 1 of formula I wherein R₁ is lower alkanoyl or furylcarbonyl, or a pharmaceutically acceptable salt thereof.

11. A compound according to claim 1 of formula I wherein R₁ is lower alkoxy-lower alkanoyl or pyridylcarbonyl, or a pharmaceutically acceptable salt thereof.

12. A compound according to claim 1 of formula I wherein R₁ is lower alkanoyl, or a pharmaceutically acceptable salt thereof.

13. A compound of formula I according to claim 1 selected from the following compounds:
N-tert-butyl-decahydro-2-[2(R)-(furan-2-carboxy)-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-pivaloyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-(N-methylaminoacetoxy)-4-phenyl-3(S)-[[N-(2-quinolylcarbonyl)-L-asparaginyl]amino]butyl]-(4aS,8aS)-isquinoline-3(S)-carboxamide,
N-tert-butyl-decahydro-2-[2(R)-(N-benzyloxycarbonyl-N-methylaminoacetoxy)-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-(N-dimethylaminoacetyloxy)-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-(N-n-butyl-N-methylaminoacetyloxy)-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-(N-methyl-N-benzylaminoacetyloxy)-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-(N-methyl-N-3-pyridylmethyl-aminoacetyloxy)-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-(3-pyridylcarboxy)-4-phenyl-3(S)-[[N-(2-quinolylcarbonyl)-L-asparaginyl]amino]butyl]-(4aS,8aS)-isoquinoline-3(S)-carboxamide, and
N-tert-butyl-decahydro-2-[2(R)-(4-(N-morpholinyl-methyl)-benzoyloxy)-4-phenyl-3(S)-[[N-(2-quinolylcarbonyl)-L-asparaginyl]amino]butyl]-(4aS,8aS)-isoquinoline-3(S)-carboxamide,
or a pharmaceutically acceptable salt thereof.

14. A compound of formula I according to claim 1 selected from the following compounds:
N-tert-butyl-decahydro-2-[2(R)-propionyloxy-4-phenyl-3(S)-[[N-(2-quinolylcarbonyl)-L-asparaginyl]amino]butyl]-(4aS,8aS)-isoquinoline-3(S)-carboxamide;
N-tert-butyl-decahydro-2-[2(R)-butyryloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-methylpropionyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-pentanoyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-octanoyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-decanoyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-dodecanoyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-palmitoyloxy-4-phenyl-3(S)-[[N-(2-quinolylcarbonyl)-L-asparaginyl]amino]butyl]-(4aS,8aS)-isoquinoline-3(S)-carboxamide
N-tert-butyl-decahydro-2-[2(R)-(3-carboxypropionyloxy)-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-(3-methoxypropionyloxy)-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-benzyloxyacetyloxy-4-phenyl-3(S)-[[N-(2-quinolylcarbonyl)-L-asparaginyl]amino]butyl]-(4aS,8aS)-isoquinoline-3(S)-carboxamide
N-tert-butyl-decahydro-2-[2(R)-{(S)-α-methoxy-α-phenylacetyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{(R)-α-methoxy-α-phenylacetyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{2-(2-methoxyethoxy)acetyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{n-butoxyacetyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{2-(2-(2-methoxyethoxy)ethoxy)acetyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{2-pyridylacetyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{3-(2-pyridyl)propionyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{4-imidazolylcarbonyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{4-imidazolylacetyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{3-(4-imidazolyl)propionyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-quinolin-2-ylcarbonyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-phenoxyacetyloxy-4-phenyl-3(S)-[[(N-(2-quinolylcarbonyl)-L-asparaginyl]amino]butyl]-(4aS,8aS)-isoquinoline-3(S)-carboxamide,
N-tert-butyl-decahydro-2-[2(R)-pyridine-4-carbonyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-pyridine-2-carbonyloxy-4-phenyl-3(S)-[[N-(2-quinolylcarbonyl)-L-asparaginyl]amino]butyl]-(4aS,8aS)-isoquinoline-3(S)-carboxamide
N-tert-butyl-decahydro-2-[2(R)-{1-pyrazolylacetyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-pyrazin-2-ylcarbonyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-isoquinoline-3-carbonyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-aminoacetyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{(L)-pyrrolidine-2-carbonyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{N-(imidazolyl-4-methyl)-N-methylaminoacetyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{N-(pyridin-2-ylmethyl)-N-methylaminoacetyloxy4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{N,N-(4-dimethylaminobutyryl)oxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-benzoyloxy-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{4-chloromethylbenzoyloxy}-4-phenyl-3(S)-[[N-(2-quinolylcarbonyl)-L-asparaginyl]amino]butyl]-(4aS,8aS)-isoquinoline-3(S)-carboxamide,
N-tert-butyl-decahydro-2-[2(R)-{3-(N,N-dimethylaminopropyl)oxyacetyl)-4-phenyl-3(S)-[[N-(2-quinolylcarbonyl)-L-asparaginyl]amino]butyl]-(4aS,8aS)-isoquinoline-3(S)-carboxamide, and
N-tert-butyl-decahydro-2-[2(R)-{2-[3-(N,N-dimethylaminopropoxy)ethoxy]acetyl}-4-phenyl-3(S)-[[N-(2-quinolylcarbonyl)-L-asparaginyl]amino]butyl]-(4aS,8aS)-isoquinoline-3(S)-carboxamide,
or a pharmaceutically acceptable salt thereof.

15. A compound of formula I according to claim 1 wherein R₁ is acetyl, or a pharmaceutically acceptable salt thereof.

16. A compound of formula I according to claim 1 wherein R₁ is furan-2-ylcarbonyl, or a pharmaceutically acceptable salt thereof.

17. A compound of formula I according to claim 1 wherein R₁ is methoxyacetyl, or a pharmaceutically acceptable salt thereof.

18. A compound of formula I according to claim 1 wherein R₁ is pyridin-2-ylcarbonyl, or a pharmaceutically acceptable salt thereof.

19. A compound of formula I or a salt thereof according to any one of claims 1 to 18 for use in a method for the therapeutic treatment of the human or animal body.

20. The use of a compound according to any one of claims 1 to 18 of formula I or a pharmaceutically acceptable salt thereof for the preparation of a pharmaceutical composition for the treatment of AIDS.

21. A pharmaceutical composition comprising a compound of formula I according to any one of claims 1 to 18 or a pharmaceutically acceptable salt of such a compound having at least one salt-forming group, together with a pharmaceutically acceptable carrier.

22. A process for the preparation of a compound of formula I according to claim 1, or of a salt of such a compound, which comprises
a) reacting a compound of formula II 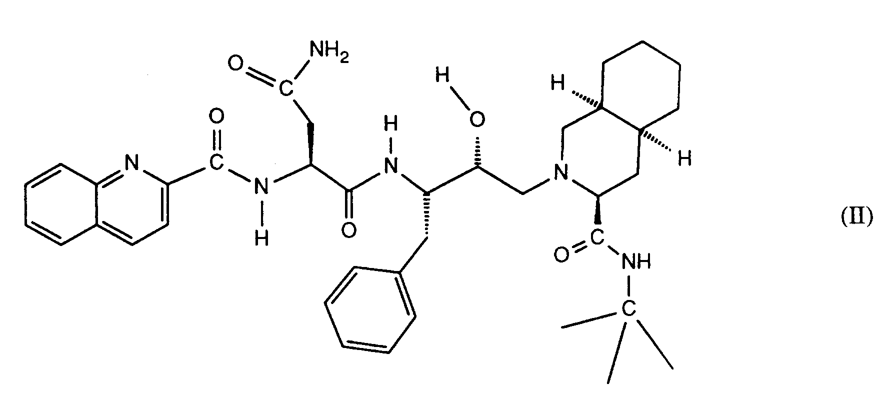 with a carboxylic acid of formula III
R₁-OH (III),
wherein R₁ is as defined in claim 1, or with a reactive derivative thereof, free functional groups in the starting materials of formulae II and III that are not intended to participate in the reaction being if necessary in protected form, and removing any protecting groups present, or
b) amidating an amino compound of formula IV 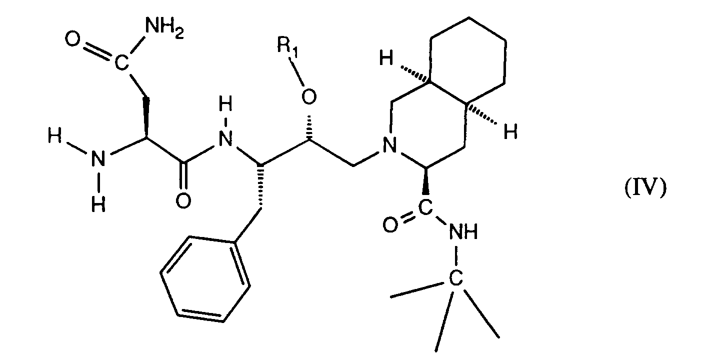 wherein R₁ is as defined, or a reactive derivative thereof, with a carboxylic acid of formula V 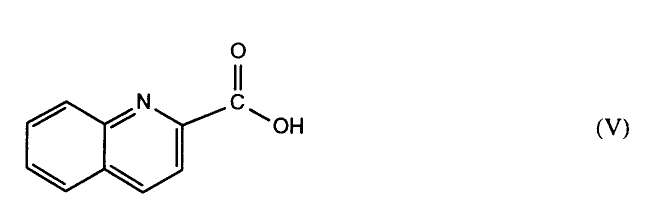 or with a reactive acid derivative thereof, free functional groups in the starting materials of formulae IV and V that are not intended to participate in the reaction being if necessary in protected form, and removing any protecting groups present, or
c) amidating an amino compound of formula VI 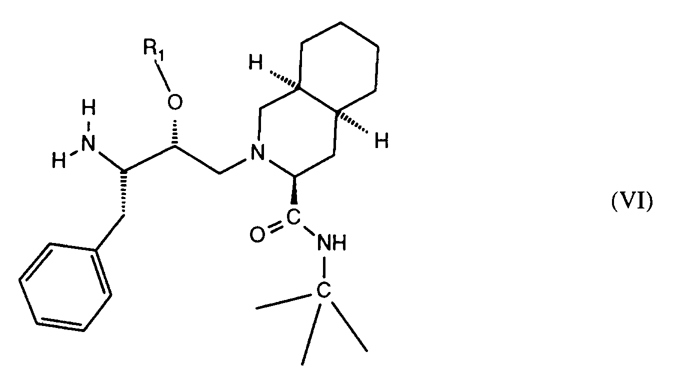 wherein R₁ is as defined, or a reactive derivative thereof, with a carboxylic acid of formula VII 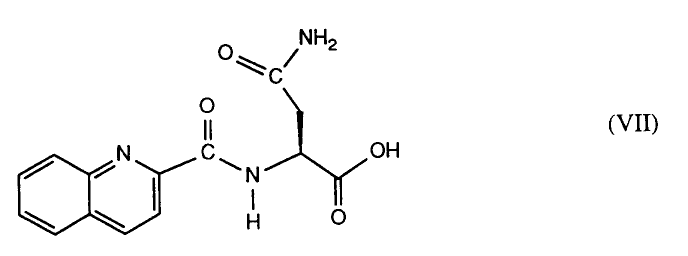 or with a reactive acid derivative thereof, free functional groups in the starting materials of formulae VI and VII that are not intended to participate in the reaction being if necessary in protected form, and removing any protecting groups present,
and, if desired, converting a compound of formula I obtainable in accordance with the above process having at least one salt-forming group into its salt and/or converting an obtainable salt into the free compound or into a different salt and/or separating any isomeric mixtures of compounds of formula I that are obtainable and/or converting a compound of formula I according to the invention into a different compound of formula I according to the invention.

## Revendications

1. Les composés de formule I 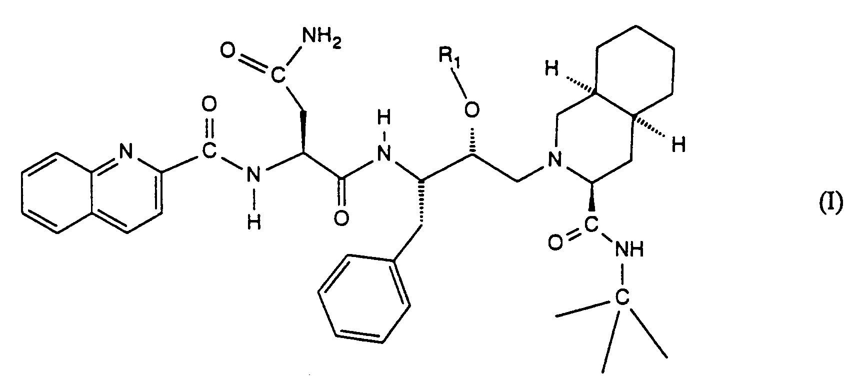 dans laquelle R₁ signifie un groupe octanoyle, décanoyle, dodécanoyle, palmitoyle, alcanoyle inférieur alcénoyle inférieur ou alcynoyle inférieur non substitués ou substitués, où les substituants sont choisis parmi un ou plusieurs restes choisis parmi le groupe hydroxy, alcoxy inférieur, alcoxy inférieur-alcoxy inférieur, alcoxy inférieur-alcoxy inférieur-alcoxy inférieur, phénoxy, naphtyloxy, phényl-alcoxy inférieur, 2-halogène-alcanoyle inférieur, amino-, alkylamino inférieur- ou di-alkylamino inférieur-alcoxy inférieur-2-alcanoyle inférieur, amino- alkylamino inférieur- ou di-alkylamino inférieur-alcoxy inférieur-alcoxy inférieur-2- alcanoyle inférieur, alcanoyloxy inférieur, phényl-alcanoyloxy inférieur, un halogène, carboxy, alcoxycarbonyle inférieur, phénylalcoxycarbonyle inférieur, carbamoyle, alkylcarbamoyle inférieur, hydroxy-alkylcarbamoyle inférieur, di-alkylcarbamoyle inférieur, bis(hydroxy-alkyl inférieur)carbamoyle, carbamoyle dont l'atome d'azote est un constituant d'un hétérocycle à 5 à 7 chaînons qui peut contenir un autre hétéroatome choisi parmi O, S, N et un azote substitué par un groupe alkyle inférieur tel que méthyle ou éthyle, cyano, oxo, cycloalkyle, bicycloalkyle, tricycloalkyle, cycloalcényle, bicycloalcényle, hétérocyclyle qui signifie un cycle saturé, partiellement saturé ou un cycle simple insaturé contenant de 5 à 7 atomes dans le cycle et jusqu'à 4 hétéroatomes choisis parmi l'azote, le soufre et/ou l'oxygène, le cycle étant présent tel quel ou pouvant être simplement sous forme benzo-, cyclopenta-, cyclohexa- ou cyclohepta-condensée; et non substitué ou substitué en particulier par un groupe alkyle inférieur, alcanoyle inférieur, hydroxy, alcoxy inférieur, phényl-alcoxy inférieur, hydroxy-alkyle inférieur, un halogène, cyano et/ou trifluorométhyle, et C₆-C₁₂-aryle qui est non substitué ou est mono- ou polysubstitué par un groupe alkyle inférieur, halogène-alkyle inférieur, un halogène, hydroxy, alcoxy inférieur, alcanoyloxy inférieur, carboxy, alkyloxycarbonyle inférieur, phényl-alcoxycarbonyle inférieur, carbamoyle, mono- ou di-alkylcarbamoyle inférieur, mono- ou di-hydroxy-alkylcarbamoyle inférieur, halo-alkyle inférieur, hétérocyclylalkyle inférieur où hétérocyclyle est tel que défini plus haut, par cyano et/ou par nitro; alcoxycarbonyle inférieur, 2-halogène-alcoxycarbonyle inférieur, aryl-alcoxycarbonyle inférieur où le groupe aryle contient de 6 à 14 atomes de carbone et est non substitué ou mono- ou polysubstitué par un groupe alkyle inférieur, halogène-alkyle inférieur, un halogène, hydroxy, alcoxy inférieur, alcanoyloxy inférieur, carboxy, alkyloxycarbonyle inférieur, phényl-alcoxycarbonyle inférieur, carbamoyle, mono- ou di-alkylcarbamoyle inférieur, mono- ou di-hydroxy-alkylcarbamoyle inférieur, haloalkyle inférieur, hétérocyclyl-alkyle inférieur où le groupe hétérocyclyle est tel que défini plus haut comme substituant du groupe alcanoyle inférieur, par cyano et/ou par nitro; hétérocyclyl-alcoxycarbonyle inférieur où le groupe hétérocyclyle est tel que défini plus haut comme substituant du groupe alcanoyle inférieur; aminocarbonyle, un reste aminocarbonyle où le groupe amino porte 1 ou 2 substituants choisis indépendamment l'un de l'autre parmi un groupe alkyle inférieur non substitué ou substitué où les substituants sont choisis parmi un groupe hydroxy, alcoxy inférieur, phénoxy, naphtyloxy, alcanoyloxy inférieur, phényl-alcanoyloxy inférieur, un halogène, carboxy, alcoxycarbonyle inférieur, phényl-alcoxycarbonyle inférieur, carbamoyle, alkylcarbamoyle inférieur, hydroxy-alkylcarbamoyle inférieur, di-alkylcarbamoyle inférieur, bis(hydroxy-alkyl inférieur)carbamoyle, cyano, oxo et C₆-C₁₂-aryle qui est non substitué ou mono- ou polysubstitué, par exemple par un groupe alkyle inférieur, halogène-alkyle inférieur, un halogène, hydroxy, alcoxy inférieur, alcanoyloxy inférieur, carboxy, alkyloxycarbonyle inférieur, phényl-alcoxycarbonyle inférieur, carbamoyle, mono- ou di-alkylcarbamoyle inférieur, mono- ou di-hydroxyalkylcarbamoyle inférieur, halo-alkyle inférieur tel que trifluorométhyle, par cyano et/ou par nitro, et sont choisis parmi un groupe aryle qui contient de 6 à 14 atomes de carbone et est non substitué ou mono- ou polysubstitué par un groupe alkyle inférieur, halogène-alkyle inférieur, un halogène, hydroxy, alcoxy inférieur, alcanoyloxy inférieur, carboxy, alkyloxycarbonyle inférieur, phényl-alcoxycarbonyle inférieur, carbamoyle, mont ou di-alkylcarbamoyle inférieur, mont ou di-hydroxy-alkylcarbamoyle inférieur, halo-alkyle inférieur, hétérocyclyl-alkyle inférieur où le groupe hétérocyclyle est tel que défini plus haut comme substituant du groupe alcanoyle inférieur, par cyano et/ou par nitro; où pas plus d'un des substituants du reste aminocarbonyle signifiant un groupe aryle; ou le reste d'un aminoacide lié par le groupe carboxy choisi parmi la glycine, l'alanine, l'acide 2-aminobutyrique, l'acide 3-aminobutyrique, l'acide 4-aminobutyrique, l'acide 3-aminopentanoïque, l'acide 4-aminopentanoïque, l'acide 5-aminopentanoïque, l'acide 3-aminohexanoïque, l'acide 4-aminohexanoïque ou l'acide 5-aminohexanoïque, la valine, la norvaline, la leucine, l'isoleucine, la norleucine, la sérine, l'homosérine, la thréonine, la méthionine, la cystéine, la phénylalanine, la tyrosine, la 4-aminophénylalanine, la 4-chlorophénylalanine, la 4-carboxyphénylalanine, la β-phénylsérine, la phénylglycine, l'α-naphtylalanine, la cyclohexylalanine, la cyclohexylglycine, le tryptophane, l'acide aspartique, l'asparagine, l'acide aminomalonique, le monoamide de l'acide aminomalonique, l'acide glutamique, la glutamine, l'histidine, l'arginine, la lysine, la δ-hydroxylysine, l'ornithine, l'acide 3-aminopropanoïque, l'acide α,γ-diaminobutyrique et l'acide α,β-diaminopropionique, chacun des aminoacides indiqués pouvant être sous forme D, L- ou (D,L)- (sauf dans les cas où il n'y a pas d'atome de carbone asymétrique),
un groupe α-amino, lorsqu'il est présent, est non substitué ou est mono- ou di-N-alkylé par un groupe alkyle inférieur, par amino-alkyle inférieur, par phényl- ou naphtylamino-alkyle inférieur, par phényl-alkyle inférieur, par diphénylméthyle, par trityle et/ou par hétérocyclyl-alkyle inférieur où le groupe hétérocyclyle est tel que défini plus haut pour le groupe alcanoyle inférieur substitué R₁, et/ou est N-acylé par un reste alcanoyle inférieur non substitué ou substitué indiqué plus haut dans la définition de R₁, par un groupe alcoxycarbonyle inférieur ou par un groupe phénylalcoxycarbonyle inférieur;
un groupe carboxy de la chaîne latérale est présent sous forme libre, sous forme d'un groupe ester alkylique inférieur, un groupe ester arylique ou d'un groupe ester aryl-alkylique inférieur où le groupe aryle signifie un groupe phényle, 4-nitrophényle, naphtyle, fluorényle ou biphénylyle, sous forme d'un groupe carbamoyle, alkylcarbamoyle inférieur, di-alkylaminocarbamoyle inférieur, mono- ou di(hydroxy-alkyl inférieur)carbamoyle ou mont ou di-(carboxy-alkyl inférieur)-carbamoyle,
un groupe amino de la chaîne latérale qui n'est pas en position α, est présent sous forme libre, sous forme d'un groupe mono- ou dialkylamino inférieur, alcanoylamino inférieur, amino-alcanoylamino inférieur, aryl-alcanoylamino inférieur où le groupe aryle contient de 6 à 14 atomes de carbone et est non substitué ou substitué par un groupe alkyle inférieur, hydroxy, alcoxy inférieur, carboxy, carbamoyle ou sulfamoyle, sous forme d'un groupe alcoxycarbonylamino inférieur, arylméthoxycarbonylamino où le groupe aryle contient de 6 à 14 atomes de carbone, sous forme d'un groupe pipéridyl-1-carbonyle, morpholinocarbonyle, thiomorpholinocarbonyle ou S,S-dioxothiomorpholinocarbonyle, et/ou
un groupe hydroxy de la chaîne latérale est présent sous forme libre, sous forme d'un groupe alcoxy inférieur, phényl-alcoxy inférieur, alcanoyloxy inférieur ou alkyloxycarbonyloxy inférieur et leurs sels.

2. Les composés selon la revendication 1 de formule I, où R₁ signifie un groupe alcanoyle inférieur, alcénoyle inférieur ou alcynoyle inférieur non substitués ou substitués, où les substituants sont choisis parmi un ou plusieurs restes choisis parmi un groupe hydroxy, alcoxy inférieur, phénoxy, naphtyloxy, alcanoyloxy inférieur, phényl-alcanoyloxy inférieur, un halogène, carboxy, alcoxycarbonyle inférieur, phénylalcoxycarbonyle inférieur, carbamoyle, alkylcarbamoyle inférieur, hydroxy-alkylcarbamoyle inférieur, di-alkylcarbamoyle inférieur, bis(hydroxy-alkyl inférieur)carbamoyle, carbamoyle dont l'atome d'azote est un constituant d'un hétérocycle à 5 à 7 chaînons qui peut contenir un autre hétéroatome choisi parmi O, S, N et un azote substitué par un groupe alkyle inférieur tel que méthyle ou éthyle, cyano, oxo, cycloalkyle, bicycloalkyle, tricycloalkyle, cycloalcényle, bicycloalcényle, hétérocyclyle qui signifie un cycle saturé, partiellement saturé ou un cycle simple insaturé contenant de 5 à 7 atomes dans le cycle et jusqu'à 4 hétéroatomes choisis parmi l'azote, le soufre et l'oxygène, le cycle étant présent tel quel ou pouvant être simplement sous forme benzo-, cyclopenta-, cyclohexa- ou cyclohepta-condensée; et non substitué ou substitué en particulier par un groupe alkyle inférieur, alcanoyle inférieur, hydroxy, alcoxy inférieur, phényl-alcoxy inférieur, hydroxy-alkyle inférieur, un halogène, cyano et/ou trifluorométhyle, et C₆-C₁₂aryle qui est non substitué ou mono- ou polysubstitué par un groupe alkyle inférieur, halogène-alkyle inférieur, un halogène, hydroxy, alcoxy inférieur, alcanoyloxy inférieur, carboxy, alkyloxycarbonyle inférieur, phényl-alcoxycarbonyle inférieur, carbamoyle, mont ou di-alkylcarbamoyle inférieur, mono- ou di-hydroxy-alkylcarbamoyle inférieur, halo-alkyle inférieur, hétérocyclylalkyle inférieur où le groupe hétérocyclyle est tel que défini plus haut, par cyano et/ou par nitro; alcoxycarbonyle inférieur, 2-halogène-alcoxycarbonyle inférieur, arylalcoxycarbonyle inférieur, où le groupe aryle contient de 6 à 14 atomes de carbone et est non substitué ou est mono- ou polysubstitué par un groupe alkyle inférieur, halogène-alkyle inférieur, un halogène, hydroxy, alcoxy inférieur, alcanoyloxy inférieur, carboxy, alkyloxycarbonyle inférieur, phényl-alcoxycarbonyle inférieur, carbamoyle, mono- ou di-alkylcarbamoyle inférieur, mont ou di-hydroxyalkylcarbamoyle inférieur, halo-alkyle inférieur, hétérocyclyl-alkyle inférieur où le groupe hétérocyclyl est tel que défini plus haut comme substituant du groupe alcanoyle inférieur, par cyano et/ou par nitro; hétérocyclyl-alcoxycarbonyle inférieur où le groupe hétérocyclyle est tel que défini plus haut comme substituant du groupe alcanoyle inférieur; aminocarbonyle, un reste aminocarbonyle où le groupe amino porte 1 ou 2 substituants choisis indépendamment l'un de l'autre parmi un groupe alkyle inférieur non substitué ou substitué où les substituants sont choisis parmi un groupe hydroxy, alcoxy inférieur, phénoxy, naphtyloxy, alcanoyloxy inférieur, phénylalcanoyloxy inférieur, un halogène, carboxy, alcoxycarbonyle inférieur, phénylalcoxycarbonyle inférieur, carbamoyle, alkylcarbamoyle inférieur, hydroxyalkylcarbamoyle inférieur, di-alkylcarbamoyle inférieur, bis(hydroxy-alkyl inférieur)carbamoyle, cyano, oxo et C₆-C₁₂-aryle qui est non substitué ou mono- ou polysubstitué, par exemple par un groupe alkyle inférieur, halogène-alkyle inférieur, un halogène, hydroxy, alcoxy inférieur, alcanoyloxy inférieur, carboxy, alkyloxycarbonyle inférieur, phényl-alcoxycarbonyle inférieur, carbamoyle, mont ou di-alkylcarbamoyle inférieur, mont ou di-hydroxy-alkyl-carbamoyle inférieur, haloalkyle inférieur, tel que le groupe trifluorométhyle, par cyano et/ou par nitro, et sont choisis parmi le groupe aryle qui contient de 6 à 14 atomes de carbone et est non substitué ou mono- ou polysubstitué par un groupe alkyle inférieur, halogène-alkyle inférieur, un halogène, hydroxy, alcoxy inférieur, alcanoyloxy inférieur, carboxy, alkyloxycarbonyle inférieur, phényl-alcoxycarbonyle inférieur, carbamoyle, mono- ou di-alkylcarbamoyle inférieur, mono- ou di-hydroxy-alkylcarbamoyle inférieur, haloalkyle inférieur, hétérocyclyl-alkyle inférieur où le groupe hétérocyclyle est tel que défini plus haut comme substituant du groupe alcanoyle inférieur, par cyano et/ou par nitro; où pas plus d'un des substituants du reste aminocarbonyle signifiant un groupe aryle; ou le reste d'un aminoacide lié par un groupe carboxy choisi parmi la glycine, l'alanine, l'acide 2-aminobutyrique, l'acide 3-aminobutyrique, l'acide 4-aminobutyrique, l'acide 3-amino-pentanoïque, l'acide 4-aminopentanoïque, l'acide 5-aminopentanoïque, l'acide 3-aminohexanoïque, l'acide 4-aminohexanoïque ou l'acide 5-aminohexanoïque, la valine, la norvaline, la leucine, l'isoleucine, la norleucine, la sérine, l'homosérine, la thréonine, la méthionine, la cystéine, la phénylalanine, la tyrosine, la 4-aminophényl-alanine, la 4-chlorophénylalanine, la 4-carboxyphénylalanine, la β-phénylsérine, la phénylglycine, l'α-naphtylalanine, la cyclohexylalanine, la cyclohexylglycine, le tryptophane, l'acide aspartique, l'asparagine, l'acide aminomalonique, le monoamide de l'acide aminomalonique, l'acide glutamique, la glutamine, l'histidine, l'arginine, la lysine, la δ-hydroxylysine, l'ornithine, l'acide 3-aminopropanoïque, l'acide α,γ-di-aminobutyrique et l'acide α,β-diaminopropionique, chacun des aminoacides indiqués pouvant être sous forme D,L- ou (D,L)- (sauf dans les cas où il n'y a pas d'atome de carbone asymétrique),
un groupe α-amino, lorsqu'il est présent, est non substitué ou est mono- ou di-N-alkylé par un groupe alkyle inférieur, par amino-alkyle inférieur, par phényl- ou naphtylamino-alkyle inférieur, par phényl-alkyle inférieur, par diphénylméthyle, par trityle et/ou par hétérocyclyl-alkyle inférieur où le groupe hétérocyclyle est tel que défini plus haut pour le groupe alcanoyle inférieur substitué R₁, et/ou est N-acylé par un reste alcanoyle inférieur non substitué ou substitué indiqué plus haut dans la définition de R₁, par un groupe alcoxycarbonyle inférieur ou par un groupe phénylalcoxycarbonyle inférieur;
un groupe carboxy de la chaîne latérale est présent sous forme libre, sous forme d'un groupe ester alkylique inférieur, un groupe ester arylique inférieur ou un groupe ester aryl-alkylique inférieur où le groupe aryle signifie un groupe phényle, 4-nitrophényle, naphtyle, fluorényle ou biphénylyle, sous forme d'un groupe carbamoyle, alkylcarbamoyle inférieur, di-alkylaminocarbamoyle inférieur, mono- ou di(hydroxy-alkyl inférieur)carbamoyle ou mono- ou di-(carboxy-alkyl inférieur)-carbamoyle,
un groupe amino de la chaîne latérale qui n'est pas en position α, est présent sous forme libre, sous forme d'un groupe mono- ou di-alkylamino inférieur, alcanoylamino inférieur, amino-alcanoylamino inférieur, aryl-alcanoylamino inférieur où le groupe aryle contient de 6 à 14 atomes de carbone et est non substitué ou substitué par un groupe alkyle inférieur, hydroxy, alcoxy inférieur, carboxy, carbamoyle ou sulfamoyle, sous forme d'un groupe alcoxycarbonylamino inférieur, arylméthoxycarbonylamino où le groupe aryle contient de 6 à 14 atomes de carbone, sous forme d'un groupe pipéridyl-1-carbonyle, morpholinocarbonyle, thiomorpholinocarbonyle ou S,S-dioxothiomorpholinocarbonyle, et/ou
un groupe hydroxy de la chaîne latérale est présent sous forme libre, sous forme d'un groupe alcoxy inférieur, phényl-alcoxy inférieur, alcanoyloxy inférieur ou alkyloxycarbonyloxy inférieur et leurs sels.

3. Les composés selon la revendication 1 ou 2 de formule I où R₁ signifie un groupe octanoyle, décanoyle, dodécanoyle, palmitoyle, alcanoyle inférieur non substitué ou substitué, où les substituants sont choisis parmi un jusqu'à 3 restes choisis parmi le groupe hydroxy, alcoxy inférieur, alcoxy inférieur-alcoxy inférieur, alcoxy inférieur-alcoxy inférieur-alcoxy inférieur, phénoxy, naphtyloxy, phényl-alcoxy inférieur, alcanoyloxy inférieur, phényl-alcanoyloxy inférieur, amino-, alkylamino inférieur- ou di-alkylamino inférieur-alcoxy inférieur-2-alcanoyle inférieur, amino-, alkylamino inférieur- ou di-alkylamino inférieur-alcoxy inférieur-alcoxy inférieur-2-alcanoyle inférieur, un halogène, carboxy, alcoxycarbonyle inférieur, phényl-alcoxycarbonyle inférieur, carbamoyle, alkylcarbamoyle inférieur, hydroxy-alkylcarbamoyle inférieur, di-alkylcarbamoyle inférieur, bis(hydroxy-alkyl inférieur)carbamoyle, cyano, oxo, C₃-C₈-cycloalkyle, C₄-C₈-cycloalcényle; pyrrolyle, 2,5-dihydropyrrolyle, furanyle, thiényle, tétrahydrofuranyle, pyrrolyle, pyrrolidinyle, imidazolyle, imidazolidinyle, pyrazolyle, pyrazolidinyle, tétrahydro-oxazolyle, tétrahydro-isoxazolyle, tétrahydro-thiazolyle, tétrahydro-isothiazolyle, indolyle, isoindolyle, quinolyle, isoquinolyle, benzimidazolyle, benzofuranyle, pyridyle, pyrimidinyle, pipéridinyle, pipérazine-1-yle, morpholino, thiomorpholino, S,S-di-oxothiomorpholino, 1,2-dihydro- ou 1,2,3,4-tétrahydroquinolyle ou 1,2-dihydro- ou 1,2,3,4-tétrahydroisoquinolyle, lesdits restes hétérocycliques étant non substitués ou substitués par un groupe alkyle inférieur, alcanoyle inférieur, hydroxy, alcoxy inférieur, phényl-alcoxy inférieur, hydroxy-alkyle inférieur, un halogène, par cyano et/ou par trifluorométhyle; et le groupe aryle étant choisi parmi un groupe phényle, naphtyle, indanyle, indényle et fluorényle, ces restes étant non substitués ou mono- ou polysubstitués par un groupe alkyle inférieur, halogène-alkyle inférieur, un halogène, hydroxy, alcoxy inférieur, alcanoyloxy inférieur, carboxy, alkyloxycarbonyle inférieur, phénylalcoxycarbonyle inférieur, carbamoyle, mono- ou di-alkylcarbamoyle inférieur, mono- ou di-hydroxy-alkylcarbamoyle inférieur, halo-alkyle inférieur, pipéridinométhyle, pipérazine-1-ylméthyle, 4-alkyl inférieur-pipérazine -1-ylméthyle, 4-alcanoyl inférieur-pipérazine -1-ylméthyle, morpholinométhyle, thiomorpholinométhyle, par cyano et/ou par nitro; ou le reste d'un aminoacide lié par un groupe carboxy choisi parmi la glycine, l'alanine, l'acide 2-aminobutyrique, l'acide 3-aminobutyrique, l'acide 4-aminobutyrique, l'acide 3-aminopentanoïque, l'acide 4-aminopentanoïque, l'acide 5-aminopentanoïque, l'acide 3-aminohexanoïque, l'acide 4-aminohexanoïque ou l'acide 5-aminohexanoïque, la valine, la norvaline, la leucine, l'isoleucine, la norleucine, la sérine, l'homosérine, la thréonine, la méthionine, la cystéine, la phénylalanine, la tyrosine, la 4-aminophénylalanine, la 4-chlorophénylalanine, la 4-carboxyphénylalanine, la β-phénylsérine, la phénylglycine, l'α-naphtylalanine, la cyclohexylalanine, la cyclohexylglycine, le tryptophane, l'acide aspartique, l'asparagine, l'acide aminomalonique, le monoamide de l'acide aminomalonique, l'acide glutamique, la glutamine, l'histidine, l'arginine, la lysine, la δ-hydroxylysine, l'ornithine, l'acide 3-aminopropanoïque, l'acide α,γ-diaminobutyrique et l'acide α,β-diaminopropionique, chacun des aminoacides indiqués pouvant être sous forme D, L- ou (D,L)- (sauf dans les cas où il n'y a pas d'atome de carbone asymétrique), où
un groupe α-amino, lorsqu'il est présent, est non substitué ou est mono- ou di-N-alkylé par un groupe alkyle inférieur, par amino-alkyle inférieur, par phényl- ou naphtylamino-alkyle inférieur, par phényl-alkyle inférieur, par diphénylméthyle, par trityle, par furanyl-alkyle inférieur, par thiényl-alkyle inférieur, par imidazolyl-alkyle inférieur, et/ou par un groupe 2-, 3- ou 4-pyridyl-alkyle inférieur, et/ou N-acylé par un reste alcanoyle inférieur non substitué ou substitué indiqué plus haut dans la définition de R₁, par alcoxycarbonyle inférieur ou par phényl-alcoxycarbonyle inférieur; et leurs sels pharmaceutiquement acceptables.

4. Les composés selon la revendication 2 de formule I où R₁ signifie un groupe alcanoyle inférieur non substitué ou substitué où les substituants sont choisis parmi 1 jusqu'à 3 restes choisis parmi le groupe hydroxy, alcoxy inférieur, phénoxy, naphtyloxy, alcanoyloxy inférieur, phényl-alcanoyloxy inférieur, un halogène, carboxy, alcoxycarbonyle inférieur, phényl-alcoxycarbonyle inférieur, carbamoyle, alkylcarbamoyle inférieur, hydroxy-alkylcarbamoyle inférieur, di-alkylcarbamoyle inférieur bis(hydroxy-alkyl inférieur)carbamoyle, cyano, oxo, C₃-C₈-cycloalkyle, C₄-C₈-cycloalcényle; pyrrolyle, 2,5-dihydropyrrolyle, furanyle, thiényle, tétrahydrofuranyle, pyrrolyle, pyrrolidinyle, imidazolyle, imidazolidinyle, pyrazolyle, pyrazolidinyle, tétrahydrooxazolyle, tétrahydro-isoxazolyle, tétrahydro-thiazolyle, tétrahydro-isothiazolyle, indolyle, isoindolyle, quinolyle, isoquinolyle, benzimidazolyle, benzofuranyle, pyridyle, pyrimidinyle, pipéridinyle, pipérazine-1-yle, morpholino, thiomorpholino, S,S-dioxothiomorpholino, 1,2-dihydro- ou 1,2,3,4-tétrahydroquinolyle ou 1,2-dihydro- ou 1,2,3,4-tétrahydroisoquinolyle, où lesdits restes hétérocycliques sont non substitués ou substitués par un groupe alkyle inférieur, alcanoyle inférieur, hydroxy, alcoxy inférieur, phényl-alcoxy inférieur, hydroxy-alkyle inférieur, un halogène, par cyano et/ou par trifluorométhyle; et le groupe aryle est choisi parmi un groupe phényle, naphtyle, indanyle, indényle et fluorényle, ces restes étant non substitués ou mono- ou polysubstitués par un groupe alkyle inférieur, halogène-alkyle inférieur, un halogène, hydroxy, alcoxy inférieur, alcanoyloxy inférieur, carboxy, alkyloxycarbonyle inférieur, phényl-alcoxycarbonyle inférieur, carbamoyle, mono- ou dialkyl-carbamoyle inférieur, mono- ou di-hydroxy-alkylcarbamoyle inférieur, halo-alkyle inférieur, pipéridinométhyle, pipérazine-1-ylméthyle, 4-alkyl inférieur-pipérazine-1-ylméthyle, 4-alcanoyl inférieur-pipérazine-1-ylméthyle, morpholinométhyle, thiomorpholinométhyle, par cyano et/ou par nitro; ou le reste d'un aminoacide lié par un groupe carboxy choisi parmi la glycine, l'alanine, l'acide 2-aminobutyrique, l'acide 3-aminobutyrique, l'acide 4-aminobutyrique, l'acide 3-aminopentanoïque, l'acide 4-aminopentanoïque, l'acide 5-aminopentanoïque, l'acide 3-aminohexanoïque, l'acide 4-aminohexanoïque ou l'acide 5-aminohexanoïque, la valine, la norvaline, la leucine, l'isoleucine, la norleucine, la sérine, l'homosérine, la thréonine, la méthionine, la cystéine, la phénylalanine, la tyrosine, la 4-aminophénylalanine, la 4-chlorophénylalanine, la 4-carboxyphénylalanine, la β-phénylsérine, la phénylglycine, l'α-naphtylalanine, la cyclohexylalanine, la cyclohexylglycine, le tryptophane, l'acide aspartique, l'asparagine, l'acide aminomalonique, le monoamide de l'acide aminomalonique, l'acide glutamique, la glutamine, l'histidine, l'arginine, la lysine, la δ-hydroxylysine, l'ornithine, l'acide 3-aminopropanoïque, l'acide α,γ-diaminobutyrique et l'acide α,β-diaminopropionique, chacun des aminoacides indiqués pouvant être sous forme D,L- ou (D,L)- (sauf dans les cas où il n'y a pas d'atome de carbone asymétrique),
un groupe α-amino, lorsqu'il est présent, est non substitué ou est mono- ou di-N-alkylé par un groupe alkyle inférieur, par amino-alkyle inférieur, par phényl- ou naphtylamino-alkyle inférieur, par phényl-alkyle inférieur, par diphénylméthyle, par trityle, par furanyl-alkyle inférieur, par thiényl-alkyle inférieur et/ou par 2-, 3- ou 4-pyridyl-alkyle inférieur, et/ou est N-acylé par un reste alcanoyle inférieur non substitué ou substitué indiqué plus haut dans la définition de R₁ ou par un groupe phénylalcoxycarbonyle inférieur, et leurs sels pharmaceutiquement acceptables.

5. Les composés de formule I selon la revendication 1 où R₁ signifie un groupe octanoyle, décanoyle, dodécanoyle, parmitoyle, alcanoyle inférieur, hydroxyalcanoyle inférieur, alcoxy inférieur-alcanoyle inférieur, alcoxy inférieur-alcoxy inférieur-alcanoyle inférieur, alcoxy inférieur-alcoxy inférieur-alcoxy inférieur-alcanoyle inférieur, phénoxy-alcanoyle inférieur, phényl-alcoxy inférieur-alcanoyle inférieur, amino-, alkylamino inférieur- ou di-alkylamino inférieur-alcoxy inférieur-2-alcanoyle inférieur, amino-, alkylamino inférieur- ou di-alkylamino inférieur-alcoxy inférieur-alcoxy inférieur-2-alcanoyle inférieur, alcanoyloxy inférieur-alcanoyle inférieur, carboxy-alcanoyle inférieur, oxo-alcanoyle inférieur, 5-hydroxy-méthyl-furyl-2- carbonyle, 2- ou 3-pyrrolylcarbonyle, furylcarbonyle, thiénylcarbonyle, pyridyl-alcanoyle inférieur, quinolylcarbonyle, isoquinolylcarbonyle, 2-, 3- ou 5-indolylcarbonyle, pyrrolidinyl-(2- ou 3-)carbonyle, 2-,3- ou 4-pipéridinylcarbonyle, 1,2,3,4-tétrahydroquinolyl-2-, -3- ou -4-carbonyle, 1,2,3,4-tétrahydroisoquinolyl-1-, -3- ou 4-carbonyle, imidazolyl-alcanoyle inférieur, pyrazolyl-alcanoyle inférieur, morpholinocarbonyle, thiomorpholinocarbonyle, morpholinoacétyle, thiomorpholinoacétyle, 4-alkyl inférieur-1-pipérazinoacétyle, indolylacétyle, benzofuranylacétyle, phényl-alcanoyle inférieur, un reste phényle non substitué, mono- ou polysubstitué par un groupe alkyle inférieur, halo-alkyle inférieur, un halogène, hydroxy, alcoxy inférieur, pipéridinométhyle, pipérazine-1-ylméthyle, 4-alkyl inférieur-pipérazine-1-ylméthyle, morpholinométhyle, thiomorpholinométhyle, par cyano et/ou par nitro, ou bien le reste d'un aminoacide aliphatique lié par le groupe carboxy choisi parmi l'alanine, la valine, la norvaline, la leucine, l'acide 3-aminopropionique, l'acide 2-aminobutyrique et l'isoleucine ou d'un aminoacide choisi parmi la glycine, l'asparagine, la glutamine, la méthionine, la lysine, la phénylalanine, l'acide 3-aminobutyrique, l'acide 4-aminobutyrique, l'acide 3-aminopentanoïque, l'acide 4-aminopentanoïque, l'acide 5-aminopentanoïque, l'acide 3-aminohexanoïque, l'acide 4-aminohexanoïque et l'acide 5-aminohexanoïque, chacun des aminoacides indiqués pouvant se présenter sous forme D-, L- ou (D,L)- (sauf en l'absence d'atomes de carbone asymétriques), un groupe α-amino, lorsqu'il est présent, est non substitué ou mont ou di-N-alkylé par un groupe alkyle inférieur, par phényl-alkyle inférieur, par 2-, 3- ou 4-pyridyl-alkyle inférieur, par imidazolyl-alkyle inférieur, et/ou par 2-, 3- ou 4-pyridyl-alkyle inférieur, tel que 2-, 3- ou 4-pyridylméthyle, et/ou N-acylé par un groupe alcoxycarbonyle inférieur ou par benzyloxycarbonyle, et leurs sels pharmaceutiquement acceptables.

6. Les composés selon la revendication 1 de formule I où R₁ signifie un groupe alcanoyle inférieur, hydroxy-alcanoyle inférieur, alcoxy inférieur-alcanoyle inférieur, phénoxy-alcanoyle inférieur, alcanoyloxy inférieur-alcanoyle inférieur, oxoalcanoyle inférieur, 5-hydroxyméthyl-furyl-2-carbonyle, 2- ou 3-pyrrolylcarbonyle, furylcarbonyle, par exemple 2-furylcarbonyle, thiénylcarbonyle, pyridylcarbonyle, 2-, 3- ou 5-indolylcarbonyle, pyrrolidinyl-3-carbonyle, 2-, 3- ou 4-pipéridinyl-carbonyle, 1,2,3,4-tétrahydroquinolyl-2-, -3- ou -4-carbonyle, 1,2,3,4-tétrahydroisoquinolyl-1-, -3- ou -4-carbonyle, imidazolylcarbonyle, morpholinocarbonyle, thiomorpholinocarbonyle, morpholinoacétyle, thiomorpholinoacétyle, 4-alkyl inférieur-1-pipérazinoacétyle, indolylacétyle, benzofuranylacétyle, phényl-alcanoyle inférieur qui est non substitué ou mono- ou polysubstitué dans le reste phényle par un groupe alkyle inférieur, halo-alkyle inférieur, un halogène, hydroxy, alcoxy inférieur, pipéridinométhyle, pipérazine-1-ylméthyle, 4-alkyl inférieur-pipérazine-1-yl- méthyle, morpholinométhyle, thiomorpholinométhyle, par cyano et/ou par nitro; ou le reste d'un aminoacide aliphatique lié par le groupe carboxy choisi parmi l'alanine, la valine, la norvaline, la leucine, l'acide 3-aminopropionique, l'acide 2-aminobutyrique, l'acide 3-aminobutyrique, l'acide 4-aminobutyrique, l'acide 3-aminopentanoïque, l'acide 4-aminopentanoïque, l'acide 5-aminopentanoïque, l'acide 3-aminohexanoïque, l'acide 4-aminohexanoïque, l'acide 5-aminohexanoïque et l'isoleucine ou un aminoacide choisi parmi la glycine, l'asparagine, la glutamine, la méthionine, la lysine, la proline et la phénylalanine, chacun des aminoacides indiqués pouvant être sous forme D,L- ou (D,L)- (sauf dans les cas où il n'y a pas d'atome de carbone asymétrique); un groupe α-amino, lorsqu'il est présent, est non substitué ou est mont ou di-N-alkylé par un groupe alkyle inférieur, phénylalkyle inférieur et/ou par 2-, 3- ou 4-pyridyl-alkyle inférieur et/ou est N-acylé par un groupe alcoxycarbonyle inférieur ou par un groupe phényl-alcoxycarbonyle inférieur, et leurs sels pharmaceutiquement acceptables.

7. Les composés de formule I selon la revendication 1 où R₁ signifie un groupe octanoyle, décanoyle, dodécanoyle, palmitoyle, alcanoyle inférieur, alcoxy inférieur-alcanoyle inférieur, alcoxy inférieur-alcoxy inférieur-alcanoyle inférieur, alcoxy inférieur-alcoxy inférieur-alcanoyle inférieur, phénoxy-alcanoyle inférieur, phényl-alcoxy inférieur-alcanoyle inférieur, amino-, alkylamino inférieur- ou di-alkylamino inférieur-alcoxy inférieur-2-alcanoyle inférieur, amino-, alkylamino inférieur- ou di-alkylamino inférieur-alcoxy inférieur-alcoxy inférieur-2-alcanoyle inférieur, 2-alcoxy inférieur-2-phénylacétyle, furylcarbonyle, pyridyl-alcanoyle inférieur, quinolylcarbonyle, isoquinolylcarbonyle, pyrrolidinyl-2-carbonyle, imidazolyl-alcanoyle inférieur, pyrazolyl-alcanoyle inférieur, phényl-alcanoyle inférieur, 4-chlorométhylbenzoyle, 4-morpholinométhylbenzoyle, 4-thiomorpholinométhylbenzoyle, aminoacétyle, N-alkylaminoacétyle inférieur, N,N-dialkylaminoacétyle inférieur, N-alkyl inférieur-N-phényl-alkylaminoacétyle inférieur, N-alkyl inférieur-N-benzyloxycarbonylaminoacétyle, N-imidazolyl-alkyl inférieur-N-alkylaminoacétyle inférieur, N-alkyl inférieur-N-pyridyl-alkylaminoacétyle inférieur ou 4-(N,N-diméthylamino)butyryle, et leurs sels pharmaceutiquement acceptables.

8. Les composés selon la revendication 1 de formule I où R₁ signifie un groupe alcanoyle inférieur, furylcarbonyle, pyridylcarbonyle, phényl-alcanoyle inférieur, 4-morpholinométhylbenzoyle, 4-thiomorpholinométhylbenzoyle, aminoacétyle, N-alkylaminoacétyle inférieur, N,N-di-alkylaminoacétyle inférieur, N-alkyl inférieur-N-phényl-alkylaminoacétyle inférieur, N-alkyl inférieur-N-pyridyl-alkylaminoacétyle inférieur ou N-phényl-alcoxycarbonyl inférieur-N-alkylaminoacétyle inférieur et leurs sels pharmaceutiquement acceptables.

9. Les composés de formule I selon la revendication 1 où R₁ signifie un groupe alcanoyle inférieur, alcoxy inférieur-alcanoyle inférieur, pyridylcarbonyle ou furylcarbonyle et leurs sels pharmaceutiquement acceptables.

10. Les composés selon la revendication 1 de formule I où R₁ signifie un groupe alcanoyle inférieur ou furylcarbonyle, et leurs sels pharmaceutiquement acceptables.

11. Les composés selon la revendication 1 de formule I où R₁ signifie un groupe alcoxy inférieur-alcanoyle inférieur ou pyridylcarbonyle et leurs sels pharmaceutiquement acceptables.

12. Les composés selon la revendication 1 de formule I où R₁ signifie un groupe alcanoyle inférieur et leurs sels pharmaceutiquement acceptables.

13. Les composés de formule I selon la revendication 1 choisis parmi les composés suivants:
N-tert-butyl-décahydro-2-[2(R)-(furyl-2-carboxy)-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-pivaloyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2[2(R)-(N-méthylaminoacétoxy)-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-(N-benzyloxycarbonyl-N-méthylaminoacétoxy)-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-(N-diméthylaminoacétoxy)-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-(N-n-butyl-N-méthylaminoacétoxy)-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-(N-méthyl-N-benzylaminoacétoxy)-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-(N-méthyl-N-3-pyridylméthyl-aminoacétoxy)-4-phényl-3(S)-[[N-(2-quinolylcarbonyl)-L-asparaginyl]amino]butyl]-(4aS,8aS)-isoquinoline-3(S)-carboxamide,
N-tert-butyl-décahydro-2-[2(R)-(3-pyridylcarboxy)-4-phényl-3(S)-[[N-(2-quinolylcarbonyl)-L-asparaginyl]amino]butyl]-(4aS,8aS)-isoquinoline-3(S)-carboxamide, et
N-tert.-butyl-décahydro-2-[2(R)-(4-(N-morpholinyl-méthyl)-benzoyloxy)-4-phényl-3(S)-[[N-(2-quinolylcarbonyl)-L-asparaginyl]amino]butyl]-(4aS,8aS)-isoquinoline-3(S)-carboxamide,
ou leurs sels pharmaceutiquement acceptables.

14. Les composés de formule I selon la revendication 1 choisis parmi les composés suivants:
N-tert.-butyl-décahydro-2-[2(R)-propionyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-butyryloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-méthylpropionyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-pentanoyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-octanoyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-décanoyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-dodécanoyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-palmitoyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-(3-carboxypropionyloxy)-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-(3-méthoxypropionyloxy)-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-benzyloxyacétyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{(S)-α-méthoxy-α-phénylacétyloxy}-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{(R)-α-méthoxy-α-phénylacétyloxy}-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{2-(2-méthoxyéthoxy)acétyloxy}-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{n-butyloxyacétyloxy}-4-phényl-3(S)-[[N-(2-quinolyl-carbonyl)-L-asparaginyl]amino]butyl]-(4aS,8aS)-isoquinoline-3(S)-carboxamide,
N-tert.-butyl-décahydro-2-[2(R)-{2-(2-(2-méthoxyéthoxy)éthoxy)acétoxy}-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{2-pyridylacétyloxy}-4-phényl-3(S)-[[N-(2-quinolylcarbonyl)-L-asparaginyl]amino]butyl](4aS,8aS)-isoquinoline-3(S)-carboxamide,
N-tert.-butyl-décahydro-2-[2(R)-{3-(2-pyridyl)propionyloxy}-4-phényl-3(S)-[[N-(2-quinolylcaroonyl)-L-asparaginyl]amino]butyl]-(4aS,8aS)-isoquinoline-3(S)-carboxamide,
N-tert.-butyl-décahydro-2-[2(R)-{4-imidazolylcarbonyloxy}-4-phényl-3(S)-[[N-(2-quinolylcarbonyl)-L-asparaginyl]amino]butyl]-(4aS,8aS)-isoquinoline-3(S)-carboxaamide,
N-tert.-butyl-décahydro-2-[2(R)-{4-imidazolylacétyloxy}-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{3(4-imidazolyl)propionyloxy}-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-quinoline-2-ylcarbonyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-phénoxyacétyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-pyridine-4-carbonyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-pyridine-2-carbonyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{1-pyrazolylacétyloxy}-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-pyrazine-2-ylcarbonyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-isoquinoline-3-carbonyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-aminoacétyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{(L)-pyrrolidine-2-carbonyloxy}-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{N-(imidazolyl-4-méthyl)-N-méthylaminoacétyloxy}-4-phényl-3(S)-[[N-(2-quinolylcarbonyl)-L-asparaginyl]amino]-butyl]-(4aS,8aS)-isoquinoline-3(S)-carboxamide,
N-tert.-butyl-décahydro-2-[2(R)-{N-(pyridyl-2-méthyl)-N-méthylaminoacétyloxy}-4-phényl-3(S)-[[N-(2-quinolylcarbonyl)-L-asparaginyl]amino]butyl]-(4aS,8aS)- isoquinoline-3(S)-carboxamide,
N-tert.-butyl-décahydro-2-[2(R)-{N,N-(4-diméthylaminobutyryl)oxy}-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-benzoyloxy-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{4-chlorométhylbenzoyloxy}-4-phényl-3(S)-[[N-(2-quinolylcarbonyl)-L-asparaginyl]amino]butyl]-(4aS,8aS)-isoquinoline-3(S)-carboxamide,
N-tert.-butyl-décahydro-2-[2(R)-{3-(N,N-diméthylaminopropyl)oxyacétyl}-4-phényl-3(S)-[[N-(2-quinolylcarbonyl)-L-asparaginyl]amino]butyl]-(4aS,8aS)-isoquinoline-3(S)-carboxamide, et
N-tert.-butyl-décahydro-2-[2(R)-{2-[3-(N,N-diméthylaminopropyloxy)éthoxy]acétyl}-4-phényl-3(S)-[[N-(2-quinolylcarbonyl)-L-asparaginyl]amino]butyl]-(4aS,8aS)-isoquinoline-3(S)-carboxamide,
ou leurs sels pharmaceutiquement acceptables.

15. Un composé de formule I selon la revendication 1 où R₁ signifie un groupe acétyle et ses sels pharmaceutiquement acceptables.

16. Un composé de formule I selon la revendication 1 où R₁ signifie un groupe furyl-2-carbonyle et ses sels pharmaceutiquement acceptables.

17. Un composé de formule I selon la revendication 1 où R₁ signifie un groupe méthoxyacétyle et ses sels pharmaceutiquement acceptables.

18. Un composé de formule I selon la revendication 1 où R₁ signifie un groupe pyridyl-2-carbonyle et ses sels pharmaceutiquement acceptables.

19. Un composé de formule I ou un de ses sels selon l'une quelconque des revendications 1 à 18 pour l'utilisation dans un procédé pour le traitement thérapeutique d'un corps humain ou animal.

20. L'utilisation d'un composé selon l'une quelconque des revendications 1 à 18 de formule I ou d'un de ses sels pharmaceutiquement acceptables, pour la production de préparations pharmaceutiques peur le traitement du SIDA.

21. Des préparations pharmaceutiques contenant les composés de formule I selon l'une quelconque des revendications 1 à 18 ou un sel pharmaceutiquement acceptable de ces composés avec au moins un groupe formant un sel ensemble avec un véhicule pharmaceutiquement acceptable.

22. Un procédé de préparation d'un composé de formule I selon la revendication 1 et des sels de ce composé, caractérisé en ce que
a) on fait réagir un composé de formule II 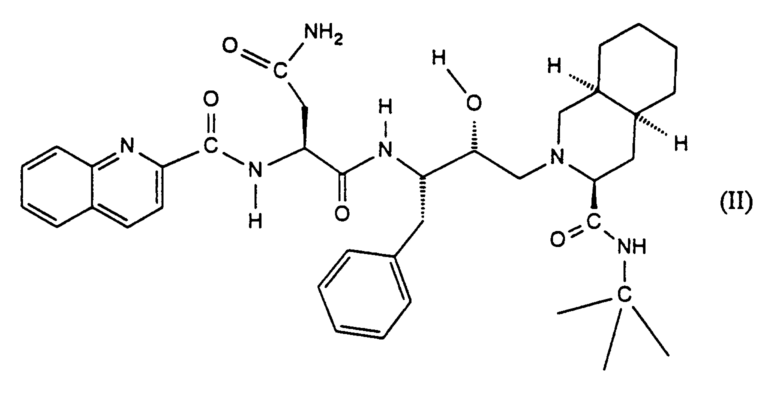 avec un acide carboxylique de formule III
R₁-OH (III)
où R₁ est tel que défini à la revendication 1 ou avec un de ses dérivés réactifs, les groupes fonctionnels libres dans les produits de départ de formule II et III qui ne sont pas destinés à participer à la réaction étant si nécessaire sous forme protégée, et on élimine les groupes protecteurs présents, ou bien
b) on amide un composé amino de formule IV 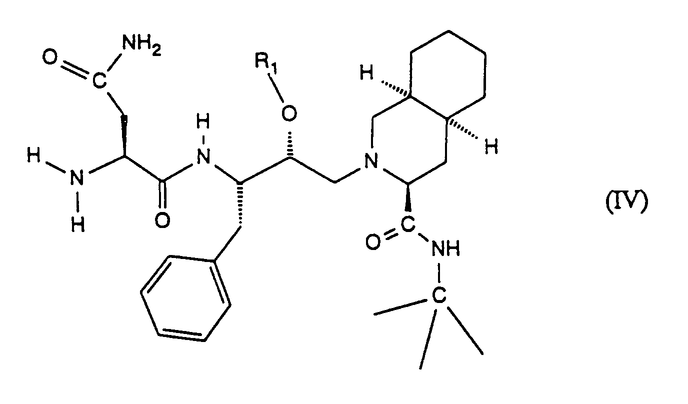 où R₁ est tel que défini ou un de ses dérivés réactifs, avec un acide carboxylique de formule V 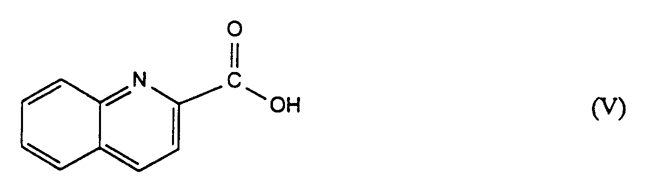 ou avec un de ses dérivés acides réactifs, les groupes fonctionnels libres dans les produits de départ de formule IV et V qui ne sont pas destinés à participer à la réaction, étant si nécessaire sous forme protégée, et on élimine les groupes protecteurs présents, ou bien
c) on amide un composé amino de formule VI 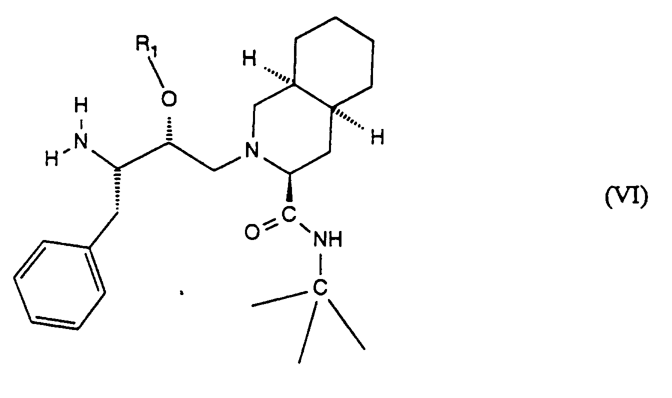 où R₁ est tel que défini, ou un de ses dérivés réactifs, avec un acide carboxylique de formule VII 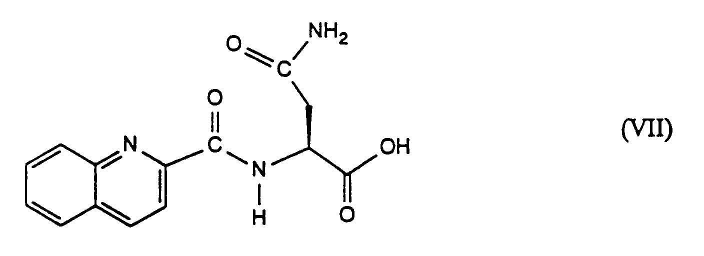 ou avec un de ses dérivés acides réactifs, les groupes fonctionnels libres dans les produits de départ de formule VI et VII qui ne sont pas destinés à participer à la réaction, étant si nécessaire sous forme protégée, et on élimine les groupes protecteurs présents,
et, si nécessaire, on transforme en son sel un composé de formule I obtenu selon le procédé ci-dessus ayant au moins un groupe formant un sel et/ou on transforme un sel obtenu en composé libre ou en un autre sel et/ou on sépare les mélanges isomères des composés de formule I éventuellement obtenus et/ou on transforme un composé de formule I de l'invention en un autre composé de formule I de l'invention.
